# EUROPEAN PATENT APPLICATION

(11) **EP 4 074 711 A1**
(43) Date of publication of application: **19.10.2022**
(21) Application number: 20898857.6
(22) Date of filing: 10.12.2020
(51) Int. Cl.: C07D 471/04, C07D 471/10, A61K 31/437, A61K 31/506, A61P 39/00

(54) **NITROGEN-CONTAINING HETEROCYCLIC AUTOTAXIN INHIBITOR, AND COMPOSITION CONTAINING SAME AND USE THEREOF**

(30) Priority: 11.12.2019 CN 201911248204; 15.04.2020 CN 202010292562; 17.06.2020 CN 202010541043
(71) Applicant: Sichuan Haisco Pharmaceutical Co., Ltd., Sichuan 611130 (CN)
(72) Inventor: LI, Yao, Chengdu, Sichuan 611130 (CN); SHI, Zongjun, Chengdu, Sichuan 611130 (CN); WANG, Wenjing, Chengdu, Sichuan 611130 (CN); WANG, Yongli, Chengdu, Sichuan 611130 (CN); PEI, Yunpeng, Chengdu, Sichuan 611130 (CN); SONG, Changwei, Chengdu, Sichuan 611130 (CN); LI, Yonghong, Chengdu, Sichuan 611130 (CN); TANG, Pingming, Chengdu, Sichuan 611130 (CN); YU, Yan, Chengdu, Sichuan 611130 (CN); ZHANG, Chen, Chengdu, Sichuan 611130 (CN); NI, Jia, Chengdu, Sichuan 611130 (CN); YAN, Pangke, Chengdu, Sichuan 611130 (CN)
(74) Representative: Zacco Sweden AB
(86) International application number: PCT/CN2020/135220
(87) International publication number: WO 2021/115375

(57) **Abstract**

Disclosed are a nitrogen-containing heterocyclic compound as shown in formula (I), or a stereoisomer, a solvate, a deuterated form, a pharmaceutically acceptable salt, or a cocrystal thereof or a pharmaceutical composition containing same, and the use thereof in the preparation of a drug for treating/preventing diseases mediated by autotaxin. Each group in formula (I) is as defined in the description.

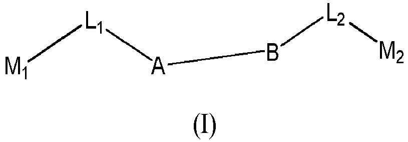

## Description

### Technical Field

The present invention relates to a nitrogen-containing heterocyclic compound, or a stereoisomer, a solvate, a deuterated form, a pharmaceutically acceptable salt, or a cocrystal thereof or a pharmaceutical composition containing same, and the use thereof in the preparation of a drug for treating/preventing diseases mediated by autotaxin.

### Background Art

Autotaxin (ATX) is an enzyme which is responsible for the increase in lysophosphatidic acid in ascites and plasma, and is a secretory enzyme important for converting lysophosphatidylcholine (LPC) into lysophosphatidic acid (LPA) as a biologically active signaling molecule. Autotaxin (ATX) is a secreted enzyme also referred to as an ectonucleotide pyrophosphatase/phosphodiesterase 2 or lysophospholipase D, and is important for converting lysophosphatidylcholine (LPC) into lysophosphatidic acid (LPA) as a biologically active signaling molecule. ATX plays a role in causing pathological conditions including fibrosis, arthritis, neurodegeneration, neuropathic pain, and cancer.

LPA is a physiologically active lipid having an influence on the migration, proliferation, and survival of various types of cells. Since the LPA level in plasma is highly related to the activity of ATX, it is believed that ATX is an important supply source of extracellular LPA. It has been shown that, in pathological conditions, inhibition of ATX reduces the LPA level. Early experiments with a prototype ATX inhibitor have shown that such a compound is able to inhibit the LPA synthesizing activity in mouse plasma. Early work conducted has demonstrated that LPA can elicit a wide range of cellular response including smooth muscle cell contraction, platelet activation, cell proliferation, chemotaxis, *etc.* LPA mediates its effects via signaling to several G protein coupled receptors (GPCRs); the first members were originally denoted Edg (endothelial cell differentiation gene) receptors or ventricular zone gene-1 but are now called LPA receptors. The prototypic group now consists of LPA1/Edg-2, VZG-1, LPA2/Edg-4 and LPA3/Edg-7. Recently, three additional LPA receptors LPA4/p2y9/GPR23, LPA5/GPR92 and LPA6/p2y5 have been described that are more closely related to nucleotide-selective purinergic receptors than to the prototypic LPA1-3 receptors. The ATX-LPA signaling axis is involved in a large range of physiological and pathophysiological functions, including, for example, nervous system function, vascular development, cardiovascular physiology, reproduction, immune system function, chronic inflammation, tumor metastasis and progression, organ fibrosis and obesity and/or other metabolic diseases such as diabetes.

Therefore, increased activity of ATX and/or increased levels of LPA, altered LPA receptor expression and altered responses to LPA may contribute to the initiation, progression and/or outcome of a number of different pathophysiological conditions related to the ATX/LPA axis.

### Summary of the Invention

As a first technical solution of the present invention, the present invention first provides a nitrogen-containing heterocyclic compound of formula (I) having an inhibitory activity against ATX, or a stereoisomer, a solvate, a deuterated form, a pharmaceutically acceptable salt or a cocrystal thereof, 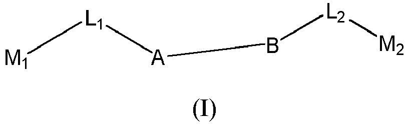
wherein, M₁ is 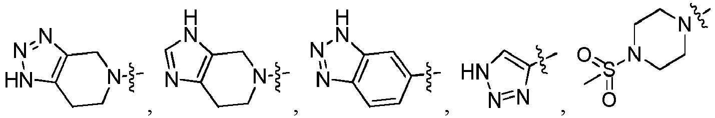 or 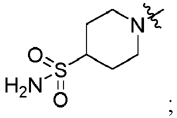
L₁ is a bond, -(CR₁R₂)ₐ-(NR₇)_{b}-W-(CR₃R₄)_{c}-(NR₈)_{d}-(CR₅R₆)ₑ-, or -C(O)-(CR₃R₄),-C=CR₇-; W is -C(=X)-, or a 3-6 membered heterocyclene containing 1-3 heteroatoms selected from N, O or S;
X is O, S or NRₓ, and Rₓ is H or cyano;
each of R₁ to R₆ is independently selected from H, halogen, C₁₋₄ alkyl or C₃₋₆ cycloalkyl; R₇ and R₈ are each independently selected from H, C₁₋₄ alkyl or C₃₋₆ cycloalkyl;
alternatively, R₁ and R₂ on the same carbon atom, R₃ and R₄ on the same carbon atom, or R₅ and R₆ on the same carbon atom together with the carbon atom to which they are attached form a 3-6-membered carbocyclic ring, wherein the carbocyclic ring is optionally substituted with 1-4 substituents selected from halogen or C₁₋₄ alkyl;
a, c and e are independently selected from an integer of 0-5, and b and d are independently 0 or 1;
A is C₃₋₆ cycloalkylene, 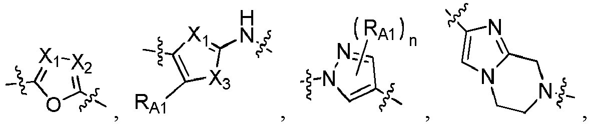 C₂₋₄ alkynylene, -RaC(O)NRa'-, -RaNRa'C(O)-, -RaNRa'-, -RaC(O)-, -Ra(CRa' Ra")ₙ- or a bond;
Ra is 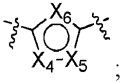
Ra' and Ra" are independently H or C₁₋₄ alkyl;
n is an integer of 1-2;
X₁ and X₂ are independently N or CR_{A1}, and are not both CR_{A1} at the same time, and X₃ is S, O or NR_{A1};
X₄, X₅ and X₆ are independently N, NR_{A1}, S, O or CR_{A1}, and are not all CR_{A1} at the same time;
each R_{A1} is independently H, cyano, -R_{A}, halogen, -C₁₋₄ alkyl R_{A}, -NHC(O)R_{A}, -C(O)R_{A}, - C₁₋₄ alkyl-O-C₁₋₄ alkyl, -NHR_{A}, -C(O)NHR_{A} or -OR_{A};
R_{A} is C₁₋₄ alkyl, C₃₋₆ cycloalkyl, C₃₋₆ cycloalkyloxy, C₁₋₄ alkoxy, C₁₋₄ haloalkyl, C₁₋₄ haloalkoxy or a 3-6 membered heterocyclyl containing 1-3 heteroatoms selected from N, O or S, the alkyl, cycloalkyl, cycloalkyloxy, alkoxy, haloalkyl, haloalkoxy and heterocyclyl are optionally further substituted with 1-6 groups selected from C₃₋₆ cycloalkyl, C₁₋₄ alkyl, halogen, -S(O)₂C₁₋₄ alkyl, -OC₁₋₄ alkyl or cyano;
B is 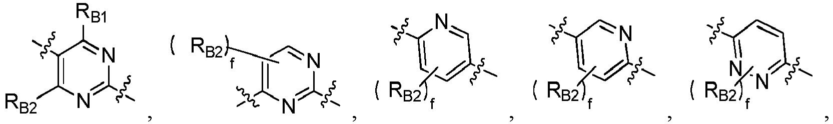 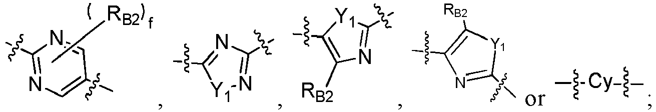
Y₁ is O, S or NR_{B3};
Cy has a structure: 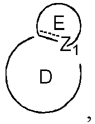 wherein, 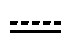 represents a single bond or a double bond, Z₁ is C or N, ring E is a 5-membered heterocycle containing 1-3 heteroatoms selected from N, O or S, and ring D is a 6-membered ring containing 0-3 heteroatoms selected from N, O or S; the 5-membered heterocycle and the 6-membered ring are independently and optionally substituted with 1-3 R_{B2};
R_{B1} and R_{B2} are each independently selected from H, oxo, OH, halogen, cyano, C₁₋₄ alkyl, halo C₁₋₄ alkyl, C₁₋₄ alkyloxy or C₃₋₆ cycloalkyl; R_{B3} is selected from H, C₁₋₄ alkyl or C₃₋₆ cycloalkyl;
alternatively, group R_{A1} on A and group R_{B1} on B together with the atoms to which they are attached form a 6-10-membered heterocycle containing 1-3 heteroatoms selected from N, S, and O;
f is an integer of 0-3;
L₂ is -O-, -NR₉-, -C(O)NR₉-, -NR₉C(O)NR₉-, -(CR₁₀R₁₁)_{g}-, -NR₉-(CR₁₀R₁₁)_{g}- or a bond, wherein g is an integer of 1-3;
R₉ is H or C₁₋₄ alkyl;
R₁₀ and R₁₁ are each independently H, halogen, C₁₋₄ alkyl or C₃₋₆ cycloalkyl; alternatively, R₁₀ and R₁₁ on the same carbon atom together with the carbon atom to which they are attached form a 3-6 membered carbocyclic ring;
M₂ is 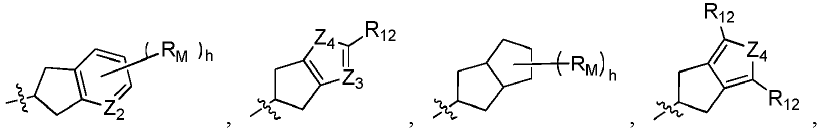 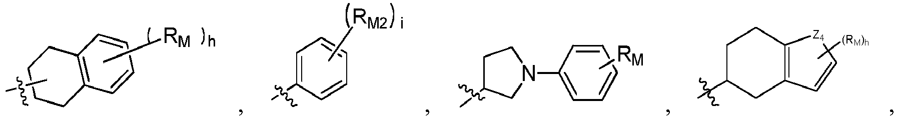 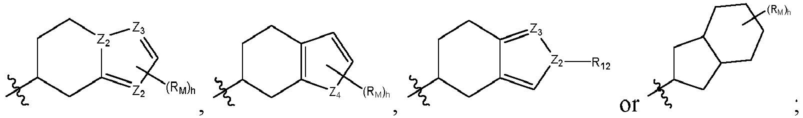
Z₂ and Z₃ are CR_{M} or N, and Z₄ is O, S or NR_{M1};
each R_{M} is independently H, C₁₋₄ alkyl, C₁₋₄ alkoxyalkyl, cyano, C₃₋₆ cycloalkyl, C₃₋₆ cycloalkyloxy, -SRm', -S(O)₂Rm', -C(O)NRmRm', -NRmC(O)Rm', C₂₋₆ alkenyl, C₂₋₆ alkynyl, -NR_{M1}, a 3-6 membered heterocyclyl containing 1-3 heteroatoms selected from N, O or S, or a halogen, the alkyl, alkenyl, alkynyl, and cycloalkyl are optionally substituted with 1-3 groups selected from halogen, cyano, C₁₋₄ alkoxy, halo C₁₋₄ alkyl or halo C₁₋₄ alkoxy, the heterocyclyl is optionally substituted with 1-3 groups selected from halogen, oxo, and C₁₋₄ alkyl;
alternatively, two R_{M} on adjacent ring carbon atoms in M₂ together with the carbon atom to which they are attached form a 3-6 membered carbocyclic ring or a 3-6 membered heterocyclyl containing 0-3 heteroatoms selected from N, O or S;
Rm is H, C₁₋₄ alkyl, and halo C₁₋₄ alkyl;
Rm' is C₁₋₄ alkyl, and halo C₁₋₄ alkyl;
R_{M1} is H, C₁₋₄ alkyl, C₃₋₆ cycloalkyl, C₁₋₄ haloalkyl or C₁₋₄ alkoxyalkyl;
R_{M2} is halo C₁₋₄ alkoxy;
each R₁₂ is independently H, halogen, C₁₋₄ alkyl, C₃₋₆ cycloalkyl or halo C₁₋₄ alkyl;
h is an integer of 0-3;
i is 0, 1 or 2;
provided that: when M₁ is 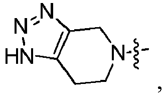 L₁ is -C(O)-CH₂-, A is 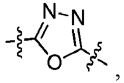 B is 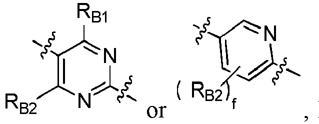 L₂ is -NH-, and M₂ is 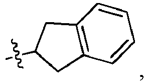 R_{B1} and R_{B2} are not both H at the same time;
when M₁ is 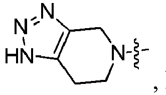 L₁ is -C(O)-CH₂-, A is 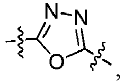 B is 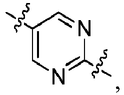 and L₂ is - NH-CH₂-, M₂ is not 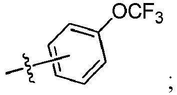
when M₁ is 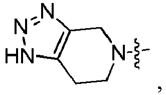 L₁ is -C(O)-CH₂-, A is 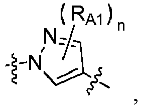 B is 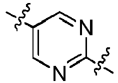 or 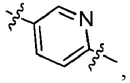 L₂ is -NH-, and M₂ is 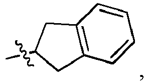 at least one substituent R_{A1} in A is selected from cyano, halogen, C₃₋₄ cycloalkyloxy, cyclopropylmethyloxy, C₁₋₄ haloalkoxy, cyclopropylmethyl, -C₁₋₄ alkyl-O-C₁₋₄ alkyl, -NHC(O) R_{A}, -C(O)NHR_{A}, -C(O)-C₃₋₆ cycloalkyl, 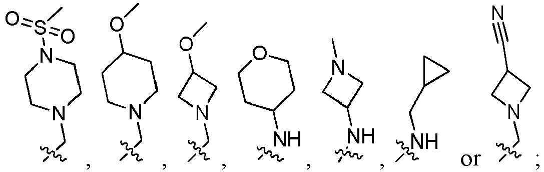
and when M₁ is 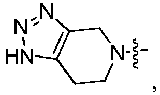 L₁ is -C(O)-CH₂-, A is 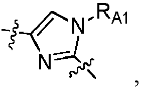 B is 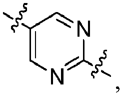 L2 is -NH-, and M₂ is 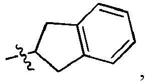 R_{A1} is not methyl, ethyl or cyclopropyl.

In some embodiments, the nitrogen-containing heterocyclic compound of formula (I), or the stereoisomer, the solvate, the deuterated form, the pharmaceutically acceptable salt or the cocrystal thereof, wherein, M₁ is 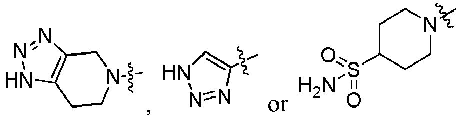
Li is -(CR₁R₂)ₐ-(NR₇)_{b}-W-(CR₃R₄)_{c}-(NR₈)_{d}-(CR₅R₆)ₑ-, or -C(O)-(CR₃R₄)_{c}-C=CR₇-;
W is -C(=X)-, or a 3-6 membered heterocyclene containing 1-3 heteroatoms selected from N, O or S;
X is O, S or NRₓ, and Rₓ is H or cyano;
each of R₁ to R₆ is independently selected from H, halogen, C₁₋₄ alkyl or C₃₋₆ cycloalkyl; R₇ and R₈ are each independently selected from H, C₁₋₄ alkyl or C₃₋₆ cycloalkyl; alternatively, R₁ and R₂ on the same carbon atom, R₃ and R₄ on the same carbon atom, or R₅ and R₆ on the same carbon atom together with the carbon atom to which they are attached form a 3-6-membered carbocyclic ring, wherein the carbocyclic ring is optionally substituted with 1-4 substituents selected from halogen or C₁₋₄ alkyl;
a, c and e are independently selected from an integer of 0-5, and b and d are independently 0 or 1;
A is C₃₋₆ cycloalkylene, 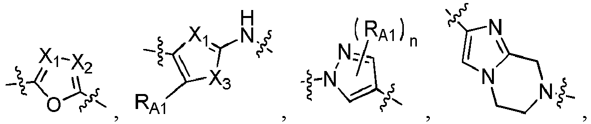 C₂₋₄ alkynylene or a bond;
n is an integer of 1-2;
X₁ and X₂ are independently N or CR_{A1}, and are not both CR_{A1} at the same time, and X₃ is S, O or NR_{A1};
each R_{A1} is independently H, cyano, -R_{A}, halogen, -C₁₋₄ alkyl R_{A}, -NHC(O) R_{A}, -C(O) R_{A}, - C₁₋₄ alkyl-O-C₁₋₄ alkyl, -NHR_{A}, -C(O)NHR_{A} or -OR_{A};
R_{A} is C₁₋₄ alkyl, C₃₋₆ cycloalkyl or a 3-6 membered heterocyclyl containing 1-3 heteroatoms selected from N, O or S, wherein the alkyl, cycloalkyl, and heterocyclyl are optionally further substituted with 1-6 groups selected from C₃₋₆ cycloalkyl, C₁₋₄ alkyl, halogen, - S(O)₂C₁₋₄ alkyl, -OC₁₋₄ alkyl or cyano;
B is 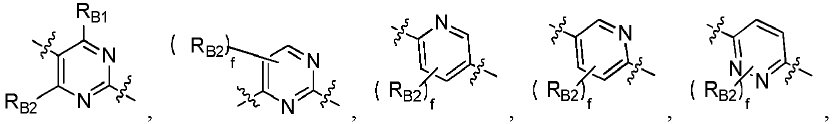 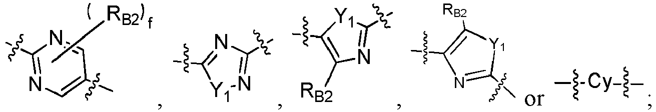
Y₁ is O, S or NR_{B3}; Cy has a structure: 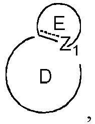 wherein, 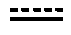 represents a single bond or a double bond, Z₁ is C or N, ring E is a 5-membered heterocycle containing 1-3 heteroatoms selected from N, O or S, and ring D is a 6-membered ring containing 0-3 heteroatoms selected from N, O or S; the 5-membered heterocycle and the 6-membered ring are independently and optionally substituted with 1-3 R_{B2};
R_{B1} and R_{B2} are each independently selected from H, oxo, OH, halogen, cyano, C₁₋₄ alkyl, halo C₁₋₄ alkyl, C₁₋₄ alkyloxy or C₃₋₆ cycloalkyl; R_{B3} is selected from H, C₁₋₄ alkyl or C₃₋₆ cycloalkyl;
alternatively, group R_{A1} on A and group R_{B1} on B together with the atoms to which they are attached form a 6-10-membered heterocycle containing 1-3 heteroatoms selected from N, S, and O;
f is an integer of 0-3;
L₂ is -O-, -NR₉-, -(CR₁₀R₁₁)_{g}- or a bond, wherein g is an integer of 1-3;
R₉ is H or C₁₋₄ alkyl;
R₁₀ and R₁₁ are each independently H, halogen, C₁₋₄ alkyl or C₃₋₆ cycloalkyl; alternatively,
R₁₀ and R₁₁ on the same carbon atom together with the carbon atom to which they are attached form a 3-6 membered carbocyclic ring;
M₂ is 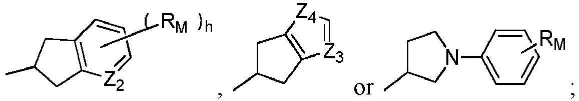
Z₂ and Z₃ are CR_{M} or N, and Z₄ is O, S or NR_{M1};
each R_{M} is independently H, C₁₋₄ alkyl, cyano, C₃₋₆ cycloalkyl or halogen, the alkyl and cycloalkyl are optionally substituted with 1-3 groups selected from halogen and cyano; R_{M1} is H, C₁₋₄ alkyl or C₃₋₆ cycloalkyl;
h is an integer of 0-3;
provided that: when M₁ is 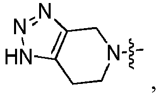 L₁ is -C(O)-CH₂-, A is 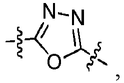 B is 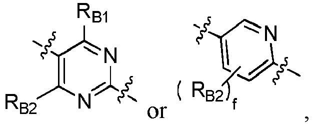 L₂ is -NH-, and M₂ is 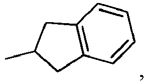 R_{B1} and R_{B2} are not both H at the same time;
when M₁ is 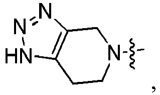 L₁ is -C(O)-CH₂-, A is 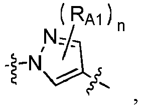 B is 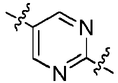 or 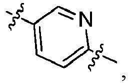 L₂ is -NH-, and M₂ is 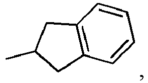 at least one of substituent R_{A1} in A is selected from cyano, halogen, C₃₋₄ cycloalkyloxy, cyclopropylmethoxy, C₁₋₄ haloalkoxy, cyclopropylmethyl, -C₁₋₄ alkyl-O-C₁₋₄ alkyl, -NHC(O) R_{A}, -C(O)NHR_{A}, -C(O)-C₃₋₆ cycloalkyl, 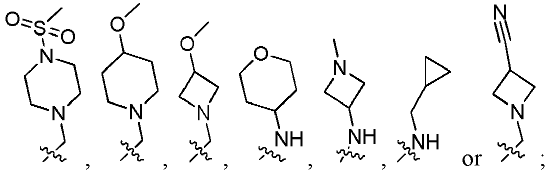 and when M₁ is 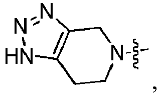 L₁ is -C(O)-CH₂-, A is 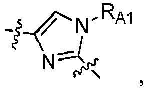 B is 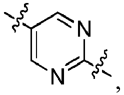 L₂ is -NH-, and M₂ is 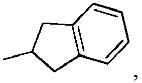 R_{A1} is not methyl, ethyl or cyclopropyl.

As a second technical solution of the present invention, provided are a nitrogen-containing heterocyclic compound of formula (I), or a stereoisomer, a solvate, a deuterated form, a pharmaceutically acceptable salt or a cocrystal thereof, wherein,
a, c and e are independently selected from an integer of 0-3, and b and d are independently 0 or 1;
W is -C(=X)- or a 5-membered heterocyclene containing 1-2 heteroatoms selected from N and O, wherein X is O, S or NRₓ, and Rₓ is cyano;
each of R₁ to R₆ is independently selected from H, halogen, and C₁₋₄ alkyl, and R₇ and R₈ are independently selected from H or C₁₋₄ alkyl;
alternatively, R₃ and R₄ on the same carbon atom, or R₅ and R₆ on the same carbon atom together with the carbon atom to which they are attached form a 3-4-membered carbocyclic ring, wherein the carbocyclic ring is optionally substituted with 1-2 substituents selected from halogen and C₁₋₂ alkyl;
A is C₃₋₆ cycloalkylene, 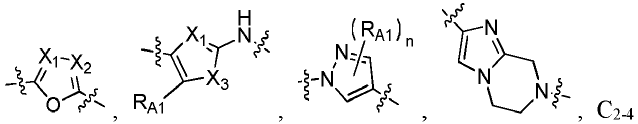 alkynylene or a bond;
R_{A} is C₁₋₄ alkyl, C₃₋₄ cycloalkyl, a 4-6-membered heterocyclyl containing 1-2 heteroatoms selected from N and O, wherein the alkyl and heterocyclyl are optionally further substituted with 1-5 groups selected from C₃₋₄ cycloalkyl, C₁₋₄ alkyl, halogen, -S(O)₂C₁₋₂ alkyl, -OC₁₋₂ alkyl or cyano,
and the rest of the groups are as defined in the above-mentioned first technical solution.

As a third technical solution of the present invention, provided are a nitrogen-containing heterocyclic compound of formula (I), or a stereoisomer, a solvate, a deuterated form, a pharmaceutically acceptable salt or a cocrystal thereof, wherein,
L₁ is -C(=O)-(NH)_{d}-CR₃R₄-, d is 0 or 1, at least one of R₃ and R₄ is halogen, or R₃ and R₄ and the carbon atom to which they are attached form a 3-4-membered carbocyclic ring; or
L₁ is -C(=S)-CR₃R₄-, wherein R₃ and R₄ are independently selected from H, halogen or C₁₋₄ alkyl; or
L₁ is -C(=N-CN)-CR₃R₄-, wherein R₃ and R₄ are independently selected from H, halogen or C₁₋₄ alkyl; or
L₁ is -C(O)-CR₃R₄-C=CR₇-, wherein R₃ and R₄ are independently selected from H, halogen or C₁₋₄ alkyl, and R₇ is H or C₁₋₄ alkyl; or
L₁ is -W-(CR₃R₄)_{c}-, wherein W is a 5-membered heterocyclene containing 1-2 heteroatoms selected from N or O, and c is an integer of 0-3,
and the rest of the groups are as defined in the above-mentioned first or second technical solution.

As a fourth technical solution of the present invention, provided are a nitrogen-containing heterocyclic compound of formula (I), or a stereoisomer, a solvate, a deuterated form, a pharmaceutically acceptable salt or a cocrystal thereof, wherein the compound has a more specific structure of formula (II): 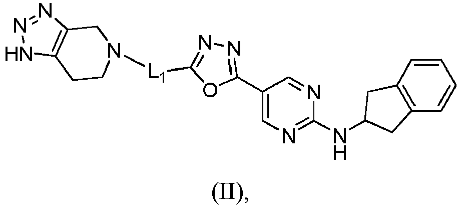 and the rest of the groups are as defined in the above-mentioned third technical solution.

As a fifth technical solution of the present invention, provided are a nitrogen-containing heterocyclic compound of formula (I), or a stereoisomer, a solvate, a deuterated form, a pharmaceutically acceptable salt or a cocrystal thereof, when L₁ is -W-(CR₃R₄)_{c}-, W is a 5-membered heterocyclene containing 1-2 heteroatoms selected from N and O, and c is an integer of 0-3, the 5-membered heterocyclene is 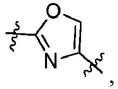 and the rest of the groups are as defined in the above-mentioned third technical solution.

As a sixth technical solution of the present invention, provided are a nitrogen-containing heterocyclic compound as shown in formula (I), or a stereoisomer, a solvate, a deuterated form, a pharmaceutically acceptable salt or a cocrystal thereof, wherein, M₁ is 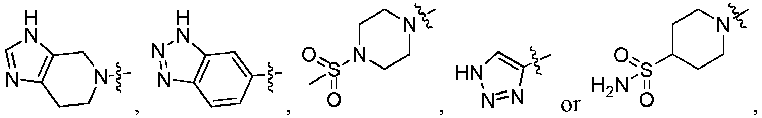 and the rest of the groups are as defined in the above-mentioned first or second technical solution.

As a seventh technical solution of the present invention, provided are a nitrogen-containing heterocyclic compound as shown in formula (I), or a stereoisomer, a solvate, a deuterated form, a pharmaceutically acceptable salt or a cocrystal thereof, wherein M₁ is 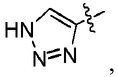 A is 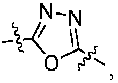 B is 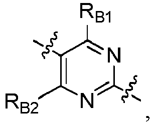 L₂ is -NH- and M₂ is 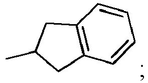 or M₁ is 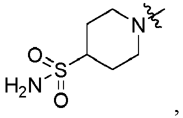 A is 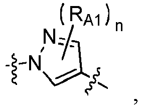 B is 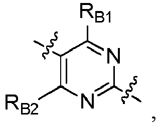 L₂ is -NH-, and M₂ is 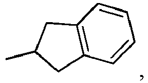 and the rest of the groups are as defined in the above-mentioned sixth technical solution.

As an eighth technical solution of the present invention, provided are a nitrogen-containing heterocyclic compound as shown in formula (I), or a stereoisomer, a solvate, a deuterated form, a pharmaceutically acceptable salt or a cocrystal thereof, wherein, L₂ is - C(O)NR₉-, -NR₉C(O)NR₉-, -O-, or -CR₁₀R₁₁-, at least one of R₁₀ and R₁₁ is halogen or C₁₋₄ alkyl, or R₁₀ and R₁₁ together with the carbon atom to which they are attached form a 3-4-membered carbocyclic ring, and the rest of the groups are as defined in the above-mentioned first or second technical solution.

As a ninth technical solution of the present invention, provided are a nitrogen-containing heterocyclic compound as shown in formula (I), or a stereoisomer, a solvate, a deuterated form, a pharmaceutically acceptable salt or a cocrystal thereof, wherein the compound has a more specific structure of formula (III) 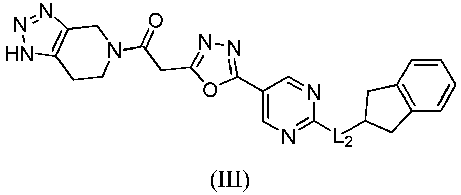 and the rest of the groups are as defined in the above-mentioned eighth technical solution.

As a tenth technical solution of the present invention, provided are a nitrogen-containing heterocyclic compound as shown in formula (I), or a stereoisomer, a solvate, a deuterated form, a pharmaceutically acceptable salt or a cocrystal thereof, wherein: A is C₃₋₆ cycloalkylene, C₂₋₄ alkynylene, 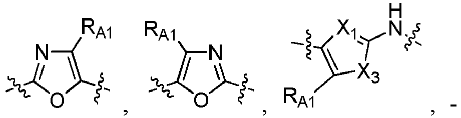 RaC(O)NRa'- or 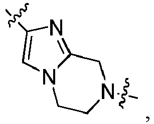 X₁ is N or CR_{A1}, X₃ is S, O or NR_{A1}, each R_{A1} is independently H, cyano, -R_{A}, halogen, -C₁₋₄ alkyl R_{A}, -NHC(O)R_{A}, -C(O)R_{A}, -C₁₋₄ alkyl-O-C₁₋₄ alkyl, -NHR_{A}, -C(O)NHR_{A} or -OR_{A}, Ra is 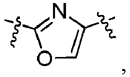 Ra' is H or C₁₋₄ alkyl, and the rest of the groups are as defined in the above-mentioned first or second technical solution; or A is 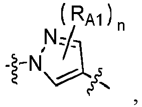 n is not 0, at least one R_{A1} is selected from cyano, halogen, C₃₋₄ cycloalkyloxy, cyclopropylmethoxy, halo C₁₋₄ alkoxy, cyclopropylmethyl, -C₁₋₄ alkyl-O-C₁₋₄ alkyl, -NHC(O)R_{A}, -C(O)NHR_{A}, -C(O)-C₃₋₆cycloalkyl, 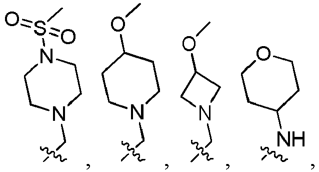 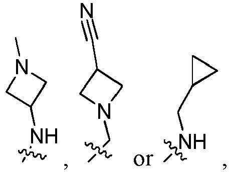 and the rest of the groups are as defined in the above-mentioned first or second technical solution.

As an eleventh technical solution of the present invention, provided are a nitrogen-containing heterocyclic compound as shown in formula (I), or a stereoisomer, a solvate, a deuterated form, a pharmaceutically acceptable salt or a cocrystal thereof, wherein the compound has a more specific structure of formula (IV) 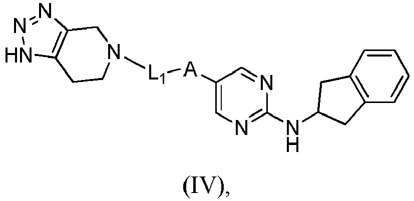 L₁ is -C(O)-(CR₅R₆)ₑ-, and the definition of the rest of the groups are as defined in the above-mentioned tenth technical solution.

As a twelfth technical solution of the present invention, provided are a nitrogen-containing heterocyclic compound as shown in formula (I), or a stereoisomer, a solvate, a deuterated form, a pharmaceutically acceptable salt or a cocrystal thereof, wherein: B is 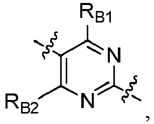 wherein at least one of R_{B1} and R_{B2} is selected from OH, halogen, cyano, halo C₁₋₄ alkyl, C₁₋₄ alkyloxy and C₃₋₆ cycloalkyl, or group R_{A1} on A and group R_{B1} on B together with the atoms to which they are attached form a 6-8-membered heterocycle containing 1 O atom, and the rest of the groups are as defined in the above-mentioned first or second technical solution; or B is 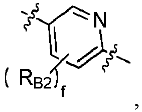 wherein R_{B2} is halogen or cyano, f is 1, 2 or 3, and the rest of the groups are as defined in the above-mentioned first or second technical solution; or B is 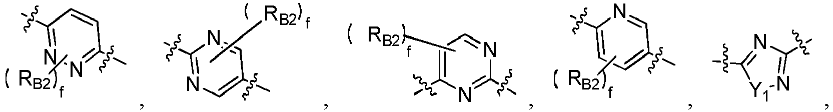 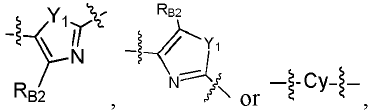 wherein the rest of the groups are as defined in the above-mentioned first or second technical solution.

As a thirteenth technical solution of the present invention, provided are a nitrogen-containing heterocyclic compound as shown in formula (I), or a stereoisomer, a solvate, a deuterated form, a pharmaceutically acceptable salt or a cocrystal thereof, wherein the compound has a more specific structure of formula (V) 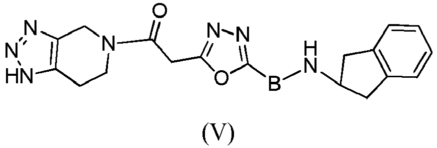 wherein B is 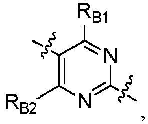 at least one of R_{B1} and R_{B2} is selected from OH, halogen, cyano, halo C₁₋₄ alkyl, C₁₋₄ alkyloxy or C₃₋₆ cycloalkyl, and the rest of the groups are as defined in the above-mentioned twelfth technical solution; or B is 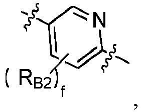 R_{B2} is halogen or cyano, f is 1, 2 or 3, and the rest of the groups are as defined in the above-mentioned twelfth technical solution; or B is 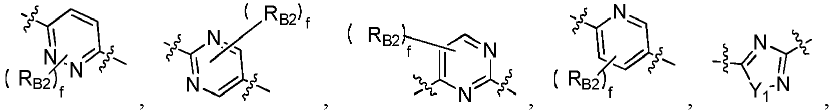 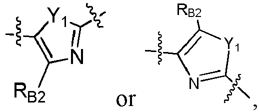 and the rest of the groups are as defined in the above-mentioned twelfth technical solution.

As a fourteenth technical solution of the present invention, provided are a nitrogen-containing heterocyclic compound as shown in formula (I), or a stereoisomer, a solvate, a deuterated form, a pharmaceutically acceptable salt or a cocrystal thereof, wherein the compound has a more specific structure of formula (VI) 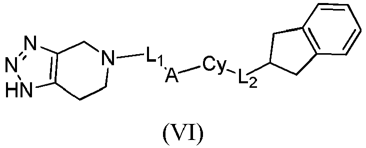
wherein, L₁ is -C(=O)-(CR₃R₄)_{c}-(NR₈)_{d}-, c is an integer of 0-3, d is 0 or 1, R₃, R₄ and R₈ are independently selected from H, C₁₋₃ alkyl or halogen, and R₃ and R₄ together with the carbon atom to which they are attached form a 3-6 membered carbocyclic ring, wherein the carbocyclic ring is optionally substituted with 1-4 substituents selected from halogen or C₁₋₄ alkyl; A is 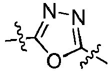 or a bond;
Cy has a structure: 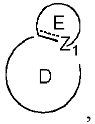 wherein, 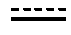 represents a single bond or a double bond, Z₁ is C or N, ring E is a 5-membered heterocycle containing 1-3 heteroatoms selected from N, O or S, ring D is a saturated or unsaturated 6-membered ring containing 0-3 heteroatoms selected from N, O or S; the 5-membered heterocycle and the 6-membered ring are independently and optionally substituted with 1-3 R_{B2};
each R_{B2} is independently selected from H, halogen, cyano, C₁₋₄ alkyl and halo C₁₋₄ alkyl;
L₂ is -NH- or a bond,
and the rest of the groups are as defined in the above-mentioned first or second technical solution.

As a fifteenth technical solution of the present invention, provided are a nitrogen-containing heterocyclic compound as shown in formula (I), or a stereoisomer, a solvate, a deuterated form, a pharmaceutically acceptable salt or a cocrystal thereof, wherein, 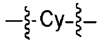 is selected from one of the following structures, and the rest of the groups are as defined in the above-mentioned fourteenth technical solution:

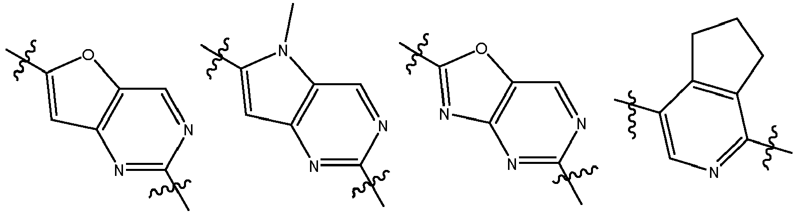

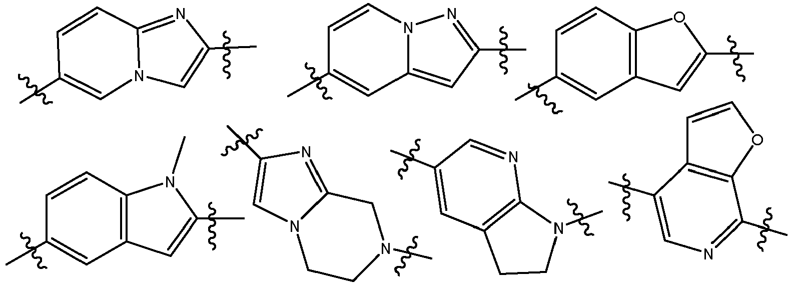

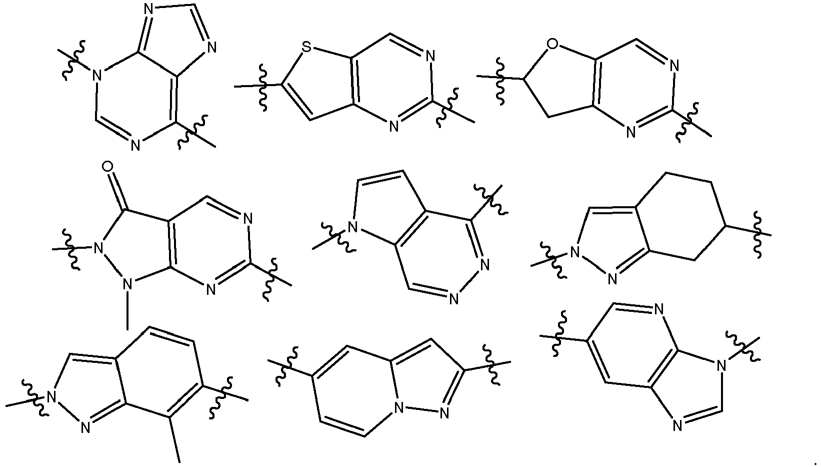

As a sixteenth technical solution of the present invention, provided are a nitrogen-containing heterocyclic compound as shown in formula (I), or a stereoisomer, a solvate, a deuterated form, a pharmaceutically acceptable salt or a cocrystal thereof, wherein: when M₂ is 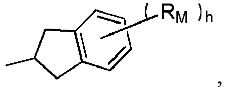 h is not 0, and at least one R_{M} is halo C₁₋₄ alkyl, cyano, C₃₋₆ cycloalkyl, C₂₋₆ alkynyl or a 3-6 membered heterocyclyl containing 1-3 heteroatoms selected from N, O or S, the alkyl is optionally substituted with 1-3 groups selected from halogen or cyano, and the heterocyclyl is optionally substituted with 1-3 groups selected from halogen, oxo or C₁₋₄ alkyl; or, alternatively, two R_{M} on adjacent ring carbon atoms in M₂ together with the carbon atoms to which they are attached form a 3-6 membered carbocyclic ring or a 3-6 membered heterocyclyl containing 0-3 heteroatoms selected from N, O or S; and the rest of the groups are as defined in the above-mentioned first or second technical solution; preferably, M₂ is 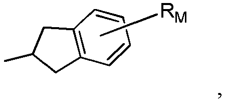 wherein R_{M} is trifluoromethyl, difluoromethyl, cyclopropyl, ethynyl, furan-3-yl, methylaminocarbonyl, azetidin-1-yl, oxetan-3-yl, 3,3-difluoro-azetidin-1-yl, acetamido, 1-methyl-1H-pyrazol-4-yl, tetrahydrofuran-3-yl, methylthio, 4-methyl-5-oxo-1,2,4-triazol-1-yl, 2-oxo-pyrrolidin-1-yl, methylsulfonyl, methoxymethyl, difluoromethoxymethyl or trifluoromethyl ethynyl; more preferably, M₂ is 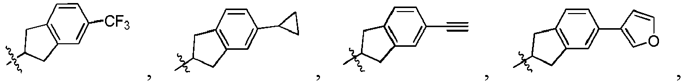 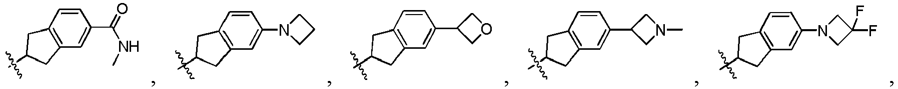 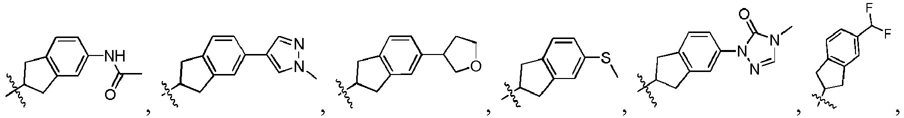 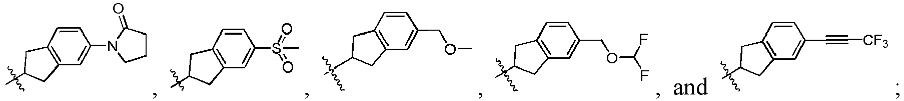 preferably, M₂ is 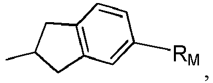 wherein R_{M} is C₁₋₄ alkyl, and the alkyl is substituted with 1-3 halogens (preferably F); preferably trifluoromethyl or difluoromethyl; when M₂ is 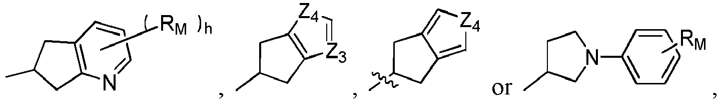 each substituent is as defined in the above-mentioned first or second technical solution.

As a seventeenth technical solution of the present invention, provided are a nitrogen-containing heterocyclic compound as shown in formula (I), or a stereoisomer, a solvate, a deuterated form, a pharmaceutically acceptable salt or a cocrystal thereof, wherein the compound has a more specific structure of formula (VII) 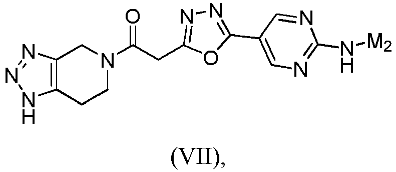 and M₂ is as defined in the above-mentioned sixteenth technical solution.

As a eighteenth technical solution of the present invention, provided are a nitrogen-containing heterocyclic compound as shown in formula (I), or a stereoisomer, a solvate, a deuterated form, a pharmaceutically acceptable salt or a cocrystal thereof,
wherein, M₁ is 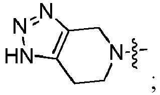
L₁ is -(CR₁R₂)ₐ-(NR₇)_{b}-W-(CR₃R₄)_{c}-(NR₈)_{d}-(CR₅R₆)ₑ-;
W is -C(=X)-, and X is O or S;
a and b are 0, c is 1 or 2, and d and e are 0;
R₃ and R₄ are each independently selected from H, halogen and C₁₋₄ alkyl, and are not both
H at the same time;
A is 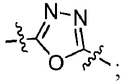
B is 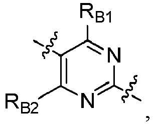 wherein both R_{B1} and R_{B2} are H;
L₂ is -NR₉-, and R₉ is H;
M₂ is 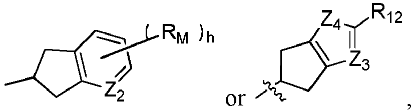 wherein Z₂ and Z₃ are CR_{M}, and Z₄ is O or S;
each R₁₂ is independently H, halogen, C₁₋₄ alkyl, C₃₋₆ cycloalkyl or halo C₁₋₄ alkyl; h is 0, 1 or 2;
each R_{M} is independently H or C₁₋₄ alkyl, the alkyl is optionally substituted with 1-3 halogens; and the rest of the groups are as defined in the above-mentioned first or second technical solution.

As a nineteenth technical solution of the present invention, provided are a nitrogen-containing heterocyclic compound as shown in formula (I), or a stereoisomer, a solvate, a deuterated form, a pharmaceutically acceptable salt or a cocrystal thereof,
wherein, M₁ is 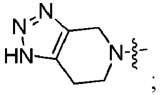
L₁ is a bond or -(CR₁R₂)ₐ-(NR₇)_{b}-W-(CR₃R₄)_{c}-(NR₈)_{d}-(CR₅R₆)ₑ-;
W is -C(=X)-;
X is O, S or NRₓ, and Rₓ is H or cyano;
each of R₁ to R₆ is independently selected from H, halogen, C₁₋₄ alkyl or C₃₋₆ cycloalkyl;
R₇ and R₈ are each independently selected from H, C₁₋₄ alkyl or C₃₋₆ cycloalkyl;
alternatively, R₁ and R₂ on the same carbon atom, R₃ and R₄ on the same carbon atom, or R₅
and R₆ on the same carbon atom together with the carbon atom to which they are attached
form a 3-6-membered carbocyclic ring, wherein the carbocyclic ring is optionally substituted
with 1-4 substituents selected from halogen or C₁₋₄ alkyl;
a, c and e are independently selected from 0, 1, 2 and 3, and b and d are independently 0 or 1;
A is 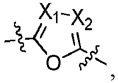 -RaC(O)NRa'-, -RaNRa'C(O)-, -RaNRa'-, -RaC(O)-, or -Ra(CRa' Ra")ₙ-;
Ra is 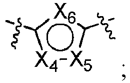 Ra' and Ra" are H or C₁₋₄ alkyl;
n is 1 or 2;
X₁ and X₂ are independently N, NR_{A1} or CR_{A1}, and are not both CR_{A1} at the same time;
X₄, X₅ and X₆ are independently N, NR_{A1}, S, O or CR_{A1}, and are not all CR_{A1} at the same time;
each R_{A1} is independently substituted with substituents of H, cyano, -R_{A}, halogen, -C₁₋₄ alkyl R_{A}, -NHC(O)R_{A}, -C(O)R_{A}, -C₁₋₄ alkyl-O-C₁₋₄ alkyl, -NHR_{A}, -C(O)NHR_{A}, and -OR_{A};
R_{A} is C₁₋₄ alkyl, C₃₋₆ cycloalkyl or a 3-6 membered heterocyclyl containing 1-3 heteroatoms selected from N, O or S, the alkyl, cycloalkyl, and heterocyclyl are optionally further substituted with 1-6 groups selected from C₃₋₆ cycloalkyl, C₁₋₄ alkyl, halogen, -S(O)₂C₁₋₄ alkyl, -OC₁₋₄ alkyl or cyano;
B is 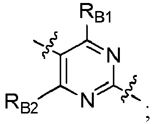 R_{B1} and R_{B2} are each independently selected from H, OH, halogen, cyano, C₁₋₄ alkyl, halo C₁₋₄ alkyl, C₁₋₄ alkyloxy or C₃₋₆ cycloalkyl; L₂ is -O-, -NR₉-, -(CR₁₀R₁₁)_{g}-, -NR₉-(CR₁₀R₁₁)_{g}- or a bond, wherein g is an integer of 1-3; R₉ is H or C₁₋₄ alkyl;
R₁₀ and R₁₁ are each independently H, halogen, C₁₋₄ alkyl or C₃₋₆ cycloalkyl; alternatively,
R₁₀ and R₁₁ on the same carbon atom together with the carbon atom to which they are attached form a 3-6 membered carbocyclic ring;
M₂ is 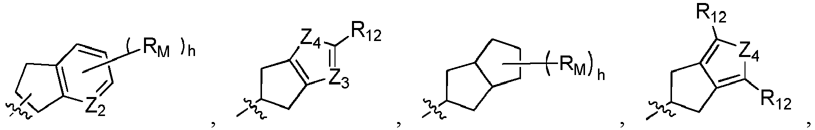 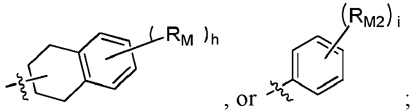
Z₂ and Z₃ are CR_{M} or N, and Z₄ is O, S or NR_{M1};
each R_{M} is independently H, C₁₋₄ alkyl, cyano, C₃₋₆ cycloalkyl, -SRm', -S(O)₂Rm', - C(O)NRmRm', -NRmC(O)Rm', C₂₋₆ alkynyl, a 3-6 membered heterocyclyl containing 1-3 heteroatoms selected from N, O or S, or halogen, the alkyl, alkynyl, and cycloalkyl are optionally substituted with 1-3 groups selected from halogen, cyano, C₁₋₄ alkoxy, halo C₁₋₄ alkyl or halo C₁₋₄ alkoxy, the heterocyclyl is optionally substituted with 1-3 groups selected from halogen, oxo, and C₁₋₄ alkyl;
Rm is H, C₁₋₄ alkyl, and halo C₁₋₄ alkyl, Rm' is C₁₋₄ alkyl and halo C₁₋₄ alkyl;
alternatively, two R_{M} on adjacent ring carbon atoms in M₂ together with the carbon atom to which they are attached form a 3-6 membered carbocyclic ring or a 3-6 membered heterocyclyl containing 0-2 heteroatoms selected from N, O or S;
R_{M1} is H, C₁₋₄ alkyl or C₃₋₆ cycloalkyl;
R_{M2} is halo C₁₋₄ alkoxy;
each R₁₂ is independently H, halogen, C₁₋₄ alkyl, C₃₋₆ cycloalkyl or halo C₁₋₄ alkyl;
h is an integer of 0-3;
i is 0, 1 or 2;
provided that, the compound is not:

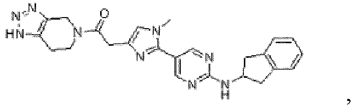

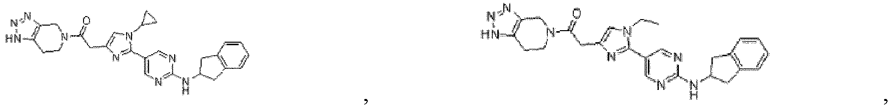

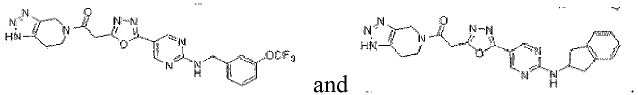

As a twentieth technical solution of the present invention, provided are a nitrogen-containing heterocyclic compound as shown in formula (I), or a stereoisomer, a solvate, a deuterated form, a pharmaceutically acceptable salt or a cocrystal thereof,
wherein, L₁ is a bond or -W-(CR₃R₄)_{c} -;
W is -C(=X)-;
X is O or S;
R₃ and R₄ are each independently selected from H, halogen or C₁₋₄ alkyl;
alternatively, R₃ and R₄ on the same carbon atom together with the carbon atom to which they are attached form a 3-6 membered carbocyclic ring;
c is selected from 0, 1 and 2; A is 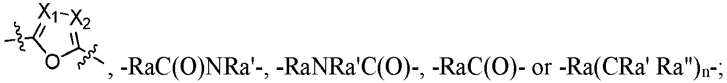
Ra is 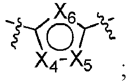
Ra' and Ra" are H or C₁₋₄ alkyl;
n is 1 or 2;
X₁ and X₂ are independently N, NR_{A1} or CR_{A1}, and are not both CR_{A1} at the same time;
X₄, X₅ and X₆ are independently N, NR_{A1}, S, O or CR_{A1}, and are not all CR_{A1} at the same time;
each R_{A1} is independently H;
B is

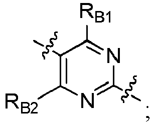

both R_{B1} and R_{B2} are H;
L₂ is -NR₉- or -NR₉-(CR₁₀R₁₁)_{g}-, wherein g is an integer of 1-3;
R₉ is H;
R₁₀ and R₁₁ are each independently H;
M₂ is 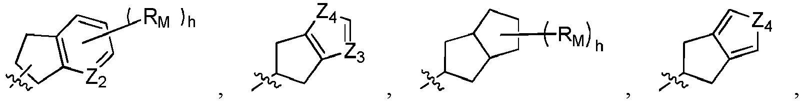 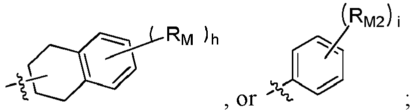
Z₂ and Z₃ are CR_{M}, and Z₄ is O or S;
each R_{M} is independently H, C₁₋₄ alkyl, C₃₋₆ cycloalkyl, -SRm', -S(O)₂Rm', -C(O)NRmRm', -NRmC(O)Rm', C₂₋₆ alkynyl, a 3-6 membered heterocyclyl containing 1-3 heteroatoms selected from N, O or S, or halogen, the alkyl, alkynyl, and cycloalkyl are optionally substituted with 1-3 groups selected from halogen, C₁₋₄ alkoxy, halo C₁₋₄ alkyl or halo C₁₋₄ alkoxy, the heterocyclyl is optionally substituted with 1-3 groups selected from halogen, oxo, and C₁₋₄ alkyl;
Rm is H or C₁₋₄ alkyl, and Rm' is C₁₋₄ alkyl;
alternatively, two R_{M} on adjacent ring carbon atoms in M₂ together with the carbon atom to which they are attached form a 3-6 membered carbocyclic ring or a 3-6 membered heterocyclyl containing 0-2 heteroatoms selected from N, O or S;
R_{M2} is halo C₁₋₄ alkoxy;
h is 0, 1 or 2;
i is 0, 1 or 2;
and the rest of the groups are as defined in the above-mentioned nineteenth technical solution.

As a twenty-first technical solution of the present invention, provided are a nitrogen-containing heterocyclic compound as shown in formula (I), or a stereoisomer, a solvate, a deuterated form, a pharmaceutically acceptable salt or a cocrystal thereof,
wherein, M₂ is 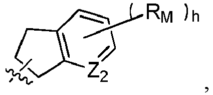
h is 1 or 2,
R_{M} is C₁₋₄ alkyl, C₃₋₆ cycloalkyl, -SRm', -S(O)₂Rm', -C(O)NRmRm', -NRmC(O)Rm', C₂₋₆ alkynyl or a 3-6 membered heterocyclyl containing 1-3 heteroatoms selected from N, O or S, the alkyl is optionally substituted with 1-3 groups selected from halogen, C₁₋₄ alkoxy or halo C₁₋₄ alkoxy, and the heterocyclyl is optionally substituted with 1-3 groups selected from halogen, oxo, and C₁₋₂ alkyl;
alternatively, two R_{M} on adjacent ring carbon atoms in M₂ together with the carbon atom to which they are attached form a 3-6 membered carbocyclic ring or a 3-6 membered heterocyclyl containing 0-2 heteroatoms selected from N, O or S; and the rest of the groups are as defined in the above-mentioned twentieth technical solution.

As a twenty-second technical solution of the present invention, provided are a nitrogen-containing heterocyclic compound as shown in formula (I), or a stereoisomer, a solvate, a deuterated form, a pharmaceutically acceptable salt or a cocrystal thereof,
wherein, M2 is 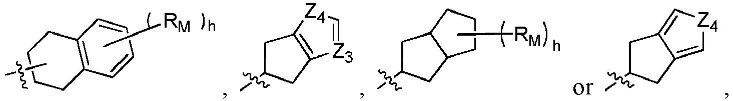 and the rest of the groups are as defined in the above-mentioned twentieth technical solution.

As a twenty-third technical solution of the present invention, provided are a nitrogen-containing heterocyclic compound as shown in formula (I), or a stereoisomer, a solvate, a deuterated form, a pharmaceutically acceptable salt or a cocrystal thereof, wherein, c is 1 or 2, and R₃ and R₄ on the same carbon atom together with the carbon atom to which they are attached form a 3-6 membered carbocyclic ring, and the rest of the groups are as defined in the above-mentioned twentieth technical solution.

As a twenty-fourth technical solution of the present invention, provided are a nitrogen-containing heterocyclic compound as shown in formula (I), or a stereoisomer, a solvate, a deuterated form, a pharmaceutically acceptable salt or a cocrystal thereof, wherein,
M₁ is 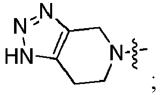
L₁ is -W-(CR₃R₄)_{c}- or -W-(NR₈)_{d}-(CR₅R₆)ₑ-;
W is -C(=X)-;
X is O or S;
R₃, R₄, R₅ and R₆ are each independently selected from H, halogen or C₁₋₄ alkyl;
each R₅ is independently selected from H, C₁₋₄ alkyl or C₃₋₆ cycloalkyl;
alternatively, R₃ and R₄ on the same carbon atom, or R₅ and R₆ on the same carbon atom together with the carbon atom to which they are attached form a 3-6 membered carbocyclic ring;
c and e are independently selected from 0 or 1, and d is 1;
A is 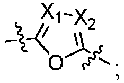
X₁ and X₂ are independently N or CR_{A1}, and are not both CR_{A1} at the same time;
R_{A1} is cyano, C₁₋₄ alkyl, -R_{A} or halogen;
R_{A} is C₃₋₆ cycloalkyl, C₃₋₆ cycloalkyloxy, C₁₋₄ alkoxy, C₁₋₄ haloalkyl, and C₁₋₄ haloalkoxy,
wherein the cycloalkyl, cycloalkyloxy, alkoxy, haloalkyl, and haloalkoxy are optionally further substituted with 1-3 groups selected from C₁₋₄ alkyl, halogen and cyano;
B is 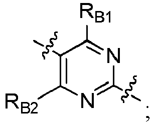
both R_{B1} and R_{B2} are H;
L₂ is -NR₉-;
R₉ is H or C₁₋₄ alkyl;
M₂ is 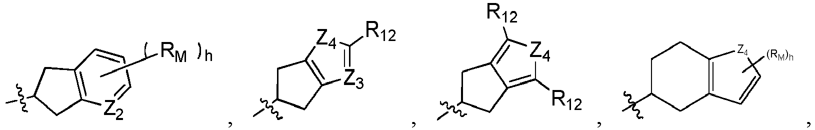 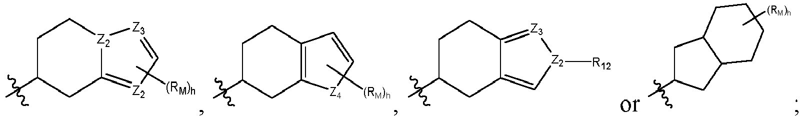
Z₂ and Z₃ are CR_{M} or N, and Z₄ is O or S;
each R_{M} is independently H, C₁₋₄ alkyl, C₁₋₄ alkoxyalkyl, cyano, C₃₋₆ cycloalkyl, C₃₋₆ cycloalkyloxy, C₂₋₆ alkenyl, C₂₋₆ alkynyl, -NR_{M1}, or a 3-6 membered heterocyclyl containing 1-3 heteroatoms selected from N, O or S, wherein the alkyl, alkenyl, alkynyl, and cycloalkyl are optionally substituted with 1-3 groups selected from halogen and cyano, and the heterocyclyl is optionally substituted with 1-3 groups selected from halogen or C₁₋₄ alkyl;
alternatively, two R_{M} on adjacent ring carbon atoms in M₂ together with the carbon atoms to which they are attached form a 3-6 membered carbocyclic ring or a 3-6 membered heterocyclyl containing 0-3 heteroatoms selected from N, O or S;
R_{M1} is H, C₁₋₄ alkyl, C₃₋₆ cycloalkyl, C₁₋₄ haloalkyl or C₁₋₄ alkoxyalkyl;
each R₁₂ is independently H, halogen or C₁₋₄ alkyl;
h is an integer of 0-3;
and i is 0, 1 or 2.

As a twenty-fifth technical solution of the present invention, provided are a nitrogen-containing heterocyclic compound as shown in formula (I), or a stereoisomer, a solvate, a deuterated form, a pharmaceutically acceptable salt or a cocrystal thereof, wherein,
M₂ is 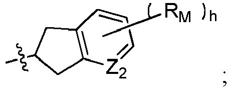
Z₂ is CR_{M};
each R_{M} is independently H, C₁₋₄ alkyl, C₁₋₄ alkoxyalkyl, C₃₋₆ cycloalkyloxy, C₂₋₆ alkenyl, C₂₋₄ alkynyl, -NR_{M1}, or a 3-6 membered heterocyclyl containing 1-3 heteroatoms selected from N, O or S, the alkyl, alkenyl and alkynyl are optionally substituted with 1-3 halogens and cyano;
alternatively, two R_{M} on adjacent ring carbon atoms in M₂ together with the carbon atoms to which they are attached form a 3-6 membered carbocyclic ring or a 3-6 membered heterocyclyl containing 0-3 heteroatoms selected from N, O or S;
R_{M1} is H, C₁₋₄ alkyl or C₃₋₆ cycloalkyl;
h is 1 or 2;
and the rest of the groups are as defined in the above-mentioned seventeenth or twenty-fourth technical solution.

As a twenty-sixth technical solution of the present invention, provided are a nitrogen-containing heterocyclic compound as shown in formula (I), or a stereoisomer, a solvate, a deuterated form, a pharmaceutically acceptable salt or a cocrystal thereof, wherein,
L₁ is -C(O)CR₃R₄-;
R₃ and R₄ are independently selected from H, halogen and C₁₋₄ alkyl;
A is 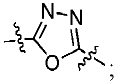
B is 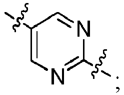
L2 is NH;
M₂ is 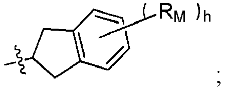
h is 1 or 2;
each R_{M} is independently H, C₁₋₄ alkyl or C₂₋₄ alkynyl; the alkyl or alkynyl is optionally substituted with 1-3 groups selected from halogen and cyano;
and R₃, R₄ and R_{M} are not all H at the same time.

As a twenty-seventh technical solution of the present invention, provided are a nitrogen-containing heterocyclic compound as shown in formula (I), or a stereoisomer, a solvate, a deuterated form, a pharmaceutically acceptable salt or a cocrystal thereof, wherein, the compound is selected from one of the following structures:

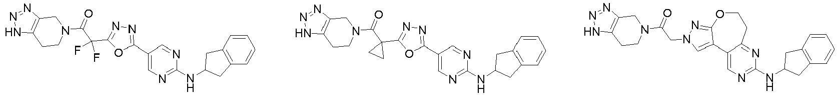

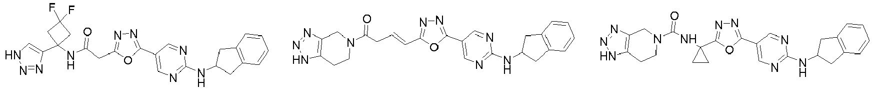

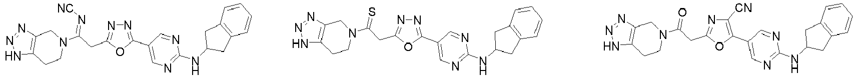

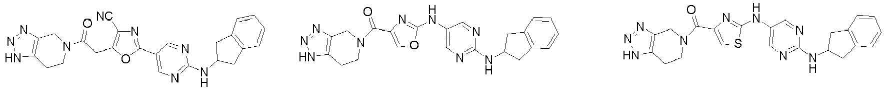

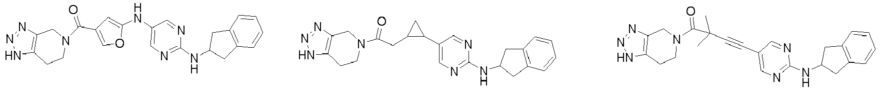

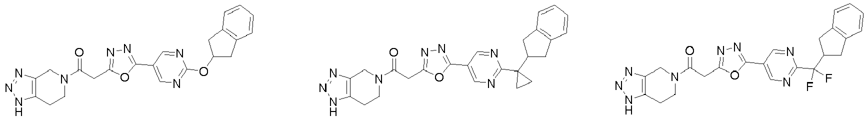

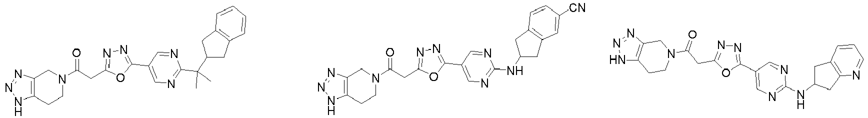

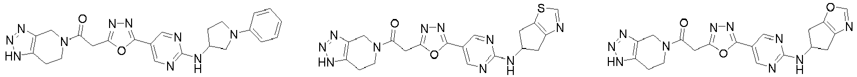

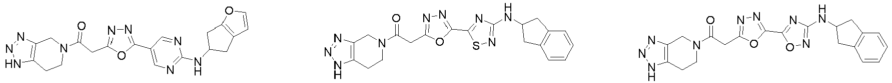

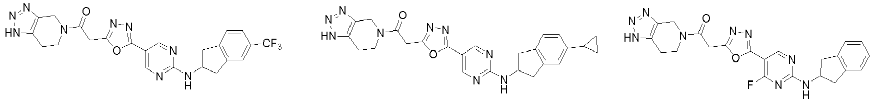

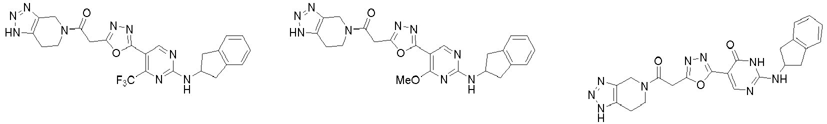

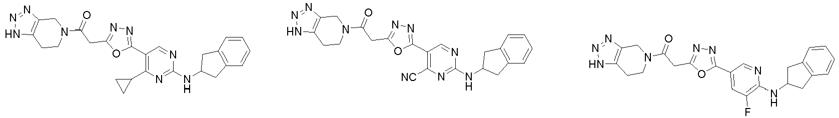

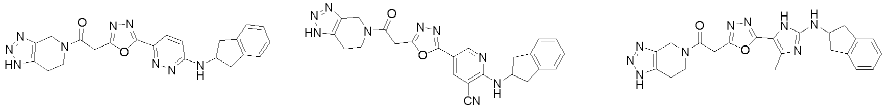

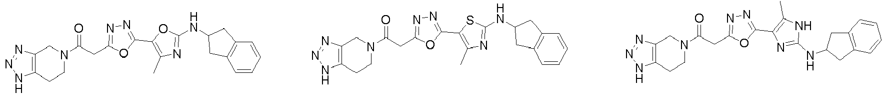

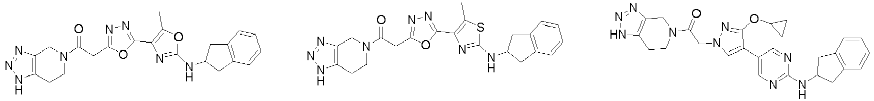

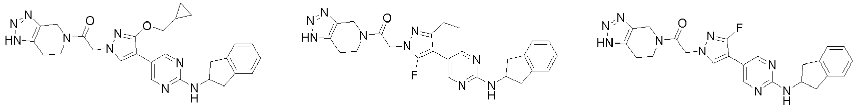

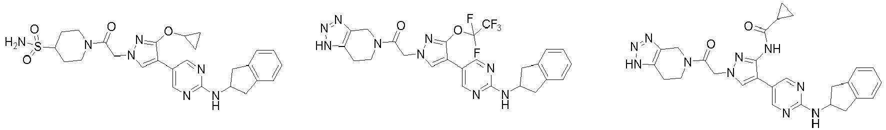

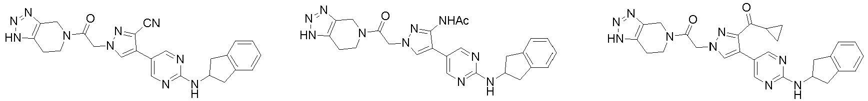

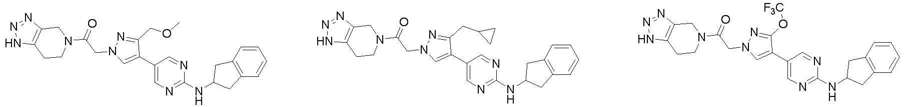

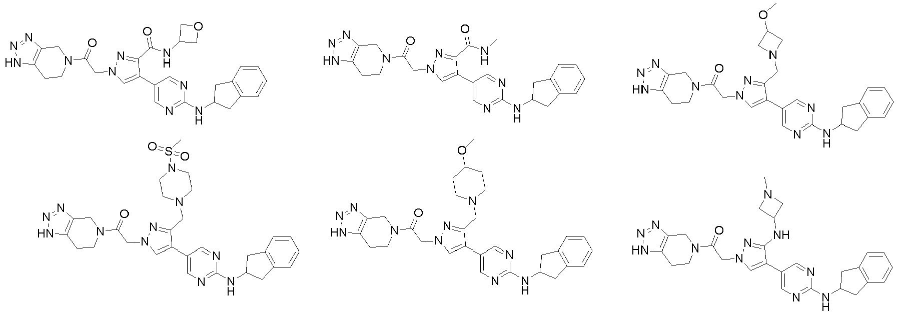

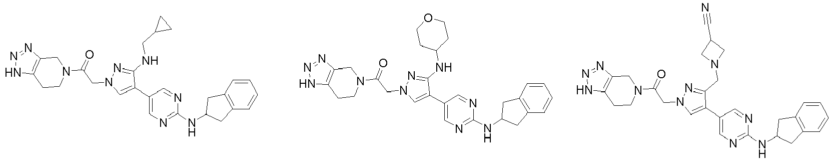

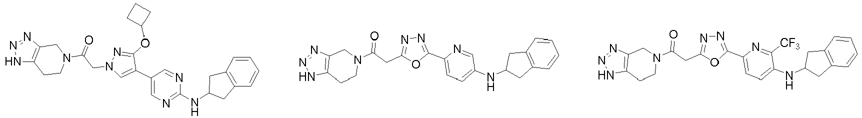

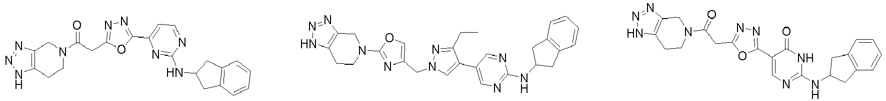

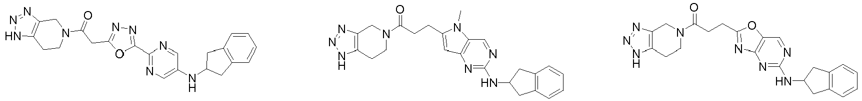

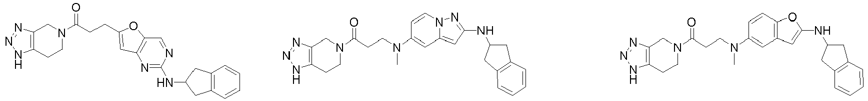

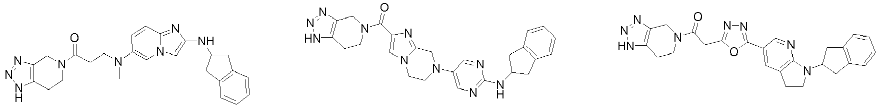

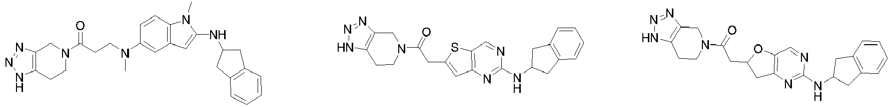

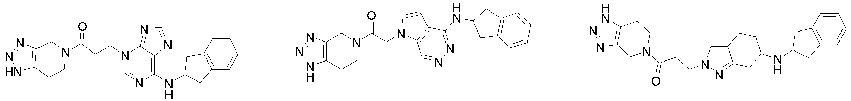

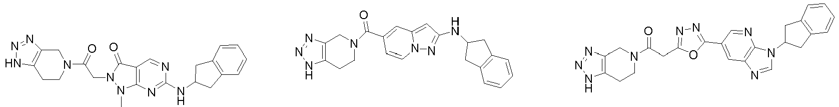

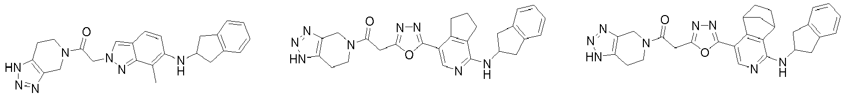

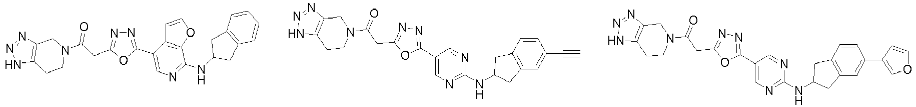

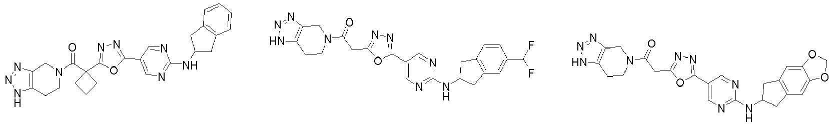

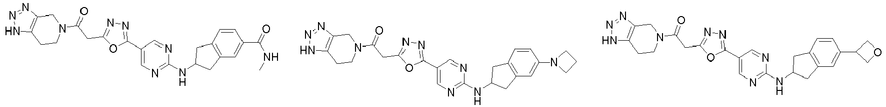

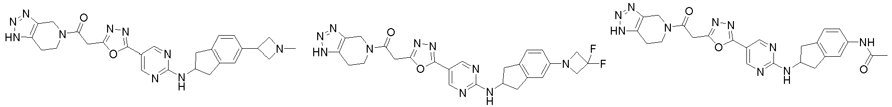

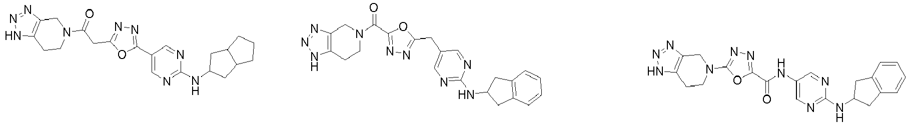

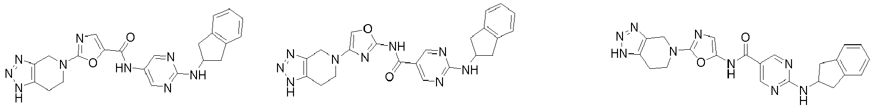

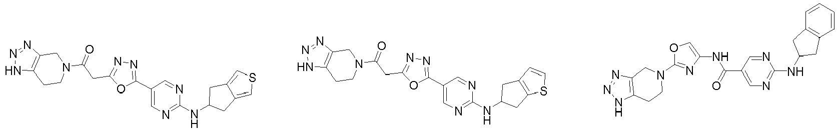

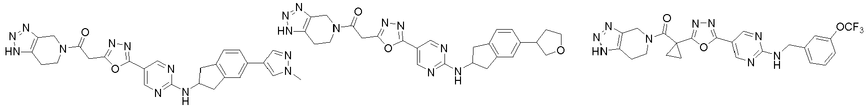

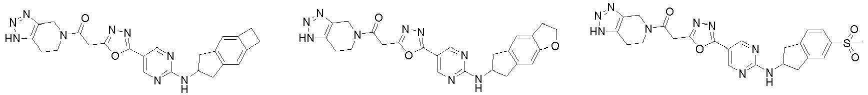

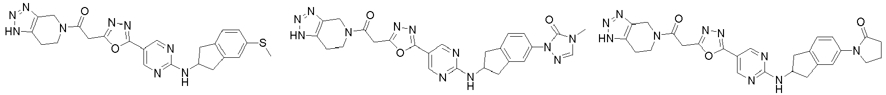

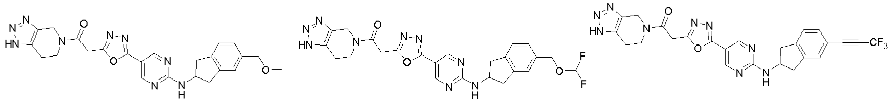

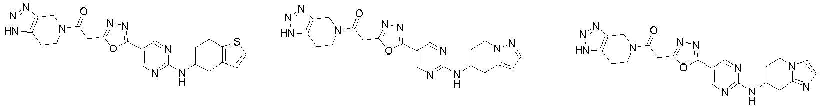

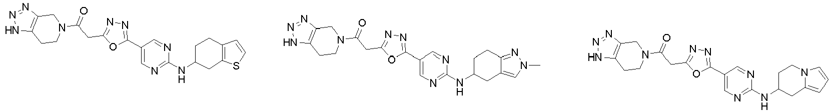

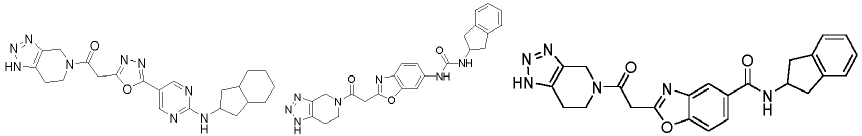

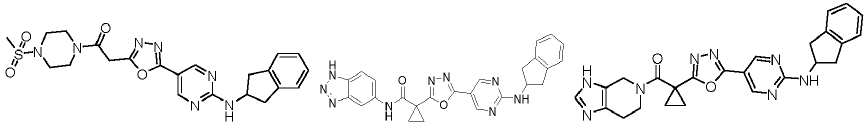

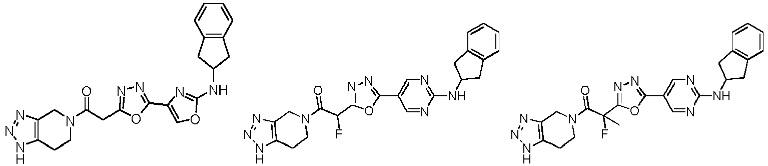

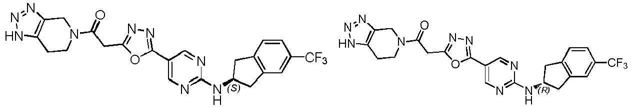

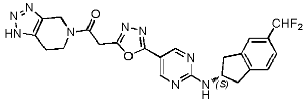

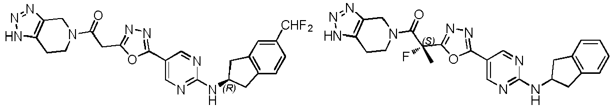

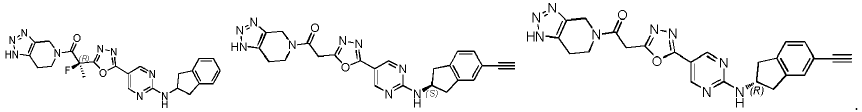

Secondly, the present invention also provides a pharmaceutical composition comprising a pharmaceutically effective amount of the nitrogen-containing heterocyclic compound of any one of the above-mentioned embodiments, or the stereoisomer, the solvate, the deuterated form, the pharmaceutically acceptable salt or the cocrystal thereof, and a pharmaceutically acceptable adjuvant and/or carrier.

Further, the present invention also provides the use of the nitrogen-containing heterocyclic compound of any one of the above-mentioned embodiments, or the stereoisomer, the solvate, the deuterated form, the pharmaceutically acceptable salt, or the cocrystal thereof or the composition containing same in the preparation of a drug for treating/preventing diseases mediated by autotaxin; and
the use of the nitrogen-containing heterocyclic compound of any one of the above-mentioned embodiments, or the stereoisomer, the solvate, the deuterated form, the pharmaceutically acceptable salt or the cocrystal thereof, or the composition containing same for treating or preventing diseases mediated by autotaxin.

Use of the above-mentioned two items, wherein, the diseases mediated by autotaxin are selected from cardiovascular conditions, cancers, metabolism disorders, kidney conditions, hepatic conditions, inflammatory conditions, nervous system conditions, respiratory system conditions, fibrotic diseases, ophthalmic conditions, cholestasis and other forms of chronic itch and acute or chronic organ-graft rejection.
preferably, the inflammatory conditions include but are not limited to arthritis, atopic dermatitis, arthritis and asthma.

A nitrogen-containing heterocyclic compound as shown in formula (I) having an inhibitory activity against ATX, or a stereoisomer, a solvate, a deuterated form, a pharmaceutically acceptable salt or a cocrystal thereof, 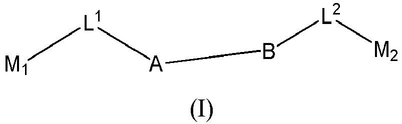
in some embodiments, M₁ is 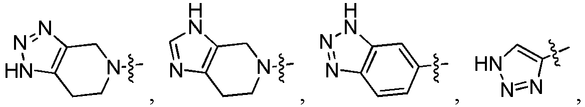 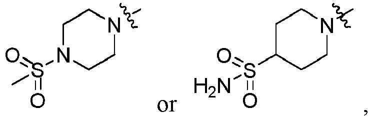 and in some embodiments, M₁ is 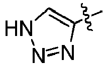 or 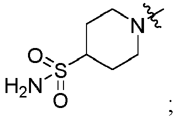 in some embodiments, M₁ is 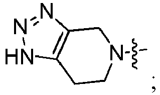
in some embodiments, L₁ is a bond, -(CR₁R₂)ₐ-(NR₇)_{b}-W-(CR₃R₄)_{c}-(NR₈)_{d}-(CR₅R₆)ₑ-, or - C(O)-(CR₃R₄)_{c}-C=CR₇-; in some embodiments, L₁ is -C(=O)-(NH)_{d}-CR₃R₄-; in some embodiments, L₁ is -C(=S)-CR₃R₄-; in some embodiments, L₁ is C(=N-CN)-CR₃R₄-; in some embodiments, L₁ is -C(O)-CR₃R₄-C=CR₇-; in some embodiments, L₁ is -W-(CR₃R₄)_{c}-; in some embodiments, L₁ is -W-(CR₃R₄)_{c}- or -W-(NR₈)_{d}-(CR₅R₆)ₑ-; in some embodiments, L₁ is -C(O)-CF₂-; in some embodiments, L₁ is -C(O)-CHF-; in some embodiments, L₁ is - C(O)-C(CH₃)(F) -; in some embodiments, L₁ is -C(O)-(CR₃R₄)-, wherein R₃ and R₄ form a 3-4-membered carbocyclic ring together, such as cyclopropyl; in some embodiments, L₁ is - C(O)-NH-(CR₅R₆)-, wherein R₅ and R₆ form a 3-4-membered carbocyclic ring together; in some embodiments, W is -C(=X)-, or a 3-6 membered heterocyclene containing 1-3 heteroatoms selected from N, O or S; in some embodiments, W is -C(=X)-, or a 5-membered heterocyclene containing 1-2 heteroatoms selected from N and O; in some embodiments, the 5-membered heterocyclene is 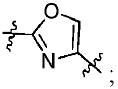
in some embodiments, X is O, S or NRₓ, wherein Rₓ is H or cyano; in some embodiments, X is O, S or NRₓ, wherein Rₓ is cyano; in some embodiments, X is O or S;
in some embodiments, each of R₁ to R₆ is independently selected from H, halogen, C₁₋₄ alkyl or C₃₋₆ cycloalkyl; in some embodiments, each of R₁ to R₆ is independently selected from H, halogen or C₁₋₄ alkyl;
in some embodiments, R₁ and R₂ on the same carbon atom, R₃ and R₄ on the same carbon atom, or R₅ and R₆ on the same carbon atom together with the carbon atom to which they are attached form a 3-6 membered carbocyclic ring, wherein the carbocyclic ring is optionally substituted with 1-4 substituents selected from halogen or C₁-₄ alkyl; in some embodiments, R₃ and R₄ on the same carbon atom, or R₅ and R₆ on the same carbon atom together with the carbon atom to which they are attached form a 3-4-membered carbocyclic ring, the carbocyclic ring is optionally substituted with 1-2 substituents selected from halogen or C₁₋₂ alkyl;
in some embodiments, R₇ and R₅ are each independently selected from H, C₁₋₄ alkyl or C₃₋₆ cycloalkyl;
in some embodiments, a, c and e are independently selected from an integer of 0-5; in some embodiments, a, c and e are independently selected from an integer of 0-3; in some embodiments, a, c and e are independently 0 or 1;
in some embodiments, b and d are independently 0 or 1;
in some embodiments, L₁ is -C(O)-CF₂-, -C(O)-CHF-, -C(O)-C(CH₃)F-, 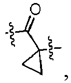 -C(O)-CH₂-, 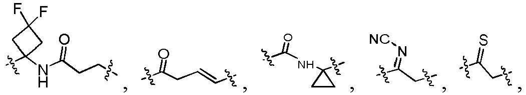 -C(O)-, 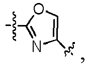 -C(O)-CH₂CH₂-, - C(O)-CH₂CH₂-N(CH₃)- 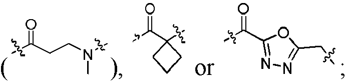
in some embodiments, A is C₃₋₆ cycloalkylene, 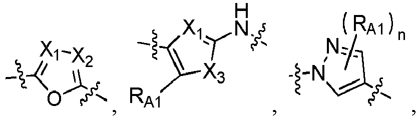 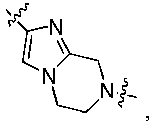 C₂₋₄ alkynylene, -RaC(O)NRa'-, -RaNRa'C(O)-, -RaNRa'-, -RaC(O)-, - Ra(CRa' Ra")ₙ- or a bond; in some embodiments, A is C₃₋₆ cycloalkylene, C₂₋₄ alkynylene, 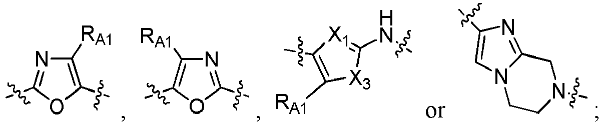 in some embodiments, A is 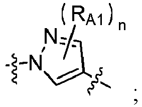 Ra is 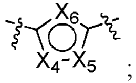 in some embodiments, Ra is 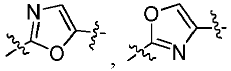 or 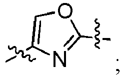 in some embodiments, A is 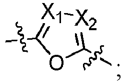 in some embodiments, A is 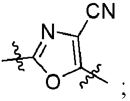 Ra' and Ra" are independently H or C₁₋₄ alkyl;
in some embodiments, n is an integer of 1-2;
in some embodiments, X₁ and X₂ are independently N or CR_{A1}; in some embodiments, X₁ and X₂ are not both CR_{A1} at the same time; in some embodiments, X₃ is S, O or NR_{A1};
in some embodiments, X₄, X₅ and X₆ are independently N, NR_{A1}, S, O or CR_{A1}, and are not all CR_{A1} at the same time;
in some embodiments, each R_{A1} is independently H, cyano, -R_{A}, halogen, -C₁₋₄ alkyl R_{A}, - NHC(O) R_{A}, -C(O) R_{A}, -C₁₋₄ alkyl-O-C₁₋₄ alkyl, -NHR_{A}, -C(O)NHR_{A} or -OR_{A};
in some embodiments, R_{A} is C₁₋₄ alkyl, C₃₋₆ cycloalkyl, C₃₋₆ cycloalkyloxy, C₁₋₄ alkoxy, C₁₋₄ haloalkyl, C₁₋₄ haloalkoxy or a 3-6 membered heterocyclyl containing 1-3 heteroatoms selected from N, O or S, the alkyl, cycloalkyl, cycloalkyloxy, alkoxy, haloalkyl, haloalkoxy and heterocyclyl are optionally further substituted with 1-6 groups selected from C₃₋₆ cycloalkyl, C₁₋₄ alkyl, halogen, -S(O)₂C₁₋₄ alkyl, -OC₁₋₄ alkyl or cyano; in some embodiments, R_{A} is C₁₋₄ alkyl, C₃₋₆ cycloalkyl or a 3-6 membered heterocyclyl containing 1-3 heteroatoms selected from N, O or S, the alkyl, cycloalkyl, and heterocyclyl are optionally further substituted with 1-6 groups selected from C₃₋₆ cycloalkyl, C₁₋₄ alkyl, halogen, -S(O)₂C₁₋₄ alkyl, -OC₁₋₄ alkyl or cyano; in some embodiments, R_{A} is C₁₋₄ alkyl, C₃₋₄ cycloalkyl, a 4-6-membered heterocyclyl containing 1-2 heteroatoms selected from N and O, the alkyl and heterocyclyl are optionally further substituted with 1-5 groups selected from C₃₋₄ cycloalkyl, C₁₋₄ alkyl, halogen, -S(O)₂C₁₋₂ alkyl, -OC₁₋₂ alkyl or cyano; in some embodiments, R_{A} is C₃₋₆ cycloalkyl, C₃₋₆ cycloalkyloxy, C₁₋₄ alkoxy, C₁₋₄ haloalkyl, and C₁₋₄ haloalkoxy, the cycloalkyl, cycloalkyloxy, alkoxy, haloalkyl, and haloalkoxy are optionally further substituted with 1-3 groups selected from C₁₋₄ alkyl, halogen and cyano;
in some embodiments, A is 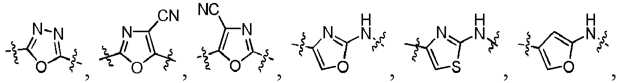 cyclopropylidene, ethynylene, 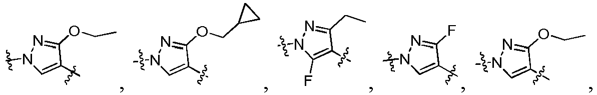 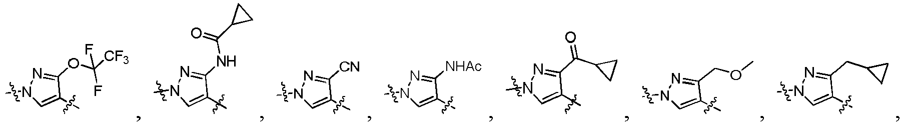 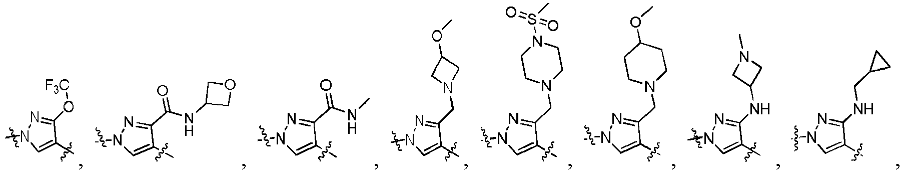 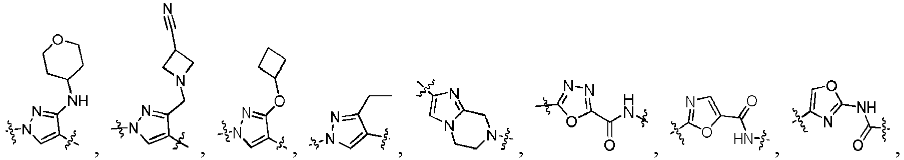 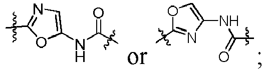 in a preferred embodiment, A is 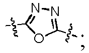
in some embodiments, B is 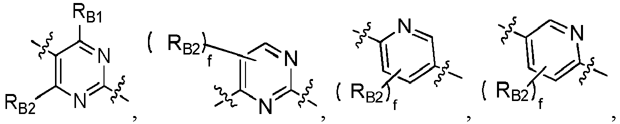 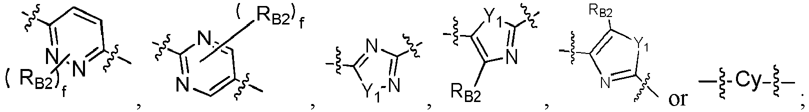 in some embodiments, B is 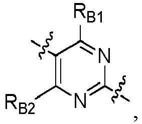 wherein at least one of R_{B1} and R_{B2} is selected from OH, halogen, cyano, halo C₁₋₄ alkyl, C₁₋₄ alkyloxy and C₃₋₆ cycloalkyl, or group R_{A1} on A and group R_{B1} on B together with the atoms to which they are attached form a 6-8-membered heterocycle containing 1 O atom; in some embodiments, B is 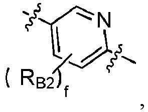 wherein R_{B2} is halogen or cyano, and f is not 0; in some embodiments, B is 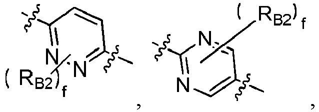 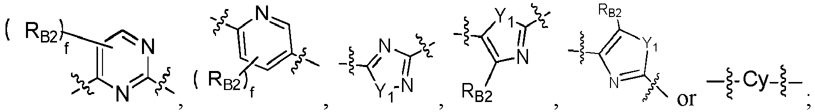 in some embodiments, B is 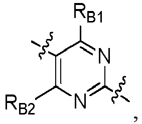 wherein both R_{B1} and R_{B2} are H;
in some embodiments, Y₁ is O, S or NR_{B3};

In some embodiments, Cy has a structure: 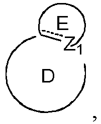 wherein, 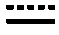 represents a single bond or a double bond, Z₁ is C or N, ring E is a 5-membered heterocycle containing 1-3 heteroatoms selected from N, O or S, and ring D is a 6-membered ring containing 0-3 heteroatoms selected from N, O or S; the 5-membered heterocycle and the 6-membered ring are independently and optionally substituted with 1-3 R_{B2}; in some embodiments, Cy is selected from one of the following structures: 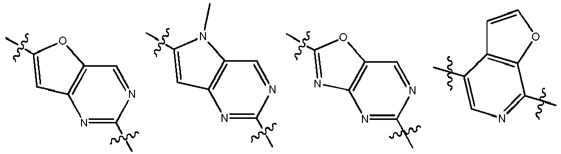 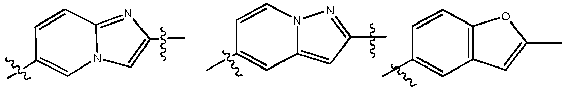 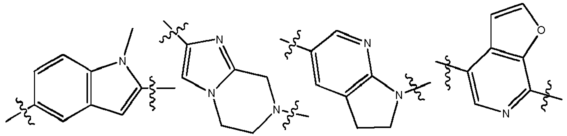 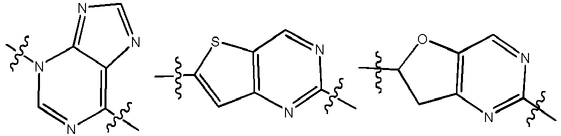 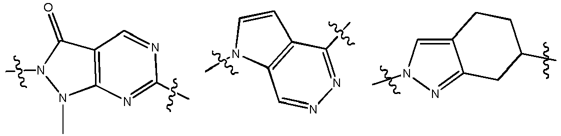 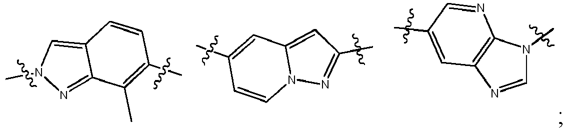
in some embodiments, R_{B1} and R_{B2} are each independently selected from H, oxo, OH, halogen, cyano, C₁₋₄ alkyl, halo C₁₋₄ alkyl, C₁₋₄ alkyloxy, C₃₋₆ cycloalkyl, and R_{B3} is selected from H, C₁₋₄ alkyl, C₃₋₆ cycloalkyl; in some embodiments, group R_{A1} on A and group R_{B1} on B together with the atoms to which they are attached form a 6-10-membered heterocycle containing 1-3 heteroatoms selected from N, S, and O; in some embodiments, f is an integer of 0-3;
in some embodiments, B is 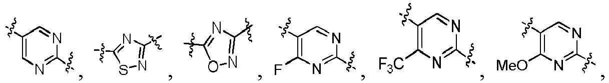 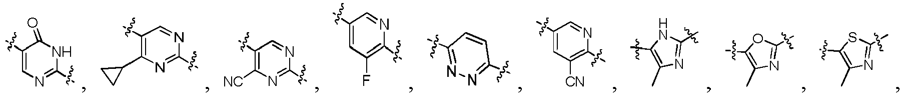 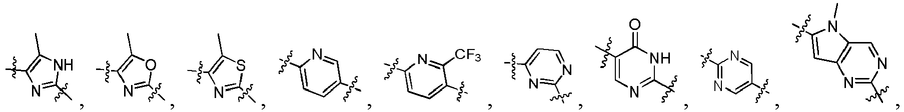 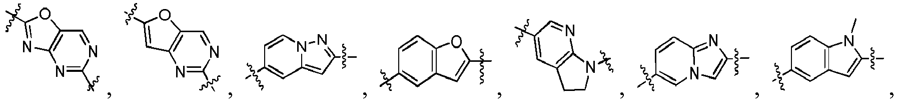 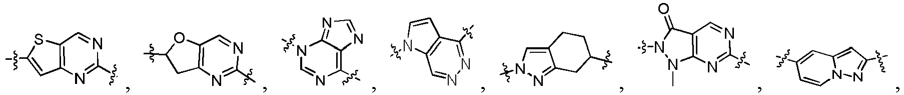 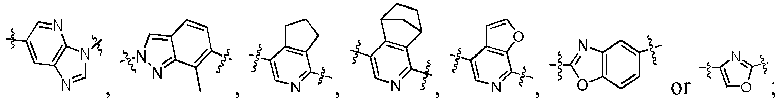
in some embodiments, L₂ is -O-, -C(O)NR₉-, -NR₉C(O)NR₉-, -NR₉-, -(CR₁₀R₁₁)_{g}-, -NR₉-(CR₁₀R₁₁)_{g}- or a bond, wherein g is an integer of 1-3;
in some embodiments, L₂ is -O- or -CR₁₀R₁₁-, wherein at least one of R₁₀ and R₁₁ is halogen or C₁₋₄ alkyl, or R₁₀ and R₁₁ together with the carbon atom to which they are attached form a 3-4-membered carbocyclic ring, such as cyclopropyl;
in some embodiments, L₂ is -NR₉-, wherein R₉ is H or C₁₋₄ alkyl;
in some embodiments, R₁₀ and R₁₁ are each independently H, halogen, C₁₋₄ alkyl or C₃₋₆ cycloalkyl; alternatively, R₁₀ and R₁₁ on the same carbon atom together with the carbon atom to which they are attached form a 3-6 membered carbocyclic ring;
in some embodiments, L2 is -NH-, -O-, 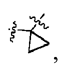 -CF2-, -C(CH3)2-, -NHC(O)NH- or -C(O)NH-;
in some embodiments, M₂ is 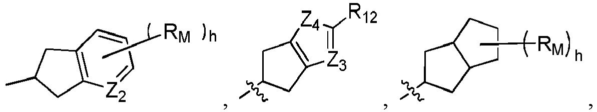 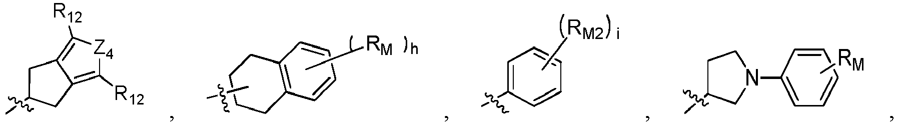 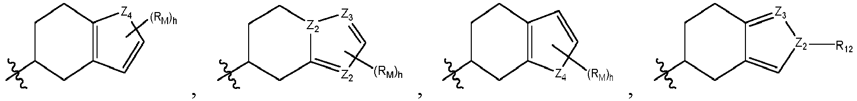 or 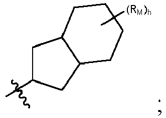 in some embodiments, M₂ is 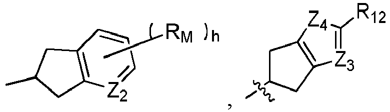 or 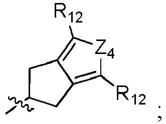 in some embodiments, M₂ is 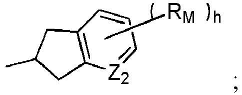 and in some embodiments, 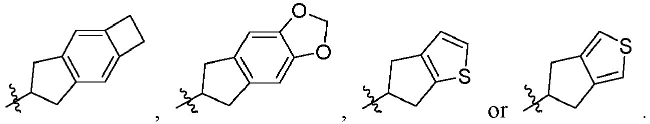 In some embodiments, Z₂ and Z₃ are CR_{M} or N, and Z₄ is O, S or NR_{M1}; in some embodiments, Z₂ is CR_{M};
in some embodiments, each R_{M} is independently H, C₁₋₄ alkyl, C₁₋₄ alkoxyalkyl, cyano, C₃₋₆ cycloalkyl, C₃₋₆ cycloalkyloxy, -SRm', -S(O)₂Rm', -C(O)NRmRm', -NRmC(O)Rm', C₂₋₆ alkenyl, C₂₋₆ alkynyl, -NR_{M1}, a 3-6 membered heterocyclyl containing 1-3 heteroatoms selected from N, O or S, or a halogen, the alkyl, alkenyl, alkynyl, and cycloalkyl are optionally substituted with 1-3 groups selected from halogen, cyano, C₁₋₄ alkoxy, halo C₁₋₄ alkyl or halo C₁₋₄ alkoxy, the heterocyclyl is optionally substituted with 1-3 groups selected from halogen, oxo, and C₁₋₄ alkyl; in some embodiments, each R_{M} is independently H, C₁₋₄ alkyl, C₂₋₆ alkynyl or a 3-6 membered heterocyclyl containing 1-3 heteroatoms selected from N, O or S, wherein the alkyl is optionally substituted with 1-3 groups selected from halogen; in some embodiments, each R_{M} is independently cyano, difluoroalkyl, trifluoroalkyl, C₂₋₄ alkynyl, C₃₋₄ cycloalkyl, a 5-6-membered heterocyclyl containing 1-3 heteroatoms selected from N, O or S, the heterocyclyl is optionally substituted with 1-3 groups selected from C₁₋₄ alkyl;
alternatively, two R_{M} on adjacent ring carbon atoms in M₂ together with the carbon atom to which they are attached form a 3-6 membered carbocyclic ring or a 3-6 membered heterocyclyl containing 0-3 heteroatoms selected from N, O or S; further, two R_{M} on adjacent ring carbon atoms in M₂ together with the carbon atom to which they are attached form a 3-5 membered carbocyclic ring or a 3-5 membered heterocyclyl containing 0-3 heteroatoms selected from N, O or S; furthermore, two R_{M} on adjacent ring carbon atoms in M₂ together with the carbon atom to which they are attached form a 4-5 membered carbocyclic ring or a 4-5 membered heterocyclyl containing 0-3 heteroatoms selected from N, O or S; furthermore, two R_{M} on adjacent ring carbon atoms in M₂ together with the carbon atom to which they are attached form cyclobutane or a 5-membered heterocyclyl containing 1-2 heteroatoms selected from N, O or S;
Rm is H, C₁₋₄ alkyl or halo C₁₋₄ alkyl;
Rm' is C₁₋₄ alkyl or halo C₁₋₄ alkyl; in some embodiments, M₂ is 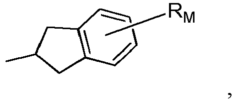 wherein R_{M} is trifluoromethyl, difluoromethyl, cyclopropyl, ethynyl, furan-3-yl, methylaminocarbonyl, azetidin-1-yl, oxetan-3-yl, 3,3-difluoro-azetidin-1-yl, acetamido, 1-methyl-1H-pyrazol-4-yl, tetrahydrofuran-3-yl, methylthio, 4-methyl-5-oxo-1,2,4-triazol-1-yl, 2-oxo-pyrrolidin-1-yl, methylsulfonyl, methoxymethyl, difluoromethoxymethyl or trifluoromethyl ethynyl;
in some embodiments, M₂ is 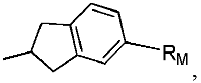 wherein R_{M} is C₁₋₄ alkyl, and the alkyl is substituted with 1-3 halogens (preferably F); preferably trifluoromethyl or difluoromethyl;
in some embodiments, M₂ is 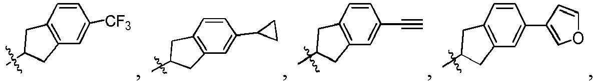 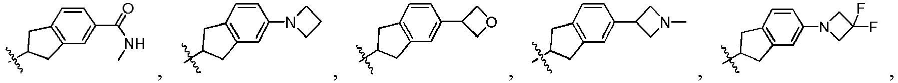 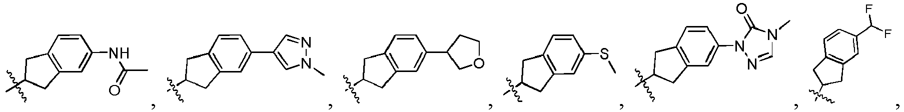 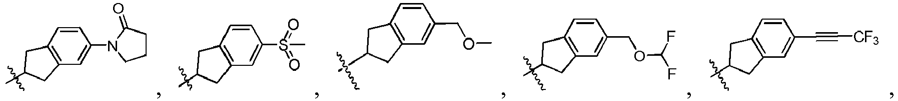 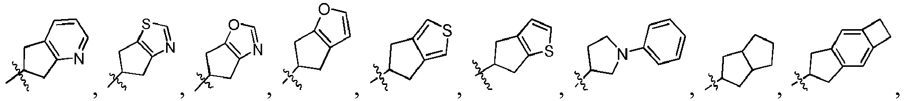 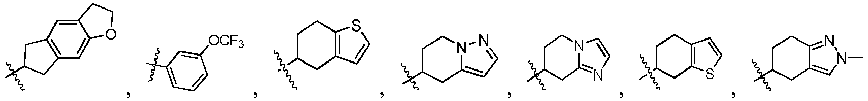 or 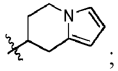
in some embodiments, R_{M1} is H, C₁₋₄ alkyl, C₃₋₆ cycloalkyl, C₁₋₄ haloalkyl or C₁₋₄ alkoxyalkyl; in some embodiments, R_{M1} is H, C₁₋₄ alkyl or C₃₋₆ cycloalkyl;
In some embodiments, each R₁₂ is independently H, halogen, C₁₋₄ alkyl, C₃₋₆ cycloalkyl or halo C₁₋₄ alkyl; In some embodiments, each R₁₂ is independently H;
In some embodiments, h is an integer of 0-3; In some embodiments, h is 1 or 2;
i is 0, 1 or 2;
provided that: when M₁ is 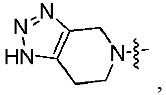 L₁ is -C(O)-CH₂-, A is 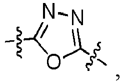 B is 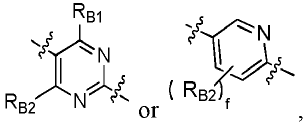 L₂ is -NH-, and M₂ is 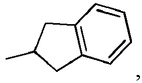 R_{B1} and R_{B2} are not both H at the same time;
when M₁ is 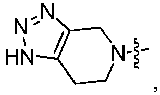 L₁ is -C(O)-CH₂-, A is 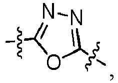 B is 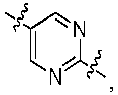 and L₂ is - NH-CH₂-, M₂ is not 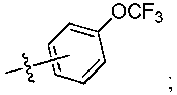
when M₁ is 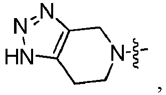 L₁ is -C(O)-CH₂-, A is 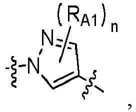 B is 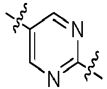 or 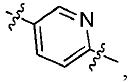 L₂ is -NH-, and M₂ is 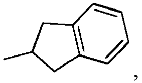 at least one of substituent R_{A1} in A is selected from cyano, halogen, C₃₋₄ cycloalkyloxy, cyclopropylmethoxy, C₁₋₄ haloalkoxy, cyclopropylmethyl, -C₁₋₄ alkyl-O-C₁₋₄ alkyl, -NHC(O) R_{A}, -C(O)NHR_{A}, -C(O)-C₃₋₆ cycloalkyl, 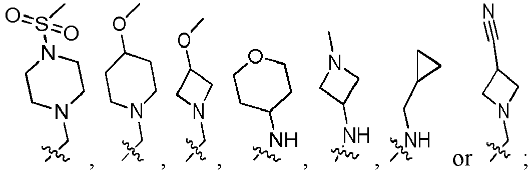
and when M₁ is 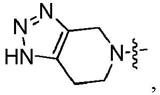 L₁ is -C(O)-CH₂-, A is 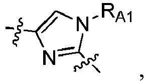 B is 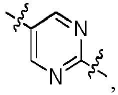 L₂ is -NH-, and M₂ is 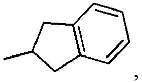 R_{A1} is not methyl, ethyl or cyclopropyl.

### Synthetic route

Patent document CN 109476664 A introduces a method for preparing a class of ATX inhibitors. One skilled in the art may combine the document and known organic synthesis techniques to prepare the compound of the present invention. The starting materials for the compound are commercially available chemicals and (or) the compounds described in the chemical literature. "Commercially available chemicals" are obtained from formal commercial sources, and the suppliers include: Titan Technology Co., Ltd., Energy Chemical Co., Ltd., Shanghai Demo Co., Ltd., Chengdu Kelong Chemical Co., Ltd., Accela ChemBio Co., Ltd., Nanjing PharmaBlock Co., Ltd., WuXi Apptec Co., Ltd., and J & K Scientific Co., Ltd. and other companies.

Reference books and monographs in the field introduce in detail the synthesis of reactants useful for the preparation of the compound described herein, or provide articles describing the preparation method for reference. These reference books and monographs include: "Synthetic Organic Chemistry", John Wiley & Sons, Inc., New York; S. R. Sandler et al., "Organic Functional Group Preparations," 2nd Ed., Academic Press, New York, 1983; H. O. House, "Modern Synthetic Reactions", 2nd Ed., W. A. Benjamin, Inc. Menlo Park, Calif. 1972; T. L. Gilchrist, "Heterocyclic Chemistry", 2nd Ed., John Wiley & Sons, New York, 1992; J. March, "Advanced Organic Chemistry: Reactions, Mechanisms and Structure", 4th Ed., Wiley-Interscience, New York, 1992; Fuhrhop, J. and Penzlin G. "Organic Synthesis: Concepts, Methods, Starting Materials", Second, Revised and Enlarged Edition (1994) John Wiley & Sons ISBN: 3-527-29074-5; Hoffman, R.V. "Organic Chemistry, An Intermediate Text" (1996) Oxford University Press, ISBN 0-19-509618-5; Larock, R. C. "Comprehensive Organic Transformations: A Guide to Functional Group Preparations" 2nd Edition (1999) Wiley-VCH, ISBN: 0-471-19031-4; March, J. "Advanced Organic Chemistry: Reactions, Mechanisms, and Structure" 4th Edition (1992) John Wiley & Sons, ISBN: 0-471-60180-2; Otera, J. (editor) "Modern Carbonyl Chemistry" (2000) Wiley-VCH, ISBN: 3-527-29871-1; Patai, S. "Patai's 1992 Guide to the Chemistry of Functional Groups" (1992) Interscience ISBN: 0-471-93022-9; Solomons, T. W. G. "Organic Chemistry" 7th Edition (2000) John Wiley & Sons, ISBN: 0-471-19095-0; Stowell, J.C., "Intermediate Organic Chemistry" 2nd Edition (1993) Wiley-Interscience, ISBN: 0-471-57456-2; "Industrial Organic Chemicals: Starting Materials and Intermediates: An Ullmann's Encyclopedia" (1999) John Wiley & Sons, ISBN: 3-527-29645-X, in 8 volumes; "Organic Reactions" (1942-2000) John Wiley & Sons, in over 55 volumes; and "Chemistry of Functional Groups" John Wiley & Sons, in 73 volumes.

Specific and similar reactants can be selectively identified through indexes of known chemicals made by the Chemical Abstracts Service of the American Chemical Society, and these indexes are available in most public and university libraries and online. Chemicals that are known but not commercially available in the catalog are optionally prepared by custom chemical synthesis facilities, wherein many standard chemical supply facilities (e.g., those listed above) provide custom synthesis services. A reference document for the preparation and selection of pharmaceutically acceptable salts of the compound described herein is P. H. Stahl & C. G. Wermuth "Handbook of Pharmaceutical Salts", Verlag Helvetica Chimica Acta, Zurich, 2002.

### Terms

"Halogen" as used herein refers to F, Cl, Br, I, or their isotopes.

"Halo" or "substituted with halogen" refers to substitution with more than one halogens selected from F, Cl, Br, I, or their isotopes, and the upper limit of the number of halogen substituents is equal to the sum of the number of hydrogens that can be substituted with substituent groups. Without particular limitation, the number of halogen substituents is any integer between 1 and the upper limit. When the number of halogen substituents is greater than 1, identical or different halogens may be used for substitution.

The term "alkyl" refers to a monovalent straight or branched chain saturated aliphatic hydrocarbon group. Unless otherwise specified, the term "alkyl" refers to alkyl containing 1 to 20 carbon atoms, preferably alkyl containing 1 to 8 carbon atoms, more preferably alkyl containing 1 to 6 carbon atoms, and further preferably alkyl containing 1 to 4 carbon atoms. Non-limiting examples include methyl, ethyl, n-propyl, isopropyl, n-butyl, sec-butyl, neobutyl, tert-butyl, n-pentyl, isoamyl, neopentyl, n-hexyl and various branched isomers thereof.

The term "alkylene" refers to a bivalent straight or branched chain saturated alkyl. Examples of alkylene include, but are not limited to, methylene, ethylene, *etc.*

The definition of "ring" includes a carbocyclic ring and heterocycle. Unless otherwise specified, it is usually a 3-12-membered monocyclic ring containing 0 to 3 heteroatoms selected from N, O or S, preferably a 4-7-membered ring, more preferably a five-membered ring or a six-membered ring.

The term "cycloalkyl" refers to a monovalent saturated, substituted or unsubstituted carbocyclic hydrocarbon group, unless otherwise specified, usually has 3 to 10 carbon atoms, preferably 3-6 carbon atoms, and further preferably 3-4 carbon atoms. Non-limiting examples include cyclopropyl, cyclobutyl, cyclopentyl, cyclohexyl, or cycloheptyl, etc.

The term "cycloalkylene" refers to a divalent saturated, substituted or unsubstituted cycloalkyl. Non-limiting examples include

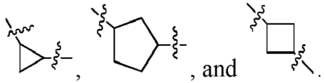

The term "carbocyclic ring" or "carbocyclyl" refers to a substituted or unsubstituted, saturated or unsaturated carbocyclic group, and includes a monocyclic carbocyclic ring, a bicyclic bridged ring, a bicyclic fused ring, a bicyclic spiro ring, *etc.,* usually having 3 to 12 carbon atoms, preferably 3-10 carbon atoms, and further preferably 3-6 carbon atoms. In non-limiting examples, a monocyclic carbocyclic ring includes cyclopropyl, cyclobutyl, cyclopentyl, cyclohexyl, cycloheptyl or phenyl, *etc.,* a bicyclic bridged ring includes 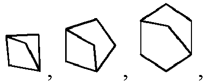 *etc.,* a bicyclic fused ring includes 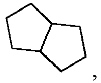 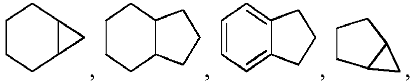 *etc.,* and a bicyclic spiro ring includes 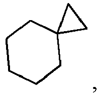 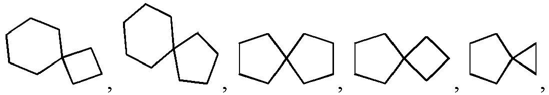 *etc.*

The term "heterocycle" or "heterocyclyl" refers to a substituted or unsubstituted, saturated or unsaturated aromatic ring or non-aromatic ring, and when not particularly limited, contains 1 to 3 heteroatoms selected from N, O or S, including a monocyclic heterocycle, a bicyclic bridged heterocycle, a bicyclic fused heterocycle, a bicyclic spiro heterocycle, *etc.,* preferably a 3- to 12-membered heterocycle, more preferably a 4-12-membered heterocycle, and more preferably a 4-10-membered heterocycle. The selectively substituted N and S in the heterocyclyl can be oxidized to various oxidation states. Heterocyclyl can be connected to a heteroatom or carbon atom. Non-limiting examples include oxiranyl, azacyclopropyl, oxetanyl, azetidinyl, 1,3-dioxolane, 1,4-dioxolane, 1,3-dioxane, piperazinyl, azacycloheptyl, pyridyl, furanyl, thienyl, pyranyl, N-alkylpyrrolyl, pyrimidinyl, pyrazinyl, pyrazolyl, pyridazinyl, imidazolyl, piperidyl, piperidinyl, morpholinyl, thiomorpholinyl, 1,3-dithianyl, dihydrofuranyl, dihydropyranyl, dithiolanyl, tetrahydrofuranyl, tetrahydropyrrolyl, tetrahydroimidazolyl, oxazolyl, dihydrooxazolyl, tetrahydrooxazolyl, tetrahydrothiazolyl, tetrahydropyranyl, benzimidazolyl, benzopyridyl, pyrrolopyridyl, benzodihydrofuranyl, azabicyclo[3.2.1]octyl, azabicyclo[5.2.0]nonyl, oxatricyclic[5.3.1.1]dodecyl, azaadamantyl and oxaspiro[3.3]heptyl, 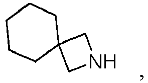 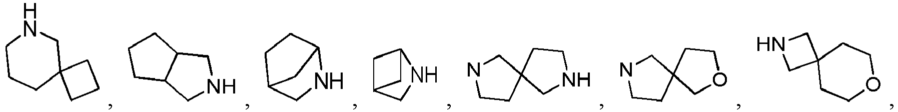 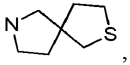 *etc.*

The term "heterocyclene" refers to a substituted or unsubstituted, saturated or unsaturated, aromatic or non-aromatic, divalent heterocyclic group. Non-limiting examples include 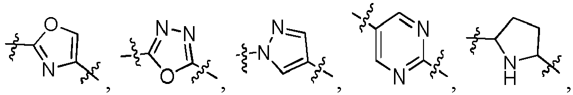 *etc.*

The term "alkynyl" refers to a straight or branched chain monovalent unsaturated hydrocarbon group containing a carbon-carbon triple bond. Unless otherwise specified, alkynyl contains 2-6 carbon atoms, preferably 2-4 carbon atoms, and non-limiting example includes ethynyl. The term "alkynylene" refers to a straight or branched chain divalent unsaturated hydrocarbon group containing a carbon-carbon triple bond.

The term "alkoxy" or "alkyloxy" refers to -O-alkyl. Non-limiting examples include methoxy, ethoxy, n-propoxy, isopropoxy, n-butoxy, sec-butoxy, tert-butoxy, n-pentoxy, n-hexyloxy, cyclopropoxy, cyclobutoxy, etc.

The expression "optional" or "optionally" refers to that events or circumstances subsequently described may but not necessarily occur, and the description includes the occasions where the events or circumstances occur or do not occur. For example, the expression "alkyl optionally substituted with F" means that the alkyl may but not necessarily be substituted with F, and the description includes the case where the alkyl is substituted with F and the case where the alkyl is not substituted with F.

"Pharmaceutically acceptable salt" refers to a salt of the compound of the present invention maintaining the biological effectiveness and characteristics of the free acid or free base, and obtained by reacting the free acid with a non- toxic inorganic base or organic base, reacting the free base with a non- toxic inorganic acid or organic acid.

The term "deuterated form" refers to a compound in which one or more hydrogen atoms are substituted with a corresponding number of deuterium atoms.

The term "pharmaceutical composition" refers to a mixture of one or more compounds described herein, or stereoisomers, solvates, deuterated forms, pharmaceutically acceptable salts or cocrystals thereof, and other components, wherein the other components comprise physiologically/pharmaceutically acceptable carriers and/or excipients.

The term "carrier" refers to: a system that does not cause significant irritation to the organism, does not eliminate the biological activity and characteristics of the administered compound, can change the way the drug enters the human body and the distribution of the drug in the body, controls the release rate of the drug, and delivers the drug to targeted organs, and non-limiting examples include microcapsules and microspheres, nanoparticles, liposomes, *etc.*

The term "excipient" refers to: an excipient which itself is not a therapeutic agent, but used as a diluent, adjuvant, binder, and/or vehicle for addition to a pharmaceutical composition to improve its handling or storage properties or to permit or facilitate formation of a compound or pharmaceutical composition into a unit dosage form for administration. As will be known to those in the art, pharmaceutically acceptable excipients can provide a variety of functions and can be described as wetting agents, buffering agents, suspending agents, lubricating agents, emulsifiers, disintegrants, absorbents, preservatives, surfactants, colorants, flavorants, and sweeteners. Examples of pharmaceutically acceptable excipients include, but are not limited to: (1) sugars, such as lactose, glucose and sucrose; (2) starches, such as corn starch and potato starch; (3) cellulose and its derivatives, such as sodium carboxymethyl cellulose, ethyl cellulose, cellulose acetate, hydroxypropylmethylcellulose, hydroxypropylcellulose, microcrystalline cellulose, and croscarmellose, such as croscarmellose sodium; (4) tragacanth powder; (5) malt; (6) gelatin; (7) talc; (8) excipients, such as cocoa butter and suppository waxes; (9) oils, such as peanut oil, cottonseed oil, safflower oil, sesame oil, olive oil, corn oil and soybean oil; (10) glycols, such as propylene glycol; (11) polyols, such as glycerin, sorbitol, mannitol and polyethylene glycol; (12) esters, such as ethyl oleate and ethyl laurate; (13) agar; (14) buffering agents, such as magnesium hydroxide and aluminum hydroxide; (15) alginic acid; (16) pyrogen-free water; (17) isotonic saline; (18) Ringer's solution; (19) ethanol; (20) pH buffer solutions; (21) polyesters, polycarbonates and/or polyanhydrides; and (22) other non-toxic compatible substances employed in pharmaceutical formulations.

The term "stereoisomer" refers to an isomer produced by different arrangements of atoms in a molecule in space, including cis-trans isomers, enantiomers and conformational isomers.

The compounds of the present invention also include tautomers thereof, for example, when the present invention describes the left side compound in which the pyrimidine ring is substituted with OH, the right side tautomer compound is also included.

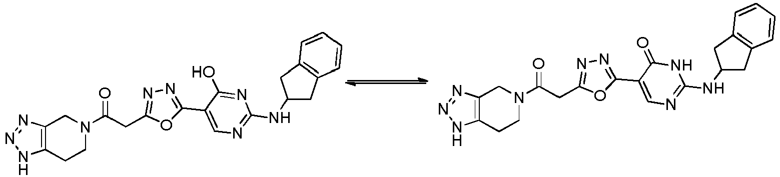

The term "solvate" refers to substance formed by combining compounds of the present invention or salts thereof with stoichiometric or non-stoichiometric solvents bound by non-covalent intermolecular forces. When the solvent is water, the solvate is a hydrate.

"Co-crystal" refers to a crystal formed by the combination of active pharmaceutical ingredient (API) and co-crystal former (CCF) under the action of hydrogen bonds or other non-covalent bonds. The pure states of API and CCF are both solid at room temperature, and there is a fixed stoichiometric ratio between various components. The co-crystal is a multi-component crystal, which includes both a binary co-crystal formed between two neutral solids and a multi-element co-crystal formed between a neutral solid and a salt or solvate.

### Detailed Description of Embodiments

The contents of the present invention will be described below in detail by examples. In the examples, experimental methods without specifying specific conditions are performed under conventional conditions. The listed examples are intended to better illustrate the contents of the present invention, but this should not be understood as that the contents of the present invention are only limited to the listed examples. Non-essential improvements and adjustments made by one of ordinary skill in the art to the embodiments based on the above-mentioned contents of the present invention should still fall within the protection scope of the present invention.

The following abbreviations are used in the present invention:

| | |
|---|---|
| CDI | N,N'-carbonyldiimidazole |
| DAST | Diethylaminosulphur trifluoride |
| DIPEA | N,N-diisopropylethylamine |
| DCM | Dichloromethane |
| DMA | N,N-dimethylacetylamide |
| DPPA | Diphenylphosphoryl azide |
| EA or EtOH | Ethyl acetate or ethanol |
| HATU | O-(7-azabenzotriazol-1-yl)-N,N,N',N'-tetramethyluronium hexafluorophosphate |
| NMP | N-methylpyrrolidone |
| PE | Petroleum ether |
| TFA | Trifluoroacetic acid |

### Detection method

The structures of the compounds are determined by nuclear magnetic resonance (NMR) and/or mass spectrometry (MS). The NMR shift (δ) is given in the unit of 10⁻⁶ (ppm). NMR is measured with (Bruker Avance III 400 and Bruker Avance 300) NMR instrument, wherein the solvent for determination is deuterated form of dimethyl sulfoxide (DMSO-d6), deuterated form of chloroform (CDCl₃), deuterated form of methanol (CD₃OD), and the internal standard is tetramethylsilane (TMS); MS is measured with (Agilent 6120B(ESI) and Agilent 6120B(APCI)); HPLC is measured with Agilent 1260DAD high pressure liquid chromatography (Zorbax SB-C18 100 × 4.6 mm, 3.5 µM); Yantai Huanghai HSGF254 or Qingdao GF254 silica gel plate is used for thin layer chromatography silica plate, and the silica gel plate for the thin layer chromatography (TLC) is of the specification of 0.15 mm-0.20 mm, and the specification when separating and purifying a product by thin layer chromatography is 0.4 mm - 0.5 mm. For the column chromatography, Yantai Huanghai silica gel of 200-300 mesh silica gel is generally used as a carrier.

### Preparation of compounds

### Intermediate 1: N-(2,3-dihydro-1H-inden-2-yl)pyrimidine-2,5-diamine (intermediate 1)

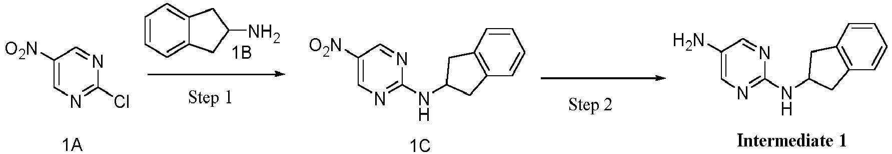

### Step 1: N-(2,3-dihydro-1H-inden-2-yl)-5-nitropyrimidin-2-amine (1C)

2-chloro-5-nitro pyrimidin (**1A**) (4.8 g, 30 mmol), 2-aminoindan (**1B**) (4.01 g, 30 mmol) and N,N-diisopropylethylamine (5.2 g, 40 mmol) were dissolved in ethanol (50 mL), and stirred at 90°C for 2 hours. After the reaction was completed, the mixture was cooled to room temperature, and the resulting solid was filtered, washed with ethanol (20 mL), and dried to obtain the title compound **1C** as a beige solid (5.6 g, 73%). LC-MS (ESI): m/z = 257.1 [M+H]⁺

### Step 2: N-(2,3-dihydro-1H-inden-2-yl)pyrimidine-2,5-diamine (intermediate 1)

**1C** (5.6 g, 22 mmol), iron powder (5.6 g, 100 mmol) and ammonium chloride (0.54 g, 10 mmol) were dissolved in ethanol (50 mL) and water (15 mL), and stirred at 80°C for 2 hours. After the reaction was completed, the mixture was cooled to room temperature, and filtered. The filtrate was concentrated under reduced pressure, and then separated by silica gel column chromatography to obtain the title compound **intermediate 1** as a yellow solid (3.6 g, 72%). LC-MS (ESI): m/z = 227.2 [M+H]⁺.

### Intermediate 2: ethyl 2-(5-(2-chloropyrimidin-5-yl)-1,3,4-oxadiazol-2-yl)acetate (intermediate 2)

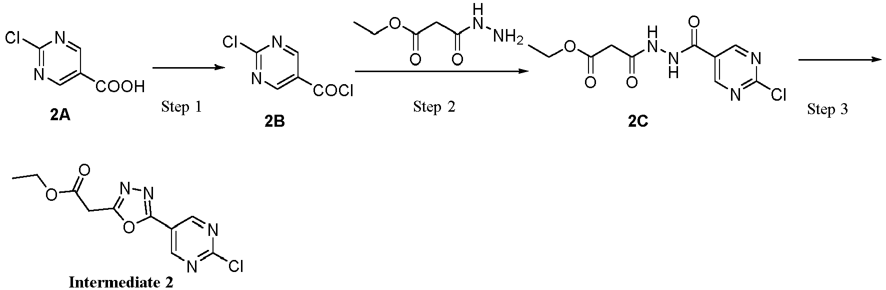

### Step 1: 2-chloropyrimidine-5-carbonyl chloride (2B)

Compound **2A** (2.5 g, 15.8 mmol) was dissolved in DCM (40 ml), and DMF (0.1 ml) was added. The resulting solution was cooled to 0°C with an ice bath, and oxalyl chloride (3.0 g, 23.7 mmol) was added dropwise. After the addition was completed, the reaction mixture was slowly returned to room temperature and stirred overnight. The reaction mixture was evaporated to dryness under reduced pressure to obtain compound **2B** (2.8 g, 99%).

### Step 2: ethyl 3-(2-(2-chloropyrimidine-5-carbonyl)hydrazinyl)-3-oxopropanoate (2C)

Ethyl 3-oxo-3-hydrazinyl propanoate (3.5 g, 23.7 mmol) and triethylamine (4.8 g, 47.4 mmol) were dissolved in DCM (60 ml), and cooled to 0°C with an ice bath. A solution of compound **2B** (2.8 g, 15.8 mmol) in DCM (15 ml) was slowly added dropwise. After the addition was completed, the mixture was slowly returned to room temperature and stirred overnight. The reaction mixture was evaporated to dryness under reduced pressure, and the residue was directly purified by silica gel column chromatography (PE/EA = 2/1) to obtain **2C** (2.7 g, 60.0%).

### Step 3: ethyl 2-(5-(2-chloropyrimidin-5-yl)-1,3,4-oxadiazol-2-yl)acetate (intermediate 2)

**2C** (2.7 g, 9.4 mmol) was placed into a 100 ml single-necked flask, anhydrous THF (50 ml) was added, and Burgess reagent (3.4 g, 14.1 mmol) was added at room temperature. After the addition was completed, the mixture was warmed to 70°C, and stirred for 2 hours. The reaction mixture was directly purified by silica gel column chromatography (PE/EA = 2/1) to obtain **intermediate 2** (1.6 g, 59.3%).

### Example 1: 2-(3-cyclopropoxy-4-(2-((2,3-dihydro-1H-inden-2-yl)amino)pyrimidin-5-yl)-1H-pyrazol-1-yl)-1-(1,4,6,7-tetrahydro-5H-[1,2,3]triazolo[4,5-c]pyridin-5-yl)ethan-1-one (compound 1)

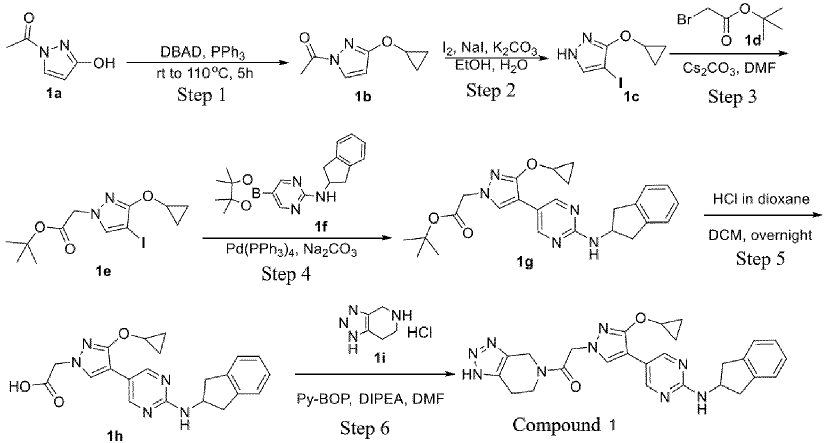

### Step 1: 1-(3-cyclopropoxy-1H-pyrazol-1-yl) ethenone (1b)

Compound **1a** (5.04 g, 40 mmol) was dissolved in toluene (50 mL). At 0°C, cyclopropanol (2.9 g, 50 mmol) and triphenylphosphine (15.8 g, 60 mmol) were added successively, and stirred uniformly, then DBAD (13.8 g, 60 mmol) was added to the system in portions, and the mixture was reacted under nitrogen protection at 110°C for 5 hours. After the reaction was completed, the system was concentrated under reduced pressure, and then directly separated by silica gel column chromatography to obtain target compound (**1b**) (1.2 g, 18%). LC-MS (ESI): m/z = 167.2 [M+H]⁺. ¹H NMR (400 MHz, CDCl₃) *δ* 8.08 (d, 1H), 6.04 (d, 1H), 4.12-4.05 (m, 1H), 2.61 (s, 3H), 0.87 - 0.68 (m, 4H).

### Step 2: 3-cyclopropoxy-4-iodo-1H-pyrazole (1c)

Sodium iodide (1.08 g, 7.2 mmol), iodine (2.79 g, 11 mmol) and potassium carbonate (3.86 g, 28 mmol) were added successively to a solution of compound **1b** (1.2 g, 7.2 mmol) in a mixed solvent of water (10 mL) and ethanol (6 mL), and the mixture was stirred at room temperature for 1.5 hours. After the reaction was completed, the mixture was diluted with saturated sodium bicarbonate solution (30 mL), and extracted with ethyl acetate (50 mL). The organic phase was dried over anhydrous sodium sulfate and filtered. The filtrate was concentrated under reduced pressure, and then directly used in the next step of reaction. LC-MS (ESI): m/z = 251.1 [M+H]⁺

### Step 3: tert-butyl 2-(3-cyclopropoxy-4-iodo-1H-pyrazol-1-yl) acetate (1e)

Cesium carbonate (2.28 g, 7 mmol) and tert-butyl bromoacetate (0.99 g, 5.1 mmol) were added successively to a solution of compound **1c** (0.85 g, 3.4 mmol) in N,N-dimethylformamide (10 mL), and the mixture was stirred at room temperature for 1 hour. After the reaction was completed, the mixture was diluted with saturated brine (30 mL), and extracted with ethyl acetate (30 mL × 2). The organic phase was dried over anhydrous sodium sulfate, filtered and concentrated. The residue was purified by silica gel column chromatography to obtain white solid **1e** (1.1 g, 89%). LC-MS (ESI): m/z = 365.1 [M+H]⁺. ¹H NMR (400 MHz, DMSO-d₆) δ 7.69 (s, 1H), 4.77 (s, 2H), 4.06 - 4.00 (m, 1H), 1.42 (s, 9H), 0.68 - 0.64 (m, 4H).

### Step 4: tert-butyl 2-(3-cyclopropoxy-4-(2-((2,3-dihydro-1H-inden-2-yl)amino)pyrimidin-5-yl)-1H-pyrazol-1-yl)acetate (1 g)

Tetrakis(triphenylphosphine)palladium(0) (0.35 g, 0.6 mmol) and sodium carbonate (0.64 g, 6 mmol) were added to a mixture of compound **1e** (1.1 g, 3 mmol) and compound **1f** (1.0 g, 3 mmol) in a mixed solvent of 1,4-dioxane (9 mL) and distilled water (3 mL), and the mixture was stirred at 80°C under nitrogen protection for 90 minutes. After the reaction was completed, the mixture was cooled to room temperature, diluted with saturated brine (50 mL), and extracted with ethyl acetate (80 mL). The organic layer was dried over anhydrous sodium sulfate, and concentrated. The residue was separated and purified by silica gel column chromatography to obtain the title compound **1g** as a yellow solid (0.84 g, 63%). LC-MS (ESI): m/z = 448.3 [M+H]⁺

### Step 5: 2-(3-cyclopropoxy-4-(2-((2,3-dihydro-1H-inden-2-yl)amino)pyrimidin-5-yl)-1H-pyrazol-1-yl)acetic acid (1 h)

A solution (3 mL) of 4N hydrogen chloride in dioxane was added to a solution of compound **1g** (0.45 g, 1 mmol) in dichloromethane (3 mL), and the mixture was stirred at room temperature overnight. After the reaction was completed, the solvent was removed by filtration to obtain a solid, which was slurried with diethyl ether, filtered and dried to obtain the title compound **1h** as a light brown solid (0.22 g, 56%). LC-MS (ESI): m/z = 392.3 [M+H]⁺

### Step 6: 2-(3-cyclopropoxy-4-(2-((2,3-dihydro-1H-inden-2-yl)amino)pyrimidin-5-yl)-1H-pyrazol-1-yl)-1-(1,4,6,7-tetrahydro-5H-[1,2,3]triazolo[4,5-c]pyridin-5-yl)ethan-1-one (compound 1)

At 0°C, compound **1h** (0.22 g, 0.58 mmol), N,N-diisopropylethylamine (1 mL) and benzotriazol-1-yl-oxy-tripyrrolidinophosphonium hexafluorophosphate (0.45 g, 0.87 mmol) were sequentially added slowly to a solution of compound **1i** (0.11 g, 0.68 mmol) in N,N-dimethylformamide (10 mL), and the mixture was stirred at room temperature under nitrogen protection overnight. After the reaction was completed, the reaction mixture was diluted with distilled water (30 mL), and extracted with ethyl acetate (50 mL × 2). The organic layer was washed with distilled water and saturated brine (50 mL), dried over anhydrous sodium sulfate and concentrated. The silica gel column chromatography was used for separation and purification to obtain the title **compound 1** (12 mg). LC-MS (ESI): m/z = 498.3 [M+H]⁺. ¹H NMR (400 MHz, DMSO-d₆) δ 8.45 (s, 2H), 7.87 (d, 1H), 7.37 (d, 1H), 7.24 - 7.19 (m, 2H), 7.16 - 7.11 (m, 2H), 5.18 - 5.09 (m, 2H), 4.82 - 4.54 (m, 4H), 4.12 - 4.06 (m, 2H), 3.86 - 3.78 (m, 3H), 2.93 - 2.85 (m, 4H), 0.73 - 0.65 (m, 4H).

### Example 2: 4-(2-((2,3-dihydro-1H-inden-2-yl)amino)pyrimidin-5-yl)-1-(2-oxo-2-(1,4,6,7-tetrahydro-5H-[1,2,3]triazolo[4,5-c]pyridin-5-yl)ethyl)-1H-pyrazole-3-carbonitrile (compound 2)

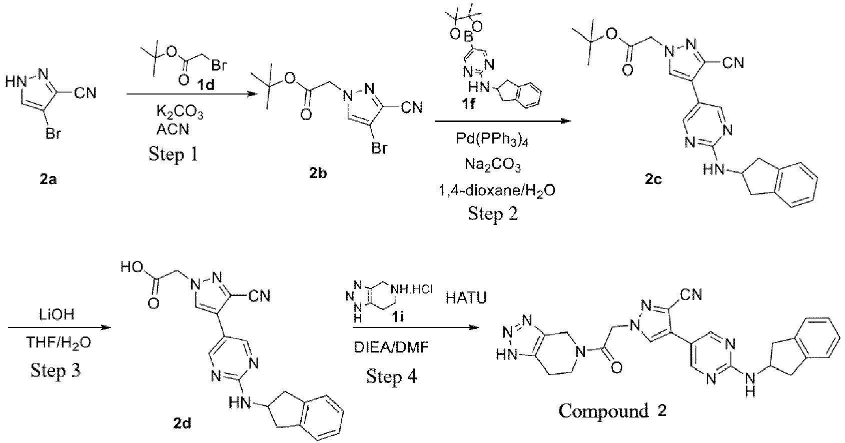

### Step 1: tert-butyl 2-(4-bromo-3-cyano-1H-pyrazol-1-yl)acetate (2b)

Compound **2a** (3.3 g, 19 mmol) was dissolved in acetonitrile (30 mL). **1d** (4.1 g, 21 mmol) and potassium carbonate (8.1 g, 58 mmol) were added, and the mixture was reacted at room temperature for 5 hours. After the reaction was completed, water was added, and the resulting solution was extracted with ethyl acetate, washed with saturated brine, and dried over anhydrous sodium sulfate. The concentrate was separated and purified by silica gel column chromatography to obtain target compound (**2b**) (4.3 g, 78%). ¹H NMR (400 MHz, CDCl₃) δ 7.61 (s, 1H), 4.83 (s, 2H), 1.49 (s, 9H).

### Step 2: tert-butyl-2-(3-cyano-4-(2-((2,3-dihydro-1H-inden-2-yl)amino)pyrimidin-5-yl)-1H-pyrazol-1-yl)acetate (2c)

Compound **2b** (0.31 g, 1.08 mmol) was dissolved in a mixed solvent of 1,4-dioxane/water (4 : 1), and **1f** (0.4 g, 1.18 mmol), tetratriphenylphosphine palladium (0.12 g, 1.08 mmol) and potassium carbonate (0.35 g, 3.23 mmol) were added successively. The mixture was reacted under nitrogen protection at 80°C for 5 h. After the reaction was completed, the concentrate was separated and purified by silica gel column chromatography to obtain target compound (**2c**) (0.15 g, 33%). LC-MS (ESI): m/z = 417.3 [M+H]⁺

### Step 3: 2-(3-cyano-4-(2-((2,3-dihydro-1H-inden-2-yl)amino)pyrimidin-5-yl)-1H-pyrazol-1-yl)acetic acid (2d)

Compound **2c** (0.13 g, 0.31 mmol) was dissolved in a mixed solvent of THF/H₂O = 4 : 1. After dissolution, LiOH (0.02 g, 0.62 mmol) was added and the mixture was reacted at room temperature for 2 h. Water was added, and the resulting solution was extracted with DCM. The aqueous phase was collected, diluted to pH = 4, and extracted with DCM. The extraction solution was washed with saturated brine, dried over anhydrous sodium sulfate and concentrated. A white solid **2d** (0.12 g, 90%) was obtained. LC-MS (ESI): m/z = 361.2 [M+H]⁺

### Step 4: 4-(2-((2,3-dihydro-1H-inden-2-yl)amino)pyrimidin-5-yl)-1-(2-oxo-2-(1,4,6,7-tetrahydro-5H-[1,2,3]triazolo[4,5-c]pyridin-5-yl)ethyl)-1H-pyrazole-3-carbonitrile (compound 2)

Compound **2d** (67 mg, 0.19 mmol), compound **1i** (46 mg, 0.37 mmol), DPEA(72 mg, 0.56 mmol) and HATU (106 mg, 0.28 mmol) were dissolved in DMF, and the mixture was reacted at room temperature overnight. After the reaction was completed, water was added, and the resulting solution was extracted with ethyl acetate. The extraction solution was washed with saturated brine, dried over anhydrous sodium sulfate, and concentrated. The concentrate was separated and purified by silica gel column chromatography to obtain compound **2** (7 mg, 8%). LC-MS (ESI): m/z = 467.3 [M+H]⁺. ¹H NMR (400 MHz, DMSO-d₆) δ 8.57 (s, 2H), 8.26 (d, 1H), 7.80 (d, 1H), 7.23 - 7.13 (m, 4H), 5.52 - 5.48 (m, 2H), 4.77-4.63 (m, 3H), 3.84 - 3.82 (m, 2H), 3.26 - 3.24 (m, 2H), 2.95 - 2.89 (m, 4H), 2.77 - 2.74 (m, 1H).

### Example 3: 4-(2-((2,3-dihydro-1H-inden-2-yl)amino)pyrimidin-5-yl)-N-methyl-1-(2-oxo-2-(1,4,6,7-tetrahydro-5H-[1,2,3]triazolo[4,5-c]pyridin-5-yl)ethyl)-1H-pyrazole-3-carboxamide (compound 3)

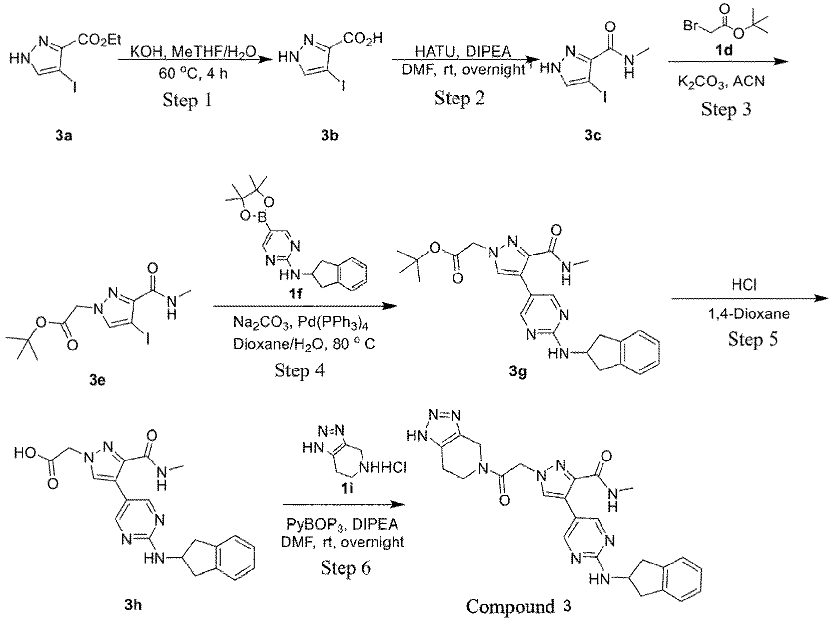

### Step 1: 4-iodo-1H-pyrazole-3-carboxylic acid (3b)

Compound **3a** (0.5 g, 1.88 mmol) was dissolved in 2-methyl tetrahydrofuran (10 mL) and distilled water (2 ml), and then KOH (0.2 g, 3.76 mmol) was added; Then the mixture was reacted at 60°C for 2 hours. After the reaction was completed, the reaction solution was cooled, and 20 mL of water was added. The resulting solution was extracted with ethyl acetate (20 mL × 3). The aqueous phase was adjusted to pH = 3 with acid, and then extracted with ethyl acetate (50 mL × 3). The organic phase was dried over anhydrous sodium sulfate, and filtered. The filtrate was concentrated under reduced pressure to obtain target compound (**3b**) (0.43 g, 95%). LC-MS (ESI): m/z = 239.2 [M+H]⁺. ¹H NMR (400 MHz, DMSO-d₆) δ 13.66 (s, 2H), 7.88 (s, 1H).

### Step 2: 4-iodo-N-methyl-1H-pyrazole-3-carboxamide (3c)

N,N-diisopropylethylamine (0.65 g, 5.04 mmol), methanamine hydrochloride (0.08 g, 2.52 mmol) and 2-(7-azabenzotriazole)-N,N,N',N'-tetramethyluronium hexafluorophosphate (0.528 g, 1.39 mmol) were added successively to a solution of compound **3b** (0.3 g, 1.26 mmol) in N,N-dimethylformamide (10 mL), and the mixture was stirred at room temperature overnight. After the reaction was completed, the reaction was quenched by adding water, and extracted with ethyl acetate (50 mL × 3). The organic phase was dried over anhydrous sodium sulfate, and filtered. The filtrate was concentrated under reduced pressure, and separated and purified by silica gel column chromatography (eluent: petroleum ether : ethyl acetate = 1 : 0-0 : 1) to obtain **3c** as an off-white solid (0.150 g, 47%). LC-MS (ESI): m/z = 252.1 [M+H]⁺. ¹H NMR (400 MHz, DMSO-d₆) δ 13.60 (s, 1H), 8.07 (s, 1H), 7.97 (s, 1H), 2.71 (s, 3H).

### Step 3: tert-butyl 2-(4-iodo-3-(methylcarbamoyl)-1H-pyrazol-1-yl)acetate (3e)

Cesium carbonate (0.25 g, 1.79 mmol) and tert-butyl bromoacetate (0.13 g, 0.66 mmol) were added successively to a solution of compound **3c** (0.15 g, 0.60 mmol) in acetonitrile (10 mL), and the mixture was stirred at room temperature for 2 hours. After the reaction was completed, the mixture was diluted with saturated brine (30 mL), and extracted with ethyl acetate (30 mL × 2). The organic phase was dried over anhydrous sodium sulfate, filtered and concentrated. The residue was separated and purified by silica gel column chromatography (eluent: petroleum ether : ethyl acetate = 1 : 0-0 : 1) to obtain **3e** as a white solid (0.170 g, 78%). LC-MS (ESI): m/z = 366.1 [M+H]⁺. ¹H NMR (400 MHz, DMSO-d₆) δ 8.04 (s, 1H), 7.98 (s, 1H), 4.99 (s, 2H), 2.70 (s, 3H), 1.43 (s, 9H).

### Step 4: tert-butyl2-(4-(2-((2,3-dihydro-1H-inden-2-yl)amino)pyrimidin-5-yl)-3-(methylcarbamoyl)-1H-pyrazol-1-yl)acetate (3g)

Tetrakis(triphenylphosphine)palladium(0) (0.057 g, 0.05 mmol) and sodium carbonate (0.16 g, 1.48 mmol) were added to a mixture of compound **3e** (0.18 g, 0.49 mmol) and compound **1f** (0.2 g, 0.59 mmol) in a mixed solvent of 1,4-dioxane (18 mL) and distilled water (4 mL), and the mixture was stirred at 80°C under nitrogen protection for 2 hours. After the reaction was completed, the mixture was cooled to room temperature, diluted with saturated brine (50 mL), and extracted with ethyl acetate (80 mL). The organic phase was dried over anhydrous sodium sulfate, and concentrated. The silica gel column chromatography (eluent: petroleum ether : ethyl acetate = 1 : 0-0 : 1) was used for separation and purification to obtain the title compound **3g** as light yellow solid (0.115 g, 52%). LC-MS (ESI): m/z = 449.3 [M+H]⁺. ¹H NMR (400 MHz, DMSO-d₆) δ 8.46 (s, 2H), (d, 1H), 8.01 (s, 1H),7.48 (d, 1H), 7.23 - 7.21 (m, 2H), 7.16 - 7.13 (m, 2H), 5.01 (s, 2H), 4.67 - 4.61 (m, 1H), 3.25 (t, 2H), 2.91 (q, 2H), 2.71 (d, 3H), 1.45 (s, 9H).

### Step 5: 2-(4-(2-((2,3-dihydro-1H-inden-2-yl)amino)pyrimidin-5-yl)-3-(methylcarbamoyl)-1H-pyrazol-1-yl)acetic acid (3h)

A solution (6 mL) of 4N hydrogen chloride in dioxane was added to a solution of compound **3g** (0.115 g, 0.26 mmol) in dichloromethane (4 mL), and the mixture was stirred at room temperature overnight. After the reaction was completed, the solvent was removed by filtration to obtain a solid, which was slurried with diethyl ether, filtered and dried to obtain the title compound **3h** as a light brown solid (0.08 g, 80%). LC-MS (ESI): m/z = 393.3 [M+H]⁺. ¹H NMR (400 MHz, DMSO-d₆) δ 8.46 (s, 2H), 8.05 (d, 1H), 8.01 (s, 1H),7.48 (d, 1H), 7.23 - 7.21 (m, 2H), 7.16 - 7.13 (m, 2H), 5.01 (s, 2H), 4.67 - 4.61 (m, 1H), 3.25 (t, 2H), 2.91 (q, 2H), 2.71 (d, 3H).

### Step 6: 4-(2-((2,3-dihydro-1H-inden-2-yl)amino)pyrimidin-5-yl)-N-methyl-1-(2-oxo-2-(1,4,6,7-tetrahydro-5H-[1,2,3]triazolo[4,5-c]pyridin-5-yl)ethyl)-1H-pyrazole-3-carboxamide (compound 3)

At 0°C, compound **3h** (0.080 g, 0.20 mmol), N,N-diisopropylethylamine (0.09 g, 0.71 mmol) and benzotriazol-1-yl-oxy-tripyrrolidinophosphonium hexafluorophosphate (0.16 g, 0.31 mmol) were sequentially added slowly to a solution of compound **1i** (0.05 g, 0.41 mmol) in N,N-dimethylformamide (6 mL), and the mixture was stirred at room temperature under nitrogen protection overnight. After the reaction was completed, the reaction mixture was diluted with distilled water (20 mL), and extracted with ethyl acetate (30 mL × 3). The organic phase was washed with distilled water and saturated brine (50 mL), dried over anhydrous sodium sulfate and concentrated. The silica gel column chromatography (eluent: dichloromethane : ethyl acetate : methanol = 1 : 0 : 0 - : 2 : 10 : 1) was used for separation and purification to obtain **compound 3** (14 mg). LC-MS (ESI): m/z = 499.3 [M+H]⁺. ¹H NMR (400 MHz, DMSO-d₆) δ 8.47 (s, 2H), 7.94 (d, 2H), 7.46 (d, 1H), 7.23 - 7.21 (m, 2H), 7.15 - 7.13 (m, 2H), 5.34 (d, 2H), 4.79 (s, 1H), 4.68 - 4.62 (m, 2H), 3.83 (d, 2H), 3.25 (t, 2H), 2.91 (q, 2H), 2.71 (d, 3H).

### Example 4: (1-(5-(2-((2,3-dihydro-1H-inden-2-yl)amino)pyrimidin-5-yl)-1,3,4-oxadiazol-2-yl)cyclopropyl)(1,4,6,7-tetrahydro-5H-[1,2,3]triazolo[4,5-c]pyridin-5-yl)methanone (compound4)

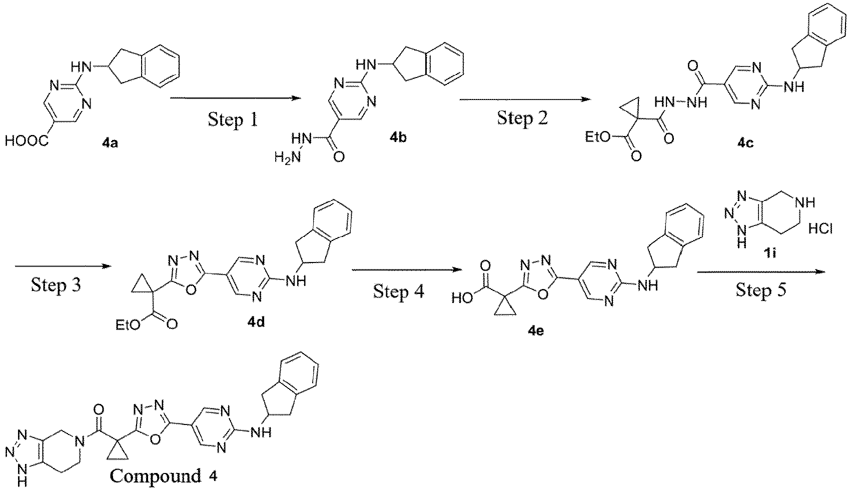

### Step 1: 2-((2,3-dihydro-1H-inden-2-yl)amino)pyrimidine-5-carbohydrazide (4b)

Compound **4a** (500 mg, 1.96 mmol) was dissolved in DMF (10 mL), HATU (1.11 g, 2.94 mmol) and DIPEA (758 mg, 5.88 mmol) were added and stirred at room temperature, and then hydrazine hydrate (0.25 mL) was added. The mixture was reacted at room temperature overnight. The reaction was completed and then directly used in the next step.

### Step 2: Ethyl 1-(2-(2-((2,3-dihydro-1H-inden-2-yl)amino)pyrimidine-5-carbonyl)hydrazine-1-carbonyl)cyclopropane-1-carboxylate (4c)

HATU (1.11 g, 2.94 mmol) and DIEA (758 mg, 5.88 mmol) were added to the reaction solution of the first step, then 1,1-cyclopropanedicarboxylic acid monoethyl ester (620 mg, 3.92 mmol) was added and the mixture was stirred at room temperature for 3 hours. After the reaction was completed, water was added, and the resulting solution was extracted with ethyl acetate. The organic phase was combined and dried to obtain compound **4c** (675 mg, 84%).

LC-MS (ESI): m/z = 410.2 [M+H]⁺.

### Step 3: Ethyl-1-(5-(2-((2,3-dihydro-1H-inden-2-yl)amino)pyrimidin-5-yl)-1,3,4-oxadiazol-2-yl)cyclopropane-1-carboxylate (4d)

Compound **4c** (500 mg, 1.22 mmol) was dissolved in anhydrous tetrahydrofuran, (methoxycarbonylsulfamoyl)triethylammonium hydroxide (582 mg, 2.44 mmol) was added, and the mixture was heated to 75°C and stirred for 1 hour. After the reaction was completed, the reaction solution was extracted twice with ethyl acetate and water. The organic phase was dried and concentrated, and separated and purified by silica gel column chromatography (DCM : MeOH = 40 : 1) to obtain compound **4d** (465 mg, 97%). LC-MS (ESI): m/z = 392.2 [M+H]⁺.

### Step 4: 1-(5-(2-((2,3-dihydro-1H-inden-2-yl)amino)pyrimidin-5-yl)-1,3,4-oxadiazol-2-yl)cyclopropane-1-carboxylic acid (4e)

Compound **4d** (465 mg, 1.19 mmol) was dissolved in anhydrous ethanol (10 mL), and lithium hydroxide (143 mg, 5.95 mmol) dissolved in 2 mL of water was added. The mixture was stirred at room temperature and reacted for 2 hours. After TLC detected that the reaction was completed, the reaction solution was concentrated, and diluted with water, and then 2N hydrochloric acid aqueous solution was added to adjust pH to 3. A large number of solids were precipitated and filtered by suction. The filter cake was dried to obtain compound **4e** (205 mg, 48%). LC-MS (ESI): m/z = 364.2 [M+H]⁺.

### Step 5: (1-(5-(2-((2,3-dihydro-1H-inden-2-yl)amino)pyrimidin-5-yl)-1,3,4-oxadiazol-2-yl)cyclopropyl)(1,4,6,7-tetrahydro-5H-[1,2,3]triazolo[4,5-c]pyridin-5-yl)methanone (compound 4)

Compound **4e** (205 mg, 0.56 mmol) was dissolved in 10 mL of N,N-dimethylformamide; HATU (322 mg, 0.85 mmol), N,N-diisopropylethylamine (0.3 mL, 1.68 mmol) and compound **1i** (139 mg, 1.12 mmol) were added; and the mixture was reacted at room temperature for 2 hours. After TLC detected that the reaction was completed, the reaction solution was extracted with ethyl acetate and water, and washed twice with saturated brine. The organic phase was dried and concentrated to obtain a crude product, which was separated and purified by silica gel column chromatography (DCM : MeOH = 20 : 1) to obtain **compound 4** (25 mg, 9%).

LC-MS (ESI): m/z = 470.2 [M+H]⁺.

¹H NMR (400 MHz, DMSO-*d₆*): 8.79-8.85 (m, 2H), 8.38-8.40 (d, 1H), 7.14-7.24 (m, 4H), 4.81- 4.68 (m, 3H), 4.44 (d, 2H), 3.28 (m, 2H), 2.91-3.00 (m, 3H), 2.73-2.76 (m, 1H), 1.88- 1.91 (m, 4H).

### Compound 5: 2-(5-(6-((2,3-dihydro-1H-inden-2-yl)amino)pyridazin-3-yl)-1,3,4-oxadiazol-2-yl)-1-(1,4,6,7-tetrahydro-5H-[1,2,3]triazolo[4,5-c]pyridin-5-yl)ethan-1-one (compound 5)

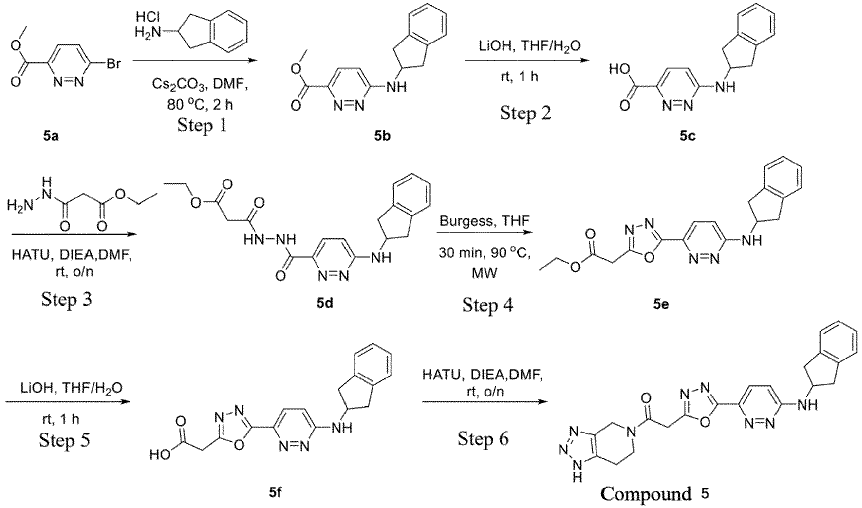

### Step 1: methyl 6-((2,3-dihydro-1H-inden-2-yl)amino)pyridazine-3-carboxylate (5b)

A solution of compound **5a** (6.95 g, 32 mmol) dissolved in DMF (100 mL) was added to a 250 mL single-necked flask, and 2,3-dihydro-1H-inden-2-amine hydrochloride (5.71 g, 33.6 mmol) and cesium carbonate (22.95 g, 70.4 mmol) were added. The mixture was reacted at 80°C for 2 h, cooled to room temperature, filtered, poured into 300 ml of water, and extracted with ethyl acetate (100 mL × 3). The organic phase was combined, washed with saturated sodium chloride (100 mL), dried over anhydrous sodium sulfate, concentrated under reduced pressure and separated and purified by silica gel column chromatography to obtain compound **5b** (4.0 g, yield 46%). LC-MS (ESI): m/z = 270.2 [M+H]⁺.

### Step 2: 6-((2,3-dihydro-1H-inden-2-yl)amino)pyridazine-3-carboxylic acid (5c)

Compound **5b** (4.0 g, 14.9 mmol) was dissolved in 30 ml of THF and 10 ml of water, and lithium hydroxide monohydrate (1.87 g, 44.6 mmol) was added. The mixture was stirred at room temperature and reacted for 1 hour. After TLC detected that the reaction was completed, potassium bisulfate aqueous solution was added to adjust pH to 4-5, and then dimethyl tetrahydrofuran was added. The organic layer was washed with saturated brine, dried over anhydrous sodium sulfate, and concentrated under reduced pressure to obtain compound **5c** (3.2 g, 84%). LC-MS (ESI): m/z = 256.2 [M+H]⁺.

### Step 3: Ethyl 3-(2-(6-((2,3-dihydro-1H-inden-2-yl)amino)pyridazine-3-carbonyl)hydrazinyl)-3-oxopropanoate (5d)

Compound **5c** (2.84 g, 11.1 mmol) was dissolved in DMF (50 mL), and then HATU (5.1 g, 13.4 mmol) and DIPEA (4.3 g, 33.5 mmol) were added. The mixture was stirred and reacted for 10 minutes, and then ethyl 3-oxo-3-hydrazinyl propanoate (2.0 g, 13.4 mmol) was added. The mixture was stirred at room temperature overnight. The reaction solution was poured into water (200 mL), extracted with ethyl acetate (50 mL × 3), washed with saturated brine, dried over anhydrous sodium sulfate and concentrated under reduced pressure to obtain compound **5d** (3.0 g, 71%). LC-MS (ESI): m/z = 384.2 [M+H]⁺.

### Step 4: ethyl 2-(5-(6-((2,3-dihydro-1H-inden-2-yl)amino)pyridazin-3-yl)-1,3,4-oxadiazol-2-yl)acetate (5e)

Compound **5d** (1.0 g, 2.6 mmol) was dissolved in anhydrous tetrahydrofuran (15 mL), and Burgess reagent (1.24 g, 5.2 mmol) was added. The mixture was heated to 90°C and reacted under microwave and nitrogen protection for 30 minutes. After the reaction was completed, water was added and the resulting solution was extracted with ethyl acetate. The organic layer was washed with saturated brine, dried over anhydrous sodium sulfate, concentrated, and separated and purified by silica gel column chromatography (PE/EA = 1/1) to obtain compound **5e** (480 mg, 51%). LC-MS (ESI): m/z = 366.2 [M+H]⁺.

### Step 5: 2-(5-(6-((2,3-dihydro-1H-inden-2-yl)amino)pyridazin-3-yl)-1,3,4-oxadiazol-2-yl)acetic acid (5f)

Compound **5e** (480 mg, 1.32 mmol) was dissolved in 10 ml of THF and 5 ml of water, and lithium hydroxide monohydrate (166 mg, 3.95 mmol) was added. The mixture was stirred at room temperature and reacted for 1 hour. After TLC detected that the reaction was completed, the reaction solution was concentrated, and diluted with water, and then potassium bisulfate aqueous solution was added to adjust pH to 4-5. A large number of solids were precipitated and filtered by suction. The filter cake was dried to obtain compound **5f** (420 mg, 94%). LC-MS (ESI): m/z = 338.2 [M+H]⁺.

### Step 6: 2-(5-(6-((2,3-dihydro-1H-inden-2-yl)amino)pyridazin-3-yl)-1,3,4-oxadiazol-2-yl)-1-(1,4,6,7-tetrahydro-5H-[1,2,3]triazolo[4,5-c]pyridin-5-yl)ethan-1-one (compound 5)

Compound **5f** (100 mg, 0.3 mmol) was dissolved in 3 ml of DMF, and CDI (58 mg, 0.36 mmol) was added. The mixture was stirred at room temperature for 30 minutes. In addition, 4,5,6,7-tetrahydro-1H-[1,2,3]triazolo[4,5-c]pyridine hydrochloride (192 mg, 1.2 mmol) was dissolved in 2 ml of DMF and DIEA (155 mg, 1.2 mmol) was added, and then the resulting solution was added to the reaction solution of **5f**, reacted for 1 h, concentrated, separated firstly with HPLC (acetonitrile and water), and then separated with a preparative silica gel plate (MeOH/DCM = 1/8) to obtain compound **5** (6 mg, 5%).

LC-MS (ESI): m/z = 444.2 [M+H]⁺.

¹H NMR (400 MHz, DMSO-d₆) *δ* 7.91 - 7.88 (m, 2H), 7.28 - 7.25 (m, 2H), 7.18 - 7.16 (m, 2H), 6.99 (d, 1H), 4.82 - 4.75 (m, 2H), 4.69 (s, 1H), 4.48 (d, 2H), 3.88 - 3.82 (m, 2H), 3.40 - 3.34 (m, 2H), 2.94 - 2.89 (m, 3H), 2.76 - 2.74 (m, 1H).

### Compound 6: 2-(5-(2-((1-phenylpyrrolidin-3-yl)amino)pyrimidin-5-yl)-1,3,4-oxadiazol-2-yl)-1-(1,4,6,7-tetrahydro-5H-[1,2,3]triazolo[4,5-c]pyridin-5-yl)ethan-1-one (compound 6)

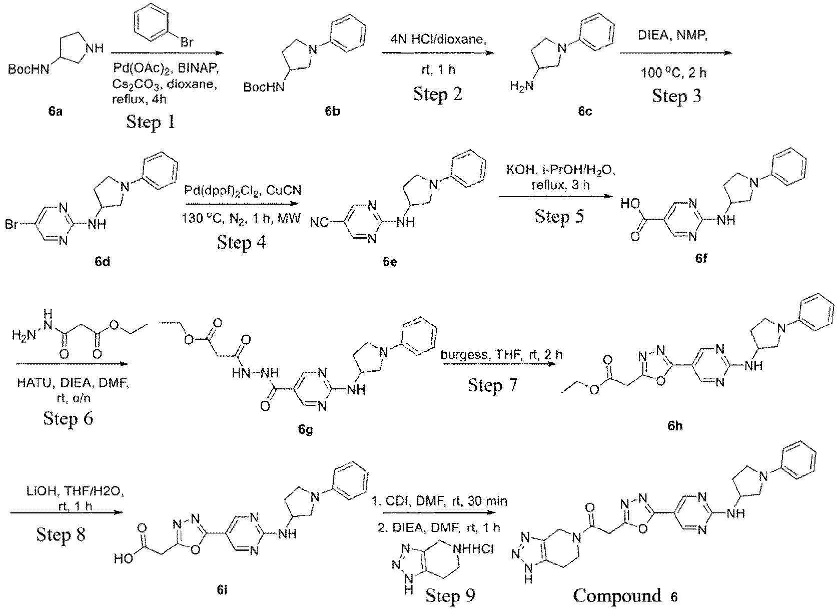

### Step 1: tert-butyl (1-phenylpyrrolidin-3-yl)carbamate (6b)

Compound **6a** (8.0 g, 43.0 mmol) was dissolved in DMF (100 mL), and then compounds bromobenzene (13.5 g, 85.9 mmol), 1,1'-binaphthyl-2,2'-bisdiphenylphosphino (4.0 g, 6.44 mmol), cesium carbonate (22.5 g, 68.9 mmol) and palladium acetate (965 mg, 4.3 mmol) were successively added. Under nitrogen protection, the mixture was reacted at 100°C for 4 hours. The reaction solution was cooled to room temperature and filtered. The resulting solution was poured into water (200 mL), and extracted with ethyl acetate (60 mL × 3). The organic phase was washed with saturated brine, dried over anhydrous sodium sulfate, concentrated, and separated and purified by silica gel column chromatography (PE/EA = 4/1) to obtain compound **6b** (5.6 g, 50%). LC-MS (ESI): m/z = 263.2 [M+H]⁺.

### Step 2: 1-phenylpyrrolidin-3-amine (6c)

At room temperature, a solution (60 mL) of 4N hydrochloric acid in 1,4-dioxane was added to compound **6b** (5.6 g, 21.4 mmol). The mixture was stirred and reacted for 1 hour. After the reaction was completed, the reaction solution was concentrated to obtain **6c** (5.5 g, a crude product), which was directly used in the next step. LC-MS (ESI): m/z = 163.2 [M+H]⁺.

### Step 3: 5-bromo-N-(1-phenylpyrrolidin-3-yl)pyrimidin-2-amine (6d)

Compound **6c** (5.5 g, a crude product) was dissolved in NMP (80 mL), DIEA (8.27 g, 64.1 mmol) and 5-bromo-2-chloropyridine (4.13 g, 21.4 mmol) were added, and the mixture was stirred and reacted at 100°C for 2 hours. The reaction solution was poured into water (300 mL). A large number of solids were precipitated and filtered. The filter cake was dried to obtain compound **6d** (4.0 g, two-step yield: 58%). LC-MS (ESI): m/z = 319.2 and 321.2 [M+H]⁺.

### Step 4: 2-((1-phenylpyrrolidin-3-yl)amino)pyrimidine-5-carbonitrile (6e)

Compound **6d** (1.0 g, 3.1 mmol) was dissolved in N,N-dimethylacetylamide (15 mL), and copper cyanide (563 mg, 6.29 mmol), triethylamine (951 mg, 9.42 mmol) and 1,1'-bis(diphenylphosphine)ferrocene palladium dichloride (234 mg, 0.32 mmol) were successively added. Under nitrogen protection, the mixture was heated to 130°C, and reacted under microwave for 1 hour. After the reaction was completed, a system of water and ethyl acetate was poured into the reaction solution. The resulting solution was filtered, and then extracted twice with ethyl acetate. The organic layer was washed with saturated brine, dried over anhydrous sodium sulfate, and concentrated to obtain compound **6e** (600 mg, 73%). LC-MS (ESI): m/z = 266.2 [M+H]⁺.

### Step 5: 2-((1-phenylpyrrolidin-3-yl)amino)pyrimidine-5-carboxylic acid (6f)

Compound **6e** (1.2 g, 4.5 mmol) was dissolved in 20 ml of isopropanol and 5 ml of water, potassium hydroxide (1.26 g, 22.5 mmol) was added, and the reaction mixture was refluxed for 3 hours. After TLC detected that the reaction was completed, the reaction solution was concentrated, diluted with water and extracted with ethyl acetate. The organic phase was adjusted to pH = 4-5 with potassium bisulfate aqueous solution, and then extracted with ethyl acetate. The organic layer was washed with saturated brine, dried over anhydrous sodium sulfate and concentrated to obtain compound **6f** (510 mg, 40%). LC-MS (ESI): m/z = 285.2 [M+H]⁺.

### Step 6: ethyl 3-oxo-3-(2-(2-((1-phenylpyrrolidin-3-yl)amino)pyrimidine-5-carbonyl)hydrazinyl)propanoate (6g)

Compound **6f** (500 mg, 1.76 mmol) was dissolved in DMF (10 mL), and then HATU (802 mg, 2.11 mmol) and DIPEA (681 mg, 5.28 mmol) were added. The mixture was stirred and reacted for 10 minutes, and then ethyl 3-hydrazinyl-3-oxopropanoate (309 mg, 2.11 mmol) was added and stirred at room temperature overnight. The reaction solution was poured into water (200 mL), extracted with ethyl acetate (50 mL × 3), washed with saturated brine, dried over anhydrous sodium sulfate and concentrated under reduced pressure to obtain compound **6g** (400 mg, 55%). LC-MS (ESI): m/z = 413.2 [M+H]⁺.

### Step 7: ethyl 2-(5-(2-((1-phenylpyrrolidin-3-yl)amino)pyrimidin-5-yl)-1,3,4-oxadiazol-2-yl)acetate (6h)

Compound **6g** (1.0 g, 2.4 mmol) was dissolved in anhydrous tetrahydrofuran (15 mL), and Burgess reagent (1.15 g, 4.9 mmol) was added. The mixture was stirred at room temperature and reacted under nitrogen protection for 1 hour. After TLC detected that the reaction was completed, water was added and the resulting solution was extracted with ethyl acetate. The organic layer was washed with saturated brine, dried over anhydrous sodium sulfate, concentrated, and then subjected to column chromatography (PE/EA = 1/1) to obtain compound **6h** (300 mg, 32%). LC-MS (ESI): m/z = 395.2 [M+H]⁺.

### Step 8: 2-(5-(2-((1-phenylpyrrolidin-3-yl)amino)pyrimidin-5-yl)-1,3,4-oxadiazol-2-yl)acetic acid(6i)

Compound **6h** (250 mg, 0.63 mmol) was dissolved in 9 ml of THF and 3 ml of water, and lithium hydroxide monohydrate (133 mg, 3.17 mmol) was added. The mixture was stirred at room temperature and reacted for 1 hour. After TLC detected that the reaction was completed, the reaction solution was concentrated, diluted with water and extracted with ethyl acetate. Then the aqueous layer was adjusted to pH = 4-5 with potassium bisulfate aqueous solution, and extracted with ethyl acetate. The organic layer was washed with saturated brine, dried over anhydrous sodium sulfate and concentrated to obtain compound 6i (120 mg, 52%). LC-MS (ESI): m/z = 367.2 [M+H]⁺.

### Step 9: 2-(5-(2-((1-phenylpyrrolidin-3-yl)amino)pyrimidin-5-yl)-1,3,4-oxadiazol-2-yl)-1-(1,4,6,7-tetrahydro-5H-[1,2,3]triazolo[4,5-c]pyridin-5-yl)ethan-1-one (compound 6)

Compound **6i** (120 mg, 0.33 mmol) was dissolved in 3 ml of DMF, and CDI (64 mg, 0.39 mmol) was added. The mixture was stirred at room temperature for 30 minutes. In addition, 4,5,6,7-tetrahydro-1H-[1,2,3]triazolo[4,5-c]pyridine hydrochloride (212 mg, 1.32 mmol) was dissolved in 2 ml of DMF and DIEA (170 mg, 1.32 mmol) was added, and then the resulting solution was added to the reaction solution of **6i,** reacted for 1 h, separated firstly with HPLC (acetonitrile and water), and then separated and purified by silica gel column chromatography (MeOH/DCM = 1/8) to obtain compound **6** (11 mg, 7%).

LC-MS (ESI): m/z = 473.2 [M+H]⁺.

¹H NMR (400 MHz, DMSO-d₆) *δ* 8.83 - 8.79 (m, 2H), 8.38 (d, 1H), 7.15 (t, 2H), 6.61 - 6.52 (m, 3H), 4.81 (s, 1H), 4.68 - 4.62 (m, 2H), 4.41 (d, 2H), 3.85 - 3.83 (m, 2H), 3.62 - 3.58 (m, 1H), 3.34 - 3.28 (m, 1H), 3.24 - 3.20 (m, 1H), 2.92 - 2.89 (m, 1H), 2.76 - 2.73 (m, 1H), 2.34 - 2.29 (m, 1H), 2.10 - 2.05 (m, 1H).

### Compound 7: 2-(5-(2-((5-cyclopropyl-2,3-dihydro-1H-inden-2-yl)amino)pyrimidin-5-yl)-1,3,4-oxadiazol-2-yl)-1-(1,4,6,7-tetrahydro-5H-[1,2,3]triazolo[4,5-c]pyridin-5-yl)ethan-1-one (compound 7)

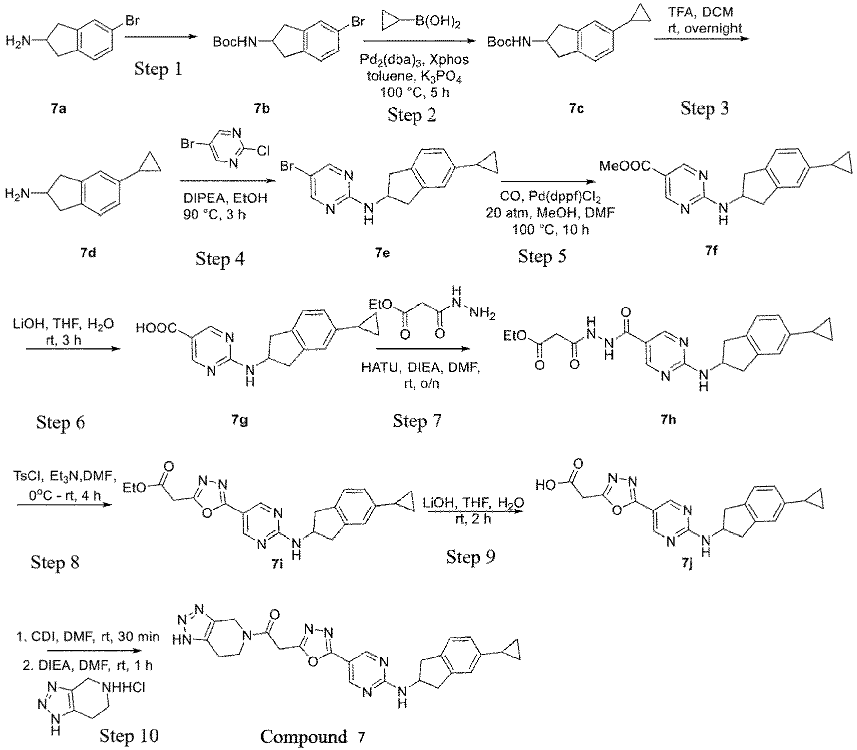

### Step 1: tert-butyl (5-bromo-2,3-dihydro-1H-inden-2-yl)carbamate (7b)

Starting material **7a** (20 g, 68.2 mmol) was dissolved in 200 mL of methanol, and then di-tert-butyl dicarbonate (16.4 g, 75.1 mmol) and triethylamine (20.7 g, 204.6 mmol) were added. The mixture was stirred at room temperature overnight until the reaction was completed (monitored by TLC). Most of the solvent was removed by rotation. 300 mL of saturated sodium bicarbonate solution was added, and then the resulting solution was extracted three times with 300 mL of ethyl acetate. The organic phase was combined, dried over anhydrous sodium sulfate and spun to dryness to obtain white solid **7b** (21 g, yield 71.2%). LC-MS (ESI): m/z = 313.2 [M+H]⁺.

### Step 2: tert-butyl (5-cyclopropyl-2,3-dihydro-1H-inden-2-yl)carbamate (7c)

Compound **7b** (6.0 g, 19.2 mmol) was added to a 250 mL single-necked flask, and dissolved with toluene (60 mL) and water (6 mL). Cyclopropyl boronic acid (2.2 g, 25.0 mmol), potassium phosphate (8.2 g, 38.4 mmol), Pd₂(dba)₃ (1.8 g, 1.9 mmol) and Xphos (1.8 g, 3.8 mmol) were added, and the mixture was subj ected to nitrogen replacement 3 times, reacted at 100°C for 5 h, quenched with aqueous solution (100 mL), and extracted with EA (50 mL × 3). The organic phase was combined, washed with saturated sodium chloride (5 mL × 1), dried over anhydrous sodium sulfate, concentrated under reduced pressure, and subjected to column chromatography (PE : EA = 4 : 1) to obtain compound **7c** (6.2 g, a crude product), which was directly used in the next reaction. LC-MS (ESI): m/z = 218.2 [M-55]⁺.

### Step 3: 5-cyclopropyl-2,3-dihydro-1H-inden-2-amine (7d)

Compound **7c** (6.2 g, a crude product) was added to a 250 mL single-necked flask, and dissolved with DCM (50 mL). TFA (50 mL) was added. The mixture was stirred at room temperature for 18 h, adjusted to pH = 9 with saturated sodium bicarbonate (10 mL), and extracted with DCM (50 mL × 2). The organic phase was combined, washed with saturated sodium chloride (5 mL × 1), dried over anhydrous sodium sulfate, concentrated under reduced pressure, and separated and purified by silica gel column chromatography (PE/EA = 2/1) to obtain compound **7d** (2.3 g, 68%). LC-MS (ESI): m/z = 174.2 [M+H]⁺.

### Step 4: 5-bromo-N-(5-cyclopropyl-2,3-dihydro-1H-inden-2-yl)pyrimidin-2-amine (7e)

Compound **7d** (2.3 g, 11.9 mmol) was added to a 100 mL single-necked flask, and dissolved with EtOH (15 mL). DIPEA (3.8 g, 29.7 mmol) and 5-bromo-2-chloropyrimidin (3.8 g, 29.7 mmol) were added. The mixture was stirred at 90°C for 3 h, cooled to room temperature, filtered by suction, and dried to obtain compound **7e** as a white solid (2.2 g, 56%). LC-MS (ESI): m/z = 330.1 [M+H]⁺.

### Step 5: methyl 2-((5-cyclopropyl-2,3-dihydro-1H-inden-2-yl)amino)pyrimidine-5-carboxylate (7f)

Compound **7e** (2.2 g, 6.7 mmol) was added to a 250 mL autoclave, and dissolved with methanol (20 mL) and DMF (20 mL). Pd(dppf)Cl₂ (2.0 g, 2.7 mmol) was added. The mixture was subjected to carbon monoxide replacement 3 times, reacted at 20 atm and 100°C for 10 h, filtered with diatomite, concentrated, separated and purified by silica gel column chromatography (PE/EA = 3/1) to obtain compound **7f** as a white solid (0.45 g, 22%). LC-MS (ESI): m/z = 310.3 [M+H]⁺.

### Step 6: 2-((5-cyclopropyl-2,3-dihydro-1H-inden-2-yl)amino)pyrimidine-5-carboxylicacid (7g)

Compound **7f** (0.45 g, 1.5 mmol) was added to a 100 mL eggplant-shaped flask, and dissolved with THF (5 mL) and water (5 mL). The mixture was stirred and reacted at room temperature for 3 h, adjusted to pH = 4-5 with HCl (1 M), and filtered to obtain compound **7g** as a white solid (0.36 g, 84%).

LC-MS (ESI): m/z = 296.2 [M+H]⁺.

### Step 7: ethyl 3-(2-(2-((5-cyclopropyl-2,3-dihydro-1H-inden-2-yl)amino)pyrimidine-5-carbonyl)hydrazinyl)-3-oxopropanoate (7h)

Compound **7g** (0.36 g, 1.2 mmol) was added to a 100 mL eggplant-shaped flask, and dissolved with DMF (5 mL). DIPEA (0.39 g, 3.0 mmol) and HATU (0.6 g, 1.6 mmol) were added. The mixture was stirred at room temperature for 10 minutes, and ethyl 3-oxo-3-hydrazinyl propanoate (0.36 g, 1.2 mmol) was added. The mixture was continuously stirred and reacted at room temperature overnight, and water (10 mL) was added. The resulting solution was extracted with EA (2 × 20 mL), separated and purified by silica gel column chromatography (PE/EA= 2/3) to obtain compound **7h** as a white solid (0.6 g). LC-MS (ESI): m/z = 424.3 [M+H]⁺.

### Step 8: ethyl 2-(5-(2-((5-cyclopropyl-2,3-dihydro-1H-inden-2-yl)amino)pyrimidin-5-yl)-1,3,4-oxadiazol-2-yl)acetate (7i)

Compound **7h** (550 mg, 1.3 mmol) was dissolved in dichloromethane (10 mL) and DMF (2 mL), triethylamine (328 mg, 3.3 mmol) was added, and p-toluenesulfonyl chloride (297 mg, 1.56 mmol) was added under an ice bath. The mixture was warmed to room temperature, and reacted under nitrogen protection for 4 hours with stirring. After TLC detected that the reaction was completed, the reaction solution was extracted with sodium bicarbonate aqueous solution and dichloromethane. The organic layer was washed with saturated brine, dried over anhydrous sodium sulfate, concentrated, and separated and purified by silica gel column chromatography (PE/EA = 1/1) to obtain compound **7i** (200 mg, 38%). LC-MS (ESI): m/z = 406.2 [M+H]⁺.

### Step 9: 2-(5-(2-((5-cyclopropyl-2,3-dihydro-1H-inden-2-yl)amino)pyrimidin-5-yl)-1,3,4-oxadiazol-2-yl)acetic acid (7j)

Compound **7i** (200 mg, 0.49 mmol) was dissolved in 9 ml of THF and 3 ml of water, and lithium hydroxide monohydrate (104 mg, 2.5 mmol) was added. The mixture was stirred at room temperature and reacted for 1 hour. After TLC detected that the reaction was completed, the reaction solution was concentrated, diluted with water and extracted with ethyl acetate. Then the aqueous layer was adjusted to pH = 4-5 with potassium bisulfate aqueous solution, and extracted with ethyl acetate. The organic layer was washed with saturated brine, dried over anhydrous sodium sulfate and concentrated to obtain compound **7j** (140 mg, 76%). LC-MS (ESI): m/z = 378.2 [M+H]⁺.

### Step 10: 2-(5-(2-((5-cyclopropyl-2,3-dihydro-1H-inden-2-yl)amino)pyrimidin-5-yl)-1,3,4-oxadiazol-2-yl)-1-(1,4,6,7-tetrahydro-5H-[1,2,3]triazolo[4,5-c]pyridin-5-yl)ethan-1-one (compound 7)

Compound **7j** (120 mg, 0.32 mmol) was dissolved in 3 ml of DMF, and CDI (62 mg, 0.38 mmol) was added. The mixture was stirred at room temperature for 30 minutes. Then 4,5,6,7-tetrahydro-1H-[1,2,3]triazolo[4,5-c]pyridine hydrochloride (205 mg, 1.28 mmol) was dissolved in 2 ml of DMF and dissociated with DIEA (165 mg, 1.28 mmol), and then the resulting solution was added to the reaction solution of 6i, reacted for 1 h, separated firstly with preparative HPLC (acetonitrile and water), and then separated and purified with a preparative silica gel plate (MeOH/DCM (v/v) = 1/8) to obtain compound **7** (5 mg, 3%).

LC-MS (ESI): m/z = 484.3 [M+H]⁺.

¹H NMR (400 MHz, DMSO-d₆) *δ* 14.63 (s, 1H), 8.82 - 8.79 (m, 2H), 8.36 (d, 1H), 7.08 (d, 1H), 6.92 - 6.87 (m, 2H), 4.81 - 4.66 (m, 3H), 4.41 (d, 2H), 3.86 - 3.82 (m, 2H), 3.25 - 3.21 (m, 2H), 2.91 - 2.88 (m, 3H), 2.78 - 2.76 (m, 1H), 1.90 - 1.86 (m, 1H), 0.91 - 0.89 (m, 2H), 0.62 - 0.60 (m, 2H).

### Example 8: 2-((5-(5-(2-(6,7-dihydro-1H-[1,2,3]triazolo[4,5-c]pyridin-5(4H)-yl)-2-oxoethyl)-1,3,4-oxadiazol-2-yl)pyrimidin-2-yl)amino)-2,3-dihydro-1H-indene-5-carbonitrile (compound 8)

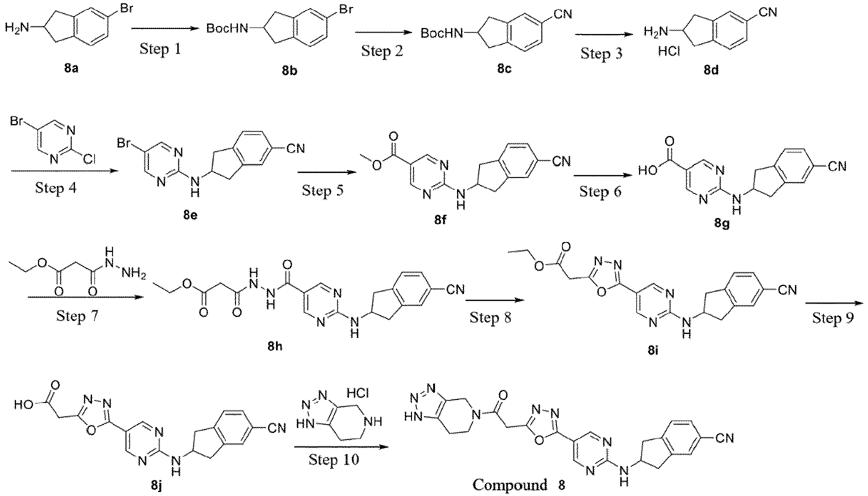

### Step 1: tert-butyl (5-bromo-2,3-dihydro-1H-inden-2-yl)carbamate (8b)

Compound **8a** (28.9 g, 98.6 mmol) was dissolved in methanol (500 ml), and triethylamine (29.9 g, 296 mmol) was added. The mixture was stirred at room temperature for 10 minutes, and (Boc)₂O(21.5 g, 98.6 mmol) was added in portions. After the addition was completed, the resulting solution was stirred at room temperature overnight. The reaction solution was evaporated to dryness under reduced pressure, 600 ml of water was added to the residue and the mixture was stirred for 1 hour, and then filtered. The filter cake was dried to obtain compound **8b** (30.0g, 97.4%).

### Step 2: tert-butyl (5-cyano-2,3-dihydro-1H-inden-2-yl)carbamate (8c)

Compound **8b** (6.8 g, 21.8 mmol) was dissolved in DMF (80 ml), and Zn(CN)₂ (3.5 g, 29.9 mmol) was added. The mixture was subjected to nitrogen replacement 3 times, then Pd(PPh₃)₄ (3.9 g, 3.4 mmol) was added, the resulting mixture was subjected to nitrogen replacement 3 times, and then warmed to 100°C and stirred overnight. The reaction solution was subjected to evaporation under increased pressure to remove DMF, and the residue was separated and purified by silica gel column chromatography (PE/EA = 5/1) to obtain **8c** (4.8 g, 85.2%). LC-MS (ESI): m/z = 259.3 [M+H]⁺.

### Step 3: 2-amino-2,3-dihydro-1H-indene-5-carbonitrile hydrochloride (8d)

**8c** (4.8 g, 18.6 mmol) was placed into a 100 ml single-necked flask, and HCl-dioxane (60 ml) was added. After the addition was completed, the mixture was stirred at room temperature overnight. The reaction solution was evaporated to dryness under increased pressure to obtain compound **8d** (3.6 g, 100%).

### Step 4: 2-((5-bromopyrimidin-2-yl)amino)-2,3-dihydro-1H-indene-5-carbonitrile (8e)

Compound **8d** (3.6 g, 18.6 mmol) and 5-bromopyrimidin-2-amine (3.2 g, 18.6 mmol) were dissolved in anhydrous ethanol (100 ml), and DIPEA (12.0 g, 93.0 mmol) was added. After the addition was completed, the mixture was warmed to 90°C and stirred overnight. The reaction solution was evaporated to dryness under reduced pressure, 100 ml of water was added to the residue and the mixture was stirred for 30 minutes, and then filtered. The filter cake was dried to obtain compound **8e** (5.1 g, 87.0%). LC-MS (ESI): m/z = 315.3 [M+H]⁺.

### Step 5: methyl 2-((5-cyano-2,3-dihydro-1H-inden-2-yl)amino)pyrimidine-5-carboxylate (8f)

Compound **8b** (4.0 g, 12.7 mmol) was placed into an autoclave, DMF (30 ml), MeOH (30 ml) and DIPEA (4.9 g, 38.1 mmol) were added, and finally Pd(dppf)Cl₂·CH₂Cl₂ (490 mg, 0.6 mmol) was added. After the addition was completed, the mixture was subjected to carbon monoxide replacement 3 times, then carbon monoxide was introduced until a pressure of 3.0 MPa, and the resulting mixture was heated to 110°C and reacted for 15 hours. The reaction solution was evaporated to dryness under reduced pressure, and the residue was separated and purified by silica gel column chromatography (PE/EA= 4/1) to obtain **8f** (2.8 g, 74.9%). LC-MS (ESI): m/z = 295.3 [M+H]⁺.

### Step 6: 2-((5-cyano-2,3-dihydro-1H-inden-2-yl)amino)pyrimidine-5-carboxylic acid (8g)

**8f** (2.8 g, 9.5 mmol) was placed into a 100 ml single-necked flask, methanol/water = 1/1 (40 ml) and LiOH·H₂O (2.0 g, 47.5 mmol) were successively added. After the addition was completed, the mixture was stirred at room temperature for 5 hours, and then subjected to evaporation under reduced pressure to remove methanol. The aqueous phase was adjusted to pH = 5 with 1N dilute hydrochloric acid, stirred for 10 minutes, and then filtered. The filter cake was dried to obtain compound **8g** (2.5 g, 93.9%).

### Step 7: Ethyl 3-(2-(2-((5-cyano-2,3-dihydro-1H-inden-2-yl)amino)pyrimidine-5-carbonyl)hydrazinyl)-3-oxopropanoate (8h)

**8g** (2.5 g, 8.9 mmol) and ethyl 3-oxo-3-hydrazinyl propanoate (1.4 g, 9.3 mmol) were dissolved in DMF (50 ml), and DIPEA (2.3 g, 17.8 mmol) and HATU (4.1 g, 10.7 mmol) were successively added. After the addition was completed, the mixture was stirred at room temperature overnight. 80 ml of water was added to the reaction solution, and the resulting solution was extracted 4 times with ethyl acetate. The organic phase was washed 3 times with saturated NaCl aqueous solution, dried over anhydrous sodium sulfate, and filtered. The filtrate was evaporated to dryness under reduced pressure. The residue was separated and purified by silica gel column chromatography (PE/EA = 2/1) to obtain compound **8h** (1.9 g, 52.3%).

### Step 8: Ethyl 2-(5-(2-((5-cyano-2,3-dihydro-1H-inden-2-yl)amino)pyrimidin-5-yl)-1,3,4-oxadiazol-2-yl)acetate (8i)

Compound **8h** (1.9 g, 6.8 mmol) was dissolved in anhydrous THF (30 ml), and Burgess reagent (3.2 g, 13.6 mmol) was added. After the addition was completed, the mixture was stirred at room temperature overnight. The reaction solution was evaporated to dryness under reduced pressure. The residue was separated and purified by silica gel column chromatography (PE/EA = 2/1) to obtain compound **8i** (1.0 g, 37.7%). LC-MS (ESI): m/z = 391.2 [M+H]⁺.

### Step 9: 2-(5-(2-((5-cyano-2,3-dihydro-1H-inden-2-yl)amino)pyrimidin-5-yl)-1,3,4-oxadiazol-2-yl)acetic acid (8j)

Compound **8i** (1.0 g, 2.6 mmol) was dissolved in THF/H₂O = 2/1 (15 ml), and LiOH·H₂O (328 mg, 7.8 mmol) was added. After the addition was completed, the mixture was stirred at room temperature overnight. The reaction solution was subjected to evaporation under reduced pressure to remove THF. The aqueous phase was adjusted to pH = 5 with 1N dilute hydrochloric acid, stirred for 10 minutes and then filtered. The filter cake was dried to obtain **8j** (0.8 g, 84.9%).

### Step 10: 2-((5-(5-(2-(6,7-dihydro-1H-[1,2,3]triazolo[4,5-c]pyridin-5(4H)-yl)-2-oxoethyl)-1,3,4-oxadiazol-2-yl)pyrimidin-2-yl)amino)-2,3-dihydro-1H-indene-5-carbonitrile (compound 8)

**8j** (0.8 g, 2.2 mmol) and 4,5,6,7-tetrahydro-1H-[1,2,3]triazolo[4,5-c]pyridine hydrochloride (707 mg, 4.4 mmol) were dissolved in DMF (20 ml), and DIPEA (1.4 g, 11 mmol) and HATU (988 mg, 2.6 mmol) were successively added. After the addition was completed, the mixture was stirred at room temperature overnight. The reaction solution was slowly added dropwise into ice water, stirred for 2 minutes and then filtered. The filter cake was separated and purified by silica gel column chromatography (DCM/MeOH = 15/1) to obtain compound **8** (47 mg, 4.6%).

¹H NMR (400 MHz, DMSO-d₆) *δ* 8.81 (brs, 2H), 8.42 (d, 1H), 7.69 (s, 1H), 7.63 (d, 1H), 7.45 (s, 1H), 8.81 (s, 1H), 4.77-4.72 (m, 1H), 4.68 (s, 1H), 4.43 (s, 1H), 4.39 (S, 1H), 3.87-381 (m, 2H), 3.41-3.32 (m, 1H), 3.06-2.97 (m, 2H), 2.91 (t, 1H), 2.75 (t, 1H). LC-MS (ESI): m/z = 469.1 [M+H]⁺.

### Compound 9: 2-(5-(2-((5-(difluoromethyl)-2,3-dihydro-1H-inden-2-yl)amino)pyrimidin-5-yl)-1,3,4-oxadiazol-2-yl)-1-(1,4,6,7-tetrahydro-5H-[1,2,3]triazolo[4,5-c]pyridin-5-yl)ethan-1-one (compound 9)

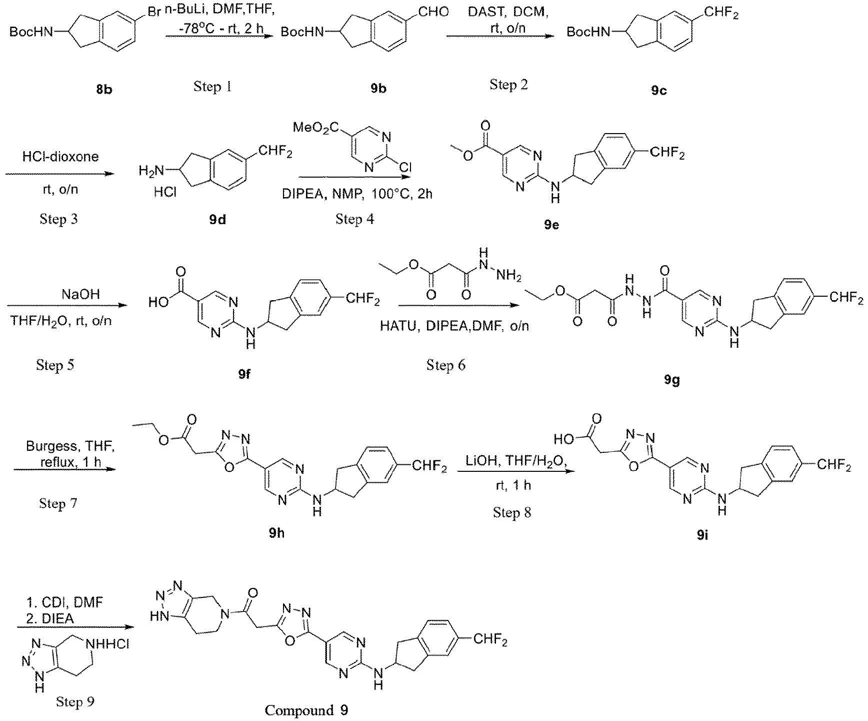

### Step 1: tert-butyl (5-formyl-2,3-dihydro-1H-inden-2-yl)carbamate (9b)

Starting material **8b** (10 g, 32.1 mmol) was dissolved in 400 mL of anhydrous tetrahydrofuran, and then n-butyl lithium (2.5 M, 32 mL, 80.1 mmol) was added under the condition of -78°C. The reaction mixture was stirred for 30 minutes under the same temperature, and then DMF (12.5 mL, 160.5 mmol) was added under the condition of -78°C. The mixture was returned to room temperature slowly, and then stirred for 15 minutes. After the reaction was completed, saturated ammonium chloride solution was added. Then the mixture was extracted three times with ethyl acetate. The organic phase was combined, dried over anhydrous sodium sulfate, spun to dryness, and separated and purified by silica gel column chromatography (PE/EA = 6/1) to obtain **9b** as a white solid (5.8 g, yield 69%). LC-MS (ESI): m/z = 262.2 [M+H]⁺.

### Step 2: tert-butyl (5-(difluoromethyl)-2,3-dihydro-1H-inden-2-yl)carbamate (9c)

Compound **9b** (5.8 g, 22.2 mmol) and dichloromethane (100 mL) were added to a 250 mL single-necked flask, and DAST (7.2 g, 44.4 mmol) was added at -10°C. The mixture was warmed to room temperature, reacted for 48 h, quenched with aqueous solution (100 mL), and extracted with dichloromethane (50 mL × 3). The organic phase was combined, washed with saturated sodium chloride (50 mL × 1), dried over anhydrous sodium sulfate, concentrated under reduced pressure, and separated and purified by silica gel column chromatography (PE/EA = 9/1) to obtain **9c** as a white solid (4.2 g, 67%). LC-MS (ESI): m/z = 284.2 [M-55]⁺.

### Step 3: 5-(difluoromethyl)-2,3-dihydro-1H-inden-2-amine (9d)

At room temperature, a solution (60 mL) of 4N hydrochloric acid in 1,4-dioxane was added to compound **9c** (4.2 g, 14.8 mmol). The mixture was stirred and reacted for 1 hour. After the reaction was completed, the reaction solution was concentrated to obtain **9d** (3.3 g, a crude product), which was directly used in the next step. LC-MS (ESI): m/z = 184.2 [M+H]⁺.

### Step 4: methyl 2-((5-(difluoromethyl)-2,3-dihydro-1H-inden-2-yl)amino)pyrimidine-5-carboxylate (9e)

Compound **9d** (3.3 g, 18.0 mmol) was added to a 100 mL single-necked flask, and dissolved with NMP (40 mL). DIPEA (7.0 g, 54.1 mmol) and methyl 2-chloropyrimidin-5-carboxylate (3.1 g, 18.0 mmol) were added. The mixture was stirred at 100°C for 2 h, cooled to room temperature, and poured into water. Solids were precipitated, filtered by suction, and dried to obtain compound **9e** as a light yellow solid (2.5 g, 44%). LC-MS (ESI): m/z = 320.1 [M+H]⁺.

### Step 5: 2-((5-(difluoromethyl)-2,3-dihydro-1H-inden-2-yl)amino)pyrimidine-5-carboxylic acid (9f)

Compound **9e** (2.5 g, 7.84 mmol) was added to a 100 mL eggplant-shaped flask, and dissolved with THF (30 mL) and water (20 mL). Sodium hydroxide (940 mg, 23.51 mmol) was added. The mixture was stirred and reacted for 3 h under reflux, concentrated to remove tetrahydrofuran, adjusted to pH = 4-5 with HCl (1 M), and filtered to obtain compound **9f** as a white solid (2.0 g, 84%). LC-MS (ESI): m/z = 306.2 [M+H]⁺.

### Step 6: ethyl 3-(2-(2-((5-(difluoromethyl)-2,3-dihydro-1H-inden-2-yl)amino)pyrimidine-5-carbonyl)hydrazinyl)-3-oxopropanoate (9g)

Compound **9f** (3.6 g, 11.76 mmol) was added to a 100 mL eggplant-shaped flask, and dissolved with DMF (50 mL). DIPEA (4.55 g, 35.29 mmol) and HATU (6.7 g, 17.64 mmol) were added. The mixture was stirred at room temperature for 10 minutes, and ethyl 3-oxo-3-hydrazinyl propanoate (2.06 g, 14.11 mmol) was added. The resulting mixture was continuously stirred and reacted at room temperature overnight, and poured into water. A yellow solid was precipitated and filtered. The filter cake was dried to obtain compound **9g** as a yellow solid (4.0 g, 78%). LC-MS (ESI): m/z = 434.3 [M+H]⁺.

### Step 7: ethyl 2-(5-(2-((5-(difluoromethyl)-2,3-dihydro-1H-inden-2-yl)amino)pyrimidin-5-yl)-1,3,4-oxadiazol-2-yl)acetate (9h)

Compound **9g** (4.0 g, 9.24 mmol) was dissolved in anhydrous tetrahydrofuran (50 mL), and Burgess reagent (4.4 g, 18.48 mmol) was added. The mixture was reacted under nitrogen protection at 70°C for 1 hour. After TLC detected that the reaction was completed, water was added and the resulting solution was extracted with ethyl acetate. The organic layer was washed with saturated brine, dried over anhydrous sodium sulfate, concentrated, and separated and purified by silica gel column chromatography (PE/EA = 1/1) to obtain compound **9h** (2.8 g, 73%). LC-MS (ESI): m/z = 416.2 [M+H]⁺.

### Step 8: 2-(5-(2-((5-(difluoromethyl)-2,3-dihydro-1H-inden-2-yl)amino)pyrimidin-5-yl)-1,3,4-oxadiazol-2-yl)acetic acid (9i)

Compound **9h** (2.8 g, 6.75 mmol) was dissolved in 30 ml of THF and 10 ml of water, and lithium hydroxide monohydrate (850 mg, 20.24 mmol) was added. The mixture was stirred at room temperature and reacted for 1 hour. After TLC detected that the reaction was completed, the reaction solution was concentrated, diluted with water and extracted with ethyl acetate. Then the aqueous layer was adjusted to pH = 4-5 with potassium bisulfate aqueous solution, and extracted with ethyl acetate. The organic layer was washed with saturated brine, dried over anhydrous sodium sulfate and concentrated to obtain compound **9i** (2.3 g, 88%). LC-MS (ESI): m/z = 388.2 [M+H]⁺.

### Step 9: 2-(5-(2-((5-(difluoromethyl)-2,3-dihydro-1H-inden-2-yl)amino)pyrimidin-5-yl)-1,3,4-oxadiazol-2-yl)-1-(1,4,6,7-tetrahydro-5H-[1,2,3]triazolo[4,5-c]pyridin-5-yl)ethan-1-one (compound 9)

Compound **9i** (387 mg, 1.0 mmol) was dissolved in 5 ml of DMF, and CDI (195 mg, 1.2 mmol) was added. The mixture was stirred at room temperature for 30 minutes. Then 4,5,6,7-tetrahydro-1H-[1,2,3]triazolo[4,5-c]pyridine hydrochloride (482 mg, 3.0 mmol) was dissolved in 2 ml of DMF, and dissociated with DIEA (516 mg, 4.0 mmol), and the resulting solution was added to the reaction solution of **9i,** reacted for 1 h, poured into water, and extracted with a mixed solvent of DCM/MeOH/MeCN. The extraction solution was spun to dryness, firstly separated with HPLC (acetonitrile and water), and then separated and purified by silica gel column chromatography (THF/DCM = 1/3) to obtain compound **9** (70 mg, 14%). LC-MS (ESI): m/z = 494.2 [M+H]⁺. ¹H NMR (400 MHz, DMSO-d₆) *δ* 8.83 - 8.79 (m, 2H), 8.38 - 8.36 (m, 1H), 7.40 (d, 3H), 6.97 (t, 1H), 4.81 - 4.68 (m, 3H), 4.43 - 4.39 (m, 2H), 3.87 - 3.81 (m, 2H), 3.35 - 3.31 (m, 2H), 3.02 - 2.72 (m, 4H).

### Example 10: N-(2,3-dihydro-1H-inden-2-yl)-2-(2-oxo-2-(1,4,6,7-tetrahydro-5H-[1,2,3]triazolo[4,5-c]pyridin-5-yl)ethyl)benzo[d]oxazole-5-carboxamide (compound 10)

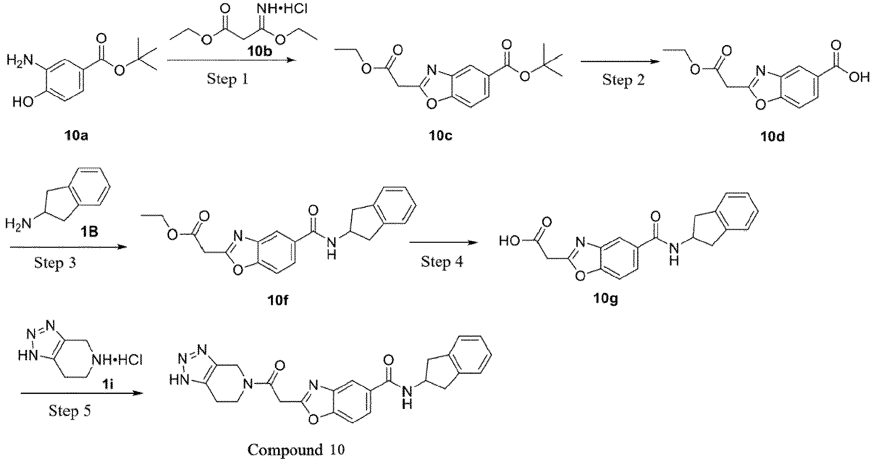

### Step 1: tert-butyl 2-(2-ethoxy-2-oxoethyl)benzo[d]oxazole-5-carboxylate (10c)

Tert-butyl 3-amino-4-hydroxybenzoate (**10a**) (3 g, 14.34 mmol) and ethyl 3-ethoxy-3-iminopropionate hydrochloride **10b** (3.37 g, 17.20 mmol) were dissolved in ethanol (20 mL), and stirred at 80°C overnight. After the reaction was completed, the reaction was cooled to room temperature and the ethanol was concentrated under reduced pressure. Then the residue was dissolved in dichloromethane. The organic phase was washed with distilled water. The layers were separated. The organic phase was concentrated under reduced pressure to obtain target compound (**10c**) as a white solid (4 g, 91.37%). LC-MS (ESI): m/z = 306.1 [M+H]⁺

### Step 2: 2-(2-ethoxy-2-oxoethyl)benzo[d]oxazole-5-carboxylic acid (10d)

Trifluoroacetic acid (2 mL) was added to a solution of compound **10c** (4.9 g, 4.5 mmol) in dichloromethane (10 mL), and stirred at room temperature for 3 hours. After the reaction was completed, saturated sodium bicarbonate aqueous solution was added thereto to adjust the pH value to neutral, and then the resulting solution was extracted with dichloromethane. The organic layer was successively washed with water and saturated brine, dried over anhydrous sodium sulfate, and concentrated to obtain the title compound **10d** as a light yellow solid (3.9 g, 97.51%). LC-MS (ESI): m/z = 250.3 [M+H]⁺

### Step 3: ethyl 2-(5-((2,3-dihydro-1H-inden-2-yl)carbamoyl)benzo[d]oxazol-2-yl)acetate (10f)

Compound **10d** (1.2 g, 4.81 mmol), DMF (20 mL), HATU (2.2 g, 5.78 mmol) and N,N-diisopropylethylamine (1.3 g, 10 mmol) were successively added into a single-necked flask. The mixture was stirred and reacted for 15 minutes, then compound **1B** (0.77 g, 5.78 mmol) was added, and the resulting mixture was continuously stirred and reacted at room temperature for 1 hour. After the reaction was completed, the reaction solution was diluted with water (30 mL), and extracted with ethyl acetate. The organic layer was washed with water and saturated brine, dried over anhydrous sodium sulfate and concentrated. The silica gel column chromatography (DCM : MeOH = 60 : 1) was used for separation to obtain target compound **10f** as a white solid (1.2 g, 68.4%). LC-MS (ESI): m/z = 365.3 [M+H]⁺

### Step 4: 2-(5-((2,3-dihydro-1H-inden-2-yl)carbamoyl)benzo[d]oxazol-2-yl)acetic acid (10g)

Lithium hydroxide (0.44g, 11 mmol) was added to a mixed solvent of compound **10f** (1.2 g, 3.29 mmol) in tetrahydrofuran (10 mL) and water (5 mL), and the mixture was stirred at room temperature for 1 hour. After the reaction was completed, the mixture was treated with 10% citric acid aqueous solution to adjust to pH = 6, and then extracted with ethyl acetate. The organic layer was washed with saturated brine, dried over anhydrous sodium sulfate, and concentrated. The residue was slurried with diethyl ether to obtain target compound **10g** as a yellow solid (1.0 g, 90.2%). LC-MS (ESI): m/z = 337.2 [M+H]⁺

### Step 5: N-(2,3-dihydro-1H-inden-2-yl)-2-(2-oxo-2-(1,4,6,7-tetrabydro-5H-[1,2,3]triazolo[4,5-c]pyridin-5-yl)ethyl)benzo[d]oxazole-5-carboxamide (compound 10)

At room temperature, intermediate **1i** (0.11 g, 0.61 mmol) and N,N-diisopropylethylamine (0.13 g, 1 mmol) were sequentially added slowly to a solution of compound **10g** (0.12 g, 0.35 mmol), chloridized tetramethyluronium hexafluorophosphate (0.12 g, 0.42 mmol) and N-methyl imidazole (0.04 g, 0.52 mmol) in N,N-dimethylformamide (10 mL), and the mixture was stirred at room temperature under nitrogen protection for 3 h. After the reaction was completed, the reaction mixture was diluted with water (30 mL), and extracted with ethyl acetate (50 mL × 2). The organic layer was washed with saturated brine (50 mL), dried over anhydrous sodium sulfate and concentrated to obtain a crude product. The crude product was separated and purified by silica gel column chromatography (DCM : MeOH = 25 : 1) to obtain **compound 10** (15 mg, 9.5%).

LC-MS (ESI): m/z = 443.2 [M+H]⁺.

¹H NMR (400 MHz, DMSO-d₆) *δ* 8.77 - 8.75 (m, 1H), 8.26 - 8.24 (m, 1H), 7.96 - 7.93 (m, 1H), 7.77-7.73 (m, 1H), 7.25-7.22 (m, 2H), 7.18-7.13 (m, 2H), 4.81 - 4.69 (m, 3H), 4.43 - 4.40 (m, 2H), 3.88 - 3.81 (m, 2H), 3.31 - 3.32 (m, 2H), 3.03- 2.97 (m, 2H), 2.87-2.74 (m, 2H).

### Example 11: 1-(2,3-dihydro-1H-inden-2-yl)-3-(2-(2-oxo-2-(1,4,6,7-tetrahydro-5H-[1,2,3]triazolo[4,5-c]pyridin-5-yl)ethyl)benzo[d]oxazol-6-yl)urea (compound 11)

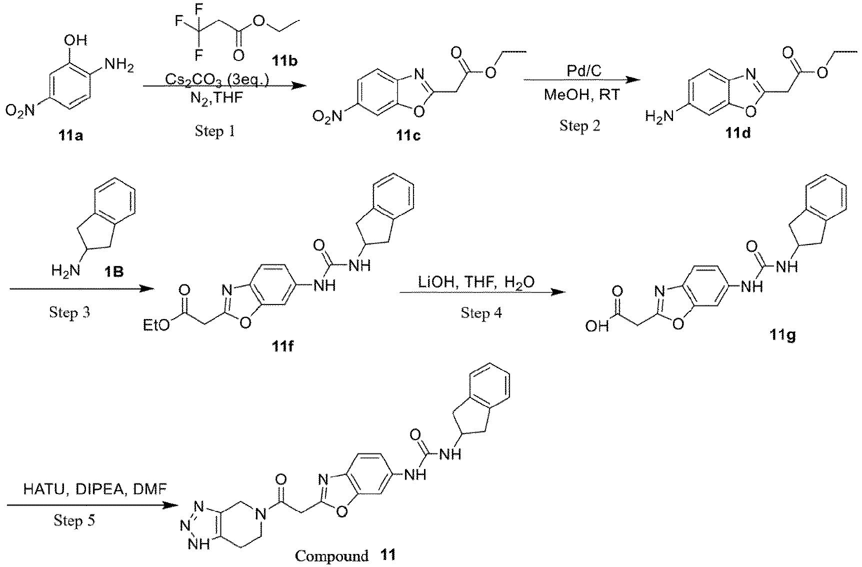

### Step 1: ethyl 2-(6-nitrobenzo[d]oxazol-2-yl)acetate (11c)

Compound **11a** (2.00 g, 12.98 mmol) and compound **11b** (2.03 g, 12.98 mmol) were dissolved in tetrahydrofuran (40 mL), and Cs₂CO₃ (8.46g, 25.95 mmol) was slowly added under an ice bath. The mixture was subjected to nitrogen replacement, and then reacted at 45°C for 5 hours. After the reaction was completed, the reaction solution was cooled to room temperature. Water was added, and the resulting solution was extracted three times with ethyl acetate, washed with saturated brine, dried over anhydrous sodium sulfate, spun to dryness, and separated and purified by silica gel column chromatography (PE : EA = 40 : 1) to obtain (**11c**) (3.00 g, 92.4%). LC-MS (ESI): m/z = 251.1 [M+H]⁺. ¹H NMR (400 MHz, CDCl₃) *δ* 8.45 (d, 1H), 8.32 (dd, 1H), 7.85 - 7.80 (m, 1H), 4.27 (q, 2H), 4.10 - 4.05 (m, 2H), 2.04 (s, 1H), 1.31 (dd, 3H).

### Step 2: ethyl 2-(6-aminobenzo[d]oxazol-2-yl)acetate (11d)

Compound **11c** (1.6 g, 6.4 mmol) was dissolved in methanol, and Pd/C (160 mg) was added. The mixture was subjected to hydrogen replacement, and reacted overnight. DCM : MeOH = 10 : 1 showed that the reaction was completed. The reaction solution was filtered, and concentrated under reduced pressure to obtain (**11d**) (1.18 g, 84 %).

### Step 3: ethyl 2-(6-(3-(2,3-dihydro-1H-inden-2-yl)ureido)benzo[d]oxazol-2-yl)acetate (11f)

Compound **11d** (1.18 g, 5.36 mmol) and compound **1B** (1.09 g, 6.43 mmol) were dissolved in anhydrous dichloromethane, triphosgene (557 mg, 1.88 mmol) was added, and DIPEA (2.71 g, 26.8 mmol) was slowly added dropwise. The mixture was reacted at room temperature for 10 min. After the reaction was completed, the reaction solution was quenched with saturated ammonium chloride, and extracted with ethyl acetate (30 mL × 2). The organic phase was washed with saturated brine, dried over anhydrous sodium sulfate, filtered and concentrated. The silica gel column chromatography (DCM : MeOH = 60 : 1) was used for separation and purification to obtain product **11f** as a white solid (1.15 g, 56.6%).

### Step 4: 2-(6-(3-(2,3-dihydro-1H-inden-2-yl)ureido)benzo[d]oxazol-2-yl)acetic acid (11g)

Compound **11f** (1.15 g, 3.03 mmol) was dissolved in methanol, and an aqueous solution of lithium hydroxide (318 mg, 7.58 mmol) was slowly added dropwise. The mixture was reacted at room temperature. After the reaction was completed, the mixture was concentrated, water was added and the resulting solution was filtered. The filtrate was adjusted to pH = 3-4 with HCl, and extracted with ethyl acetate (30 mL × 2). The organic phase was washed with saturated brine, dried over anhydrous sodium sulfate, filtered and concentrated. Product **11g** as a white solid (1.0 g, 93.9%) was obtained.

### Step 5: 1-(2,3-dihydro-1H-inden-2-yl)-3-(2-(2-oxo-2-(1,4,6,7-tetrabydro-5H-[1,2,3]triazolo[4,5-c]pyridin-5-yl)ethyl)benzo[d]oxazol-6-yl)urea (compound 11)

Compound **11g** (400 mg, 1.138 mmol) and **11h** (212 mg, 1.707 mmol) were dissolved in DMF, HATU (866 mg, 2.277 mmol) was added, and DIPEA (0.79 ml, 5.692 mmol) was added dropwise. The mixture was reacted at room temperature. After the reaction was completed, the mixture was diluted with water, and extracted with ethyl acetate. The organic layer was washed with saturated brine, dried over anhydrous sodium sulfate, and concentrated. The silica gel column chromatography (DCM : MeOH = 15 : 1) was used for separation and purification to obtain **compound 11** (386 mg, 74.1%).

LC-MS (ESI): m/z = 458.2 [M+H]⁺.

¹H NMR (400 MHz, DMSO-d₆) *δ* 8.58 (d, 1H), 7.94 (dd, 1H), 7.54 - 7.48 (m, 1H), 7.25 (dt, 2H), 7.18 - 7.13 (m, 2H), 7.08 (m, 1H), 6.50 (d, 1H), 4.80 (s, 1H), 4.68 (s, 1H), 4.49 - 4.39 (m, 1H), 4.30 (d, 2H), 3.91 - 3.80 (m, 2H), 3.22 (d, 1H), 3.18 (d, 1H), 2.88 - 2.72 (m, 4H).

### Example 12: 1-(6,7-dihydro-1H-[1,2,3]triazolo[4,5-c]pyridin-5(4H)-yl)-2-(5-(2-((2,3-dihydro-1H-inden-2-yl)amino)-4-methoxypyrimidin-5-yl)-1,3,4-oxadiazol-2-yl)ethanone (compound 12)

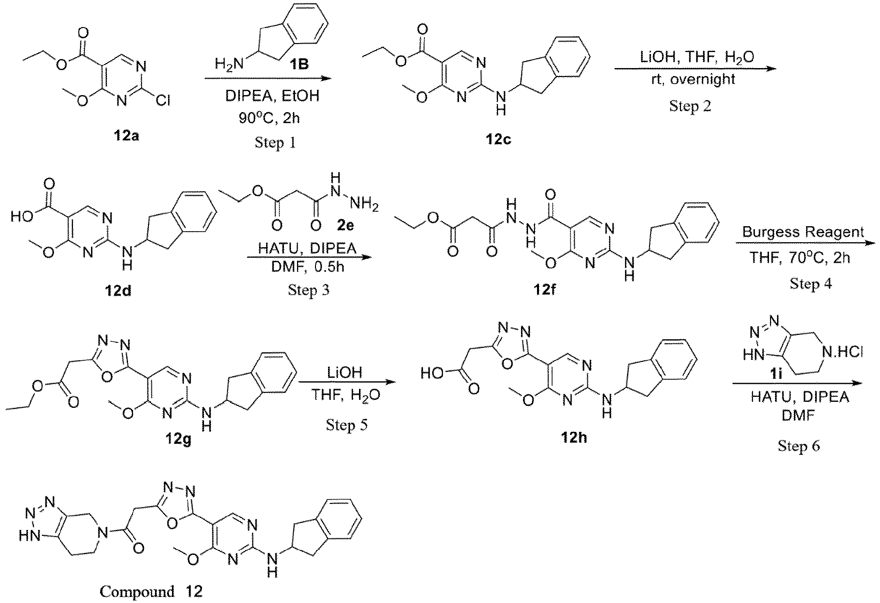

### Step 1: ethyl 2-((2,3-dihydro-1H-inden-2-yl)amino)-4-methoxypyrimidine-5-carboxylate (12c)

Ethyl 2-chloro-4-methoxypyrimidine-5-carboxylate (**12a**) (2.2 g, 10 mmol), 2-aminoindan (**1B**) (1.33g, 10 mmol) and N,N-diisopropylethylamine (2.6 g, 20 mmol) were dissolved in ethanol (20 mL), and stirred at 90°C for 2 hours. After the reaction was completed, the mixture was cooled to room temperature, and the resulting solid was filtered, washed with ethanol (20 mL), and dried to obtain the title compound (**12c**) as a beige solid (1.4 g, 45%). LC-MS (ESI): m/z = 314.1 [M+H]⁺.

### Step 2: 2-((2,3-dihydro-1H-inden-2-yl)amino)-4-methoxypyrimidine-5-carboxylic acid (12d)

Lithium hydroxide (1.08 g, 27 mmol) was added to a solution of compound ethyl 2-((2,3-dihydro 1H-inden 2-yl)amino)-4-methoxypyrimidine-5-carboxylate (**12c**) (1.2 g, 4.5 mmol) in tetrahydrofuran (30 mL) and distilled water (10 mL), and the reaction mixture was stirred at room temperature for 15 hours. After the reaction was completed, 2N hydrochloric acid aqueous solution was added thereto to adjust the pH value to 3, and then the resulting solution was extracted with ethyl acetate. The organic layer was washed with saturated brine, dried over anhydrous sodium sulfate, and concentrated to obtain the title compound (**12d**) (1.05 g) as a light yellow solid. LC-MS (ESI): m/z = 286.2 [M+H]⁺

### Step 3: ethyl 3-(2-(2-((2,3-dihydro-1H-inden-2-yl)amino)-4-methoxypyrimidine-5-carbonyl)hydrazinyl)-3-oxopropanoate (12f)

At 0°C, compound (**2e**) (0.65 g, 4 mmol), N,N-diisopropylethylamine (1.3 g, 10 mmol) and HATU (1.52 g, 4 mmol) were sequentially added slowly to a solution of compound (**12d**) (1.05 g, 3.7 mmol) in N,N-dimethylformamide (30 mL), and the mixture was stirred at room temperature under nitrogen flow for 1 hour. After the reaction was completed, the reaction mixture was cooled to room temperature, diluted with distilled water (30 mL), and extracted with ethyl acetate. The organic layer was washed with saturated brine, dried over anhydrous sodium sulfate and concentrated. The silica gel column chromatography was used for separation to obtain the title compound (**12f**) as a white solid (1.21 g, 79%). LC-MS (ESI): m/z = 414.2 [M+H]⁺

### Step 4: ethyl 2-(5-(2-((2,3-dihydro-1H-inden-2-yl)amino)-4-methoxypyrimidin-5 -yl)-1,3,4-oxadiazol-2-yl)acetate (12g)

At 0°C, methyl N-(triethylamidosulfonyl)carbamate (1.6 g, 5.5 mmol) was added to a solution of compound (**12f**) (1.21 g, 2.9 mmol) in anhydrous tetrahydrofuran (50 mL), and the mixture was stirred at 70°C under nitrogen atmosphere for 2 hours. After the reaction was completed, the mixture was cooled to room temperature, diluted with distilled water (80 mL), and extracted with ethyl acetate. The organic layer was washed with saturated brine, dried over anhydrous sodium sulfate and concentrated. The residue was separated and purified by silica gel column chromatography to obtain the title compound (**12g**) as a yellow solid (0.86 g, 75%).

LC-MS (ESI): m/z = 396.2 [M+H]⁺

### Step 5: 2-(5-(2-((2,3-dihydro-1H-inden-2-yl)amino)-4-methoxypyrimidin-5-yl)-1,3,4-oxadiazol-2-yl)acetic acid (12h)

Lithium hydroxide (0.44 g, 11 mmol) was added to a solution of compound (**12g**) (0.86 g, 2.2 mmol) in a mixed solvent of tetrahydrofuran (10 mL) and distilled water (5 mL), and the mixture was stirred at room temperature for 2 hour. After the reaction was completed, the mixture was treated with 2N hydrochloric acid aqueous solution to adjust to pH 2 or less, and then extracted with ethyl acetate. The organic layer was washed with saturated brine, dried over anhydrous sodium sulfate, and concentrated. The residue was slurried with diethyl ether to obtain target compound **12h** as a yellow solid (0.59 g, 73%). LC-MS (ESI): m/z = 368.2 [M+H]⁺

### Step 6: 1-(6,7-dihydro-1H-[1,2,3]triazolo[4,5-c]pyridin-5(4H)-yl)-2-(5-(2-((2,3-dihydro-1H-inden-2-yl)amino)-4-methoxypyrimidin-5-yl)-1,3,4-oxadiazol-2-yl)ethanone (compound 12)

At 0°C, intermediate **1i** (0.11 g, 0.66 mmol), N,N-diisopropylethylamine (0.5 mL) and HATU (0.14 g, 0.36 mmol) were sequentially added slowly to a solution of compound (**12h**) (0.12 g, 0.33 mmol) in N,N-dimethylformamide (10 mL), and the mixture was stirred at room temperature under nitrogen protection for 3 h. After the reaction was completed, the reaction mixture was diluted with distilled water (30 mL), and extracted with ethyl acetate (50 mL × 2). The organic layer was washed with saturated brine (50 mL), dried over anhydrous sodium sulfate and concentrated. The silica gel column chromatography was used for separation and purification to obtain **compound 12** (14 mg).

LC-MS (ESI): m/z = 474.2 [M+H]⁺.

¹H NMR (400 MHz, CDCl₃) *δ* 8.61 (s, 1H), 8.24 (s, 1H), 7.79 (s, 1H), 7.33 - 7.12 (m, 4H), 4.87 (s, 2H), 4.19 (s, 3H), 4.06 - 3.85 (m, 3H), 3.49 - 3.40 (m, 4H), 3.10 - 2.85 (m, 4H).

### Example 13: 2-((2,3-dihydro-1H-inden-2-yl)amino)-5-(5-(2-oxo-2-(1,4,6,7-tetrahydro-5H-[1,2,3]triazolo[4,5-c]pyridin-5-yl)ethyl)-1,3,4-oxadiazol-2-yl)nicotinonitrile (compound 13)

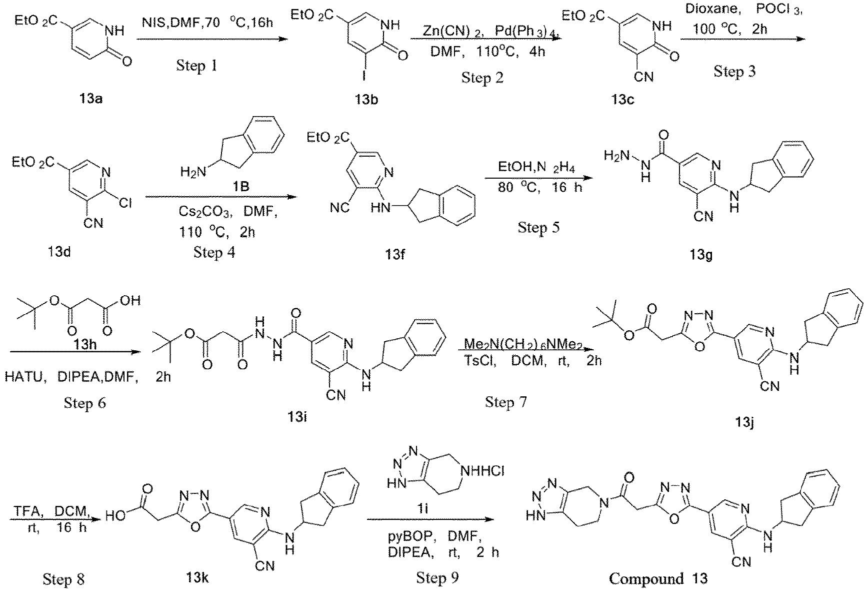

### Step 1: ethyl 5-iodo-6-oxo-1,6-dihydropyridine-3-carboxylate (13b)

N-iodosuccinimide (14.8 g, 65.8 mmol) was added to solution of compound **13a** (10.0 g, 59.8 mmol) in N,N-dimethylformamide (100 mL), and the mixture was stirred at 70°C for 16 hours. After the reaction was completed, the mixture was diluted with distilled water (30 mL), and extracted with ethyl acetate (100 mL × 3). The organic phase was washed with saturated brine (50 mL), dried over anhydrous sodium sulfate and concentrated. The silica gel column chromatography (eluent: petroleum ether : ethyl acetate = 1 : 0-0 : 1) was used for separation and purification to obtain the title compound **13b** as a white solid (10.0 g, 57%). LC-MS (ESI): m/z = 294.0 [M+H]⁺. ¹H NMR (400 MHz, DMSO-d₆) *δ* 12.48 (s, 1H), 8.36 (s, 1H), 8.08 (s, 1H), 4.24 (q, 2H), 1.28 (t, 3H).

### Step 2: ethyl 5-cyano-6-oxo-1,6-dihydropyridine-3-carboxylate (13c)

Zinc cyanide (4.0 g, 34.1 mmol) and tetrakis(triphenylphosphine)palladium (0.99 g, 0.86 mmol) were added to a solution of compound **13b** (5.0 g, 17.1 mmol) in N,N-dimethylformamide (10 mL), and the mixture was stirred at 110°C for 4 hours. After the reaction was completed, the mixture was diluted with water (100 mL), and extracted with ethyl acetate (100 mL × 4). The organic phase was washed with saturated brine, dried over anhydrous sodium sulfate and concentrated. The silica gel column chromatography (eluent: petroleum ether : ethyl acetate = 1 : 0-0 : 1) was used for separation and purification to obtain the title compound **13c** as a white solid (1.3 g, 40%).

LC-MS (ESI): m/z = 193.1 [M+H]⁺.

¹H NMR (400 MHz, DMSO-d₆) *δ* 13.12 (s, 1H), 8.46 (s, 1H), 8.34 (s, 1H), 4.26 (q, 2H), 1.29 (t, 3H).

### Step 3: ethyl 6-chloro-5-cyanonicotinate (13d)

Phosphorus oxychloride (6 ml) was added to a solution of compound **13c** (1.3 g, 6.8 mmol) in 1,4-dioxane (10 mL), and the mixture was stirred at 100°C for 2 hours. After the reaction was completed, the reaction solution was concentrated. The silica gel column chromatography (eluent: petroleum ether : ethyl acetate = 1 : 0-1 : 1) was used for separation and purification to obtain the title compound **13d** as a white solid (0.9 g, 64%). LC-M S(ESI): m/z = 211.1 [M+H]⁺. ¹H NMR (400 MHz, DMSO-d₆) *δ* 9.12 (s, 1H), 8.91 (s, 1H), 4.38 (q, 2H), 1.35 (t, 3H).

### Step 4: ethyl 5-cyano-6-((2,3-dihydro-1H-inden-2-yl)amino)nicotinate (13f)

**1B** (1.1 g, 6.4 mmol) and cesium carbonate (2.8 g, 8.5 mmol) were added to a solution of compound **13d** (0.9 g, 4.2 mmol) in N,N-dimethylformamide (10 mL), and the mixture was stirred at 110°C for 2 hours. After the reaction was completed, the mixture was diluted with distilled water (30 mL), and extracted with ethyl acetate (30 mL × 3). The organic phase was washed with saturated brine, dried over anhydrous sodium sulfate and concentrated. The silica gel column chromatography (eluent: petroleum ether : ethyl acetate = 1 : 0-0 : 1) was used for separation and purification to obtain the title compound **13f** as an off-white solid (1.25 g, 96%).

LC-MS (ESI): m/z = 308.2 [M+H]⁺.

¹H NMR (400 MHz, DMSO-d₆) *δ* 8.81 (s, 1H), 8.32 (s, 1H), 8.06 (s, 1H), 7.23 - 7.20 (m, 2H), 7.17 - 7.14 (m, 2H), 4.96 - 4.92 (m, 1H), 4.28 (q, 2H), 3.25 (dd, 2H), 3.06 (dd, 2H), 1.30 (t, 3H).

### Step 5: 5-cyano-6-((2,3-dihydro-1H-inden-2-yl)amino)nicotinohydrazide (13g)

Hydrazine hydrate (4 ml) was added to a solution of compound **13f** (0.7 g, 2.0 mmol) in ethanol (10 mL), and the mixture was reacted at 80°C for 16 hours. After the reaction was completed, the reaction solution was filtered, washed with ethanol and spun to dryness to obtain the title compound **13g** as a grey solid (350 mg, 63%). LC-MS (ESI): m/z = 294.1 [M+H]⁺. ¹H NMR (400 MHz, DMSO-d₆) *δ* 8.81 (s, 1H), 8.32 (s, 1H), 8.06 (s, 1H), 7.23 - 7.20 (m, 2H), 7.17 - 7.14 (m, 2H), 4.96 - 4.92 (m, 1H), 4.28 (q, 2H), 3.25 (dd, 2H), 3.06 (dd, 2H), 1.30 (t, 3H).

### Step 6: tert-butyl 3-(2-(5-cyano-6-((2,3-dihydro-1H-inden-2-yl)amino)nicotinoyl)hydrazinyl)-3-oxopropanoate (13i)

13h (0.23 g, 1.4 mmol), 2-(7-azabenzotriazole)-N,N,N',N'-tetramethyluronium hexafluorophosphate (0.54 g, 1.4 mmol) and N,N-diisopropylethylamine (0.39 g, 3.0 mmol) were added to a solution of compound **13g** (0.35 g, 1.2 mmol) in N,N-dimethylformamide (6 mL), and the mixture was reacted at room temperature for 2 hours. After the reaction was completed, the mixture was diluted with distilled water (30 mL), and extracted with dichloromethane (20 mL × 3). The organic phase was washed with distilled water and saturated brine, dried over anhydrous sodium sulfate and concentrated. Title compound **13i** as a brown solid (720 mg) was obtained. LC-MS (ESI): m/z = 434.1 [M-H]⁺.

### Step 7: tert-butyl 2-(5-(5-cyano-6-((2,3-dihydro-1H-inden-2-yl)amino)pyridin-3-yl)-1,3,4-oxadiazol-2-yl)acetate (13j)

4-toluene sulfonyl chloride (0.63 g, 3.3 mmol) and N,N,N',N'-tetramethyl-1,6-hexanediamine (0.57 g, 3.3 mmol) were added to a solution of compound **13i** (0.72 g, 1.7 mmol) in dichloromethane (10 mL), and the mixture was reacted at room temperature for 2 hours. After the reaction was completed, the mixture was diluted with dichloromethane (30 mL). The organic phase was washed with saturated brine, dried over anhydrous sodium sulfate and concentrated. The title compound **13j** as a grey solid (150 mg, two-step yield: 30%) was obtained. LC-MS (ESI): m/z = 418.2 [M+H]⁺. ¹H NMR (400 MHz, DMSO-d₆) *δ* 8.87 (s, 1H), 8.42 (s, 1H), 8.03 (d, 1H), 7.24 - 7.22 (m, 2H), 7.17 - 7.15 (m, 2H), 4.98 - 4.92 (m, 1H), 8.12 (s, 2H), 3.27 (dd, 2H), 3.06 (dd, 2H), 1.43 (s, 9H).

### Step 8: 2-(5-(5-cyano-6-((2,3-dihydro-1H-inden-2-yl)amino)pyridin-3-yl)-1,3,4-oxadiazol-2-yl)acetic acid (13k)

TFA (4 ml) was added to a solution of compound **13j** (0.15 g, 0.36 mmol) in dichloromethane (6 mL), and the mixture was reacted at room temperature for 16 hours. After the reaction was completed, the reaction solution was diluted with dichloromethane (20 mL). The organic phase was washed with saturated brine, dried over anhydrous sodium sulfate, and concentrated to obtain the title compound **13k** as a grey solid (120 mg, 92%). LC-MS (ESI): m/z = 362.1 [M+H]⁺

### Step 9: 2-((2,3-dibydro-1H-inden-2-yl)amino)-5-(5-(2-oxo-2-(1,4,6,7-tetrahydro-5H-[1,2,3]triazolo[4,5-c]pyridin-5-yl)ethyl)-1,3,4-oxadiazol-2-yl)nicotinonitrile (compound 13)

Compound **1i** (0.11 g, 0.66 mmol), N,N-diisopropylethylamine (0.21 g, 1.7 mmol) and benzotriazol-1-yl-oxy-tripyrrolidinophosphonium hexafluorophosphate (0.26 g, 0.50 mmol) were sequentially added slowly to a solution of compound **13k** (0.12 g, 0.33 mmol) in N,N-dimethylformamide (6 mL), and the mixture was stirred at room temperature for 2 hours. After the reaction was completed, dichloromethane (20 mL) was added to the reaction solution, and the resulting mixture was diluted with water (20 mL), and extracted with dichloromethane (20 mL × 3). The organic phase was washed with saturated brine, dried over anhydrous sodium sulfate and concentrated. The silica gel column chromatography (eluent: dichloromethane : ethyl acetate : methanol = 1 : 0 : 0-2 : 10 : 1) was used for separation and purification to obtain white **compound 13** (20 mg, 13%). LC-MS (ESI): m/z = 468.2 [M+H]+. ¹H NMR (400 MHz, DMSO-d₆) *δ* 8.84 (dd, 1H), 8.39 (dd, 1H), 8.02 (d, 1H), 7.24 - 7.20 (m, 2H), 7.17 - 7.14 (m, 2H), 4.98 - 4.92 (m, 1H), 4.81 (s, 1H), 4.68 (s, 1H), 4.41 (d, 1H), 3.27 (dd, 2H), 3.06 (dd, 2H), 2.91 (t, 1H), 2.75 (t, 1H).

### Example 14: N-(1-(5-(2-((2,3-dihydro-1H-inden-2-yl)amino)pyrimidin-5-yl)-1,3,4-oxadiazol-2-yl)cyclopropyl)-1,4,6,7-tetrahydro-5H-[1,2,3]triazolo[4,5-c]pyridine-5-carboxamide (compound 14)

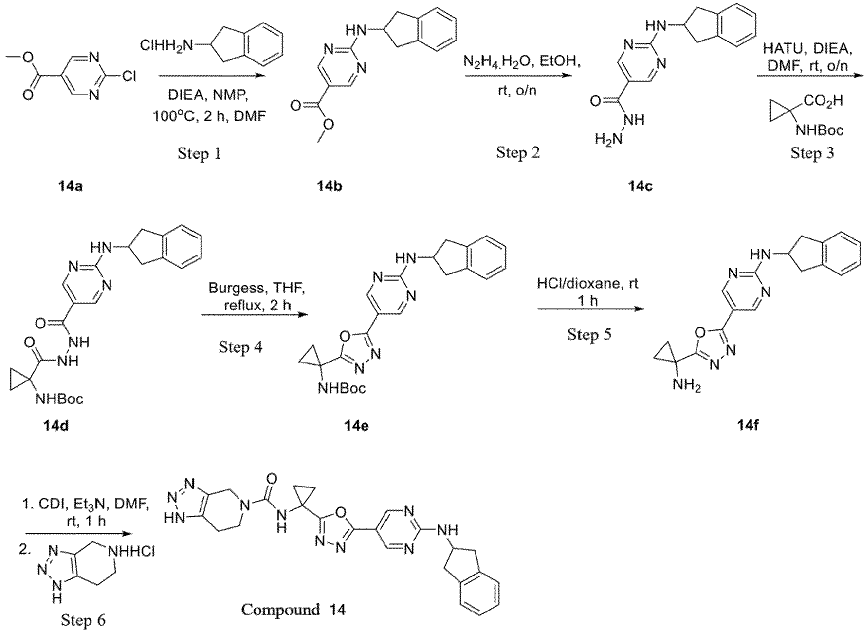

### Step 1: methyl 2-((2,3-dihydro-1H-inden-2-yl)amino)pyrimidine-5-carboxylate (14b)

Starting material **14a** (8.63 g, 50 mmol) was dissolved in 200 mL of NMP, and then 2,3-dihydro-1H-inden-2-amine hydrochloride (8.48 g, 50 mmol) and DIEA (14.2 g, 110 mmol) were added. The mixture was stirred and reacted at 100°C for 2 hours. The reaction solution was poured into water (600 mL). A large number of solids were precipitated and filtered. The filter cake was dried to obtain compound **14b** (9.8 g, 73%). LC-MS (ESI): m/z = 270.2 [M+H]⁺.

### Step 2: 2-((2,3-dihydro-1H-inden-2-yl)amino)pyrimidine-5-carbohydrazide (14c)

Compound **14b** (8.5 g, 31.6 mmol) and ethanol (100 mL) were added into a 250 mL single-necked flask, and then hydrazine hydrate (39.5 g, 632 mmol) was added. The mixture was stirred at room temperature overnight. A large number of solids were precipitated and then filtered. The filter cake was dried to obtain compound **14c** (6.8 g, 80%). LC-MS (ESI): m/z = 270.2 [M+H]⁺.

### Step 3: tert-butyl (1-(2-(2-((2,3-dihydro-1H-inden-2-yl)amino)pyrimidine-5-carbonyl)hydrazine-1-carbonyl)cyclopropyl)carbamate (14d)

Compound **14c** (6.8 g, 25.28 mmol) was dissolved in DMF (80 mL), and then HATU (14.41 g, 37.92 mmol) and DIPEA (9.78 g, 75.84 mmol) were added. The mixture was stirred and reacted for 10 minutes, and then 1-((tertbutoxycarbonyl)amino)cyclopropane-1-carboxylic acid (5.09 g, 25.28 mmol) was added. The mixture was stirred at room temperature overnight. The reaction solution was poured into water (250 mL). A large number of solids were precipitated and filtered. The filter cake was dried to obtain compound **14d** (8.0 g 70%). LC-MS (ESI): m/z = 453.2 [M+H]⁺.

### Step 4: tert-butyl (1-(5-(2-((2,3-dihydro-1H-inden-2-yl)amino)pyrimidin-5-yl)-1,3,4-oxadiazol-2-yl)cyclopropyl)carbamate (14e)

Compound **14d** (4.0 g, 8.85 mmol) was dissolved in anhydrous tetrahydrofuran (50 mL), and Burgess reagent (4.22 g, 17.7 mmol) was added. The mixture was heated to 70°C and reacted under nitrogen protection for 1 h. After the reaction was completed, water was added and the resulting solution was extracted with ethyl acetate. The organic layer was washed with saturated brine, dried over anhydrous sodium sulfate, concentrated, and separated and purified by silica gel column chromatography (PE/EA = 1/1) to obtain compound **14e** (2.1 g, 55%). LC-MS (ESI): m/z = 435.1 [M+H]⁺.

### Step 5: 5-(5-(1-aminocyclopropyl)-1,3,4-oxadiazol-2-yl)-N-(2,3-dihydro-1H-inden-2-yl)pyrimidin-2-amine (14f)

At room temperature, a solution (10 mL) of 4N hydrochloric acid in 1,4-dioxane was added to compound **14e** (500 mg, 1.15 mmol). The mixture was stirred and reacted for 1 hour. After the reaction was completed, the reaction solution was concentrated to obtain **14f** (400 mg, a crude product), which was directly used in the next step. LC-MS (ESI): m/z = 335.2 [M+H]⁺.

### Step 6: N-(1-(5-(2-((2,3-dihydro-1H-inden-2-yl)amino)pyrimidin-5-yl)-1,3,4-oxadiazol-2-yl)cyclopropyl)-1,4,6,7-tetrahydro-5H-[1,2,3]triazolo[4,5-c]pyridine-5-carboxamide (compound 14)

Compound **14f** (400 mg, 1.19 mmol) was dissolved in 5 ml of DMF, and triethylamine (721 mg, 7.14 mmol) and CDI (193 mg, 1.19 mmol) were added. The mixture was stirred at room temperature for 1 h, and then 4,5,6,7-tetrahydro-1H-[1,2,3]triazolo[4,5-c]pyridine hydrochloride (191 mg, 1.19 mmol) was added. The resulting mixture was reacted overnight, and poured into water. Solids were precipitated. The solids were directly separated and purified by silica gel column chromatography (MeOH/DCM = 1/8) to obtain compound **14** (70 mg, 12%).

LC-MS (ESI): m/z = 485.2 [M+H]⁺.

¹H NMR (400 MHz, DMSO-d₆) *δ* 8.75 (d, 2H), 8.35 (d, 1H), 7.87 (s, 1H), 7.23 - 6.95 (m, 4H), 4.73 - 4.67 (m, 1H), 4.55 (s, 2H), 3.70 - 3.67 (m, 2H), 3.29 - 3.25 (m, 2H), 2.96-2.91 (m, 2H), 2.75-2.74 (m, 2H), 1.55 -1.52 (m, 2H), 1.31-1.28 (m, 2H).

### Example 15: (1-(5-(2-((2,3-dihydro-1H-inden-2-yl)amino)pyrimidin-5-yl)-1,3,4-oxadiazol-2-yl)cyclopropyl)(3,4,6,7-tetrahydro-5H-imidazo[4,5-c]pyridin-5-yl)methanone (compound15)

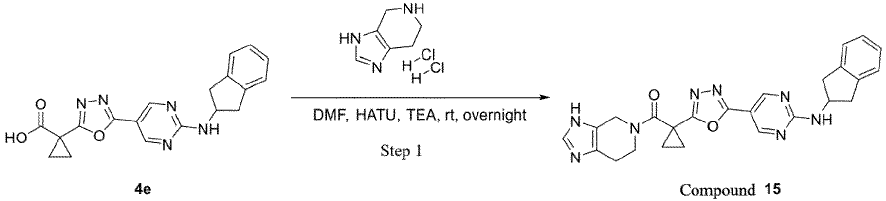

### Step 1: (1-(5-(2-((2,3-dihydro-1H-inden-2-yl)amino)pyrimidin-5-yl)-1,3,4-oxadiazol-2-yl)cyclopropyl)(3,4,6,7-tetrahydro-5H-imidazo[4,5-c]pyridin-5-yl)methanone (compound 15)

**4e** (100 mg, 0.28 mmol) was dissolved in DMF (2 mL). The resulting solution was cooled to 0°C in an ice bath. 4,5,6,7-tetrahydro-3H-imidazo[4,5-c]pyridine dihydrochloride (70 mg, 0.36 mmol) was added with stirring. HATU (136 mg, 0.36 mmol) was added, triethylamine (0.4 mL, 2.75 mmol) was added dropwise, and the mixture was stirred at room temperature overnight. Water (50 mL) was add and the resulting mixture was extracted with ethyl acetate (50 mL × 2). The organic phase was combined, and washed once with water (100 mL), dried over sodium sulfate, filtered and spun to dryness. The residue was separated and purified by silica gel column chromatography to obtain compound **15** (30 mg, 23%).

LC-MS (ESI): m/z = 469.2 [M+H]⁺.

¹H NMR (400 MHz, DMSO-d₆) δ 11.82 (s, 1H), 8.77 (m, 2H), 8.47 - 8.27 (m, 1H), 7.48 (s, 1H), 7.33 - 7.18 (m, 2H), 7.18 - 7.03 (m, 2H), 4.79 - 4.61 (m, 1H), 4.48 (s, 2H), 3.81 (s, 2H), 3.29 - 3.23 (m, 2H), 2.93 (dd, 2H), 2.64 - 2.52 (m, 2H), 1.72 - 1.63 (m, 2H), 1.63 - 1.48 (m, 2H).

### Example 16: N-(1H-benzo[d][1,2,3]triazol-5-yl)-1-(5-(2-((2,3-dihydro-1H-inden-2-yl)amino)pyrimidin-5-yl)-1,3,4-oxadiazol-2-yl)cyclopropane-1-carboxamide (compound 16)

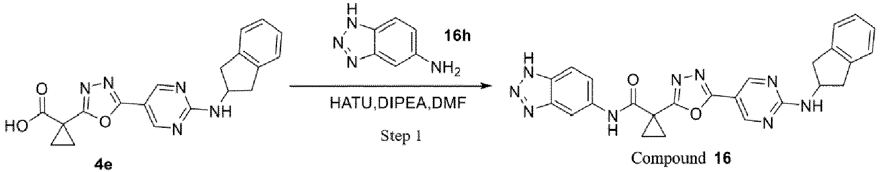

### Step 1: N-(1H-benzo[d][1,2,3]triazol-5-yl)-1-(5-(2-((2,3-dibydro-1H-inden-2-yl)amino)pyrimidin-5-yl)-1,3,4-oxadiazol-2-yl)cyclopropane-1-carboxamide (compound 16)

Compound **4e** (130 mg, 0.3577 mmol) and **16h** (72 mg, 0.5366 mmol) were dissolved in DMF, HATU (272 mg, 0.7155 mmol) was added, and DIPEA (0.25 ml, 13.789 mmol) was added dropwise. The mixture was reacted at room temperature. After the reaction was completed, the mixture was diluted with water, and extracted with ethyl acetate. The organic layer was washed with saturated brine, dried over anhydrous sodium sulfate, and concentrated. The silica gel column chromatography was used for separation and purification to obtain **compound 16** (65 mg, 37.89%).

LC-MS (ESI): m/z = 480.2 [M+H]⁺.

¹H NMR (400 MHz, DMSO-d₆) *δ* 8.80 (d, 2H), 8.39 (t, 1H), 7.75 (d, 1H), 7.25 - 7.19 (m, 4H), 7.17 - 7.13 (m, 2H), 7.09 (s, 1H), 6.96 (s, 1H), 6.83 (dd, 1H), 4.69 (dd, 1H), 3.27 (dd, 2H), 2.93 (dd, 2H), 2.07 (dd, 2H), 1.97 (dd, 2H).

### Example 17: 2-(5-(2-((2,3-dihydro-1H-inden-2-yl)amino)pyrimidin-5-yl)-1,3,4-oxadiazol-2-yl)-2,2-difluoro-1-(1,4,6,7-tetrahydro-5H-[1,2,3]triazolo[4,5-c]pyridin-5-yl)ethan-1-one (compound 17)

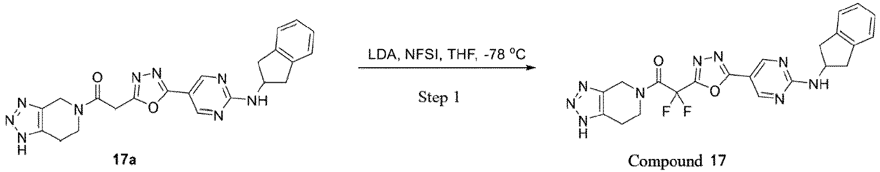

Diisopropylamine (0.038 g, 0.38 mmol) and tetrahydrofuran (4 ml) were added to a flask, and n-BuLi (0.15 ml, 0.38 mmol) was added dropwise at -78°C. The mixture was stirred for 0.5 h, and then a solution of compound **17a** (0.036 g, 0.075 mmol) in tetrahydrofuran (2 ml) was added dropwise to the flask and stirred at -78°C for 0.5 h. Then a solution of N-fluorodibenzenesulfonimide (0.12 g, 0.38 mmol) in tetrahydrofuran (2 ml) was added dropwise to the reaction solution and stirred at -78°C for 0.5 h. Then the mixture was continuously stirred to room temperature. After the reaction was completed, the reaction mixture was quenched with saturated ammonium chloride (20 mL) solution, and extracted with dichloromethane (30 mL×3). The organic phase was washed with saturated brine, dried over anhydrous sodium sulfate and concentrated. The silica gel column chromatography (eluent: dichloromethane : ethyl acetate : methanol = 1 : 0 : 0-2 : 10 :1) was used for separation and purification to obtain **compound 17** (7 mg, 18%).

LC-MS (ESI): m/z = 480.1 [M+H]⁺.

¹H NMR (400 MHz, DMSO-d₆) δ 8.95 (s, 1H), 8.89 (s, 1H), 8.59 (d, 2H), 7.24 - 7.22 (m, 2H), 7.18 - 7.14 (m, 2H), 4.84 (s, 2H), 4.76 - 4.71 (m, 1H), 3.99 (s, 1H), 3.93 (s, 1H), 3.29 (dd, 2H), 2.95 (dd, 2H), 2.87 - 2.83 (m, 2H).

### Example 18: (1-(5-(2-((2,3-dihydro-1H-inden-2-yl)amino)pyrimidin-5-yl)-1,3,4-oxadiazol-2-yl)cyclopropyl)(4-(methylsulfonyl)piperazin-1-yl)methanone (compound 18)

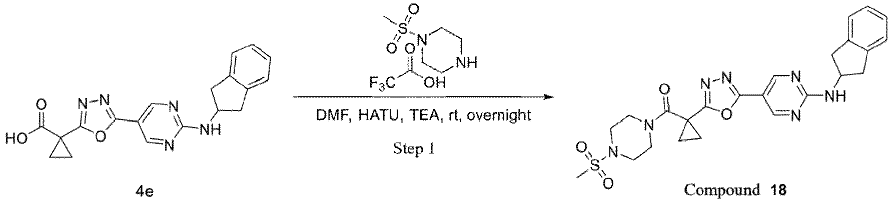

### Step 1: (1-(5-(2-((2,3-dihydro-1H-inden-2-yl)amino)pyrimidin-5-yl)-1,3,4-oxadiazol-2-yl)cyclopropyl)(4-(methylsulfonyl)piperazin-1-yl)methanone (compound 18)

**4e** (100 mg, 0.28 mmol) was dissolved in DMF (2 mL). The resulting solution was cooled to 0°C in an ice bath. 1-(methylsulfonyl)piperazine trifluoroacetic acid salt (100 mg, 0.36 mmol) was added with stirring. HATU (136 mg, 0.36 mmol) was added, triethylamine (0.4 mL, 2.75 mmol) was added dropwise, and the mixture was stirred at room temperature overnight. Water (50 mL) was added. The mixture was stirred uniformly. Solids was precipitated and filtered by suction. The solids were separated and purified by silica gel column chromatography to obtain compound **18** (20 mg, 14%).

LC-MS (ESI): m/z = 510.3 [M+H]⁺.

¹H NMR (400 MHz, DMSO-d₆) δ 8.83 (d, 2H), 8.39 (d, 1H), 7.25 - 7.19 (m, 2H), 7.18 - 7.11 (m, 2H), 4.76 - 4.64 (m, 1H), 3.73 - 3.54 (m, 4H), 3.34 - 3.30 (m, 1H), 3.28 - 3.23 (m, 1H), 3.20 - 3.07 (m, 4H), 2.94 (dd, 2H), 2.89 (s, 3H), 1.73 - 1.62 (m, 2H), 1.62 - 1.49 (m, 2H).

### Example 19: N-(2-((2,3-dihydro-1H-inden-2-yl)amino)pyrimidin-5-yl)-2-(1,4,6,7-tetrahydro-5H-[1,2,3]triazolo[4,5-c]pyridin-5-yl)oxazole-4-carboxamide (compound 19)

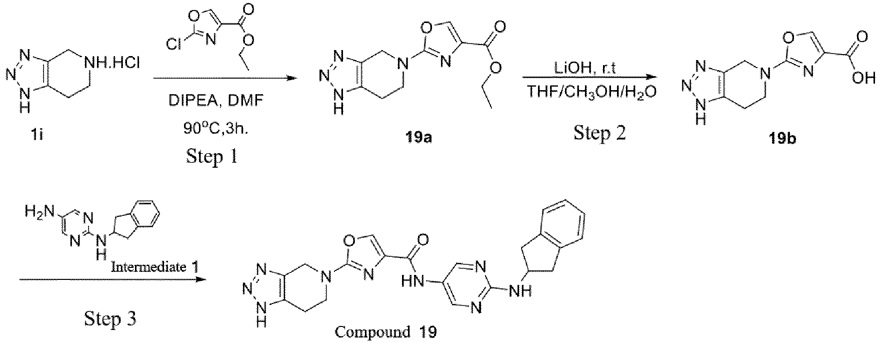

### Step 1: Ethyl 2-(1,4,6,7-tetrahydro-5H-[1,2,3]triazolo[4,5-c]pyridin-5-yl)oxazole-4-carboxylate (19a)

Ethyl 2-chlorooxazole-4-carboxylate (350 mg, 2 mmol), compound 1i (320 mg, 2 mmol) and N,N-diisopropylethylamine (1.3 g, 10 mmol) were dissolved in DMF (10 mL), and the mixture was stirred at 90°C for 2 hours. After the reaction was completed, the mixture was cooled to room temperature, quenched by adding water (10 ml × 2), extracted with dichloromethane (20 ml × 2), washed with saturated brine (20 ml), dried, spun to dryness and separated and purified by silica gel column chromatography to obtain the title compound **(19a)** (350 mg, 67%).

LC-MS (ESI): m/z = 264.1 [M+H]⁺.

### Step 2: 2-(1,4,6,7-tetrahydro-5H-[1,2,3]triazolo[4,5-c]pyridin-5-yl)oxazole-4-carboxylic acid (19b)

Compound **19a** (350 mg, 1.33 mmol), tetrahydrofuran (2 mL), methanol (5 ml), distilled water (2 mL) and lithium hydroxide monohydrate (300 mg, 7 mmol) were successively added to a single-necked flask, and the mixture was stirred at room temperature for 3 h. The reaction was quenched by adding dilute hydrochloric acid and extracted with dichloromethane (50 mL × 2). The combined organic phase was dried over anhydrous sodium sulfate and concentrated under reduced pressure to obtain a crude product of compound **19b** (280 mg, 89%). The crude product can be directly used in the next reaction.

LC-MS (ESI): m/z = 236.1 [M+H]⁺.

### Step 3: N-(2-((2,3-dihydro-1H-inden-2-yl)amino)pyrimidin-5-yl)-2-(1,4,6,7-tetrahydro-5H-[1,2,3]triazolo[4,5-c]pyridin-5-yl)oxazole-4-carboxamide (compound 19)

Under nitrogen protection, compound **19b** (100 mg, 0.42 mmol), DMF (10 mL), HATU (190 mg, 0.5 mmol), DIPEA (91 mg, 0.7 mmol) and **intermediate 1** (113 mg, 0.5 mmol) were successively added to a single-necked flask, and the mixture was stirred at room temperature for 3 h. The reaction was quenched by adding aqueous solution (30 mL), extracted and allowed to stand for phase separation. The aqueous phase was washed with dichloromethane (100 mL × 2). The combined organic phase was dried over anhydrous sodium sulfate and concentrated under reduced pressure to obtain a crude product which was separated and purified by silica gel column chromatography to obtain compound **19** (20 mg, 11%).

LC-MS (ESI): m/z = 444.2 [M+H]⁺.

¹H NMR (400 MHz, DMSO-d₆) δ 7.81 (s, 3H), 7.27 (s, 1H), 6.42-6.41 (m, 3H), 6.35-6.33 (m, 3H), 3.94-3.91 (m, 2H), 3.20-3.17 (m, 4H), 2.19-2.10 (m, 5H).

### Example 20: (1-(5-(2-((2,3-dihydro-1H-inden-2-yl)amino)pyrimidin-5-yl)-1,3,4-oxadiazol-2-yl)cyclobutyl)(1,4,6,7-tetrahydro-5H-[1,2,3]triazolo[4,5-c]pyridin-5-yl)methanone (compound 20)

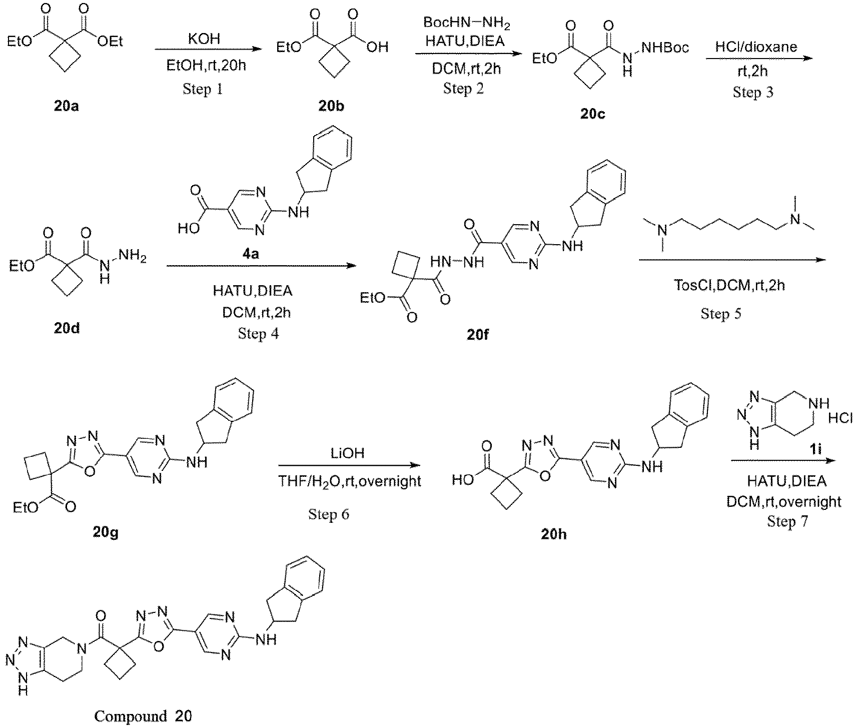

### Step 1: 1-(ethoxycarbonyl)cyclobutane-1-carboxylic acid (20b)

At room temperature, ethanol (44 mL) was added to known compound **20a** (10.0 g, 50 mmol), and then potassium hydroxide (2.8 g, 50 mmol) was added. The mixture was stirred at room temperature for 16 h and adjusted to pH to 5-6 with 6N HCl, water was added and the resulting solution was extracted with EA The EA phase was washed with saturated brine, dried over anhydrous sodium sulfate, and concentrated under reduced pressure to obtain **20b** (8.5 g, 99%). LC-MS (ESI): m/z = 173.1 [M+H]⁺.

### Step 2: tert-butyl-2-(1-(ethoxycarbonyl)cyclobutane-1-carbonyl)hydrazine-1-carboxylate (20c)

Dichloromethane (30 mL), tert-butoxycarbonyl hydrazine (2.5 g, 19.2 mmol), HATU (7.3 g, 19.2 mmol) and DIEA(6.7 g, 52.2 mmol) were added to compound **20b** (3.0 g, 17.4 mmol) at room temperature. The mixture was stirred at room temperature for 2 hours. Water was added and the resulting solution was extracted with DCM. The DCM phase was washed with saturated brine, dried over anhydrous sodium sulfate, and concentrated under reduced pressure. Then the residue was separated and purified by silica gel column chromatography (PE : EA = 5 : 1) to obtain **20c** (4.0 g, 81%). LC-MS (ESI): m/z = 231.1 [M+H-56]⁺.

### Step 3: ethyl 1-(hydrazinecarbonyl)cyclobutane-1-carboxylate (20d)

HCl/dioxane solution (50 mL) was added to compound **20c** (4.0 g, 14 mmol), and the mixture was stirred at room temperature for 2 hours, and concentrated under reduced pressure to obtain **20d** (2.6 g, a crude product).

### Step 4: ethyl 1-(2-(2-((2,3-dihydro-1H-inden-2-yl)amino)pyrimidine-5-carbonyl)hydrazine-1-carbonyl)cyclobutane-1-carboxylate (20f)

At room temperature, dichloromethane (20 mL), compound **20d** (1.6 g, 8.4 mmol), HATU (2.95 g, 7.7 mmol) and DIEA(1.6 g, 21.0 mmol) were successively added to intermediate **4a** (1.8 g, 7.0 mmol), and the mixture was stirred at room temperature for 2 hours. The reaction solution was diluted with water, and extracted with dichloromethane. The organic phase was washed with saturated brine (50 mL), dried over anhydrous sodium sulfate, and concentrated under reduced pressure. Then the residue was separated and purified by silica gel column chromatography (PE : EA= 2 : 1) to obtain **20f** (1.5 g, 50%). LC-MS (ESI): m/z = 424.2 [M+H]⁺.

### Step 5: Ethyl 1-(5-(2-((2,3-dihydro-1H-inden-2-yl)amino)pyrimidin-5-yl)-1,3,4-oxadiazol-2-yl)cyclobutane-1-carboxylate (20g)

At room temperature, dichloromethane (10 mL), N1,N1,N6,N6-tetramethylhexane-1,6-diamine (0.49 g, 2.8 mmol) and p-toluenesulfonyl chloride (0.54 g, 2.8 mmol) were successively added to compound **20f** (0.8 g, 1.9 mmol). The mixture was stirred at room temperature for 2 hours, diluted by adding water, and extracted with dichloromethane. The dichloromethane phase was washed with saturated brine (20 mL), dried over anhydrous sodium sulfate, and concentrated under reduced pressure. Then the residue was separated and purified with silica gel column chromatography (PE : EA= 1 : 1) to obtain **20g** (500 mg, 76.6%).

### Step 6: 1-(5-(2-((2,3-dihydro-1H-inden-2-yl)amino)pyrimidin-5-yl)-1,3,4-oxadiazol-2-yl)cyclobutane-1-carboxylic acid (20h)

At room temperature, tetrahydrofuran (5 mL), water (1 mL), and LiOH (0.26 g, 6.15 mmol) were successively added to compound **20g** (0.5 g, 1.23 mmol), and the mixture was stirred at room temperature for 2 hour and adjusted to pH to 5-6 with 1N dilute hydrochloric acid, and extracted with ethyl acetate. The ethyl acetate phase was washed with saturated brine (20 mL), dried over anhydrous sodium sulfate, concentrated under reduced pressure to obtain **20h** (0.45 g, 96.7%). LC-MS (ESI): m/z = 378.2 [M+H]⁺.

### Step 7: (1-(5-(2-((2,3-dihydro-1H-inden-2-yl)amino)pyrimidin-5-yl)-1,3,4-oxadiazol-2-yl)cyclobutyl)(1,4,6,7-tetrahydro-5H-[1,2,3]triazolo[4,5-c]pyridin-5-yl)methanone (compound 20)

At room temperature, DMF (2 mL), HATU (0.55 g, 1.44 mmol), **1i** (0.4 g, 2.5 mmol) and DIEA (0.67 g, 3.6 mmol) were successively added to compound **20h** (0.45 g, 1.2 mmol). The mixture was stirred at room temperature for 2 hours, diluted by adding water, and extracted with ethyl acetate. The organic phase was washed with saturated brine (20 mL), dried over anhydrous sodium sulfate, and concentrated under reduced pressure. Then the residue was separated and purified by silica gel column chromatography (DCM : MeoH = 100 : 1-20 : 1) to obtain **Compound 20** (0.012 g, 1.7%).

¹H NMR (400 MHz, CDCl₃) δ 12.36 (s, 1H), 8.86-8.47 (m, 2H), 7.18-7.16 (m, 2H), 7.19-7.10 (m, 2H), 6.18-5.94 (m, 1H), 4.82-4.78 (m, 2H), 4.44 (s, 1H), 3.91-3.89 (m, 1H), 3.59-3.57 (m, 1H), 3.37-3.31 (m, 2H), 2.97-2.77 (m, 7H), 2.57-2.55 (m, 1H), 2.21-2.13 (m, 1H), 1.95-1.93 (m, 1H). LC-MS (ESI): m/z = 484.2 [M+H]⁺.

### Compound 21: 2-(5-(2-((2,3-dihydro-1H-inden-2-yl)amino)oxazol-4-yl)-1,3,4-oxadiazol-2-yl)-1-(1,4,6,7-tetrahydro-5H-[1,2,3]triazolo[4,5-c]pyridin-5-yl)ethan-1-one (compound 21)

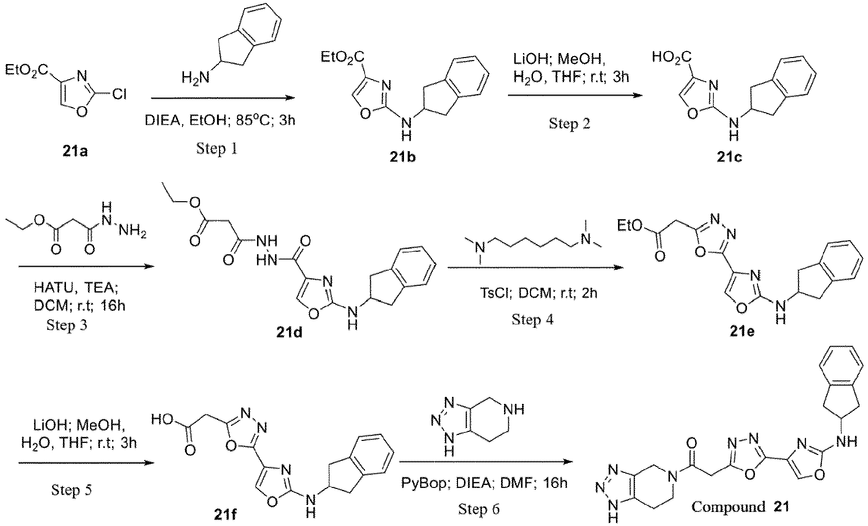

### Step 1: ethyl 2-((2,3-dihydro-1H-inden-2-yl)amino)oxazole-4-carboxylate (21b)

Compound **(21a)** (5.0 g, 26.5 mmol) was added to a 250 mL single-necked flask, and dissolved with ethanol (50 mL). DIEA (13.7 g, 106 mmol) and 2,3-dihydro-1H-inden-2-amine hydrochloride (6.75 g, 39.8 mmol) were added at room temperature. Upon completion of the addition, the mixture was warmed to 85°C and reacted for 3 hours. After the reaction was completed, the reaction solution was concentrated under reduced pressure. The crude product was directly used in the next step without purification. LC-MS (ESI): m/z = 273.1 [M+H]⁺.

### Step 2: 2-((2,3-dihydro-1H-inden-2-yl)amino)oxazole-4-carboxylic acid (21c)

Compound **(21b)** (7.0 g, 25.7 mmol) and LiOH (5.13 g, 129 mmol) were added to a 250 mL single-necked flask, and dissolved with methanol (50 mL), water (50 mL) and tetrahydrofuran (50 mL). The mixture was reacted at room temperature for 3 hours. After the reaction was completed, 100 mL of water was added. The resulting solution was concentrated under reduced pressure to remove the organic solvent, and then adjusted to pH = 2-3 with 4N hydrochloric acid. A large number of solids were precipitated and filtered. The filter cake was washed with water (20 mL × 3). The filter cake was azeotropically dried by distilling off toluene and water three times, and concentrated to dryness to obtain compound **21c** (5.0 g, 79.6%). LC-MS (ESI): m/z = 245.1 [M+H]⁺.

### Step 3: ethyl 3-(2-(2-((2,3-dihydro-1H-inden-2-yl)amino)oxazole-4-carbonyl)hydrazinyl)-3-oxopropanoate (21d)

Compound **21c** (2.0 g, 8.19 mmol) was added into a 100 mL single-necked flask, and dissolved with DCM (20 mL). HATU (4.05 g, 10.6 mmol), triethylamine (2.45 g, 24.6 mmol) and ethyl 3-hydrazinyl-3-oxopropanoate (1.56 g, 10.6 mmol) were added. The mixture was reacted at room temperature for 16 h, washed with aqueous solution (10 mL), stirred at room temperature for 10 min, and then extracted with DCM (5 mL × 3). The organic phase was combined, washed with saturated sodium chloride (5 mL × 1), dried over anhydrous sodium sulfate, and concentrated under reduced pressure to obtain compound **21d** (3.5 g, a crude product), which was directly used in the next reaction. LC-MS (ESI): m/z = 373.1 [M+H]⁺.

### Step 4: ethyl 2-(5-(2-((2,3-dihydro-1H-inden-2-yl)amino)oxazol-4-yl)-1,3,4-oxadiazol-2-yl)acetate (21e)

Compound **21d** (3.5 g) was added into a 100 mL single-necked flask, and dissolved with DCM (40 mL). TsCl (3.6 g, 19 mmol) and N¹,N¹,N⁶,N⁶-tetramethylhexane-1,6-diamine (3.2 g, 19 mmol) were added at room temperature, and then the mixture was reacted at room temperature overnight. The reaction solution was washed with aqueous solution (40 mL), stirred at room temperature for 10 min, and then extracted with DCM (50 mL × 3). The organic phase was combined, washed with saturated sodium chloride (5 mL × 1), dried over anhydrous sodium sulfate, concentrated under reduced pressure, and separated and purified by silica gel column chromatography to obtain compound **21e** (0.6 g, 1.7 mmol). LC-MS (ESI): m/z = 355.2 [M+H]⁺.

### Step 5: 2-(5-(2-((2,3-dihydro-1H-inden-2-yl)amino)oxazol-4-yl)-1,3,4-oxadiazol-2-yl)acetic acid (21f)

Compound **(21e)** (0.3 g, 0.847 mmol) and LiOH (0.169 g, 4.23 mmol) were added to a 100 mL single-necked flask, and dissolved with methanol (10 mL), water (10 mL) and tetrahydrofuran (10 mL). The mixture was reacted at room temperature for 3 hours. After the reaction was completed, 20 mL of water was added. The resulting solution was concentrated under reduced pressure to remove the organic solvent, then adjusted to pH = 2-3 with 4N hydrochloric acid, and extracted with ethyl acetate (20 mL × 3). The organic phase was combined, and concentrated to obtain compound **21f** (0.2 g, 0.61 mmol, 72%). LC-MS (ESI): m/z = 327.0 [M+H]⁺.

### Step 6: 2-(5-(2-((2,3-dihydro-1H-inden-2-yl)amino)oxazol-4-yl)-1,3,4-oxadiazol-2-yl)-1-(1,4,6,7-tetrahydro-5H-[1,2,3]triazolo[4,5-c]pyridin-5-yl)ethan-1-one (compound 21)

Compound **21f** (0.20 g, 0.61 mmol) was added into a 100 mL single-necked flask, and dissolved with DMF (5 mL). PyBop (0.431 g, 0.827 mmol), DIPEA(0.317 g, 2.45 mmol) and 4,5,6,7-tetrahydro-1H-[1,2,3]triazolo[4,5-c]pyridine hydrochloride (0.197 g, 1.23 mmol) were added. The mixture was reacted at room temperature for 16 h, washed with aqueous solution (20 mL), stirred at room temperature for 10 min, and then extracted with ethyl acetate (20 mL × 3). The organic phase was combined, washed with saturated sodium chloride (5 mL × 1), dried over anhydrous sodium sulfate, concentrated under reduced pressure, and separated and purified by silica gel column chromatography (DCM : MeOH = 20 : 1) to obtain compound **21** (0.016 g, 0.038 mmol, 6%).

LC-MS (ESI): m/z = 433.2 [M+H]⁺.

¹H NMR (400 MHz, CD₃OD) δ 8.03 (d, 1H), 7.21 (dd, 2H), 7.14 (dd, 2H), 4.86 (s, 1H), 4.53 (dd, 1H), 4.37 (d, 2H), 3.96 (dd, 2H), 3.37 (d, 2H), 3.34 (d, 2H), 2.97 (t, 2H), 2.92 (d, 1H), 2.85 (t, 1H).

### Example 22: 5-(2-((2,3-dihydro-1H-inden-2-yl)amino)pyrimidin-5-yl)-2-(2-oxo-2-(1,4,6,7-tetrahydro-5H-[1,2,3]triazolo[4,5-c]pyridin-5-yl)ethyl)oxazole-4-carbonitrile (compound 22)

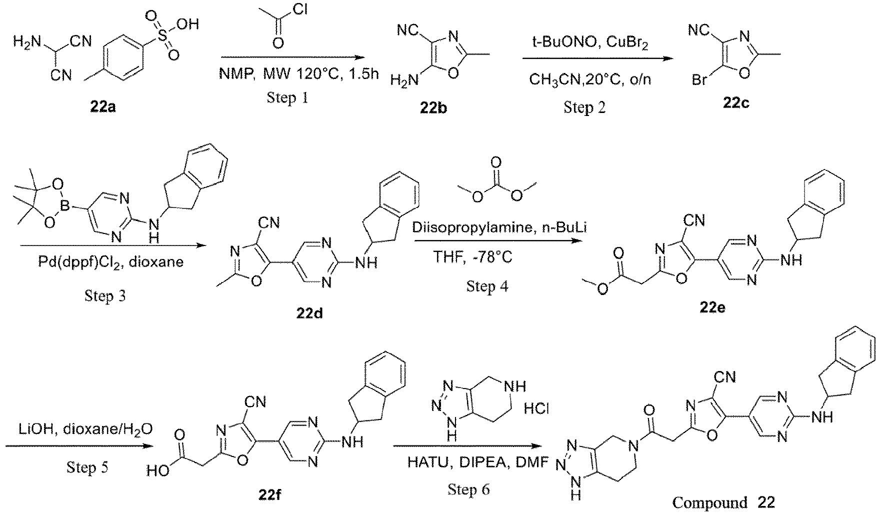

### Step 1: 5-amino-2-methyloxazole-4-carbonitrile (22b)

Compound **22a** (500 mg, 1.98 mmol), acetyl chloride (312 mg, 3.95 mmol) and NMP (5 mL) were added into a microwave tube, heated to 120°C under microwave and reacted for 1.5 hours. After the reaction was completed, the reaction system was added into water, and extracted with ethyl acetate (50 mL × 3). The organic layer was dried over anhydrous sodium sulfate, and concentrated. The silica gel column chromatography was used for separation and purification to obtain the title compound **22b** as a light yellow solid (170 mg, 70%).

¹H NMR (400 MHz, CDCl₃) δ 4.79 (s, 2H), 2.33 (s, 3H).

### Step 2: 5-bromo-2-methyloxazole-4-carbonitrile (22c)

Compound **22b** (200 mg, 1.63 mmol) was added to a solution of acetonitrile (10 mL), and copper bromide (725 mg, 3.26 mmol) and tert-butyl nitrite (335 mg, 3.26 mmol) were added successively under an ice bath. The mixture was stirred under an ice bath for 1 hour. After the reaction was completed, the mixture was diluted with a solution of saturated sodium bicarbonate (30 mL), and extracted with ethyl acetate (50 mL). The organic phase was dried over anhydrous sodium sulfate, and filtered. The filtrate was concentrated under reduced pressure, and then purified and separated by silica gel column chromatography (petroleum ether : ethyl acetate = 10 : 1) to obtain compound **22c** (100 mg, 33%).

¹H NMR (400 MHz, CDCl₃) δ 2.52 (s, 3H).

### Step 3: 5-(2-((2,3-dihydro-1H-inden-2-yl)amino)pyrimidin-5-yl)-2-methyloxazole-4-carbonitrile (22d)

Compound **22c** (400 mg, 2.14 mmol) was added to a mixed solvent of dioxane (10 mL) and water (1 mL), and then N-(2,3-dihydro-1H-inden-2-yl)-5-(4,4,5-trimethyl-1,3,2-dioxa-ol-2-yl)pyrimidin-2-amine (691 mg, 2.14 mmol), potassium carbonate (591 mg, 4.28 mmol) and Pd(dppf)Cl₂ (73 mg, 0.1 mmol) were added. Under nitrogen protection, the mixture was warmed to 100°C and stirred for 3 h. The reaction was cooled to room temperature, and filtered. The filtrate was concentrated, and separated and purified by silica gel column chromatography (petroleum ether : ethyl acetate = 2 : 1) to obtain **22d** (400 mg, 59%).

LC-MS (ESI): m/z = 318.1 [M+H]⁺.

### Step 4: methyl 2-(4-cyano-5-(2-((2,3-dihydro-1H-inden-2-yl)amino)pyrimidin-5-yl)oxazol-2-yl)acetate (22e)

Diisopropylamine (127 mg, 1.26 mmol) was added to anhydrous tetrahydrofuran (3 mL), n-butyl lithium (0.5 mL, 1.26 mmol, 2.5 M) was added under nitrogen protection and an ice bath, and the mixture was stirred for half an hour. Then the temperature was cooled to -78°C, then **22d** (200 mg, 0.63 mmol) dissolved with tetrahydrofuran was added, and the mixture was continuously stirred for half an hour. Then dimethyl carbonate (114 mg, 1.26 mmol) was added, and the resulting mixture was stirred for half an hour, then warmed to room temperature and stirred for half an hour. After the reaction was completed, the reaction was quenched with saturated aqueous ammonium chloride solution, and extracted with ethyl acetate (80 mL). The organic layer was dried over anhydrous sodium sulfate, and concentrated. The silica gel column chromatography (petroleum ether : ethyl acetate =1:2) was used for separation and purification to obtain the title compound **22e** as a yellow solid (110 mg, 47%). LC-MS (ESI): m/z = 376.1 [M+H]⁺. ¹H NMR (400 MHz, DMSO-d₆) δ 7.23-7.26 (m, 4H), 7.19-7.22 (m, 2H), 6.42-6.43 (m, 1H), 4.89-4.93 (m, 1H), 3.93 (s, 2H), 3.80 (m, 3H), 3.41-3.47 (m, 2H), 2.93-2.98 (m, 2H).

### Step 5: 2-(4-cyano-5-(2-((2,3-dihydro-1H-inden-2-yl)amino)pyrimidin-5-yl)oxazol-2-yl)acetic acid (22f)

Compound **22e** (110 mg, 0.29 mmol) was dissolved in a mixed solution of dioxane (2 mL) and water (1 mL), then lithium hydroxide monohydrate (24 mg, 0.58 mmol) was added. The mixture was stirred at room temperature. After TLC detected that the reaction was completed, the reaction was adjusted to pH to 5-6 with 1 M dilute hydrochloric acid, directly concentrated to dryness to obtain a crude product of the title compound **22f.** The crude product was directly used in the next reaction. LC-MS (ESI): m/z = 362.1 [M+H]⁺

### Step 6: 25-(2-((2,3-dihydro-1H-inden-2-yl)amino)pyrimidin-5-yl)-2-(2-oxo-2-(1,4,6,7-tetrahydro-5H-[1,2,3]triazolo[4,5-c]pyridin-5-yl)ethyl)oxazole-4-carbonitrile (compound 22)

At 0°C, compound 1i (66 mg, 0.42 mmol), N,N-diisopropylethylamine (0.5 mL) and benzotriazol-1-yl-oxy-tripyrrolidinophosphonium hexafluorophosphate (160 mg, 0.42 mmol) were sequentially added slowly to a solution of compound **22f** (100 mg, 0.28 mmol) in N,N-dimethylformamide (3 mL). The mixture was stirred and reacted at room temperature. After the reaction was completed, the reaction mixture was diluted with distilled water (30 mL), and extracted with ethyl acetate (50 mL × 2). The organic layer was washed with saturated brine (50 mL), dried over anhydrous sodium sulfate and concentrated. The silica gel column chromatography (DCM : THF = 1 : 1) was used for separation and purification to obtain **compound 22** (12 mg, 9%).

LC-MS (ESI): m/z = 468.2 [M+H]⁺.

¹H NMR (400 MHz, DMSO-d₆) δ 8.70-8.73 (m, 2H), 8.46 (d, 1H), 7.21-7.24 (m, 2H), 7.14-7.17 (m, 2H), 4.68-4.78 (m, 4H), 4.33 (d, 2H), 3.82-3.83 (m, 1H), 3.26-3.32 (m, 2H), 2.88-2.97 (m, 3H), 2.72-2.74 (m, 1H).

### Example 23: 2-(5-(2-((2,3-dihydro-1H-inden-2-yl)amino)pyrimidin-5-yl)-1,3,4-oxadiazol-2-yl)-2-fluoro-1-(1,4,6,7-tetrahydro-5H-[1,2,3]triazolo[4,5-c]pyridin-5-yl)ethan-1-one (compound 23)

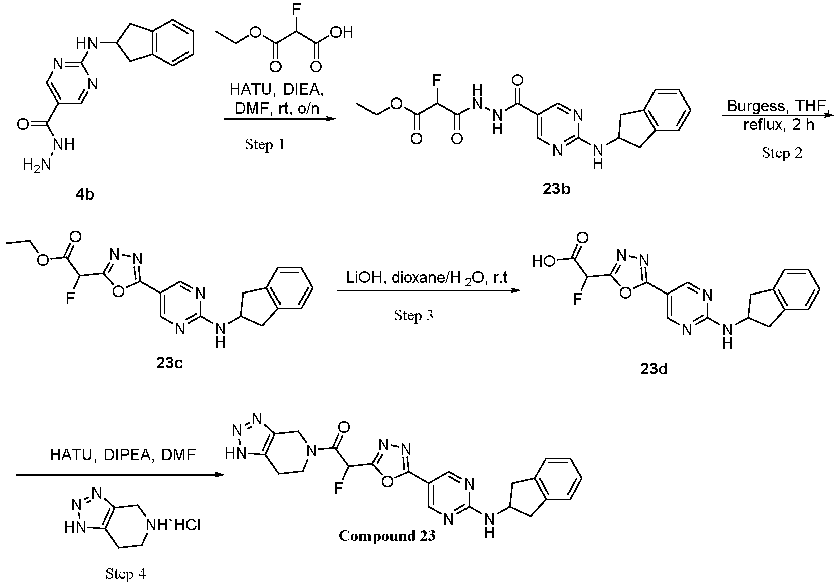

### Step 1: ethyl 3-(2-(2-((2,3-dihydro-1H-inden-2-yl)amino)pyrimidine-5-carbonyl)hydrazinyl)-2-fluoro-3-oxopropanoate (23b)

Compound **4b** (6 g, 22.3 mmol) and 3-ethoxy-2-fluoro-3-oxopropanoic acid (3.34 g, 22.3 mmol) were added to a solution of N,N-dimethylformamide (100 mL), and then N,N-diisopropylethylamine (5.75 g, 44.6 mmol) and benzotriazol-1-yl-oxy-tripyrrolidinophosphonium hexafluorophosphate (11 g, 28.99 mmol) were sequentially added slowly. The mixture was stirred and reacted at room temperature, and LCMS detected that the reaction was completed. After the reaction was completed, the reaction mixture was diluted with distilled water (200 mL), and extracted with ethyl acetate (200 mL × 2). The organic layer was washed with distilled water and saturated brine (100 mL), dried over anhydrous sodium sulfate and concentrated. The crude product was purified and separated by silica gel column chromatography (petroleum ether : ethyl acetate = 1 : 2) to obtain compound **23b** as a white solid (3.4 g, 38%). LC-MS (ESI): m/z = 402.1 [M+H]⁺

### Step 2: ethyl 2-(5-(2-((2,3-dihydro-1H-inden-2-yl)amino)pyrimidin-5-yl)-1,3,4 - oxadiazol-2-yl)-2-fluoroacetate (23c)

Compound **23b** (2.4 g, 5.99 mmol) was added to a solution of anhydrous tetrahydrofuran (80 mL), and then Burgess reagent (1.71 g, 7.18 mmol) was added. The mixture was warmed to 75°C and stirred for 2 hours. After the reaction was completed, the reaction solution was cooled, and the residue was purified by silica gel column chromatography (petroleum ether : ethyl acetate = 1 : 1) to obtain the title compound **23c** as white solid (1.6 g, 70%). LC-MS (ESI): m/z = 384.1 [M+H]⁺

### Step 3: 2-(5-(2-((2,3-dihydro-1H-inden-2-yl)amino)pyrimidin-5-yl)-1,3,4-oxadiazol-2-yl)-2-fluoroacetic acid (23d)

Compound **23c** (1.5 g, 3.92 mmol) was dissolved in a mixed solution of dioxane (15 mL) and water (4 mL), and then lithium hydroxide monohydrate (329 mg, 7.84 mmol) was added. The mixture was stirred at room temperature. After TLC detected that the reaction was completed, the reaction solution was adjusted to pH to 3-4 with 1 M dilute hydrochloric acid, extracted with ethyl acetate, dried over anhydrous sodium sulfate, filtered and concentrated to obtain the title compound **23d** as a light yellow solid (1.4 g, 100%). LC-MS (ESI): m/z = 356.1 [M+H]⁺

### Step 4: 2-(5-(2-((2,3-dihydro-1H-inden-2-yl)amino)pyrimidin-5-yl)-1,3,4-oxadiazol-2-yl)-2-fluoro-1-(1,4,6,7-tetrahydro-5H-[1,2,3]triazolo[4,5-c]pyridin-5-yl)ethan-1-one (compound 23)

At 0°C, 1i (90 mg, 0.56 mmol), N,N-diisopropylethylamine (108 mg, 0.84 mmol) and benzotriazol-1-yl-oxy-tripyrrolidinophosphonium hexafluorophosphate (160 mg, 0.42 mmol) were sequentially added slowly to a solution of compound **23d** (100 mg, 0.28 mmol) in N,N-dimethylformamide (3 mL). The mixture was stirred and reacted at room temperature, and LCMS detected that the reaction was completed. After the reaction was completed, the reaction mixture was diluted with distilled water (30 mL), and extracted with ethyl acetate (50 mL^{∗}3). The organic layer was washed with distilled water and saturated brine (50 mL), dried over anhydrous sodium sulfate and concentrated. The silica gel column chromatography (DCM : THF = 1 : 1) was used for purification to obtain compound **23** (16 mg, 12%).

LC-MS (ESI): m/z = 462.2 [M+H]⁺.

¹H NMR (400 MHz, DMSO-d₆) δ 8.77-8.90 (m, 2H), 8.50-8.51 (m, 1H), 7.22-7.24 (m, 2H), 7.11-7.17 (m, 2H), 4.60-4.82 (m, 3H), 3.76-3.88 (m, 2H), 3.23-3.32 (m, 2H), 2.90-2.97 (m, 2H), 2.67-2.89 (m, 3H).

### Example 24: 1-(1,4,6,7-tetrahydro-5H-[1,2,3]triazolo[4,5-c]pyridin-5-yl)-2-(5-(2-((5-(trifluoromethyl)-2,3-dihydro-1H-inden-2-yl)amino)pyrimidin-5-yl)-1,3,4-oxadiazol-2-yl)ethan-1-one (compound 24)

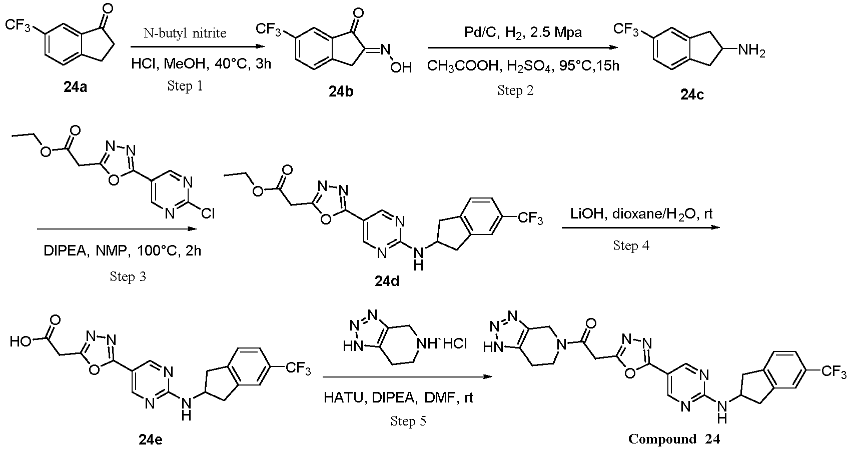

### Step 1: 2-(hydroxyimino)-6-(trifluoromethyl)-2,3-dihydro-1H-inden-1-one (24b)

Compound **24a** (18 g, 90 mmol) and n-butyl nitrite (10.8 g, 90 mmol) were added to a solution of anhydrous methanol (270 mL), and then concentrated hydrochloric acid (4.5 mL) was added. The mixture was warmed to 75°C and stirred for 10 hours. After the reaction was completed, the reaction solution was cooled and concentrated to dryness. The solid was washed with a small amount of ethyl acetate, and dried to obtain compound **24b** (13 g, 63%). LC-MS (ESI): m/z = 230.1 [M+H]⁺

### Step 2: 5-(trifluoromethyl)-2,3-dihydro-1H-inden-2-amine (24c)

A mixed solvent of compound **24b** (5 g, 21.8 mmol), glacial acetic acid (100 mL) and concentrated sulphuric acid (5 mL), and palladium-carbon (500 mg) were successively added to a 250 mL autoclave, and 4 MPa of hydrogen gas was filled. The mixture was warmed to 95°C and stirred for 15 h. The reaction was cooled to room temperature, and filtered through celite. The filtrate was concentrated, and separated and purified by silica gel column chromatography (DCM : MeOH = 10 : 1) to obtain **24c** (1.2 g, 27%). LC-MS (ESI): m/z = 202.1 [M+H]⁺

### Step 3: ethyl 2-(5-(2-((5-(trifluoromethyl)-2,3-dihydro-1H-inden-2-yl)amino) pyrimidin-5-yl)-1,3,4-oxadiazol-2-yl)acetate (24d)

Compound **24c** (400 mg, 1.99 mmol) and ethyl 2-(5-(2-chloropyrimidin-5-yl)-1,3,4-oxadiazol-2-yl) acetate (533 mg, 1.99 mmol) were added to solution of NMP (10 mL), and then DIPEA (770 mg, 5.97 mmol) was added. The mixture was warmed to 100°C and stirred for 2 hours, and cooled. The reaction mixture was diluted with distilled water (30 mL), and extracted with ethyl acetate (50 mL × 2). The organic layer was washed with distilled water and saturated brine (50 mL), dried over anhydrous sodium sulfate and concentrated. The residue was purified by silica gel column chromatography to obtain the title compound **24d** as a brown oil (120 mg, 14%). LC-MS (ESI): m/z = 434.1 [M+H]⁺

### Step 4: 2-(5-(2-((5-(trifluoromethyl)-2,3-dihydro-1H-inden-2-yl)amino)pyrimidin-5-yl)-1,3,4-oxadiazol-2-yl)acetic acid (24e)

Compound **24d** (120 mg, 0.28 mmol) was dissolved in a mixed solution of dioxane (2 mL) and water (1 mL), and then lithium hydroxide hydrate (35 mg, 0.84 mmol) was added. The mixture was stirred at room temperature. After TLC detected that the reaction was completed, the reaction solution was concentrated under reduced pressure to dryness, then adjusted to pH to 5-6 with 1M dilute hydrochloric acid, and filtered. The solid was dried to obtain the title compound **24e** as a brown solid (75 mg, 66%). LC-MS (ESI): m/z = 406.1 [M+H]⁺

### Step 5: 1-(1,4,6,7-tetrahydro-5H-[1,2,3]triazolo[4,5-c]pyridin-5-yl)-2-(5-(2-((5-(trifluoromethyl)-2,3-dihydro-1H-inden-2-yl)amino)pyrimidin-5-yl)-1,3,4-oxadiazol-2-yl)ethan-1-one (compound 24)

At 0°C, compound 1i (61 mg, 0.38 mmol), N,N-diisopropylethylamine (0.5 mL) and benzotriazol-1-yl-oxy-tripyrrolidinophosphonium hexafluorophosphate (108 mg, 0.29 mmol) were sequentially added slowly to a solution of compound **24e** (75 mg, 0.19 mmol) in N,N-dimethylformamide (3 mL), and the mixture was stirred and reacted at room temperature. LCMS detected that the reaction was completed. After the reaction was completed, the reaction mixture was diluted with distilled water (30 mL), and extracted with ethyl acetate (50 mL × 4). The organic layer was washed with distilled water and saturated brine (50 mL), dried over anhydrous sodium sulfate and concentrated. The silica gel column chromatography (DCM : THF = 1 : 1) was used for purification to obtain compound **24** (18 mg, 19%).

LC-MS (ESI): m/z = 512.2 [M+H]⁺.

¹H NMR (400 MHz, DMSO-d₆) *δ* 8.81-8.83 (m, 2H), 8.42 (d, 1H), 7.59 (s, 1H), .44-7.52 (m, 2H), 4.81 (s, 1H), 4.74-4.79 (m, 1H), 4.69 (s, 1H), 4.41 (d, 2H), 3.83-3.86 (m, 2H), 3.35-3.41 (m, 2H), 2.99-3.05 (m, 2H), 2.90-2.91 (m, 1H), 2.74-2.75 (m, 1H).

### Example 25: 1-(6,7-dihydro-1H-[1,2,3]triazolo[4,5-c]pyridin-5(4H)-yl)-2-(5-(2-((5,6-dihydro-4H-cyclopenta[b]thiophen-5-yl)amino)pyrimidin-5-yl)-1,3,4-oxadiazol-2-yl)ethanone (compound 25)

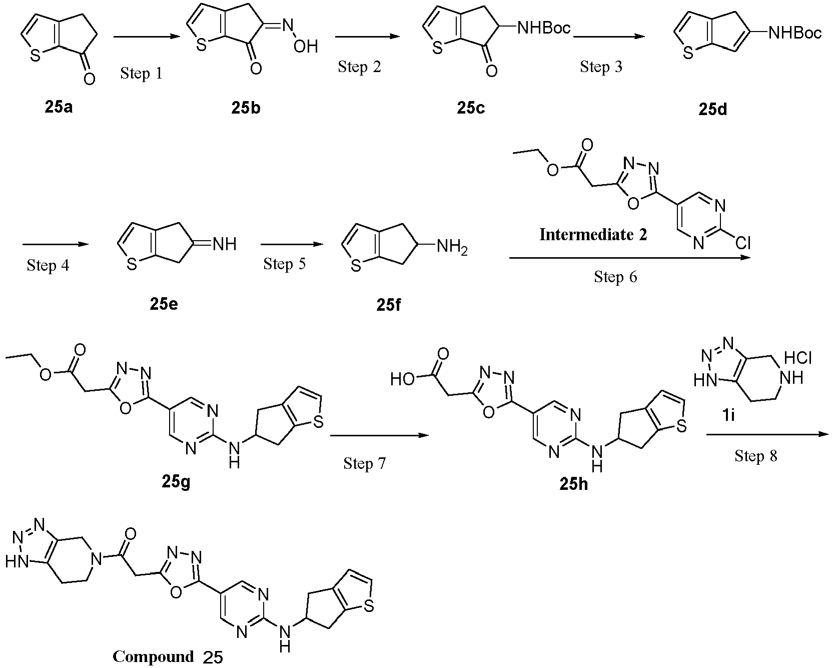

### Step 1: (Z)-5-(hydroxyimino)-4H-cyclopenta[b]thiophen-6(5H)-one (25b)

Compound **25a** (4.0 g, 28.9 mmol) was dissolved in methanol (60 ml), and warmed to 40°C. N-butyl nitrite (3.3 g, 32.0 mmol) and hydrochloric acid (1.6 ml) were added quickly. After the addition was completed, the mixture was kept at 40°C with stirring for 2 hours, and then subjected to evaporation under reduced pressure to remove methanol. PE/EtOH = 10/1 (30 ml) was added to the residue, stirred for 5 minutes, and filtered. The filter cake was dried to obtain compound **25b** (2.5 g, 52.1%).

### Step 2: tert-butyl (6-oxo-5,6-dihydro-4H-cyclopenta[b]thiophen-5-yl)carbamate (25c)

Compound **25b** (2.5 g, 15.0 mmol) was dissolved in MeOH (50 ml), and Pd/C (0.5 g) and (Boc)₂O (3.9 g, 18.0 mmol) were added. After the addition was completed, the mixture was subjected to nitrogen replacement 3 times, and then H₂ was introduced. The resulting mixture was stirred at room temperature overnight. The reaction solution was filtered. The filtrate was evaporated to dryness under reduced pressure. The residue was purified by silica gel column chromatography (PE/EA= 5/1) to obtain **25c** (2.6 g, 68.4%).

### Step 3: tert-butyl 4H-cyclopenta[b]thiophen-5-ylcarbamate (25d)

NaBH₄ (783 mg, 20.6 mmol) was placed into a 100 ml single-necked flask, THF (2.5 ml) was added, and a methanol solution (10 ml) of **25c** (2.6 g, 10.3 mmol) was added dropwise at room temperature. After the addition was completed, the mixture was stirred at room temperature overnight. Water (30 ml) was added to the reaction solution, and the resulting solution was stirred for 10 minutes, and then extracted 3 times with EA. The organic phase was combined, washed once with saturated NaCl aqueous solution, dried over anhydrous sodium sulfate, and filtered. The filtrate was evaporated to dryness under reduced pressure to obtain compound **25d** (2.2 g, 91.7%).

### Step 4: 4H-cyclopenta[b]thiophen-5(6H)-imine (25e)

Compound **25d** (2.2 g, 9.3 mmol) was dissolved in DCM (30 ml), and trifluoroacetic acid (1.0 g) was added. The resulting solution was cooled to 0°C with an ice bath, and Et₃SiH (1.1 g) was added dropwise. After the addition was completed, the mixture was slowly returned to room temperature and stirred for 2 hours. The reaction solution was evaporated to dryness under reduced pressure. Diethyl ether (30 ml) was added to the residue, and the resulting mixture was stirred for 10 minutes, and then filtered. The filter cake was dried to obtain compound **25e** (1.2 g, 94.5%).

### Step 5: 5,6-dihydro-4H-cyclopenta[b]thiophen-5-amine (25f)

Compound **25e** (1.2 g, 8.7 mmol) was dissolved in methanol (50 ml), and Pd/C (1.2 g) was added. After the addition was completed, the mixture was subjected to hydrogen gas replacement 3 times, and stirred at room temperature overnight. LC-MS detected that the reaction was completed. The reaction solution was filtered. The filtrate was evaporated to dryness under reduced pressure to obtain **25f** (0.8 g, 66.7%). LC-MS (ESI): m/z = 140.1 [M+H]⁺.

### Step 6: Ethyl 2-(5-(2-((5,6-dihydro-4H-cyclopenta[b]thiophen-5-yl)amino)pyrimidin-5-yl)-1,3,4-oxadiazol-2-yl)acetate (25g)

**25f** (200 mg, 1.4 mmol) and intermediate **2** (376 mg, 1.4 mmol) were placed into a 100 ml single-necked flask, and NMP (30 ml) and DIPEA(80 mg, 4.2 mmol) were added. After the addition was completed, the mixture was warmed to 100°C and stirred and reacted for 2 hours. LC-MS detected that the reaction was completed. The reaction solution was purified by silica gel column chromatography (PE/EA= 2/1) to obtain compound **25g** (180 mg, 34.6%). LC-MS (ESI): m/z = 372.2 [M+H]⁺.

### Step 7: 2-(5-(2-((5,6-dihydro-4H-cyclopenta[b]thiophen-5-yl)amino)pyrimidin-5-yl)-1,3,4-oxadiazol-2-yl)acetic acid (25h)

**25g** (180 mg, 0.5 mmol) was dissolved in THF (5 ml) and H₂O (2 ml), and LiOH·H₂O (63 mg, 1.5 mmol) was added. After the addition was completed, the mixture was stirred at room temperature for 3 hours. The reaction solution was evaporated to dryness under reduced pressure. Water (5 ml) was added to the residue. The mixture was adjusted to pH = 5 with 2N dilute hydrochloric acid, and extracted 4 times with ethyl acetate. The organic phase was combined, dried over anhydrous sodium sulfate, and filtered. The filtrate was evaporated to dryness under reduced pressure to obtain compound **25h** (150 mg, 87.2%). LC-MS (ESI): m/z = 344.1 [M+H]⁺.

### Step 8: 1-(6,7-dihydro-1H-[1,2,3]triazolo[4,5-c]pyridin-5(4H)-yl)-2-(5-(2-((5,6-dihydro-4H-cyclopenta[b]thiophen-5-yl)amino)pyrimidin-5-yl)-1,3,4-oxadiazol-2-yl)ethanone (compound 25)

Compound **25h** (50 mg, 0.15 mmol) was dissolved in DMF (5 ml), and CDI (29 mg, 0.18 mmol) was added. The mixture was stirred at room temperature for 10 minutes, and then a solution (2 ml) of triethylamine (606 mg, 6 mmol) and **1i** (61 mg, 0.38 mmol) in DMF was added. After the addition was completed, the resulting mixture was stirred at room temperature for 2 hours and filtered. The filtrate was purified by silica gel column chromatography (DCM/MeOH = 15/1) to obtain **compound 25** (40 mg, 59.7%).

LC-MS (ESI): m/z = 450.2 [M+H]⁺.

¹H NMR (400 MHz, DMSO-d₆) *δ* 14.72 (m, 1H), 8.84-8.77 (m, 2H), 8.53 (d, 1H), 7.37 (d, 1H), 6.89 (d, 1H), 5.16-5.07 (m, 1H), 4.81 (s, 1H), 4.68 (s, 1H), 4.43 (s, 1H), 4.39 (s, 1H), 3.87-3.81 (m, 2H), 3.35-3.32 (m, 1H), 3.18-3.12 (m, 1H), 2.92-2.83 (m, 2H), 2.78-2.72 (m, 2H).

### Example 26: 2-(5-(2-((5,6-dihydro-4H-cyclopenta[c]thiophen-5-yl)amino)pyrimidin-5-yl)-1,3,4-oxadiazol-2-yl)-1-(1,4,6,7-tetrahydro-5H-[1,2,3]triazolo[4,5-c]pyridin-5-yl)ethan-1-one (compound 26)

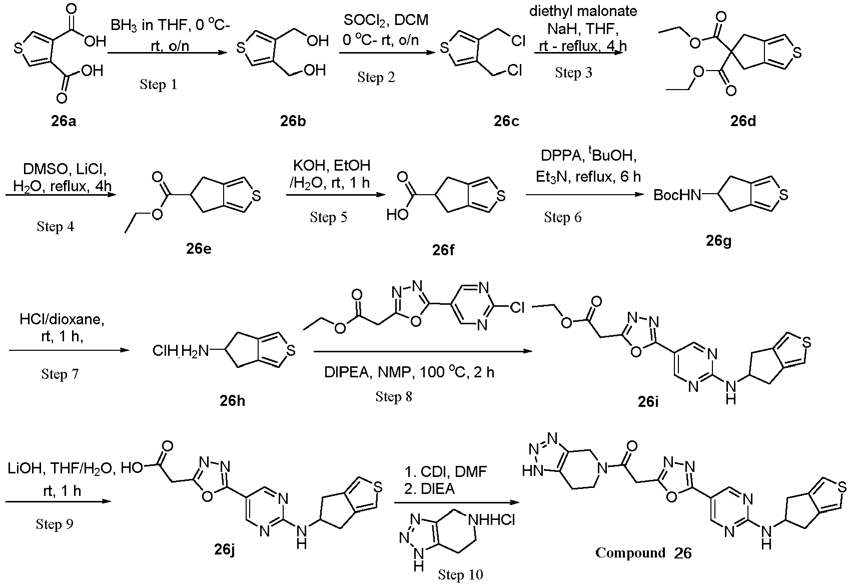

### Step 1: thiophene-3,4-diyldimethanol (26b)

Compound **26a** (10 g, 58.1 mmol) was added to a 500 mL single-necked flask, and dissolved in 40 mL of THF. A solution of borane in THF (145 mL, 145 mmol, 1.0 M) was added at 0°C. The mixture was stirred at room temperature overnight. The reaction was quenched with 100 ml THF/H₂O (1 : 1), and then 150 ml of saturated sodium bicarbonate solution was added. The resulting solution was extracted with EA (100 mL × 3). The organic phase was combined, washed with saturated sodium chloride (100 mL), dried over anhydrous sodium sulfate, and concentrated under reduced pressure to obtain compound **26b** (5.1 g, yield 61%).LC-MS (ESI): m/z = 145.2 [M+H]⁺.

### Step 2: 3,4-bis(chloromethyl)thiophene (26c)

Compound **26b** (5.1 g, 35.4 mmol) was dissolved in 60 ml of DCM, and thionyl chloride (6 mL) was added under an ice bath. Then the mixture was warmed to room temperature and reacted overnight. After TLC detected that the reaction was completed, the reaction was quenched with ice water, extracted with DCM, washed with saturated sodium bicarbonate and brine, dried over anhydrous sodium sulfate, spun to dryness, and separated and purified by silica gel column chromatography (PE/EA= 10/1) to obtain compound **26c** (2.6 g, 41%). LC-MS (ESI): m/z = 181.1 [M+H]⁺.

### Step 3: diethyl 4H-cyclopenta[c]thiophene-5,5(6H)-dicarboxylate (26d)

Diethyl malonate (6.5 g, 40.9 mmol) was dissolved in anhydrous THF (100 mL), and 60% sodium hydride (4.1 g, 102.2 mmol) was added under the condition of ice bath, and then stirred for 30 minutes under an ice bath. Then compound **26c** (7.4 g, 40.9 mmol) was added under reflux, and the reaction was continued for 3 h. The reaction was quenched with water, extracted with ethyl acetate (50 mL × 3), washed with saturated brine, dried over anhydrous sodium sulfate, concentrated, and separated and purified by silica gel column chromatography (PE/EA= 6/1) to obtain compound **26d** (6.0 g, 55%). LC-MS (ESI): m/z = 269.2 [M+H]⁺.

### Step 4: ethyl 5,6-dihydro-4H-cyclopenta[c]thiophene-5-carboxylate (26e)

Compound **26d** (5.2 g, 19.4 mmol) was dissolved in DMSO (40 mL), water (2 mL) was added, and then sodium chloride solid (5.2 g) was added. The mixture was heated to reflux and reacted for 5 hours. The reaction solution was cooled to room temperature, water was added and the resulting solution was extracted with ethyl acetate. The organic layer was washed with saturated brine, dried over anhydrous sodium sulfate, concentrated, and separated and purified by silica gel column chromatography (PE/EA = 10/1) to obtain compound **26e** (2.0 g, 52%). LC-MS (ESI): m/z = 197.2 [M+H]⁺.

### Step 5: 5,6-dihydro-4H-cyclopenta[c]thiophene-5-carboxylic acid (26f)

Compound **26e** (2.0 g, 10.2 mmol) was dissolved in 15 ml of ethanol and 5 ml of water, and potassium hydroxide (1.71 g, 30.6 mmol) was added. The mixture was stirred at room temperature and reacted for 1 hour. After TLC detected that the reaction was completed, the reaction solution was concentrated, diluted by adding water, and extracted with diethyl ether. The aqueous phase was then adjusted to a pH of less than 5 with 4N HCl, and extracted with DCM. The organic phase was washed with brine, dried over anhydrous sodium sulfate, filtered and spun to dryness to obtain compound **26f** (1.3 g, 76%). LC-MS (ESI): m/z = 169.2 [M+H]⁺.

### Step 6: tert-butyl (5,6-dihydro-4H-cyclopenta[c]thiophen-5-yl)carbamate (26g)

Compound **26f** (1.1 g, 6.5 mmol) was dissolved in 10 ml of tert-butanol, and then triethylamine (661 mg, 6.5 mmol) and DPPA (1.8 g, 6.5 mmol) were successively added. The mixture was refluxed and reacted overnight under nitrogen protection, cooled to room temperature and concentrated, and separated by silica gel column chromatography (PE/EA = 2/1) to obtain compound **26g** (1.2 g, 77%). LC-MS (ESI): m/z = 184.2 [M-55]⁺.

### Step 7: 5,6-dihydro-4H-cyclopenta[c]thiophen-5-amine hydrochloride (26h)

Compound **26g** (1.2 g, 5.0 mmol) was dissolved in a solution of 4N 1,4-dioxane (20 ml), stirred at room temperature, and reacted for 1 hour. After TLC detected that the reaction was completed, the reaction solution was concentrated to obtain compound **26h** (755 mg, 86%). LC-MS (ESI): m/z = 140.2 [M+H]⁺.

### Step 8: ethyl 2-(5-(2-((5,6-dihydro-4H-cyclopenta[c]thiophen-5-yl)amino)pyrimidin-5-yl)-1,3,4-oxadiazol-2-yl)acetate (26i)

Compound **26h** (177 mg, 1.0 mmol) was added to a 100 mL single-necked flask, and dissolved with NMP (4 mL). DIPEA (387 mg, 3.0 mmol) and intermediate **2** (268 mg, 1.0 mmol) were added. The mixture was stirred at 100°C for 2 h, cooled to room temperature, and poured into water. Solids were precipitated, filtered by suction, and dried to obtain compound **26i** as a light yellow solid (205 mg, 55%). LC-MS (ESI): m/z = 372.2 [M+H]⁺.

### Step 9: 2-(5-(2-((5,6-dihydro-4H-cyclopenta[c]thiophen-5-yl)amino)pyrimidin-5-yl)-1,3,4-oxadiazol-2-yl)acetic acid (26j)

Compound **26i** (205 mg, 0.55 mmol) was dissolved in 5 ml of THF and 2 ml of water, and lithium hydroxide monohydrate (69 mg, 1.65 mmol) was added. The mixture was stirred at room temperature and reacted for 1 hour. After TLC detected that the reaction was completed, the reaction solution was concentrated, diluted by adding water, and extracted twice with diethyl ether. The aqueous phase was adjusted to pH to 4-5 by adding additional 4N hydrochloric acid aqueous solution, extracted with dichloromethane, dried over anhydrous sodium sulfate, filtered, and spun to dryness to obtain compound **26j** (165 mg, 87%). LC-MS (ESI): m/z = 344.2 [M+H]⁺.

### Step 10: 2-(5-(2-((5,6-dihydro-4H-cyclopenta[c]thiophen-5-yl)amino)pyrimidin-5-yl)-1,3,4-oxadiazol-2-yl)-1-(1,4,6,7-tetrahydro-5H-[1,2,3]triazolo[4,5-c]pyridin-5-yl)ethan-1-one (compound 26)

Compound **26j** (165 mg, 0.48 mmol) was dissolved in 3 ml of DMF, and CDI (94 mg, 0.58 mmol) was added. The mixture was stirred at room temperature for 30 minutes. Then 4,5,6,7-tetrahydro-1H-[1,2,3]triazolo[4,5-c]pyridine hydrochloride (231 mg, 1.44 mmol) was dissolved in 2 ml of DMF, and dissociated with DIPEA (248 mg, 1.92 mmol), and the resulting solution was added to the reaction solution of **26j** for reacting for 1 h, and directly separated by silica gel column chromatography (MeOH/DCM = 10/1) to obtain compound **26** (25 mg, 12%).

LC-MS (ESI): m/z = 450.2 [M+H]⁺.

¹H NMR (400 MHz, DMSO-d₆) δ 8.84 - 8.78 (m, 2H), 8.46 (d, 1H), 7.06 (s, 2H), 4.95 - 4.90 (m, 1H), 4.81 (s, 1H), 4.68 (s, 1H), 4.41 (d, 2H), 3.87 - 3.81 (m, 2H), 3.14 - 3.08 (m, 2H), 2.69 (t, 1H), 2.74 - 2.67 (m, 3H).

### Example 27: 2-(5-(2-((2,3-dihydro-1H-inden-2-yl)amino)pyrimidin-5-yl)-1,3,4-oxadiazol-2-yl)-2-fluoro-1-(1,4,6,7-tetrahydro-5H-[1,2,3]triazolo[4,5-c]pyridin-5-yl)propan-1-one (compound 27)

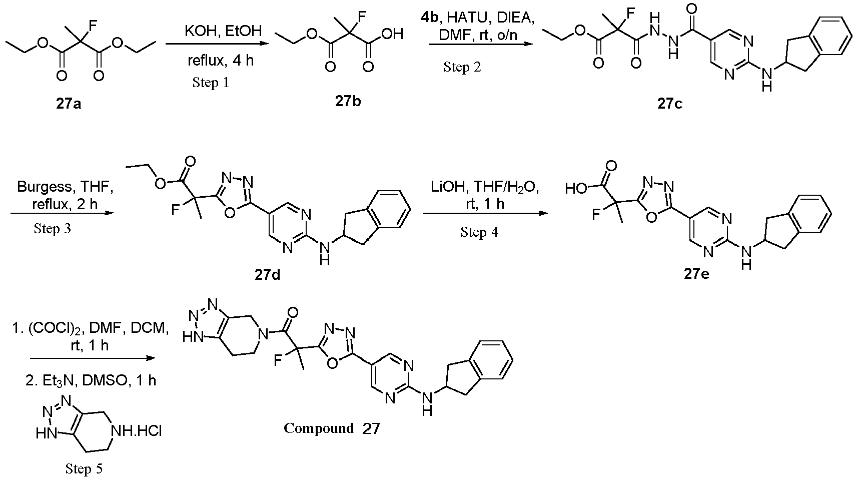

### Step 1: 3-ethoxy-2-fluoro-2-methyl-3-oxopropanoic acid (27b)

Compound **27a** (10.0 g, 52 mmol) was dissolved in anhydrous ethanol (100 mL), and then potassium hydroxide (2.92 g, 52 mmol) was added. The reaction mixture was heated to reflux and reacted for 4 hours. The reaction solution was cooled to room temperature and the ethanol was concentrated. Water and diethyl ether were added. The aqueous phase was adjusted to pH to 3-4 with additional 4N hydrochloric acid, and extracted with ethyl acetate (50 mL × 3). The organic phase was washed with saturated brine, dried over anhydrous sodium sulfate, filtered and concentrated to obtain compound **27b** (5.5 g, 64%). LC-MS (ESI): m/z = 165.2 [M+H]⁺.

### Step 2: ethyl 3-(2-(2-((2,3-dihydro-1H-inden-2-yl)amino)pyrimidine-5-carbonyl) hydrazinyl)-2-fluoro-2-methyl-3-oxopropanoate (27c)

Compound **27b** (1.8 g, 11 mmol) was dissolved in DMF (80 mL), and then HATU (5.7 g, 15 mmol) and DIPEA (3.87 g, 30 mmol) were added. The mixture was stirred and reacted for 10 minutes, and then compound **4b** (2.69 g, 10 mmol) was added. The mixture was stirred at room temperature overnight. The reaction solution was poured into water (250 mL). A large number of solids were precipitated and filtered. The filter cake was dried to obtain compound **27c** (2.4 g 58%). LC-MS (ESI): m/z = 416.2 [M+H]⁺.

### Step 3: ethyl 2-(5-(2-((2,3-dihydro-1H-inden-2-yl)amino)pyrimidin-5-yl)-1,3,4-oxadiazol-2-yl)-2-fluoropropanoate (27d)

Compound **27c** (2.48 g, 5.98 mmol) was dissolved in anhydrous tetrahydrofuran (25 mL), and Burgess reagent (4.27 g, 17.93 mmol) was added. The mixture was stirred at room temperature and refluxed and reacted under nitrogen protection for 1 hour. After TLC detected that the reaction was completed, water was added and the resulting solution was extracted with ethyl acetate. The organic layer was washed with saturated brine, dried over anhydrous sodium sulfate, concentrated, and purified by silica gel column chromatography (PE/EA= 1/1) to obtain compound **27d** (1.2 g, 50%). LC-MS (ESI): m/z = 398.2 [M+H]⁺.

### Step 4: 2-(5-(2-((2,3-dihydro-1H-inden-2-yl)amino)pyrimidin-5-yl)-1,3,4-oxadiazol-2-yl)-2-fluoropropanoic acid (27e)

Compound **27d** (1.2 g, 3.02 mmol) was dissolved in 9 ml of THF and 3 ml of water, and lithium hydroxide monohydrate (381 mg, 9.07 mmol) was added. The mixture was stirred at room temperature and reacted for 1 hour. After TLC detected that the reaction was completed, the reaction solution was concentrated, diluted with water and extracted with diethyl ether. Then the aqueous layer was adjusted to pH to 4-5 with 4N hydrochloric acid aqueous solution, and extracted with dichloromethane. The organic layer was washed with saturated brine, dried over anhydrous sodium sulfate and concentrated to obtain compound **27e** (850 mg, 76%). LC-MS (ESI): m/z = 370.2 [M+H]⁺.

### Step 9: 2-(5-(2-((2,3-dihydro-1H-inden-2-yl)amino)pyrimidin-5-yl)-1,3,4-oxadiazol-2-yl)-2-fluoro-1-(1,4,6,7-tetrahydro-5H-[1,2,3]triazolo[4,5-c]pyridin-5-yl)propan-1-one (compound 27)

Compound **27e** (123 mg, 0.33 mmol) was dissolved in 3 ml of anhydrous DCM, and oxalyl chloride (64 mg, 0.5 mmol) and a catalytic amount of DMF were added. The mixture was stirred at room temperature for 30 minutes. Then 4,5,6,7-tetrahydro-1H-[1,2,3]triazolo[4,5-c]pyridine hydrochloride (133 mg, 0.83 mmol) was dissolved in 3 ml of anhydrous DMSO, and triethylamine (134 mg, 1.32 mmol) was added. Then the solution was added to the above-mentioned reaction solution and reacted for 1 h. The resulting solution was concentrated to remove DCM, and then purified by silica gel column chromatography (THF/DCM = 1/3) to obtain compound **27** (40 mg, 26%).

LC-MS (ESI): m/z = 476.2 [M+H]⁺.

¹H NMR (400 MHz, DMSO -d6) δ 8.92 - 8.81 (m, 2H), 8.50 (d, 1H), 7.24 - 7.14 (m, 4H), 4.76 - 4.69 (m, 3H), 4.00 - 3.90 (m, 2H), 3.32 - 3.26 (m, 2H), 2.99 - 2.95 (m, 2H), 2.83 - 2.81 (m, 2H), 2.16 - 2.07 (m, 3H).

### Example 28: 2-(5-(2-((5-(furan-3-yl)-2,3-dihydro-1H-inden-2-yl)amino)pyrimidin-5-yl)-1,3,4-oxadiazol-2-yl)-1-(1,4,6,7-tetrahydro-5H-[1,2,3]triazolo[4,5-c]pyridin-5-yl)ethan-1-one (compound 28)

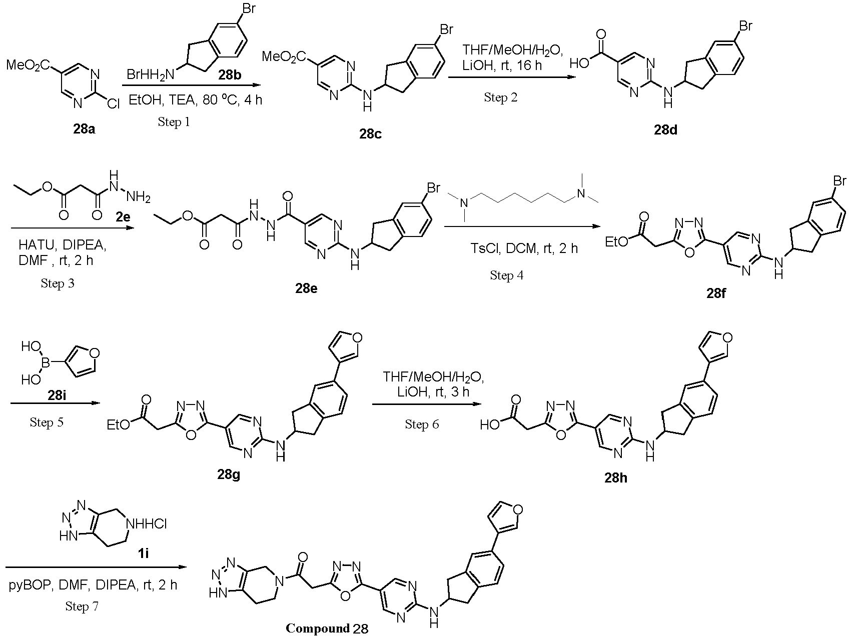

### Step 1: methyl 2-((5-bromo-2,3-dihydro-1H-inden-2-yl)amino)pyrimidine-5-carboxylate (28c)

**28b** (2.0 g, 10.71 mmol) and triethylamine (4.3 g, 42.87 mmol) were added to a solution of compound **28a** (3.0 g, 10.71 mmol) in ethanol (30 mL), and the mixture was stirred at 80°C for 4 hours. After the reaction was completed, the mixture was diluted with distilled water (30 mL), and extracted with ethyl acetate (30 mL × 3). The organic phase was washed with distilled water and saturated brine, dried over anhydrous sodium sulfate and concentrated. The silica gel column chromatography (eluent: petroleum ether : ethyl acetate = 1 : 0-0 : 1) was used for separation and purification to obtain the title compound **28c** as an off-white solid (3.7 g, 100%). LC-MS (ESI): m/z = 348.1, 350.1 [M+H]⁺. ¹H NMR (400 MHz, DMSO-d₆) δ 8.79 (s, 1H), 8.73 (s, 1H), 8.44 (d, 1H), 7.42 (s, 1H), 7.33 (d, 1H), 7.18 (d, 1H), 4.73 - 4.66 (m, 1H), 3.80 (s, 3H), 3.27 - 3.20 (m, 2H), 2.96 - 2.86 (m, 2H).

### Step 2: 2-((5-bromo-2,3-dihydro-1H-inden-2-yl)amino)pyrimidine-5-carboxylic acid (28d)

Compound **28c** (5.0 g, 14.36 mmol) was dissolved in tetrahydrofuran (50 mL)/methanol (5 ml) and distilled water (5 ml), and then LiOH (1.0 g, 43.08 mmol) was added. Then the mixture was reacted at room temperature for 16 hours. After the reaction was completed, the reaction solution was cooled, adjusted to pH = 3 by adding acid, and then extracted with ethyl acetate (50 mL × 3). The organic phase was dried over anhydrous sodium sulfate, and filtered. The filtrate was concentrated under reduced pressure to obtain target compound **28d** as an off-white solid (4.5 g, 94%). LC-MS (ESI): m/z = 334.1, 336.1 [M+H]+. ¹H NMR (400 MHz, DMSO-d₆) δ 12.67 (s, 1H), 8.77 (s, 1H), 8.71 (s, 1H), 8.31 (d, 1H), 7.42 (s, 1H), 7.33 (m, 1H), 7.18 (d, 1H), 4.73 - 4.67 (m, 1H), 3.31 - 3.17 (m, 2H), 2.96 - 2.85 (m, 2H).

### Step 3: Ethyl 3-(2-(2-((5-bromo-2,3-dihydro-1H-inden-2-yl)amino)pyrimidine-5-carbonyl)hydrazinyl)-3-oxopropanoate (28e)

**2e** (0.23 g, 1.4 mmol), 2-(7-azabenzotriazole)-N,N,N',N'-tetramethyluronium hexafluorophosphate (0.54 g, 1.4 mmol) and N,N-diisopropylethylamine (0.39 g, 3.0 mmol) were added to a solution of compound **28d** (0.35 g, 1.2 mmol) in N,N-dimethylformamide (6 mL), and the mixture was reacted at room temperature for 2 hours. After the reaction was completed, the mixture was diluted with distilled water (30 mL), and extracted with dichloromethane (20 mL × 3). The organic phase was washed with distilled water and saturated brine, dried over anhydrous sodium sulfate and concentrated. Title compound **28e** as a brown solid (720 mg) was obtained. LC-MS (ESI): m/z = 460.1, 462.1 [M-H]⁺.

### Step 4: Ethyl 2-(5-(2-((5-bromo-2,3-dihydro-1H-inden-2-yl)amino)pyrimidin-5-yl)-1,3,4-oxadiazol-2-yl)acetate (28f)

4-toluene sulfonyl chloride (0.83 g, 4.33 mmol) and N,N,N',N'-tetramethyl-1,6-hexanediamine (1.12 g, 6.49 mmol) were added to a solution of compound **28e** (1.0 g, 2.16 mmol) in dichloromethane (20 mL), and the mixture was reacted at room temperature for 2 hours. After the reaction was completed, the mixture was diluted with dichloromethane (30 mL). The organic phase was washed with distilled water and saturated brine, dried over anhydrous sodium sulfate and concentrated. The silica gel column chromatography (eluent: petroleum ether : ethyl acetate = 1 : 0-0 : 1) was used for separation and purification to obtain the title compound **28f** as a grey solid (550 mg, two-step yield: 42%). LC-MS (ESI): m/z = 444.1, 446.1, [M+H]⁺. ¹H NMR (400 MHz, DMSO-d₆) *δ* 8.86 (s, 1H), 8.81 (s, 1H), 8.43 (d, 1H), 7.43 (s, 1H), 7.33 (d, 1H), 7.19 (d, 1H), 4.74 - 4.69 (m, 1H), 4.23 (s, 2H), 4.17 (q, 2H), 3.33 - 3.22 (m, 2H), 2.98 - 2.87 (m, 2H), 1.22 (t, 3H).

### Step 5: Ethyl 2-(5-(2-((5-(furan-3-yl)-2,3-dihydro-1H-inden-2-yl)amino)pyrimidin-5-yl)-1,3,4-oxadiazol-2-yl)acetate (28g)

Compound **28i** (0.15 g, 1.33 mmol), tri-potassium phosphate monohydrate (1.23 g, 3.65 mmol) and bis(triphenylphosphine)palladium dichloride (0.04 g, 0.06 mmol) were added to a solution of compound **28f** (0.54 g, 1.22 mmol) in dioxane/water (10 ml/1 ml), and the mixture was stirred at 90°C for 4 hours. After the reaction was completed, the mixture was diluted with distilled water (30 mL), and extracted with ethyl acetate (20 mLx 4). The organic phase was washed with distilled water and saturated brine, dried over anhydrous sodium sulfate, concentrated and subjected to silica gel column chromatography (eluent: petroleum ether : ethyl acetate = 1 : 0-0 : 1) to obtain the title compound **28g** as a light yellow solid (0.3 g, 56%). LC-MS (ESI): m/z = 432.2 [M+H]⁺. ¹H NMR (400 MHz, DMSO-d₆) δ 8.87 (s, 1H), 8.81 (s, 1H), 8.43 (d, 1H), 8.12 (s, 1H), 7.71 (t, 1H), 7.48 (s, 1H), 7.41 (d, 1H), 7.24 (d, 1H), 6.92 (s, 1H), 4.77 - 4.72 (m, 1H), 4.23 (s, 2H), 4.17 (q, 2H), 3.34 - 3.26 (m, 2H), 3.00 - 2.95 (m, 2H), 1.22 (t, 3H).

### Step 6: 2-(5-(2-((5-(furan-3-yl)-2,3-dihydro-1H-inden-2-yl)amino)pyrimidin-5-yl)-1,3,4-oxadiazol-2-yl)acetic acid (28h)

Compound **28g** (0.3 g, 0.70 mmol) was dissolved in tetrahydrofuran (6 mL)/methanol (2 ml) and distilled water (2 ml), and then LiOH (0.05 g, 2.09 mmol) was added. Then the mixture was reacted at room temperature for 3 hours. After the reaction was completed, 20 mL of water was added, and the mixture was adjusted to pH = 3 by adding acid, and then extracted with DCM (30 mL × 3). The organic phase was dried over anhydrous sodium sulfate, and filtered. The filtrate was concentrated under reduced pressure to obtain target compound **28h** (0.25 g, 96%). LC-MS (ESI): m/z = 404.2 [M+H]⁺

### Step 7: 2-(5-(2-((5-(furan-3-yl)-2,3-dihydro-1H-inden-2-yl)amino)pyrimidin-5-yl)-1,3,4-oxadiazol-2-yl)-1-(1,4,6,7-tetrahydro-5H-[1,2,3]triazolo[4,5-c]pyridin-5-yl)ethan-1-one (compound 28)

Compound **1i** (0.40 g, 2.48 mmol), N,N-diisopropylethylamine (0.40 g, 3.1 mmol) and benzotriazol-1-yl-oxy-tripyrrolidinophosphonium hexafluorophosphate (0.48 g, 0.93 mmol) were sequentially added slowly to a solution of compound **28h** (0.25 g, 0.62 mmol) in N,N-dimethylformamide (8 mL), and the mixture was stirred at room temperature for 2 hours. After the reaction was completed, dichloromethane (20 mL) was added to the reaction solution, and the resulting mixture was diluted with distilled water (20 mL), and extracted with dichloromethane (20 mL × 3). The organic phase was washed with distilled water and saturated brine, dried over anhydrous sodium sulfate and concentrated. The silica gel column chromatography (eluent: dichloromethane : ethyl acetate : methanol = 1 : 0 : 0-2 : 10 : 1) was used to obtain **compound 28** (52 mg, 18%). LC-MS (ESI): m/z = 510.1 [M+H]⁺. ¹H NMR (400 MHz, DMSO-d₆) δ 8.85 (s, 1H), 8.79 (s, 1H), 8.40 (d, 1H), 8.12 (s, 1H), 7.71 (t, 1H), 7.48 (s, 1H), 7.41 (d, 1H), 7.23 (d, 1H), 6.92 (s, 1H), 4.81 (s, 1H), 4.77 - 4.71 (m, 1H), 4.68 (s, 1H), 4.41 (d, 2H), 3.87 - 3.81 (m, 2H), 3.30 (m, 2H), 2.97 (m, 2H), 2.91 (t, 1H), 2.74 (t, 1H).

### Example 29: 2-(5-(2-((6,7-dihydro-5H-indeno[5,6-d][1,3]dioxol-6-yl)amino)pyrimidin-5-yl)-1,3,4-oxadiazol-2-yl)-1-(3,4,6,7-tetrahydro-5H-[1,2,3]triazolo[4,5-c]pyridin-5-yl)ethan-1-one (compound 29)

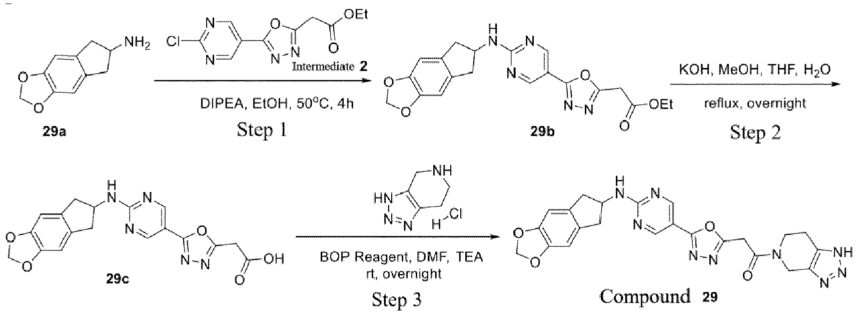

### Step 1: ethyl 2-(5-(2-((6,7-dihydro-5H-indeno[5,6-d][1,3]dioxol-6-yl)amino) pyrimidin-5-yl)-1,3,4-oxadiazol-2-yl)acetate (29b)

**29a** (180 mg, 1.02 mmol) was dissolved in ethanol (3 mL), **intermediate 2** (546 mg, 2.03 mmol) was added, and then DIPEA(0.51 ml, 3.05 mmol) was added. The mixture was warmed to 50°C and stirred for 4 hours. 1 mol/L of dilute hydrochloric acid (50 mL) was added. The mixture was extracted with dichloromethane (50 mL × 2). The organic phase was combined, washed once with water (100 mL), dried over sodium sulfate, filtered and spun to dryness to obtain crude product **29b** (200 mg) as a brown-black solid. LC-MS (ESI): m/z = 410.2 [M+H]⁺.

### Step 2: 2-(5-(2-((6,7-dihydro-5H-indeno[5,6-d][1,3]dioxol-6-yl)amino)pyrimidin-5-yl)-1,3,4-oxadiazol-2-yl)acetic acid (29c)

Crude product **29b** (200 mg) was dissolved in methanol (1.5 mL), THF (1.5 mL) was added, and then a solution of KOH (275 mg, 4.91 mmol) in water (1.5 mL) was added. The mixture was heated to reflux and stirred overnight. The reaction solution was cooled. Water (50 mL) was added. The mixture was adjusted to a pH of about 6 with dilute hydrochloric acid (1 moL/L), and extracted with ethyl acetate (50 mL × 2). The organic phase was combined, washed once with water (100 mL), dried over sodium sulfate, filtered and spun to dryness to obtain crude product **29c** (205 mg) as an ash black solid. LC-MS (ESI): m/z = 382.1 [M+H]⁺.

### Step 3: 2-(5-(2-((6,7-dihydro-5H-indeno[5,6-d][1,3]dioxol-6-yl)amino)pyrimidin-5-yl)-1,3,4-oxadiazol-2-yl)-1-(3,4,6,7-tetrahydro-5H-[1,2,3]triazolo[4,5-c]pyridin-5-yl)ethan-1-one (compound 29)

Crude product **29c** (205 mg) was dissolved in DMF (1.5 mL). 4,5,6,7-tetrahydro-3H-[1,2,3]triazolo[4,5-c]pyridine hydrochloride (169 mg, 1.05 mmol) was added, then triethylamine (0.73 mL, 5.25 mmol) was added dropwise, and BOP (348 mg, 0.79 mmol) was added. The mixture was stirred at room temperature overnight. Water (50 mL) was added. The mixture was adjusted to a pH of about 6 with dilute hydrochloric acid (2 moL/L), and extracted with ethyl acetate (50 mL × 2). The organic phase was combined, washed once with water (100 mL), dried over sodium sulfate, filtered and spun to dryness to obtain a brown oil, which was separated and purified by silica gel column chromatography to obtain **compound 29** (20 mg).

LC-MS (ESI): m/z = 488.3 [M+H]⁺.

¹H NMR (400 MHz, DMSO-d₆) δ 9.00 - 8.62 (m, 2H), 8.37 (d, 1H), 6.79 (s, 2H), 5.94 (d, 2H), 4.81 (s, 1H), 4.74 - 4.66 (m, 2H), 4.41 (d, 2H), 3.89 - 3.79 (m, 2H), 3.18 (dd, 2H), 2.90 (t, 1H), 2.82 (dd, 2H), 2.77 - 2.71 (m, 1H).

### Example 30: 2-(5-(2-((2,4,5,6-tetrahydro-1H-cyclobuta[f]inden-5-yl)amino)pyrimidin-5-yl)-1,3,4-oxadiazol-2-yl)-1-(1,4,6,7-tetrahydro-5H-[1,2,3]triazolo[4,5-c]pyridin-5-yl)ethan-1-one (compound 30)

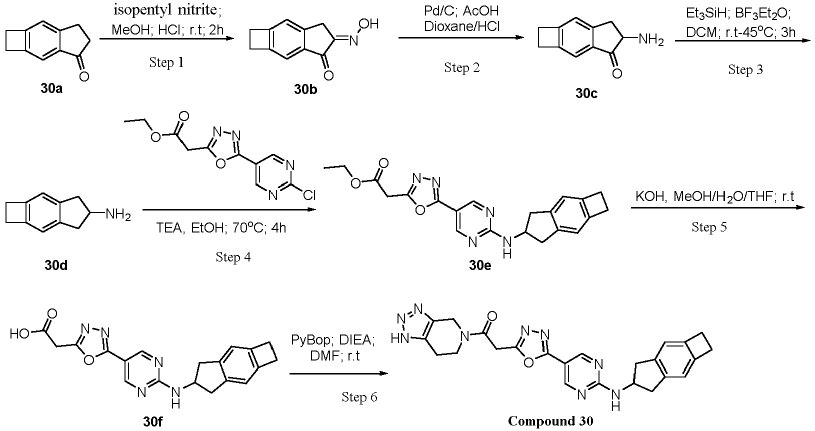

### Step 1: (E)-5-(hydroxyimino)-1,2,5,6-tetrahydro-4H-cyclobuta[f]inden-4-one (30b)

Intermediate **30a** (300 mg, 1.90 mmol) was dissolved in methanol (5 mL), concentrated hydrochloric acid (0.3 mL) was added, and then tert-pentyl nitrite (244 mg, 2.09 mmol) was added dropwise. The mixture was stirred at room temperature for 3 hours. A large number of white solids were precipitated and filtered. The filter cake was rinsed with a small amount of methanol, and spun to dryness under reduced pressure to obtain **30b** as a white solid (300 mg, 84.5%). LC-MS (ESI): m/z = 188.1 [M+H]⁺.

### Step 2: 5-amino-1,2,5,6-tetrahydro-4H-cyclobuta[f]inden-4-one (30c)

**30b** (300 mg, 1.6 mmol) was dissolved in acetic acid (10 mL), 4 N hydrochloric acid in dioxane (1 ml) was added, and then wet palladium carbon (100 mg) was added. The mixture was subjected to hydrogen gas replacement three times with a hydrogen balloon. The resulting mixture was reacted at room temperature overnight under a hydrogen atmosphere. After the reaction was completed, the reaction solution was filtered, and spun to dryness to obtain crude product **30c** as an off-white solid (250 mg). LC-MS (ESI): m/z = 174.1 [M+H]⁺.

### Step 3: 2,4,5,6-tetrahydro-1H-cyclobuta[f]inden-5-amine (30d)

Crude product **30c** (250 mg) was dissolved in DCM (10 mL), triethylhydrosilane (5 mL) was added, and then boron trifluoride diethyl etherate (5 mL) was added dropwise. The mixture was reacted at 45°C for 16 hours. After the reaction was completed, the reaction solution was concentrated, then basified by adding sodium bicarbonate aqueous solution, and extracted with ethyl acetate (30 mL × 2). The organic phase was combined, dried over sodium sulfate, filtered and spun to dryness to obtain **30d** as a reddish-brown solid (200 mg). LC-MS (ESI): m/z = 160.2 [M+H]⁺

### Steps 4, 5, and 6

2-(5-(2-((2,4,5,6-tetrahydro-1H-cyclobuta[f]inden-5-yl)amino)pyrimidin-5-yl)-1,3,4-oxadiazol-2-yl)-1-(1,4,6,7-tetrahydro-5H-[1,2,3]triazolo[4,5-c]pyridin-5-yl)ethan-1-one **(compound 30)**

With reference to the synthetic method of **the first to third steps for compound 29,** compound 30 was prepared.

LC-MS (ESI): m/z = 470.2 [M+H]⁺.

¹H NMR (400 MHz, DMSO-d₆) δ 8.89 - 8.73 (m, 2H), 8.34 (d, 1H), 6.93 (s, 2H), 4.75 (d, 2H), 4.65 (dd, 2H), 4.41 (d, 2H), 3.91 - 3.79 (m, 2H), 3.26 - 3.20 (m, 2H), 3.04 (s, 4H), 2.92 - 2.84 (m, 3H), 2.74 (t, 1H).

### Example 31: 2-(5-(2-((5-ethynyl-2,3-dihydro-1H-inden-2-yl)amino)pyrimidin-5-yl)-1,3,4-oxadiazol-2-yl)-1-(1,4,6,7-tetrahydro-5H-[1,2,3]triazolo[4,5-c]pyridin-5-yl)ethan-1-one (compound 31)

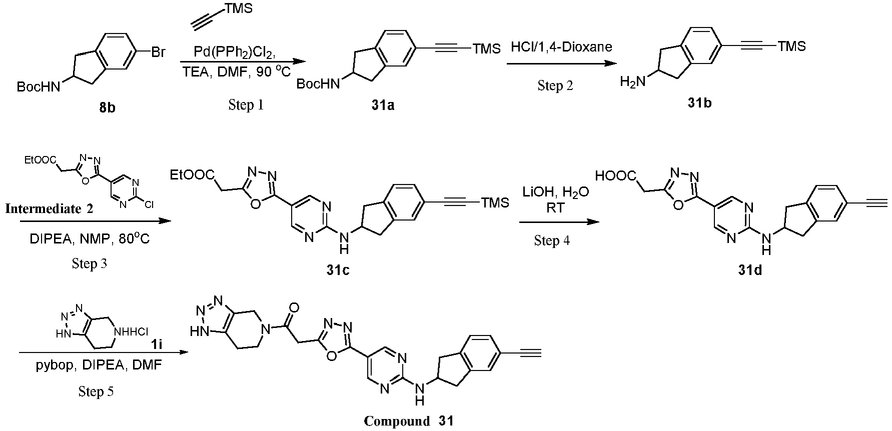

### Step 1: tert-butyl (5-((trimethylsilyl)ethynyl)-2,3-dihydro-1H-inden-2-yl) carbamate (31a)

Compound **8b** (1.0 g, 3.203 mmol), bistriphenylphosphine palladium dichloride (450 mg, 0.6406 mmol) and cuprous iodide (61 mg, 0.3203 mmol) were dissolved in DMF (32ml), and triethylamine (1.12 ml, 8.008 mmol) and trimethylsilylacetylene (378 mg, 3.844 mmol) were added. The mixture was reacted at 90°C for about 6 h under nitrogen protection. After the reaction was completed, the reaction solution was cooled to room temperature, filtered and washed with ethyl acetate. Water was added and the mixture was extracted with ethyl acetate. The organic phase was washed with saturated brine, dried over anhydrous sodium sulfate, filtered and concentrated. The silica gel column chromatography (PE : EA= 5 : 1) was used for purification to obtain product **31a** as a white solid (640 mg, 61%).

### Step 2: 5-((trimethylsilyl)ethynyl)-2,3-dihydro-1H-inden-2-amine (31b)

Compound **31a** (640 mg, 1.94 mmol) was dissolved in hydrochloric acid-1,4-dioxane (20 ml), and reacted at room temperature. After the reaction was completed, the reaction solution was concentrated. Product **31b** as a white solid (420 mg, 95%) was obtained.

### Step 3: ethyl 2-(5-(2-((5-((trimethylsilyl)ethynyl)-2,3-dihydro-1H-inden-2-yl)amino)pyrimidin-5-yl)-1,3,4-oxadiazol-2-yl)acetate (31c)

Compound **31b** (420 mg, 1.83 mmol), and **intermediate 2** (541 mg, 2.01 mmol) were dissolved in NMP (20 ml), and DIPEA (591 mg, 4.575 mmol) was added. The mixture was reacted at 80°C under nitrogen protection for 4 h. After the reaction was completed, water (100 ml) was added and the mixture was extracted with ethyl acetate. The organic phase was washed with saturated brine, dried over anhydrous sodium sulfate, filtered and concentrated. The silica gel column chromatography (DCM : MeOH = 60 : 1) was used for purification to obtain product **31c** as a white solid (640 mg, 79%).

### Step 4: 2-(5-(2-((5-ethynyl-2,3-dihydro-1H-inden-2-yl)amino)pyrimidin-5-yl)-1,3,4-oxadiazol-2-yl)acetic acid (31d)

Compound **31c** (640 mg, 1.77 mmol) was dissolved in methanol, and an aqueous solution of lithium hydroxide (149 mg, 3.54 mmol) was slowly added dropwise. The mixture was reacted at room temperature. After the reaction was completed, the mixture was concentrated, water was added and the resulting solution was filtered. The filtrate was adjusted to pH = 3-4 with HCl, and extracted with ethyl acetate (30 mL × 2). The organic phase was washed with saturated brine, dried over anhydrous sodium sulfate, filtered and concentrated to obtain product **31d** as a white solid (480 mg, 95%).

### Step 5: 2-(5-(2-((5-ethynyl-2,3-dihydro-1H-inden-2-yl)amino)pyrimidin-5-yl)-1,3,4-oxadiazol-2-yl)-1-(1,4,6,7-tetrahydro-5H-[1,2,3]triazolo[4,5-c]pyridin-5-yl)ethan-1-one (compound 31)

Compound **31d** (480 mg, 1.328 mmol) and **1i** (427 mg, 2.657 mmol) were dissolved in DMF, HATU (616 mg, 1.594 mmol) was added, and DIPEA (1.12 ml, 6.64 mmol) was added dropwise. The mixture was reacted at room temperature. After the reaction was completed, the mixture was diluted with water, and extracted with ethyl acetate. The organic layer was washed with saturated brine, dried over anhydrous sodium sulfate, and concentrated. The silica gel column chromatography (DCM : MeOH = 40 : 1) was used for purification to obtain **compound 31** (9 mg). LC-MS (ESI): m/z = 468.3 [M+H]⁺. ¹H NMR (400 MHz, DMSO-d₆) δ 8.80 (s, 2H), 8.39 (d, 1H), 7.34 (s, 1H), 7.26 (q, 2H), 4.81 (s, 1H), 4.71 (dd, 2H), 4.41 (d, 2H), 4.06 (s, 1H), 3.89 - 3.80 (m, 2H), 3.29 (m, 2H), 2.93 (m, 3H), 2.75 (t, 1H).

### Example 32: 2-(5-(2-((5-(1-methyl-1H-pyrazol-4-yl)-2,3-dihydro-1H-inden-2-yl)amino)pyrimidin-5-yl)-1,3,4-oxadiazol-2-yl)-1-(1,4,6,7-tetrahydro-5H-[1,2,3]triazolo[4,5-c]pyridin-5-yl)ethan-1-one (compound 32)

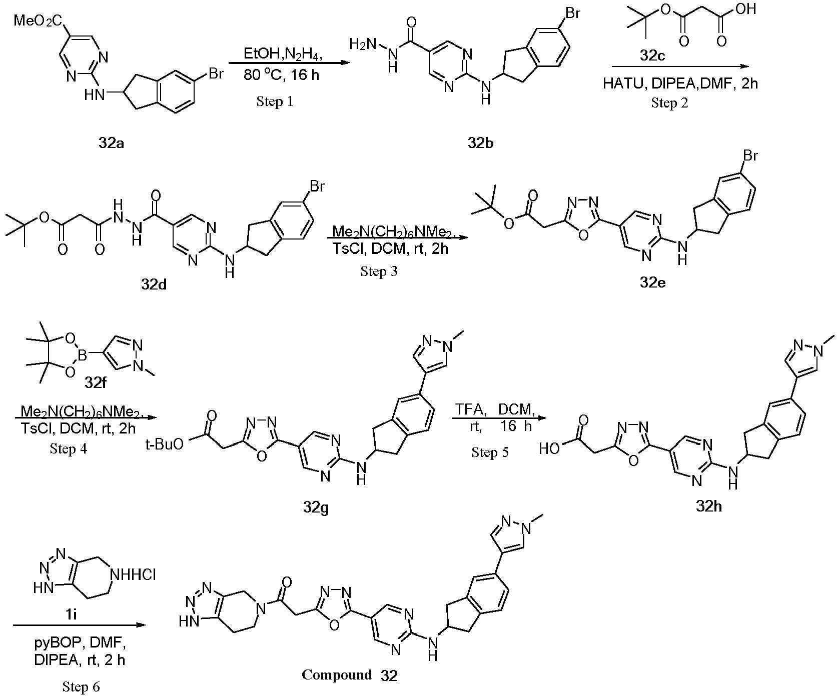

### Step 1: 2-((5-bromo-2,3-dihydro-1H-inden-2-yl)amino)pyrimidine-5-carbohydrazide (32b)

Hydrazine hydrate (16 ml) was added to a solution of compound **32a** (3.6 g, 2.0 mmol) in ethanol (40 mL), and the mixture was reacted at 80°C for 16 hours. After the reaction was completed, the reaction solution was filtered, washed with ethanol and spun to dryness to obtain the title compound **32b** as a grey solid (3.5 g, 100%). LC-MS (ESI): m/z = 348.1, 350.1 [M+H]⁺. ¹H NMR (400 MHz, DMSO-d₆) δ 9.57 (s, 1H), 8.69 (s, 2H), 8.05 (d, 1H), 7.42 (s, 1H), 7.32 (d, 1H), 7.18 (d, 1H), 4.69 - 4.64 (m, 1H), 4.40 (s, 2H), 3.28 - 3.16 (m, 2H), 2.94 - 2.83 (m, 2H).

### Step 2: tert-butyl 3-(2-(2-((5-bromo-2,3-dihydro-1H-inden-2-yl)amino)pyrimidine-5-carbonyl)hydrazinyl)-3-oxopropanoate (32d)

**32c** (0.55 g, 3.45 mmol), 2-(7-azabenzotriazole)-N,N,N',N'-tetramethyluronium hexafluorophosphate (1.30 g, 3.45 mmol) and N,N-diisopropylethylamine (0.93 g, 7.18 mmol) were added to a solution of compound **32b** (1.0 g, 2.87 mmol) in N,N-dimethylformamide (10 mL), and the mixture was reacted at room temperature for 2 hours. After the reaction was completed, the mixture was diluted with distilled water (30 mL), and extracted with dichloromethane (20 mL × 3). The organic phase was washed with distilled water and saturated brine, dried over anhydrous sodium sulfate and concentrated to obtain the title compound **32d** as brown solid (1.8 g, a crude product). LC-MS (ESI): m/z = 490.1, 492.1 [M-H]⁺.

### Step 3: tert-butyl 2-(5-(2-((5-bromo-2,3-dihydro-1H-inden-2-yl)amino)pyrimidin-5-yl)-1,3,4-oxadiazol-2-yl)acetate (32e)

4-toluene sulfonyl chloride (1.95 g, 10.20mmol) and N,N,N',N'-tetramethyl-1,6-hexanediamine (2.11 g, 12.23 mmol) were added to a solution of compound **32d** (1.8 g, 3.67 mmol) in dichloromethane (20 mL), and the mixture was reacted at room temperature for 2 hours. After the reaction was completed, the mixture was diluted with dichloromethane (30 mL). The organic phase was washed with distilled water and saturated brine, dried over anhydrous sodium sulfate and concentrated. The silica gel column chromatography (eluent: petroleum ether : ethyl acetate = 1 : 0-0 : 1) was used for purification to obtain the title compound **32e** as a grey solid (450 mg, two-step yield: 33%). LC-MS (ESI): m/z = 472.1, 474.1 [M+H]⁺. ¹H NMR (400 MHz, DMSO-d₆) δ 8.86 (s, 1H), 8.80 (s, 1H), 8.42 (d, 1H), 7.43 (s, 1H), 7.33 (d, 1H), 7.19 (d, 1H), 4.76 - 4.67 (m, 1H), 4.12 (s, 2H), 3.23 - 3.23 (m, 2H), 2.98 - 2.87 (m, 2H), 1.43 (s, 9H).

### Step 4: tert-butyl 2-(5-(2-((5-(1-methyl-1H-pyrazol-4-yl)-2,3-dihydro-1H-inden-2-yl)amino)pyrimidin-5-yl)-1,3,4-oxadiazol-2-yl)acetate (32g)

Compound **32f** (0.2 g, 0.9 mmol), tri-potassium phosphate monohydrate (1.23 g, 3.65 mmol) and bis(triphenylphosphine)palladium dichloride (0.04 g, 0.06 mmol) were added to a solution of compound **32e** (0.4 g, 0.8 mmol) in dioxane/water (10 ml/1 ml), and the mixture was stirred at 90°C for 4 hours. After the reaction was completed, the mixture was diluted with distilled water (100 mL), and extracted with ethyl acetate (100 mL × 4). The organic phase was washed with distilled water and saturated brine, dried over anhydrous sodium sulfate and concentrated. The silica gel column chromatography (eluent: petroleum ether : ethyl acetate = 1 : 0-0 : 1) was used for separation and purification to obtain the title compound **32g** as a white solid (0.2 g, 53%). LC-MS (ESI): m/z = 473.2 [M+H]⁺. ¹H NMR (400 MHz, DMSO-d₆) δ 8.86 (s, 1H), 8.81 (s, 1H), 8.42 (d, 1H), 8.06 (s, 1H), 7.78 (s, 1H), 7.42 (s, 1H), 7.35 (d, 1H), 7.19 (d, 1H), 4.76 - 4.70 (m, 1H), 4.11 (s, 2H), 3.85 (s, 3H), 3.23 - 3.24 (m, 2H), 2.98 - 2.89 (m, 2H), 1.43 (s, 9H).

### Step 5: 2-(5-(2-((5-(1-methyl-1H-pyrazol-4-yl)-2,3-dihydro-1H-inden-2-yl)amino)pyrimidin-5-yl)-1,3,4-oxadiazol-2-yl)acetic acid (32h)

Trifluoroacetic acid (4 ml) was added to a solution of compound **32g** (0.2 g, 0.42 mmol) in dichloromethane (6 mL), and the mixture was reacted at room temperature for 16 hours. After the reaction was completed, the reaction solution was diluted with dichloromethane (20 mL). The organic phase was washed with distilled water and saturated brine, dried over anhydrous sodium sulfate, and concentrated to obtain the title compound **32h** as a grey solid (165 mg, 94%). LC-MS (ESI): m/z = 418.1 [M+H]⁺.

### Step 6: 2-(5-(2-((5-(1-methyl-1H-pyrazol-4-yl)-2,3-dihydro-1H-inden-2-yl)amino)pyrimidin-5-yl)-1,3,4-oxadiazol-2-yl)-1-(1,4,6,7-tetrahydro-5H-[1,2,3]triazolo[4,5-c]pyridin-5-yl)ethan-1-one (compound 32)

Compound **1i** (0.29 g, 1.8 mmol), N,N-diisopropylethylamine (0.40 g, 3.1 mmol) and benzotriazol-1-yl-oxy-tripyrrolidinophosphonium hexafluorophosphate (0.48 g, 0.93 mmol) were sequentially added slowly to a solution of compound **32h** (0.25 g, 0.60 mmol) in N,N-dimethylformamide (6 mL), and the mixture was stirred at room temperature for 2 hours. After the reaction was completed, dichloromethane (20 mL) was added to the reaction solution, and the resulting mixture was diluted with distilled water (20 mL), and extracted with dichloromethane (20 mL × 3). The organic phase was washed with distilled water and saturated brine, dried over anhydrous sodium sulfate and concentrated. The silica gel column chromatography (eluent: dichloromethane : ethyl acetate : methanol = 1 : 0 : 0-2 : 10 : 1) was used for purification to obtain **compound 32** (19 mg, 6%).

LC-MS (ESI): m/z = 524.2 [M+H]⁺.

¹H NMR (400 MHz, DMSO-d₆) δ 8.85 (s, 1H), 8.80 (s, 1H), 8.39 (d, 1H), 8.06 (s, 1H), 7.80 (s, 1H), 7.42 (s, 1H), 7.34 (d, 1H), 7.19 (d, 1H), 4.81 (s, 1H), 4.75 - 4.70 (m, 1H), 4.68 (s, 1H), 4.43 (s, 1H), 4.39 (s, 1H), 3.87 - 3.83 (m, 5H), 3.32- 3.23 (m, 2H), 2.98 - 2.89 (m, 3H), 2.74 (t, 1H).

### Example 33: 2-(5-(2-((2,3-dihydro-1H-inden-2-yl)amino)-4-(trifluoromethyl)pyrimidin-5-yl)-1,3,4-oxadiazol-2-yl)-1-(1,4,6,7-tetrahydro-5H-[1,2,3]triazolo[4,5-c]pyridin-5-yl)ethan-1-one (compound 33)

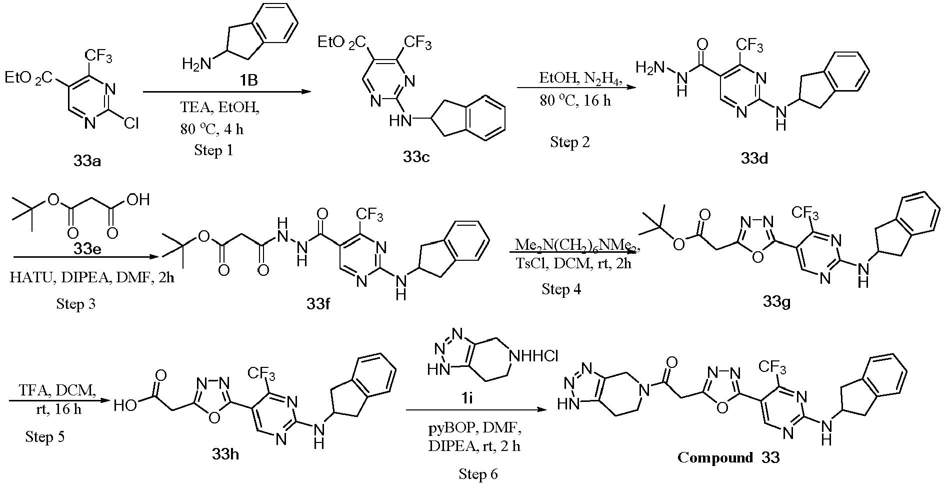

### Step 1: Ethyl 2-((2,3-dihydro-1H-inden-2-yl)amino)-4-(trifluoromethyl)pyrimidine-5-carboxylate (33c)

**1B** (1.6 g, 9.43 mmol) and triethylamine (3.18 g, 31.42 mmol) were added to a solution of compound **33a** (2.0 g, 7.86 mmol) in ethanol (40 mL), and the mixture was stirred at 80°C for 4 hours. After the reaction was completed, the mixture was diluted with distilled water (30 mL), and extracted with ethyl acetate (30 mL × 3). The organic phase was washed with distilled water and saturated brine, dried over anhydrous sodium sulfate and concentrated. The silica gel column chromatography (eluent: petroleum ether : ethyl acetate = 1 : 0-0 : 1) was used for separation and purification to obtain the title compound **33c** as an off-white solid (3.0 g, 100%). LC-MS (ESI): m/z = 352.1 [M+H]⁺. ¹H NMR (400 MHz, DMSO-d₆) δ 8.96 - 8.86 (m, 2H), 7.22 (s, 2H), 7.17 - 7.14 (m, 2H), 4.74 - 4.65 (m, 1H), 4.28 (q, 2H), 3.25 (dd, 2H), 3.06 (m, 2H), 1.29 (t, 3H).

### Step 2: 2-((2,3-dihydro-1H-inden-2-yl)amino)-4-(trifluoromethyl)pyrimidine-5-carbohydrazide (33d)

Hydrazine hydrate (6 ml) was added to a solution of compound **33c** (1.0 g, 2.85 mmol) in ethanol (10 mL), and the mixture was reacted at 80°C for 16 hours. After the reaction was completed, the reaction solution was filtered, washed with ethanol and spun to dryness to obtain the title compound **33d** as a grey solid (400 mg, 42%). LC-MS (ESI): m/z = 338.1 [M+H]⁺. ¹H NMR (400 MHz, DMSO-d₆) δ 8.52 (s, 1H), 7.22 (t, 1H), 7.17 - 7.14 (m, 2H), 7.11 - 7.08 (m, 2H), 4.68 - 4.53 (m, 1H), 3.68 (s, 1H), 3.26 (dd, 1H), 3.01 (dd, 2H), 2.91 (dd, 1H), 2.56 (dd, 2H).

### Step 3: tert-butyl 3-(2-(2-((2,3-dibydro-1H-inden-2-yl)amino)-4-(trifluoromethyl)pyrimidine-5-carbonyl)hydrazinyl)-3-oxopropanoate (33f)

33e (0.20 g, 1.2 mmol), 2-(7-azabenzotriazole)-N,N,N',N'-tetramethyluronium hexafluorophosphate (0.50 g, 1.2 mmol) and N,N-diisopropylethylamine (0.4 g, 3.0 mmol) were added to a solution of compound **33d** (0.40 g, 1.0 mmol) in N,N-dimethylformamide (6 mL), and the mixture was reacted at room temperature for 2 hours. After the reaction was completed, the mixture was diluted with distilled water (30 mL), and extracted with dichloromethane (20 mL × 3). The organic phase was washed with distilled water and saturated brine, dried over anhydrous sodium sulfate and concentrated to obtain the title compound **33f** as brown solid (800 mg, a crude product). LC-MS (ESI): m/z = 480.1 [M-H]⁺

### Step 4: tert-butyl 2-(5-(2-((2,3-dihydro-1H-inden-2-yl)amino)-4-(trifluoromethyl)pyrimidin-5 -yl)-1,3,4-oxadiazol-2-yl)acetate (33g)

4-toluene sulfonyl chloride (0.80 g, 4.0 mmol) and N,N,N',N'-tetramethyl-1,6-hexanediamine (0.90 g, 5.0 mmol) were added to a solution of compound **33f** (0.8 g, 2.0 mmol) in dichloromethane (10 mL), and the mixture was reacted at room temperature for 2 hours. After the reaction was completed, the mixture was diluted with dichloromethane (30 mL). The organic phase was washed with distilled water and saturated brine, dried over anhydrous sodium sulfate and concentrated. The silica gel column chromatography (eluent: petroleum ether : ethyl acetate = 1 : 0-0 : 1) was used for separation and purification to obtain the title compound **33g** as a grey solid (200 mg, two-step yield: 37%). LC-MS (ESI): m/z = 462.1 [M+H]⁺. ¹H NMR (400 MHz, DMSO-d₆) δ 8.96 (s, 1H), 8.28 (s, 1H), 7.24 - 7.21 (m, 2H), 7.17 - 7.13 (m, 2H), 4.76 - 4.67 (m, 1H), 4.14 (s, 2H), 3.30 (dd, 1H), 3.17 (dd, 1H), 2.97 (dd, 1H), 2.75 (dd, 1H), 1.39 (s, 9H).

### Step 5: 2-(5-(2-((2,3-dihydro-1H-inden-2-yl)amino)-4-(trifluoromethyl)pyrimidin-5 -yl)-1,3,4-oxadiazol-2-yl)acetic acid (33h)

TFA (4 ml) was added to a solution of compound **33g** (0.20 g, 0.43 mmol) in dichloromethane (6 mL), and the mixture was reacted at room temperature for 16 hours. After the reaction was completed, the reaction solution was diluted with dichloromethane (20 mL). The organic phase was washed with distilled water and saturated brine, dried over anhydrous sodium sulfate, and concentrated to obtain the title compound **33h** as a grey solid (150 mg, 86%). LC-MS (ESI): m/z = 406.1 [M+H]⁺.

### Step 6: 2-(5-(2-((2,3-dihydro-1H-inden-2-yl)amino)-4-(trifluoromethyl)pyrimidin-5-yl)-1,3,4-oxadiazol-2-yl)-1-(1,4,6,7-tetrahydro-5H-[1,2,3]triazolo[4,5-c]pyridin-5-yl)ethan-1-one (compound 33)

Compound **1i** (0.40 g, 2.48 mmol), N,N-diisopropylethylamine (0.40 g, 3.10 mmol) and benzotriazol-1-yl-oxy-tripyrrolidinophosphonium hexafluorophosphate (0.48 g, 0.93 mmol) were sequentially added slowly to a solution of compound **33h** (0.25 g, 0.62 mmol) in N,N-dimethylformamide (6 mL), and the mixture was stirred at room temperature for 2 hours. After the reaction was completed, dichloromethane (20 mL) was added to the reaction solution, and the resulting mixture was diluted with distilled water (20 mL), and extracted with dichloromethane (20 mL × 3). The organic phase was washed with distilled water and saturated brine, dried over anhydrous sodium sulfate and concentrated. The silica gel column chromatography (eluent: dichloromethane : ethyl acetate : methanol = 1 : 0: 0-2 : 10 : 1) was used for separation and purification to obtain **compound 33** (50 mg, 13%).

LC-MS (ESI): m/z = 512.1 [M+H]⁺.

¹H NMR (400 MHz, DMSO-d₆) δ 9.01 - 8.87 (m, 2H), 7.24 - 7.22 (m, 2H), 7.17 - 7.15 (m, 2H), 4.80 (s, 1H), 4.74 - 4.71 (m, 1H), 4.68 (s, 1H), 4.44 (d, 2H), 3.86 - 3.81 (m, 2H), 3.30 (dd, 2H), 2.97 (dd, 2H), 2.89 (t, 1H), 2.74 (t, 1H).

### Examples 34 and 35: (S)-1-(1,4,6,7-tetrahydro-5H-[1,2,3]triazolo[4,5-c]pyridin-5-yl)-2-(5-(2-((5-(trifluoromethyl)-2,3-dihydro-1H-inden-2-yl)amino)pyrimidin-5-yl)-1,3,4-oxadiazol-2-yl)ethan-1-one (compound 34) and (R)-1-(1,4,6,7-tetrahydro-5H-[1,2,3]triazolo [4,5 -c]pyridin-5-yl)-2-(5-(2-((5-(trifluoromethyl)-2,3 -dihydro-1H-inden-2-yl)amino)pyrimidin-5-yl)-1,3,4-oxadiazol-2-yl)ethan-1-one (compound 35)

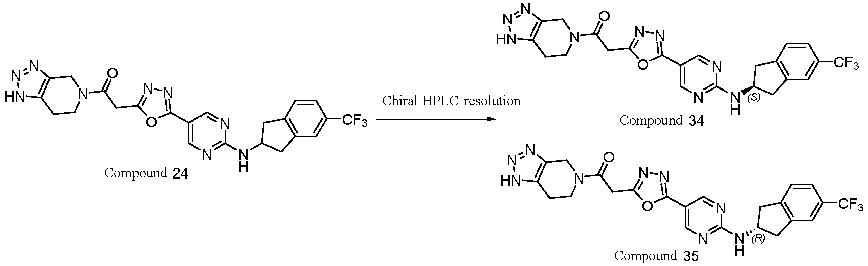

Compound **24** was resolved by chiral HPLC to obtain compound **34** and compound **35** (chiral HPLC resolution method: instrument name: MG II preparative SFC(SFC-14); chromatographic column: ChiralPak AD, 250×30mm I.D., 10µm; mobile phase: A (for CO₂) and B (for isopropanol) (0.1% NH₃H₂O); gradient: B 40%; flow rate: 80 mL/min; column pressure: 100 bar; column temperature: 38°C; absorption wavelength: 220 nm cycle time: about 8 min).

### Retention time for compound 34: 3.635 min

LC-MS (ESI): m/z = 512.2 [M+H]⁺, ¹H NMR (400 MHz, DMSO-d₆) δ 8.81-8.83 (m, 2H), 8.42 (d, 1H), 7.59 (s, 1H), .44-7.52 (m, 2H), 4.81 (s, 1H), 4.74-4.79 (m, 1H), 4.69 (s, 1H), 4.41 (d, 2H), 3.83-3.86 (m, 2H), 3.35-3.41 (m, 2H), 2.99-3.05 (m, 2H), 2.90-2.91 (m, 1H), 2.74-2.75 (m, 1H).

### Retention time for compound 35: 5.492 min

LC-MS (ESI): m/z = 512.2 [M+H]⁺, ¹H NMR (400 MHz, DMSO-d₆) δ 8.81-8.83 (m, 2H), 8.42 (d, 1H), 7.59 (s, 1H), .44-7.52 (m, 2H), 4.81 (s, 1H), 4.74-4.79 (m, 1H), 4.69 (s, 1H), 4.41 (d, 2H), 3.83-3.86 (m, 2H), 3.35-3.41 (m, 2H), 2.99-3.05 (m, 2H), 2.90-2.91 (m, 1H), 2.74-2.75 (m, 1H).

### Examples 36 and 37: (S)-2-(5-(2-((5-(difluoromethyl)-2,3-dihydro-1H-inden-2-yl)amino)pyrimidin-5-yl)-1,3,4-oxadiazol-2-yl)-1-(1,4,6,7-tetrahydro-5H-[1,2,3]triazolo[4,5-c]pyridin-5-yl)ethan-1-one (compound 36) and (R)-2-(5-(2-((5-(difluoromethyl)-2,3-dihydro-1H-inden-2-yl)amino)pyrimidin-5-yl)-1,3,4-oxadiazol-2-yl)-1-(1,4,6,7-tetrahydro-5H-[1,2,3]triazolo[4,5-c]pyridin-5-yl)ethan-1-one (compound 37)

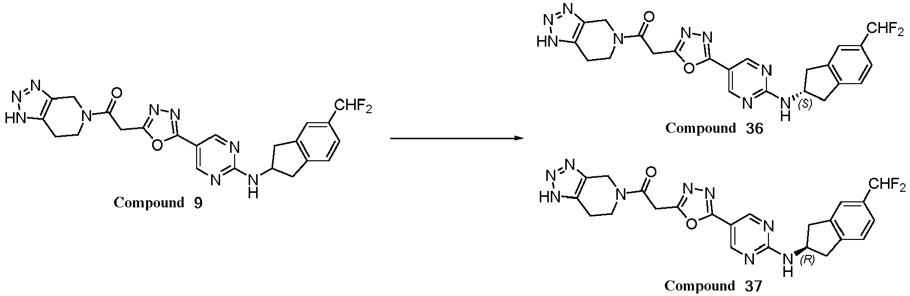

Compound **9** was resolved by chiral HPLC to obtain compound **36** and compound **37** (chiral HPLC resolution method: instrument name: MG II preparative SFC(SFC-1); chromatographic column: ChiralPak AD, 250×30mm I.D., 10µm; mobile phase: A (for CO₂) and B (for isopropanol); gradient: B 50%; flow rate: 80 mL/min; column pressure: 100 bar; column temperature: 38°C; absorption wavelength: 220 nm cycle time: about 7 min).

### Retention time for compound 36: 3.832 min

LC-MS (ESI): m/z = 494.3 [M+H]⁺, ¹H NMR (400 MHz, DMSO-d₆) *δ* 8.83 - 8.79 (m, 2H), 8.38 - 8.36 (m, 1H), 7.40 (d, *J* = 28 Hz, 3H), 6.97 (t, *J =* 56 Hz, 1H), 4.81 - 4.68 (m, 3H), 4.43 - 4.39 (m, 2H), 3.87 - 3.81 (m, 2H), 3.35 - 3.31 (m, 2H), 3.02 - 2.72 (m, 4H).

### Retention time for compound 37: 5.717 min

LC-MS (ESI): m/z = 494.3 [M+H]⁺, ¹H NMR (400 MHz, DMSO-d₆) *δ* 8.83 - 8.79 (m, 2H), 8.38 - 8.36 (m, 1H), 7.40 (d, *J* = 28 Hz, 3H), 6.97 (t, *J* = 56 Hz, 1H), 4.81 - 4.68 (m, 3H), 4.43 - 4.39 (m, 2H), 3.87 - 3.81 (m, 2H), 3.35 - 3.31 (m, 2H), 3.02 - 2.72 (m, 4H).

### Examples 38 and 39: (S)-2-(5-(2-((2,3-dihydro-1H-inden-2-yl)amino)pyrimidin-5-yl)-1,3,4-oxadiazol-2-yl)-2-fluoro-1-(1,4,6,7-tetrahydro-5H-[1,2,3]triazolo[4,5-c]pyridin-5-yl)propan-1-one and (R)-2-(5-(2-((2,3-dihydro-1H-inden-2-yl)amino)pyrimidin-5-yl)-1,3,4-oxadiazol-2-yl)-2-fluoro-1-(1,4,6,7-tetrahydro-5H-[1,2,3]triazolo[4,5-c]pyridin-5-yl)propan-1-one (compound 38 and compound 39)

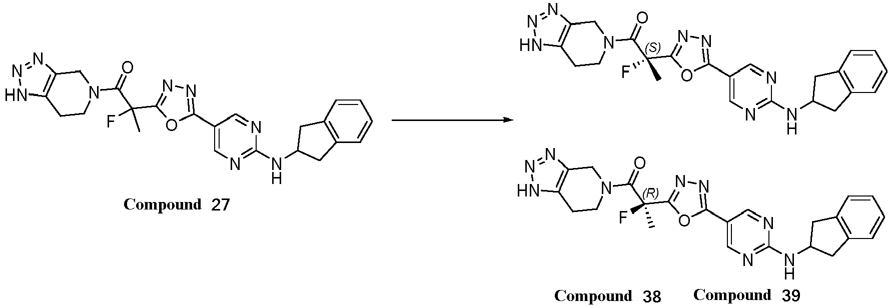

Compound 27 was resolved by chiral HPLC to obtain compound **38** (peak 1) and compound **39** (peak 2) (chiral HPLC resolution method: instrument name: MG II preparative SFC(SFC-14); chromatographic column: cellulose-2, 250 × 30 mm I.D., 5 µm; mobile phase: A (for CO₂) and B (for ethanol) (0.1% NH₃H₂O); gradient: B 45%; flow rate: 60 mL/min; column pressure: 100 bar; column temperature: 38°C; absorption wavelength: 220nm cycle time: about 9 min).

Retention time for peak 1: 4.300 min, LC-MS (ESI): m/z = 476.2 [M+H]⁺, ¹H NMR (400 MHz, DMSO-d₆) δ 8.92 - 8.81 (m, 2H), 8.50 (d, *J* = 8 Hz, 1H), 7.24 - 7.14 (m, 4H), 4.76 - 4.69 (m, 3H), 4.00 - 3.90 (m, 2H), 3.32 - 3.26 (m, 2H), 2.99 - 2.95 (m, 2H), 2.83 - 2.81 (m, 2H), 2.16 - 2.07 (m, 3H).

Retention time for peak 2: 5.784 min, LC-MS (ESI): m/z = 476.2 [M+H]⁺, ¹H NMR (400 MHz, DMSO-d₆) δ 8.92 - 8.81 (m, 2H), 8.50 *(d, J=* 8 Hz, 1H), 7.24 - 7.14 (m, 4H), 4.76 - 4.69 (m, 3H), 4.00 - 3.90 (m, 2H), 3.32 - 3.26 (m, 2H), 2.99 - 2.95 (m, 2H), 2.83 - 2.81 (m, 2H), 2.16 - 2.07 (m, 3H).

### Examples 40 and 41: (S)-2-(5-(2-((5-ethynyl-2,3-dihydro-1H-inden-2-yl)amino)pyrimidin-5-yl)-1,3,4-oxadiazol-2-yl)-1-(1,4,6,7-tetrahydro-5H-[1,2,3]triazolo[4,5-c]pyridin-5-yl)ethan-1-one and (R)-2-(5-(2-((5-ethynyl-2,3-dihydro-1H-inden-2-yl)amino)pyrimidin-5-yl)-1,3,4-oxadiazol-2-yl)-1-(1,4,6,7-tetrahydro-5H-[1,2,3]triazolo[4,5-c]pyridin-5-yl)ethan-1-one (compound 40 and compound 41)

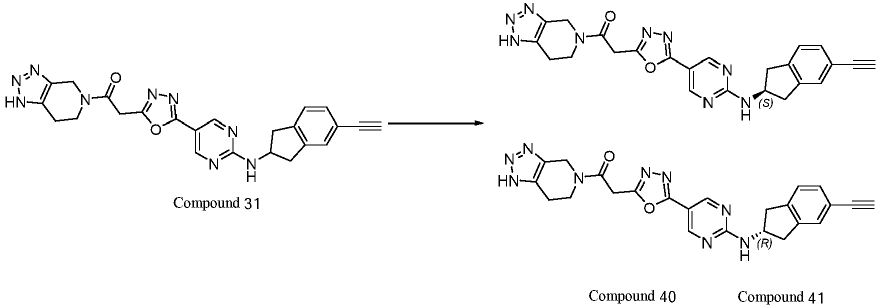

Compound **31** was resolved by chiral HPLC to obtain compound **40** (peak 1) and compound **41** (peak 2) (chiral HPLC resolution method: instrument name: MG II preparative SFC(SFC-14); chromatographic column: cellulose-2, 250 × 30 mm I.D., 5 µm; mobile phase: A (for CO₂) and B (for ethanol) (0.1% NH₃H₂O); gradient: B 45%; flow rate: 60 mL/min; column pressure: 100 bar; column temperature: 38°C; absorption wavelength: 220 nm cycle time: about 9 min).

Retention time for peak 1: 7.004 min, LC-MS (ESI): m/z = 468.2 [M+H]⁺, ¹H NMR (400 MHz, DMSO-d₆) δ 14.63 (s, 1H), 8.80 (s, 2H), 8.39 (d, 1H), 7.34 (s, 1H), 7.26 (q, 2H), 4.81 (s, 1H), 4.71 (dd, 2H), 4.41 (d, 2H), 4.07 - 4.04 (m, 1H), 3.89 - 3.79 (m, 2H), 3.27 (d, 2H), 2.99 - 2.88 (m, 3H), 2.75 (s, 1H).

Retention time for peak 2: 8.086 min, LC-MS (ESI): m/z = 468.2 [M+H]⁺, 1H NMR (400 MHz, DMSO-d₆) δ 14.65 (s, 1H), 8.80 (s, 2H), 8.39 (d, 1H), 7.34 (s, 1H), 7.30 - 7.21 (m, 2H), 4.81 (s, 1H), 4.77 - 4.63 (m, 2H), 4.41 (d, 2H), 4.06 (s, 1H), 3.89 - 3.78 (m, 2H), 3.27 (d, 2H), 2.93 (dt, 3H), 2.75 (t, 1H).

### Examples 42 and 43: (S)-2-(5-(2-((5-(furan-3-yl)-2,3-dihydro-1H-inden-2-yl)amino)pyrimidin-5-yl)-1,3,4-oxadiazol-2-yl)-1-(1,4,6,7-tetrahydro-5H-[1,2,3]triazolo[4,5-c]pyridin-5-yl)ethan-1-one and (R)-2-(5-(2-((5-(furan-3-yl)-2,3-dihydro-1H-inden-2-yl)amino)pyrimidin-5-yl)-1,3,4-oxadiazol-2-yl)-1-(1,4,6,7-tetrahydro-5H-[1,2,3]triazolo[4,5-c]pyridin-5-yl)ethan-1-one (compound 42 and compound 43)

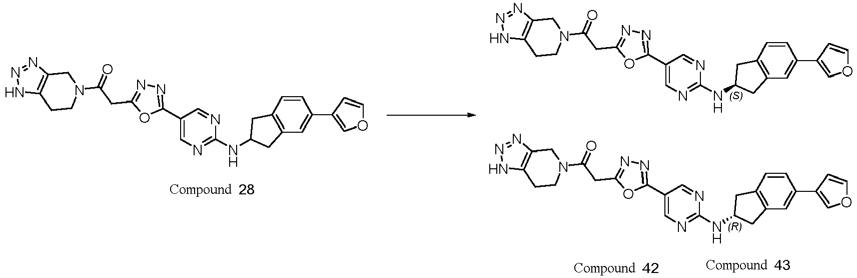

Compound **28** was resolved by chiral HPLC to obtain compound **42** (peak 1) and compound **43** (peak 2) (chiral HPLC resolution method: instrument name: MG II preparative SFC(SFC-14); chromatographic column: cellulose-2, 250 × 30 mm I.D., 5 µm; mobile phase: A (for CO₂) and B (for Ethanol) (0.1% NH₃H₂O); gradient: B 45%; flow rate: 60 mL/min; column pressure: 100 bar; column temperature: 38°C; absorption wavelength: 220 nm cycle time: about 9 min).

Retention time for peak 1: 4.886 min, LC-MS (ESI): m/z = 510.1 [M+H]⁺.

Retention time for peak 2: 5.716 min, LC-MS (ESI): m/z = 510.1 [M+H]⁺.

### Biological test

### 1. ATX inhibitory activity

Experimental subject: compounds to be tested.

ATX activity was detected by detecting LPA production in pooled human plasma. Whole blood anticoagulated with heparin was collected and centrifuged, and then plasma was collected. 5-µL gradient dilution of the compound to be tested or DMSO was added to 95-µL plasma, incubated at 37°C for 2 h, then a stop solution (40 mM disodium hydrogen phosphate buffer, containing a buffer solution (containing 30 mM citric acid, pH = 4)) was added. LPA in plasma was detected by LC-MS-MS before and after incubation.
LPA (18 : 2) and (20 : 4) LC-MS-MS analytical method

### 1 Test method

### 1.1 Instrument

| Liquid chromatography system | Shimadzu LC-30A binary pump |
|---|---|
| MS/MS system | AB Sciex API4500 tandem mass spectrometer, equipped with an electro-spray ionization source (ESI source), Sciex |
| Data acquisition | Analyst 1.6.3 software, Applied Biosystems, Inc. (USA) |
| Pipette | Pipette range 1.00 - 10.0 µL; 10.0 - 100.0 µL; 20.0 - 200 µL; 100 - 1000 µL; Eppendorf, Germany |
| Centrifuge | Legend Micro 17R multifunctional desktop centrifuge, Thermo Scientific |
| Balance | CPA225D, Sartorius Instrument and System Co., Ltd. of Beijing |
| 96-well plate vortex mixer | DMT-2500 type, Hangzhou Miu Instruments Co., Ltd. |
| Mixer | Vortex |

### 1.2 Chromatography conditions and mass spectrum conditions

### Chromatography conditions

| Analytical column | 2.1 × 50 mm ACQUITY UPLC BEH C18 1.7µm |
|---|---|
| Mobile phase | phase A: 20 mM NH₄Ac and 0.1% FA in H2O |
| | Phase B: 5 mM NH₄AC in Water/0.2% FA in ACN = 5:95 |
| Gradient elution: | 0.0 min, 55% phase B; |
| | 0.5 min, 0.3 mL/min; |
| | 0.7 min, 0.5 mL/min; |
| | 0.7 min, 55% phase B; |
| | 2.3 min, 95% phase B; |
| | 2.7 min, 95% phase B; |
| | 2.8 min, 55% phase B; |
| | 3.2 min, 0.5 mL/min; |
| | 3.3 min, 0.3 mL/min; |
| | 4 min, stop |
| Initial flow rate | 0.3 mL/min |
| Injection volume | 8 µL |
| Autosampler | 6°C |
| Column temperature | 40°C |

### Mass spectrum conditions

| Ion source | ESI | | Scanning mode: | | MRM | |
|---|---|---|---|---|---|---|
| Detection mode: | Negative ions | | Spray voltage; | | -4500 V | |
| Ion source gas 1: | 60 psi | | Ion source gas 2: | | 60 psi | |
| Ion source temperature: | 600°C | | Curtain gas: | | 25 psi | |
| Collision gas pressure (CAD): | 10 psi | | Dwell time: | | 150 ms | |

| Compounds | Detection ion pair Ion Transition | | Polarity | DP(V) | CE(eV) | CXP(V) |
|---|---|---|---|---|---|---|
| | Q1 (m/z) | Q3 (m/z) | | | | |
| LPA18:2 | 433.2 | 152.8 | Negative | -88 | -28 | -8 |
| LPA20:4 | 457.2 | 152.8 | Negative | -96 | -30 | -9 |
| LPA17:0 (IS) | 423.3 | 152.8 | Negative | -84 | -30 | -17 |

### 1.3 Plasma sample pretreatment

Using a 1.5 mL EP tube, 3.0 µL of each of the working solutions for standard curve and quality control was taken and added to 27.0 µL of water. After homogeneously mixing by vortex, 400 µL of internal standard solution was added. After homogeneously mixing by vortex, the mixture was centrifuged at 4°C for 10 min. 180 µL of supernatant was taken and added into a 96-well plate, waiting for sample injection.

### 1.4 Linear range

### 1.4.1 Stock solution

10 µL of the compound to be tested (10 mg/ml) was precisely pipetted into an EP tube, 990 µL of DMSO solution was added, and the mixture was vortexed to completely dissolve to obtain a solution (100 µg/mL).

### 1.4.2 Working solution

Solvent: n-Butanol
Series of working solution concentration for standard curve: 5 µg/mL, 2 µg/mL, 1 µg/mL, 500 ng/mL, 200 ng/mL, 100 ng/mL, 50 ng/mL, 20 ng/mL, and 10 ng/mL
Working solution concentration for quality control: 4 µg/mL, 800 ng/mL, and 30 ng/mL

### 1.4.3 Internal standard solution (solvent: n-butanol)

LPA17: 025.0 ng·mL⁻¹
Inhibition rate of ATX receptor activity was calculated in the following way: inhibition rate % = ((LPAc-LPA0)-(LPAi-LPA0))/(LPAc-LPA0)^{∗}100%, wherein LPAc was the LPA content in plasma after DMSO was added and incubated for 2 h, LPA0 was the LPA content in plasma before DMSO was added for incubation, and LPAi was the LPA content in plasma after test compound was added and incubated for 2 h.

Test results: The compounds of the present invention showed inhibitory activity against ATX receptor, and the IC50 values of the example compounds against LPA 18 : 2/20 : 4 cells were in the range of 0.01-20 nM. The test results of some examples were shown in Table 1:

**Table 1 Inhibitory activity against ATX receptor**

| Compound No. | LPA 18 : 2/20 : 4 IC50/nM | Compound No. | LPA 18 : 2/20 : 4 IC50/nM |
|---|---|---|---|
| 1 | 8.526/- | 27 | 6.26/7.08 |
| 2 | 6.19/6.85 | 28 | 5.24/5.40 |
| 3 | 5.68/5.65 | 29 | 2.75/3.27 |
| 4 | 4.93/5.33 | 30 | 1.58/1.92 |
| 7 | 7.46/7.67 | 31 | 2.61/2.85 |
| 8 | 5.94/6.18 | 35 | 2.17/2.25 |
| 9 | 3.26/3.65 | 37 | 4.84/4.78 |
| 14 | 5.97/6.56 | 38 | 5.53/5.88 |
| 20 | 3.65/3.76 | 40 | 1.51/1.56 |
| 22 | 4.97/6.84 | 42 | 2.86/2.69 |
| 26 | 8.73/8.86 | | |

Conclusion: The compounds of the present invention showed relatively high inhibitory activity against ATX receptor.

### 2. Pharmacokinetic experiment in mice

**Experimental objective:** In this experiment, a single dose of each test compound was administered to C57 mice intravenously and intragastrically, the concentrations of the compounds 35, 37 and 38 in plasma of the mice were measured, and the pharmacokinetic characteristics and bioavailability of each test compound in mice were evaluated.

**Experimental animals:** Male C57 mice, 20-25 g, 6-8 weeks old, 18 mice/compound. The experimental animals were purchased from CHENGDU DOSSY

### EXPERIMENTAL ANIMALS CO., LTD.

**Experimental method:** On the day of the experiment, 18 C57 mice were randomly grouped according to their body weight. The animals were fasted with water available for 12 to 14 hours one day before the administration of a test compound, and were fed 4 hours after the administration.

**Table 2 Administration information**

| Gr ou ps | Nu mbe r | Administration information | | | | | | |
|---|---|---|---|---|---|---|---|---|
| | Mal e | Test compo und | Administr ation dosage^{∗} (mg/kg) | Administ ration concentr ation (mg/mL) | Administr ation volume (mL/kg) | Collect ed sample s | Mode of admini stratio n | Vehicle |
| G1 | 9 | Comp ounds | 2.5 | 0.5 | 5 | Plasma | Intrave nously | 5% DMA+5% Solutol+90% Saline |
| G2 | 9 | Comp ounds | 10 | 1 | 10 | Plasma | Gavag e | 0.5%MC (methyl cellulose) |

| | | | | | | | | |
|---|---|---|---|---|---|---|---|---|
| Note: ^{∗} Dosage is calculated based on free base. | | | | | | | | |

### Sampling

Before and after the administration, 0.08 ml of blood was taken from the orbit of the rats under isoflurane anesthesia, and placed in an EDTAK2 centrifuge tube. Centrifugation was carried out at 5000 rpm at 4°C for 10 min and the plasma was collected.

Time points for plasma collection in G1 group: 0,5, 15, 30 min, 1, 2, 4, 6, 8, and 24 h.

Time points for plasma collection in G2 group: 0, 15, 30 min, 1, 2, 4, 6, 8, and 24 h.

Before analysis and detection, all samples were stored at -80°C.

### Sample pretreatment

30 µL of each of plasma sample, standard curve and quality control sample was taken, and 200 µL of acetonitrile solution containing internal standard was added, and the mixture was homogeneously mixed by vortex, and then centrifuged at 12000 rpm for 10 min at 4°C. The supernatant was taken and added into a 96-well plate and analyzed by LC-MS/MS.

The main pharmacokinetic parameters were analyzed by non-compartmental model using WinNonlin 8.0 software. The experimental results were as shown in the following table.

**Table 3 Pharmacokinetics of compounds 35, 37 and 38 in mice**

| Compound No. | Mode of administration | C₀ or Cmax (ng/ml) | AUC (h^{∗}ng/ml) | T_{1/2} (h) | Tₘₐₓ (h) | F% |
|---|---|---|---|---|---|---|
| 37 | iv 2.5 mg/kg | 6640 | 3787 | 0.888 | - | - |
| | po 10 mg/kg | 1596 | 2696 | 1.31 | 0.25 | 17.8 |
| 35 | iv 2.5 mg/kg | 6854 | 6782 | 1.02 | - | - |
| | po 10 mg/kg | 3374 | 9647 | 0.947 | 0.5 | 35.6 |
| 38 | iv 2.5 mg/kg | 3332 | 1478 | 0.552 | - | - |
| | po 10 mg/kg | 3588 | 3504 | 0.805 | 0.25 | 59.3 |

Conclusion: The compounds of the present invention have relatively high bioavailability and good pharmacokinetic characteristics.

## Claims

1. A nitrogen-containing heterocyclic compound as shown in formula (I), or a stereoisomer, a solvate, a deuterated form, a pharmaceutically acceptable salt or a cocrystal thereof, 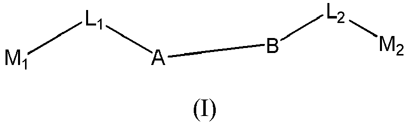
wherein M₁ is 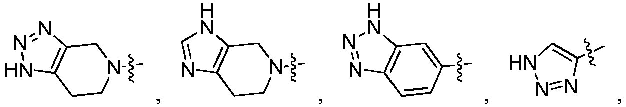 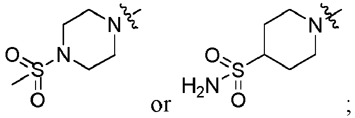
L₁ is a bond, -(CR₁R₂)ₐ-(NR₇)_{b}-W-(CR₃R₄)_{c}-(NR₈)_{d}-(CR₅R₆)ₑ- or -C(O)-(CR₃R₄)_{c}-C=CR₇-;
W is -C(=X)- or a 3-6 membered heterocyclene containing 1-3 heteroatoms selected from N, O or S;
X is O, S or NRₓ, wherein Rₓ is H or cyano;
each of R₁ to R₆ is independently selected from H, halogen, C₁₋₄ alkyl or C₃₋₆ cycloalkyl;
R₇ and Rs are each independently selected from H, C₁₋₄ alkyl or C₃₋₆ cycloalkyl;
alternatively, R₁ and R₂ on the same carbon atom, R₃ and R₄ on the same carbon atom, or R₅ and R₆ on the same carbon atom together with the carbon atom to which they are attached form a 3-6-membered carbocyclic ring, wherein the carbocyclic ring is optionally substituted with 1-4 substituents selected from halogen or C₁₋₄ alkyl;
a, c and e are independently selected from an integer of 0-5, and b and d are independently 0 or 1;
A is C₃₋₆ cycloalkylene, 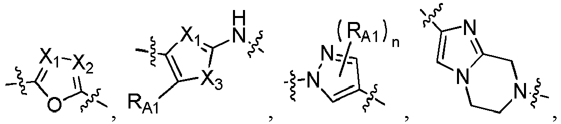 C₂₋₄ alkynylene, -RaC(O)NRa'-, -RaNRa'C(O)-, -RaNRa'-, -RaC(O)-, -Ra(CRa' Ra")ₙ- or a bond;
Ra is 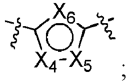
Ra' and Ra" are independently H or C₁₋₄ alkyl;
n is an integer of 1-2;
X₁ and X₂ are independently N or CR_{A1}, and are not both CR_{A1} at the same time, and X₃ is S, O or NR_{A1};
X₄, X₅ and X₆ are independently N, NR_{A1}, S, O or CR_{A1}, and are not all CR_{A1} at the same time;
each R_{A1} is independently H, cyano, -R_{A}, halogen, -C₁₋₄ alkyl R_{A}, -NHC(O)R_{A}, - C(O)R_{A}, -C₁₋₄ alkyl-O-C₁₋₄ alkyl, -NHR_{A}, -C(O)NHR_{A} or -OR_{A};
R_{A} is C₁₋₄ alkyl, C₃₋₆ cycloalkyl, C₃₋₆ cycloalkyloxy, C₁₋₄ alkoxy, C₁₋₄ haloalkyl, C₁₋₄ haloalkoxy or a 3-6 membered heterocyclyl containing 1-3 heteroatoms selected from N, O or S, wherein the alkyl, cycloalkyl, cycloalkyloxy, alkoxy, haloalkyl, haloalkoxy and heterocyclyl are optionally further substituted with 1-6 groups selected from C₃₋₆ cycloalkyl, C₁₋₄ alkyl, halogen, -S(O)₂C₁₋₄ alkyl, -OC₁₋₄ alkyl or cyano;
B is 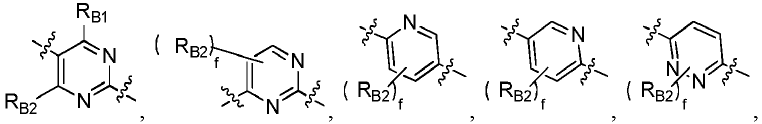 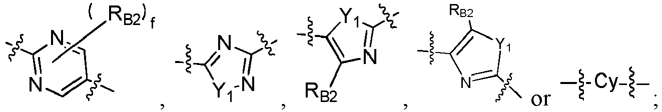
Y₁ is O, S or NR_{B3};
Cy has a structure: 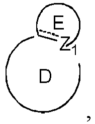 wherein, 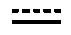 represents a single bond or a double bond, Z₁ is C or N, ring E is a 5-membered heterocycle containing 1-3 heteroatoms selected from N, O or S, and ring D is a 6-membered ring containing 0-3 heteroatoms selected from N, O or S; the 5-membered heterocycle and the 6-membered ring are independently and optionally substituted with 1-3 R_{B2};
R_{B1} and R_{B2} are each independently selected from H, oxo, OH, halogen, cyano, C₁₋₄ alkyl, halo C₁₋₄ alkyl, C₁₋₄ alkyloxy or C₃₋₆ cycloalkyl; R_{B3} is selected from H, C₁₋₄ alkyl or C₃₋₆ cycloalkyl;
alternatively, group R_{A1} on A and group R_{B1} on B together with the atoms to which they are attached form a 6-10-membered heterocycle containing 1-3 heteroatoms selected from N, S, and O;
f is an integer of 0-3;
L₂ is -O-, -NR₉-, -C(O)NR₉-, -NR₉C(O)NR₉-, -(CR₁₀R₁₁)_{g}-, -NR₉-(CR₁₀R₁₁)_{g}- or a bond, wherein g is an integer of 1-3;
R₉ is H or C₁₋₄ alkyl;
R₁₀ and R₁₁ are each independently H, halogen, C₁₋₄ alkyl or C₃₋₆ cycloalkyl; alternatively, R₁₀ and R₁₁ on the same carbon atom together with the carbon atom to which they are attached form a 3-6 membered carbocyclic ring;
M₂ is 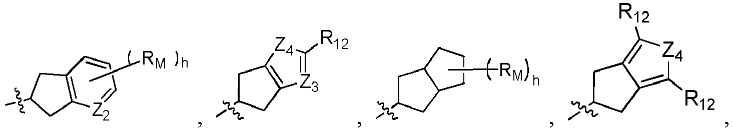 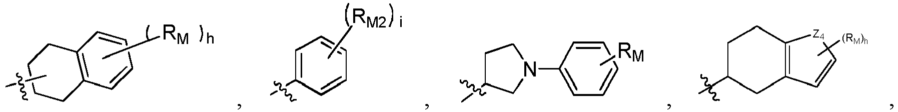 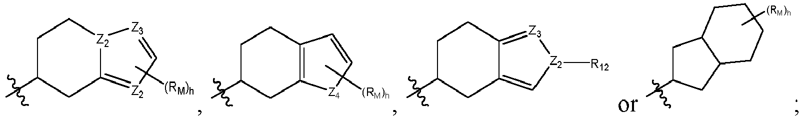
Z₂ and Z₃ are each independently CR_{M} or N, and Z₄ is O, S or NR_{M1};
each R_{M} is independently H, C₁₋₄ alkyl, C₁₋₄ alkoxyalkyl, cyano, C₃₋₆ cycloalkyl, C₃₋₆ cycloalkyloxy, -SRm', -S(O)₂Rm', -C(O)NRmRm', -NRmC(O)Rm', C₂₋₆ alkenyl, C₂₋₆ alkynyl, -NR_{M1}, 3-6 membered heterocyclyl containing 1-3 heteroatoms selected from N, O or S or halogen, wherein the alkyl, alkenyl, alkynyl and cycloalkyl are each optionally substituted with 1-3 groups selected from halogen, cyano, C₁₋₄ alkoxy, halo C₁₋₄ alkyl or halo C₁₋₄ alkoxy, and the heterocyclyl is optionally substituted with 1-3 groups selected from halogen, oxo or C₁₋₄ alkyl;
alternatively, two R_{M} on adjacent ring carbon atoms in M₂ together with the carbon atom to which they are attached form a 3-6 membered carbocyclic ring or a 3-6 membered heterocyclyl containing 0-3 heteroatoms selected from N, O or S;
Rm is H, C₁₋₄ alkyl or halo C₁₋₄ alkyl;
Rm' is C₁₋₄ alkyl or halo C₁₋₄ alkyl;
R_{M1} is H, C₁₋₄ alkyl, C₃₋₆ cycloalkyl, C₁₋₄ haloalkyl or C₁₋₄ alkoxyalkyl;
R_{M2} is halo C₁₋₄ alkoxy;
each R₁₂ is independently H, halogen, C₁₋₄ alkyl, C₃₋₆ cycloalkyl or halo C₁₋₄ alkyl;
h is an integer of 0-3;
i is 0, 1 or 2;
provided that: when M₁ is 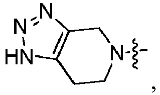 L₁ is -C(O)-CH₂-, A is 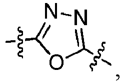 B is 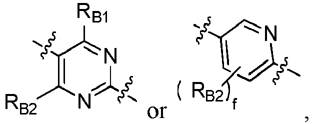 L₂ is -NH-, and M₂ is 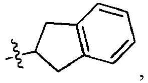 R_{B1} and R_{B2} are not both H at the same time;
when M₁ is 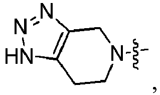 L₁ is -C(O)-CH₂-, A is 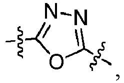 B is 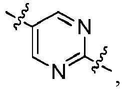 and L₂ is -NH-CH₂-, M₂ is not 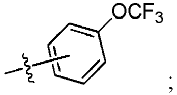
when M₁ is 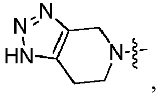 L₁ is -C(O)-CH₂-, A is 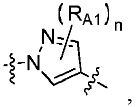 B is 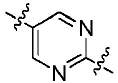 or 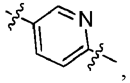 L₂ is -NH-, and M₂ is 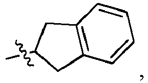 at least one substituent R_{A1} in A is selected from cyano, halogen, C₃₋₄ cycloalkyloxy, cyclopropylmethyloxy, C₁₋₄ haloalkoxy, cyclopropylmethyl, -C₁₋₄ alkyl-O-C₁₋₄ alkyl, -NHC(O) R_{A}, -C(O)NHR_{A}, -C(O)-C₃₋₆ cycloalkyl, 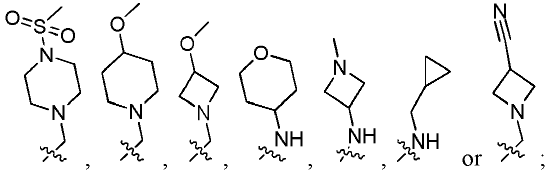
and when M₁ is 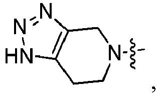 L₁ is -C(O)-CH₂-, A is 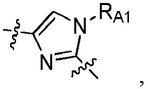 B is 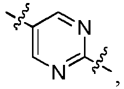 L₂ is -NH-, and M₂ is 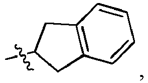 R_{A1} is not methyl, ethyl or cyclopropyl.

2. The nitrogen-containing heterocyclic compound according to claim 1, or the stereoisomer, the solvate, the deuterated form, the pharmaceutically acceptable salt or the cocrystal thereof, wherein
a, c and e are independently selected from an integer of 0-3, and b and d are independently 0 or 1;
W is -C(=X)- or a 5-membered heterocyclene containing 1-2 heteroatoms selected from N and O, wherein X is O, S or NRₓ, and Rₓ is cyano;
each of R₁ to R₆ is independently selected from H, halogen, and C₁₋₄ alkyl, and R₇ and R₈ are independently selected from H or C₁₋₄ alkyl;
alternatively, R₃ and R₄ on the same carbon atom, or R₅ and R₆ on the same carbon atom together with the carbon atom to which they are attached form a 3-4-membered carbocyclic ring, wherein the carbocyclic ring is optionally substituted with 1-2 substituents selected from halogen or C₁₋₂ alkyl;
A is C₃₋₆ cycloalkylene, 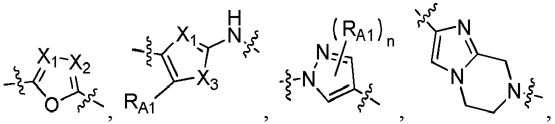 C₂₋₄ alkynylene or a bond;
R_{A} is C₁₋₄ alkyl, C₃₋₄ cycloalkyl or a 4-6-membered heterocyclyl containing 1-2 heteroatoms selected from N and O, wherein the alkyl and heterocyclyl are each optionally further substituted with 1-5 groups selected from C₃₋₄ cycloalkyl, C₁₋₄ alkyl, halogen, - S(O)₂C₁₋₂ alkyl, -OC₁₋₂ alkyl or cyano.

3. The nitrogen-containing heterocyclic compound according to claim 1 or 2, or the stereoisomer, the solvate, the deuterated form, the pharmaceutically acceptable salt or the cocrystal thereof, wherein
L₁ is -C(=O)-(NH)_{d}-CR₃R₄-, d is 0 or 1, at least one of R₃ and R₄ is halogen, or R₃ and R₄ and the carbon atom to which they are attached form a 3-4-membered carbocyclic ring; or
L₁ is -C(=S)-CR₃R₄-, wherein R₃ and R₄ are independently selected from H, halogen or C₁₋₄ alkyl; or
L₁ is -C(=N-CN)-CR₃R₄-, wherein R₃ and R₄ are independently selected from H, halogen or C₁₋₄ alkyl; or
L₁ is -C(O)-CR₃R₄-C=CR₇-, wherein R₃ and R₄ are independently selected from H, halogen or C₁₋₄ alkyl, and R₇ is H or C₁₋₄ alkyl; or
L₁ is -W-(CR₃R₄)_{c}-, wherein W is a 5-membered heterocyclene containing 1-2 heteroatoms selected from N and O, and c is an integer of 0-3.

4. The nitrogen-containing heterocyclic compound according to claim 3, or the stereoisomer, the solvate, the deuterated form, the pharmaceutically acceptable salt or the cocrystal thereof, wherein the compound has a structure as shown in formula (II): 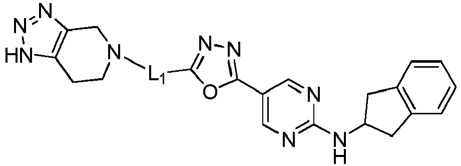 (II), wherein L₁ is as defined in formula (I).

5. The nitrogen-containing heterocyclic compound according to claim 3, or the stereoisomer, the solvate, the deuterated form, the pharmaceutically acceptable salt or the cocrystal thereof, wherein the 5-membered heterocyclene is

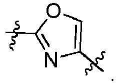

6. The nitrogen-containing heterocyclic compound according to claim 1 or 2, or the stereoisomer, the solvate, the deuterated form, the pharmaceutically acceptable salt or the cocrystal thereof, wherein:
M₁ is 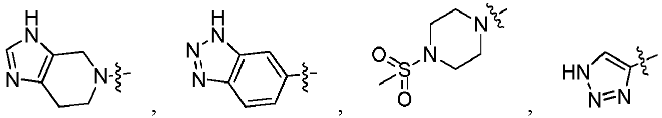 or

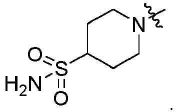

7. The nitrogen-containing heterocyclic compound according to claim 6, or the stereoisomer, the solvate, the deuterated form, the pharmaceutically acceptable salt or the cocrystal thereof, wherein M₁ is 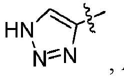 A is 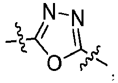 B is 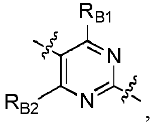 L₂ is -NH- and M₂ is 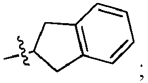 or M₁ is 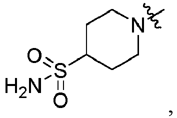 A is 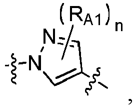 B is 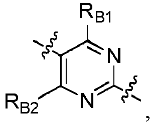 L₂ is -NH-, and M₂ is

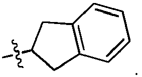

8. The nitrogen-containing heterocyclic compound according to claim 1 or 2, or the stereoisomer, the solvate, the deuterated form, the pharmaceutically acceptable salt or the cocrystal thereof, wherein, L₂ is -C(O)NR₉-, -NR₉C(O)NR₉-, -O-, or -CR₁₀R₁₁-, at least one of R₁₀ and R₁₁ is halogen or C₁₋₄ alkyl, or R₁₀ and R₁₁ together with the carbon atom to which they are attached form a 3-4-membered carbocyclic ring.

9. The nitrogen-containing heterocyclic compound according to claim 8, or the stereoisomer, the solvate, the deuterated form, the pharmaceutically acceptable salt or the cocrystal thereof, wherein the compound has a structure as shown in formula (III) 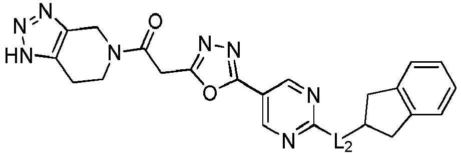 (III), wherein L₂ is as defined in formula (I).

10. The nitrogen-containing heterocyclic compound according to claim 1 or 2, or the stereoisomer, the solvate, the deuterated form, the pharmaceutically acceptable salt or the cocrystal thereof, wherein:
A is C₃₋₆ cycloalkylene, C₂₋₄ alkynylene, 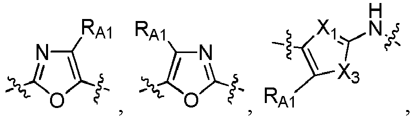 -RaC(O)NRa'- or 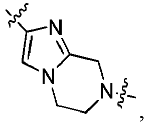 wherein X₁ is N or CR_{A1}, X₃ is S, O or NR_{A1}, each R_{A1} is independently H, cyano, -R_{A}, halogen, -C₁₋₄ alkyl R_{A}, -NHC(O)R_{A}, -C(O)R_{A}, -C₁₋₄ alkyl-O-C₁₋₄ alkyl, -NHR_{A}, -C(O)NHR_{A} or -OR_{A}, Ra is 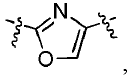 and Ra' is H or C₁₋₄ alkyl; or
A is 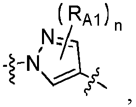 n is not 0, and at least one R_{A1} is selected from cyano, halogen, C₃₋₄ cycloalkyloxy, cyclopropylmethoxy, halo C₁₋₄ alkoxy, cyclopropylmethyl, -C₁₋₄ alkyl-O-C₁₋₄ alkyl, -NHC(O)R_{A}, -C(O)NHR_{A}, -C(O)-C₃₋₆ cycloalkyl,

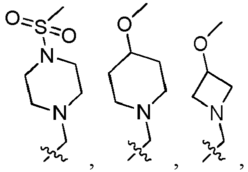

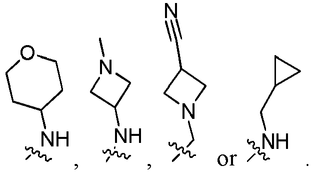

11. The nitrogen-containing heterocyclic compound according to claim 10, or the stereoisomer, the solvate, the deuterated form, the pharmaceutically acceptable salt or the cocrystal thereof, wherein the compound has a structure as shown in formula (IV) 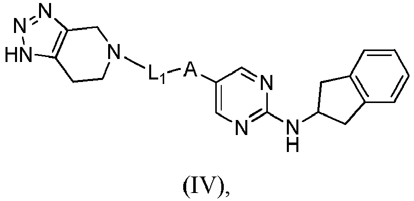 wherein L₁ is -C(O)-(CR₅R₆)ₑ-.

12. The nitrogen-containing heterocyclic compound according to claim 1 or 2, or the stereoisomer, the solvate, the deuterated form, the pharmaceutically acceptable salt or the cocrystal thereof, wherein:
B is 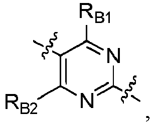 at least one of R_{B1} and R_{B2} is selected from OH, halogen, cyano, halo C₁₋₄ alkyl, C₁₋₄ alkyloxy or C₃₋₆ cycloalkyl, or group R_{A1} on A and group R_{B1} on B together with the atoms to which they are attached form a 6-8-membered heterocycle containing 1 O atom; or
B is 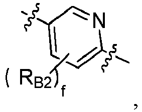 wherein R_{B2} is halogen or cyano, and f is 1, 2 or 3; or
B is 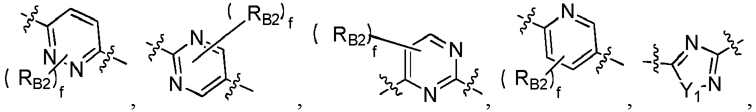 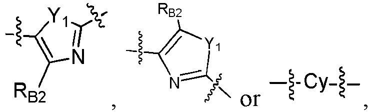 Y₁ is O, S or NR_{B3}, f is an integer of 0-3, and R_{B1} and R_{B2} are each independently selected from H, oxo, OH, halogen, cyano, C₁₋₄ alkyl, halo C₁₋₄ alkyl, C₁₋₄ alkyloxy or C₃₋₆ cycloalkyl; R_{B3} is selected from H, C₁₋₄ alkyl or C₃₋₆ cycloalkyl.

13. The nitrogen-containing heterocyclic compound according to claim 12, or the stereoisomer, the solvate, the deuterated form, the pharmaceutically acceptable salt or the cocrystal thereof, wherein the compound has a structure as shown in formula (V) 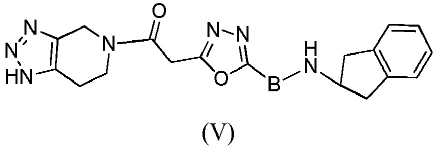
wherein B is 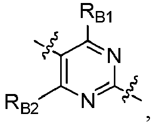 and at least one of R_{B1} and R_{B2} is selected from OH, halogen, cyano, halo C₁₋₄ alkyl, C₁₋₄ alkyloxy or C₃₋₆ cycloalkyl; or
B is 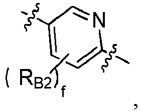 wherein R_{B2} is halogen or cyano, and f is 1, 2 or 3; or
B is 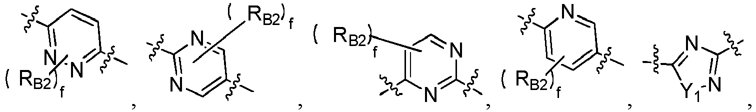 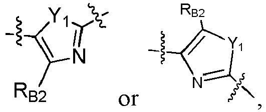 Y₁ is O, S or NR_{B3}, f is an integer of 0-3, and R_{B1} and R_{B2} are each independently selected from H, oxo, OH, halogen, cyano, C₁₋₄ alkyl, halo C₁₋₄ alkyl, C₁₋₄ alkyloxy or C₃₋₆ cycloalkyl; R_{B3} is selected from H, C₁₋₄ alkyl or C₃₋₆ cycloalkyl.

14. The nitrogen-containing heterocyclic compound according to claim 1 or 2, or the stereoisomer, the solvate, the deuterated form, the pharmaceutically acceptable salt or the cocrystal thereof, wherein the compound is as shown in formula (VI): 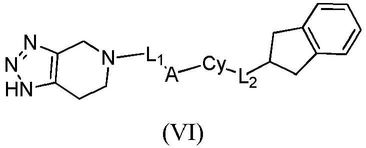
wherein, L₁ is -C(=O)-(CR₃R₄),-(NR₈)_{d}-, c is an integer of 0-3, d is 0 or 1, R₃, R₄ and R₈ are independently selected from H, C₁₋₃ alkyl or halogen, and R₃ and R₄ together with the carbon atom to which they are attached form a 3-6 membered carbocyclic ring, wherein the carbocyclic ring is optionally substituted with 1-4 substituents selected from halogen or C₁₋₄ alkyl;
A is 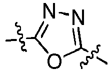 or a bond;
Cy has a structure: 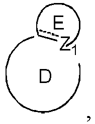 wherein, 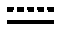 represents a single bond or a double bond, Z₁ is C or N, ring E is a 5-membered heterocycle containing 1-3 heteroatoms selected from N, O or S, ring D is a saturated or unsaturated 6-membered ring containing 0-3 heteroatoms selected from N, O or S; the 5-membered heterocycle and the 6-membered ring are independently and optionally substituted with 1-3 R_{B2};
each R_{B2} is independently selected from H, halogen, cyano, C₁₋₄ alkyl or halo C₁₋₄ alkyl;
L₂ is -NH- or a bond.

15. The nitrogen-containing heterocyclic compound according to claim 14, or the stereoisomer, the solvate, the deuterated form, the pharmaceutically acceptable salt or the cocrystal thereof, wherein 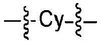 is selected from one of the following structures:

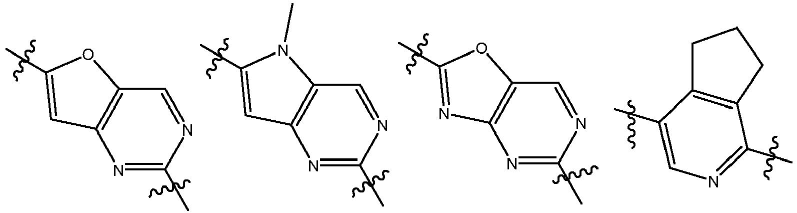

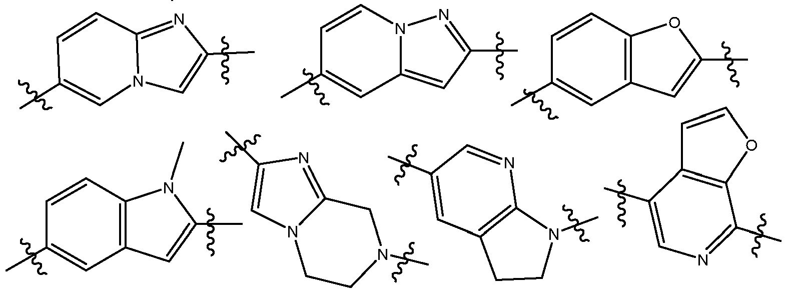

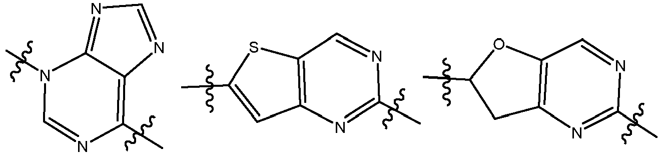

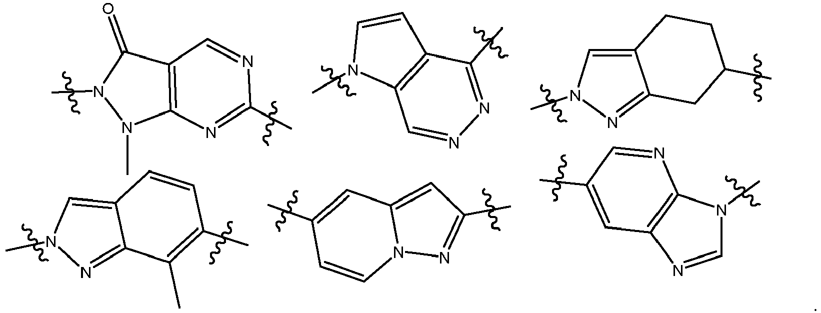

16. The nitrogen-containing heterocyclic compound according to claim 1 or 2, or the stereoisomer, the solvate, the deuterated form, the pharmaceutically acceptable salt or the cocrystal thereof, wherein:
when M₂ is 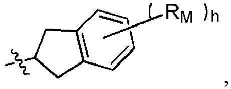 h is not 0, and at least one R_{M} is halo C₁₋₄ alkyl, cyano, C₃₋₆ cycloalkyl, C₂₋₆ alkynyl or a 3-6 membered heterocyclyl containing 1-3 heteroatoms selected from N, O or S, the alkyl is optionally substituted with 1-3 groups selected from halogen or cyano, and the heterocyclyl is optionally substituted with 1-3 groups selected from halogen, oxo or C₁₋₄ alkyl; or, alternatively, two R_{M} on adjacent ring carbon atoms in M₂ together with the carbon atoms to which they are attached form a 3-6 membered carbocyclic ring or a 3-6 membered heterocyclyl containing 0-3 heteroatoms selected from N, O or S;
and when M₂ is 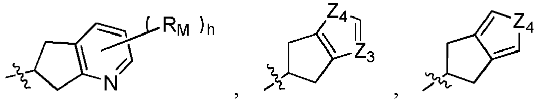 or 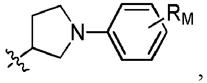 Z₃ is CR_{M} or N, Z₄ is O, S or NR_{M1}, each R_{M} is independently H or C₁₋₄ alkyl, R_{M1} is H or C₁₋₄ alkyl, and h is 0, 1, 2 or 3.

17. The nitrogen-containing heterocyclic compound according to claim 16, or the stereoisomer, the solvate, the deuterated form, the pharmaceutically acceptable salt or the cocrystal thereof, wherein the compound has a structure as shown in formula (VII)

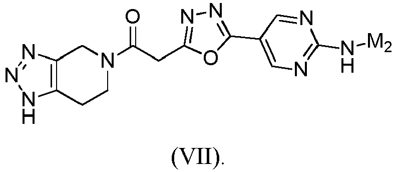

18. The nitrogen-containing heterocyclic compound according to claim 1, or the stereoisomer, the solvate, the deuterated form, the pharmaceutically acceptable salt or the cocrystal thereof, wherein,
M₁ is 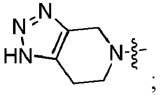
L₁ is -(CR₁R₂)ₐ-(NR₇)_{b}-W-(CR₃R₄)_{c}-(NR₈)_{d}-(CR₅R₆)ₑ-;
W is -C(=X)-, and X is O or S;
a and b are 0, c is 1 or 2, and d and e are 0;
R₃ and R₄ are each independently selected from H, halogen or C₁₋₄ alkyl, and are not both H at the same time;
A is 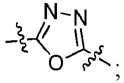
B is 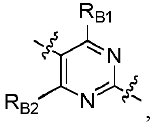 wherein both R_{B1} and R_{B2} are H;
L₂ is -NR₉-, and R₉ is H;
M₂ is 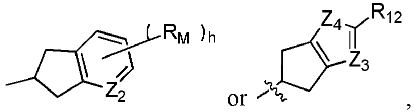 both Z₂ and Z₃ are CR_{M}, and Z₄ is O or S;
each R₁₂ is independently H, halogen, C₁₋₄ alkyl, C₃₋₆ cycloalkyl or halo C₁₋₄ alkyl;
h is 0, 1 or 2;
and each R_{M} is independently H or C₁₋₄ alkyl, the alkyl is optionally substituted with 1-3 halogens.

19. The nitrogen-containing heterocyclic compound of formula (I) according to claim 1, or the stereoisomer, the solvate, the deuterated form, the pharmaceutically acceptable salt or the cocrystal thereof,
wherein, M₁ is 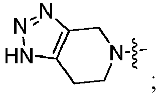
L₁ is a bond or -(CR₁R₂)ₐ-(NR₇)_{b}-W-(CR₃R₄)_{c}-(NR₈)_{d}-(CR₅R₆)ₑ-;
W is -C(=X)-;
X is O, S or NRₓ, and Rₓ is H or cyano;
each of R₁ to R₆ is independently selected from H, halogen, C₁₋₄ alkyl or C₃₋₆ cycloalkyl;
R₇ and R₈ are each independently selected from H, C₁₋₄ alkyl or C₃₋₆ cycloalkyl;
alternatively, R₁ and R₂ on the same carbon atom, R₃ and R₄ on the same carbon atom, or R₅ and R₆ on the same carbon atom together with the carbon atom to which they are attached form a 3-6-membered carbocyclic ring, wherein the carbocyclic ring is optionally substituted with 1-4 substituents selected from halogen or C₁₋₄ alkyl;
a, c and e are independently selected from 0, 1, 2 and 3, and b and d are independently 0 or 1;
A is 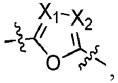 -RaC(O)NRa'-, -RaNRa'C(O)-, -RaNRa'-, -RaC(O)-, or -Ra(CRa' Ra")ₙ-;
Ra is 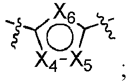
Ra' and Ra" are independently H or C₁₋₄ alkyl;
n is 1 or 2;
X₁ and X₂ are independently N, NR_{A1} or CR_{A1}, and are not both CR_{A1} at the same time;
X₄, X₅ and X₆ are independently N, NR_{A1}, S, O or CR_{A1}, and are not all CR_{A1} at the same time;
each R_{A1} is independently substituted with substituents of H, cyano, -R_{A}, halogen, -C₁₄ alkyl R_{A}, -NHC(O)R_{A}, -C(O)R_{A}, -C₁₄ alkyl-O-C₁₋₄ alkyl, -NHR_{A}, -C(O)NHR_{A}, and - OR_{A};
R_{A} is C₁₋₄ alkyl, C₃₋₆ cycloalkyl or a 3-6 membered heterocyclyl containing 1-3 heteroatoms selected from N, O or S, wherein the alkyl, cycloalkyl, and heterocyclyl are optionally further substituted with 1-6 groups selected from C₃₋₆ cycloalkyl, C₁₋₄ alkyl, halogen, -S(O)₂C₁₋₄ alkyl, -OC₁₋₄ alkyl or cyano;
B is 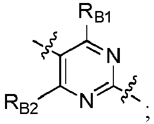
R_{B1} and R_{B2} are each independently selected from H, OH, halogen, cyano, C₁₋₄ alkyl, halo C₁₋₄ alkyl, C₁₋₄ alkyloxy or C₃₋₆ cycloalkyl;
L₂ is -O-, -NR₉-, -(CR₁₀R₁₁)_{g}-, -NR₉-(CR₁₀R₁₁)_{g}- or a bond, wherein g is an integer of 1-3;
R₉ is H or C₁₋₄ alkyl;
R₁₀ and R₁₁ are each independently H, halogen, C₁₋₄ alkyl or C₃₋₆ cycloalkyl; alternatively, R₁₀ and R₁₁ on the same carbon atom together with the carbon atom to which they are attached form a 3-6 membered carbocyclic ring;
M₂ is 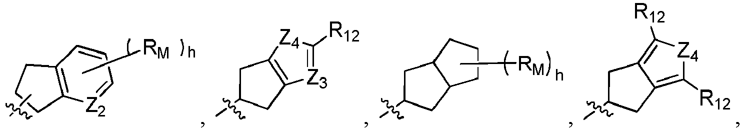 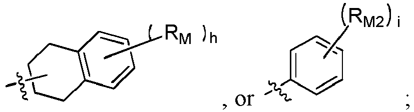
Z₂ and Z₃ are CR_{M} or N, and Z₄ is O, S or NR_{M1};
each R_{M} is independently H, C₁₋₄ alkyl, cyano, C₃₋₆ cycloalkyl, -SRm', -S(O)₂Rm', -C(O)NRmRm', -NRmC(O)Rm', C₂₋₆ alkynyl, a 3-6 membered heterocyclyl containing 1-3 heteroatoms selected from N, O or S, or halogen, the alkyl, alkynyl, and cycloalkyl are optionally substituted with 1-3 groups selected from halogen, cyano, C₁₋₄ alkoxy, halo C₁₋₄ alkyl or halo C₁₋₄ alkoxy, the heterocyclyl is optionally substituted with 1-3 groups selected from halogen, oxo, and C₁₋₄ alkyl;
Rm is H, C₁₋₄ alkyl, and halo C₁₋₄ alkyl, Rm' is C₁₋₄ alkyl and halo C₁₋₄ alkyl;
alternatively, two R_{M} on adjacent ring carbon atoms in M₂ together with the carbon atom to which they are attached form a 3-6 membered carbocyclic ring or a 3-6 membered heterocyclyl containing 0-2 heteroatoms selected from N, O or S;
R_{M1} is H, C₁₋₄ alkyl or C₃₋₆ cycloalkyl;
R_{M2} is halo C₁₋₄ alkoxy;
each R₁₂ is independently H, halogen, C₁₋₄ alkyl, C₃₋₆ cycloalkyl or halo C₁₋₄ alkyl;
h is an integer of 0-3; i is 0, 1 or 2;
provided that, the compound is not:

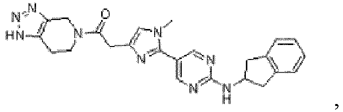

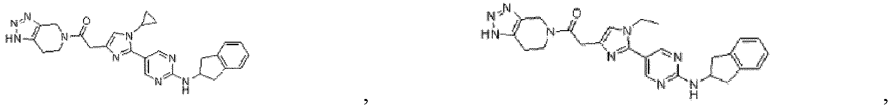

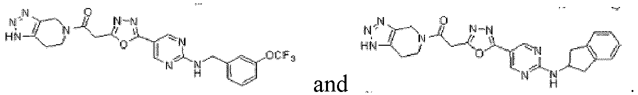

20. The nitrogen-containing heterocyclic compound according to claim 19, or the stereoisomer, the solvate, the deuterated form, the pharmaceutically acceptable salt or the cocrystal thereof,
wherein, L₁ is a bond or -W-(CR₃R₄)_{c}-;
W is -C(=X)-;
X is O or S;
R₃ and R₄ are each independently selected from H, halogen or C₁₋₄ alkyl;
alternatively, R₃ and R₄ on the same carbon atom together with the carbon atom to which they are attached form a 3-6 membered carbocyclic ring;
c is selected from 0, 1 and 2;
A is 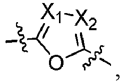 -RaC(O)NRa'-, -RaNRa'C(O)-, -RaC(O)- or -Ra(CRa' Ra")ₙ-;
Ra is 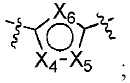
Ra' and Ra" are H or C₁₋₄ alkyl;
n is 1 or 2;
X₁ and X₂ are independently N, NR_{A1} or CR_{A1}, and are not both CR_{A1} at the same time;
X₄, X₅ and X₆ are independently N, NR_{A1}, S, O or CR_{A1}, and are not all CR_{A1} at the same time;
each R_{A1} is independently H;
B is 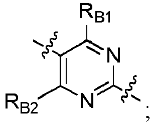
both R_{B1} and R_{B2} are H;
L₂ is -NR₉- or -NR₉-(CR₁₀R₁₁)_{g}-, wherein g is an integer of 1-3;
R₉ is H;
R₁₀ and R₁₁ are each independently H;
M₂ is 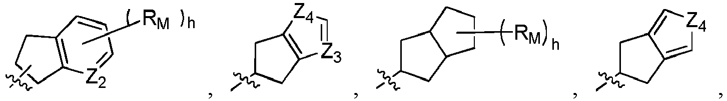 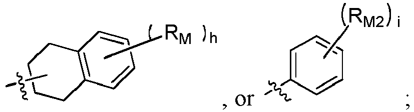
Z₂ and Z₃ are CR_{M}, and Z₄ is O or S;
each R_{M} is independently H, C₁₋₄ alkyl, C₃₋₆ cycloalkyl, -SRm', -S(O)₂Rm', - C(O)NRmRm', -NRmC(O)Rm', C₂₋₆ alkynyl, a 3-6 membered heterocyclyl containing 1-3 heteroatoms selected from N, O or S, or halogen, the alkyl, alkynyl, and cycloalkyl are optionally substituted with 1-3 groups selected from halogen, C₁₋₄ alkoxy, halo C₁₋₄ alkyl or halo C₁₋₄ alkoxy, the heterocyclyl is optionally substituted with 1-3 groups selected from halogen, oxo, and C₁₋₄ alkyl;
Rm is H or C₁₋₄ alkyl, and Rm' is C₁₋₄ alkyl;
alternatively, two R_{M} on adjacent ring carbon atoms in M₂ together with the carbon atom to which they are attached form a 3-6 membered carbocyclic ring or a 3-6 membered heterocyclyl containing 0-2 heteroatoms selected from N, O or S;
R_{M2} is halo C₁₋₄ alkoxy;
h is 0, 1 or 2;
and i is 0, 1 or 2.

21. The nitrogen-containing heterocyclic compound according to claim 20, or the stereoisomer, the solvate, the deuterated form, the pharmaceutically acceptable salt or the cocrystal thereof,
wherein, M₂ is 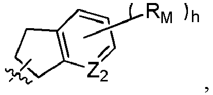
h is 1 or 2,
R_{M} is C₁₋₄ alkyl, C₃₋₆ cycloalkyl, -SRm', -S(O)₂Rm', -C(O)NRmRm', - NRmC(O)Rm', C₂₋₆ alkynyl or a 3-6 membered heterocyclyl containing 1-3 heteroatoms selected from N, O or S, the alkyl is optionally substituted with 1-3 groups selected from halogen, C₁₋₄ alkoxy or halo C₁₋₄ alkoxy, and the heterocyclyl is optionally substituted with 1-3 groups selected from halogen, oxo, and C₁₋₂ alkyl;
alternatively, two R_{M} on adjacent ring carbon atoms in M₂ together with the carbon atoms to which they are attached form a 3-6 membered carbocyclic ring or a 3-6 membered heterocyclyl containing 0-2 heteroatoms selected from N, O or S.

22. The nitrogen-containing heterocyclic compound according to claim 20, or the stereoisomer, the solvate, the deuterated form, the pharmaceutically acceptable salt or the cocrystal thereof,
wherein, M2 is 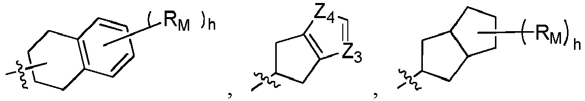 or

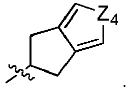

23. The nitrogen-containing heterocyclic compound according to claim 20, or the stereoisomer, the solvate, the deuterated form, the pharmaceutically acceptable salt or the cocrystal thereof, wherein c is 1 or 2, and R₃ and R₄ on the same carbon atom together with the carbon atom to which they are attached form a 3-6 membered carbocyclic ring.

24. The nitrogen-containing heterocyclic compound according to claim 1 or 2, or the stereoisomer, the solvate, the deuterated form, the pharmaceutically acceptable salt or the cocrystal thereof, wherein,
M₁ is 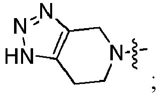
L₁ is -W-(CR₃R₄)_{c}- or -W-(NR₈)_{d}-(CR₅R₆)ₑ-;
W is -C(=X)-;
X is O or S;
R₃, R₄, R₅ and R₆ are each independently selected from H, halogen or C₁₋₄ alkyl;
each R₈ is independently selected from H, C₁₋₄ alkyl or C₃₋₆ cycloalkyl;
alternatively, R₃ and R₄ on the same carbon atom, or R₅ and R₆ on the same carbon atom together with the carbon atom to which they are attached form a 3-6 membered carbocyclic ring;
c and e are selected from 0 or 1, and d is 1;
A is 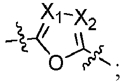
X₁ and X₂ are independently N or CR_{A1}, and are not both CR_{A1} at the same time;
R_{A1} is cyano, C₁₋₄ alkyl, -R_{A} or halogen;
R_{A} is C₃₋₆ cycloalkyl, C₃₋₆ cycloalkyloxy, C₁₋₄ alkoxy, C₁₋₄ haloalkyl, and C₁₋₄ haloalkoxy, wherein the cycloalkyl, cycloalkyloxy, alkoxy, haloalkyl, and haloalkoxy are optionally further substituted with 1-3 groups selected from C₁₋₄ alkyl, halogen and cyano;
B is 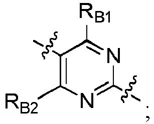
both R_{B1} and R_{B2} are H;
L₂ is -NR₉-;
R₉ is H or C₁₋₄ alkyl;
M₂ is 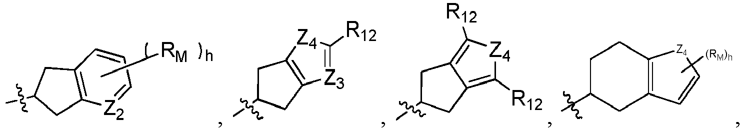 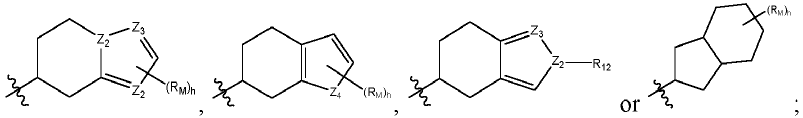
Z₂ and Z₃ are CR_{M} or N, and Z₄ is O or S;
each R_{M} is independently H, C₁₋₄ alkyl, C₁₋₄ alkoxyalkyl, cyano, C₃₋₆ cycloalkyl, C₃₋₆ cycloalkyloxy, C₂₋₆ alkenyl, C₂₋₆ alkynyl, -NR_{M1}, or a 3-6 membered heterocyclyl containing 1-3 heteroatoms selected from N, O or S, wherein the alkyl, alkenyl, alkynyl, and cycloalkyl are optionally substituted with 1-3 groups selected from halogen and cyano, and the heterocyclyl is optionally substituted with 1-3 groups selected from halogen or C₁₋₄ alkyl;
alternatively, two R_{M} on adjacent ring carbon atoms in M₂ together with the carbon atoms to which they are attached form a 3-6 membered carbocyclic ring or a 3-6 membered heterocyclyl containing 0-3 heteroatoms selected from N, O or S;
R_{M1} is H, C₁₋₄ alkyl, C₃₋₆ cycloalkyl, C₁₋₄ haloalkyl or C₁₋₄ alkoxyalkyl;
each R₁₂ is independently H, halogen or C₁₋₄ alkyl;
h is an integer of 0-3;
and i is 0, 1 or 2.

25. The nitrogen-containing heterocyclic compound according to claim 17 or 24, or the stereoisomer, the solvate, the deuterated form, the pharmaceutically acceptable salt or the cocrystal thereof, wherein,
M₂ is 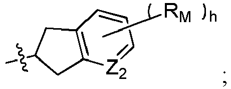
Z₂ is CR_{M};
each R_{M} is independently H, C₁₋₄ alkyl, C₁₋₄ alkoxyalkyl, C₃₋₆ cycloalkyloxy, C₂₋₆ alkenyl, C₂₋₄ alkynyl, -NR_{M1}, or a 3-6 membered heterocyclyl containing 1-3 heteroatoms selected from N, O or S, the alkyl, alkenyl and alkynyl are optionally substituted with 1-3 halogens and cyano;
alternatively, two R_{M} on adjacent ring carbon atoms in M₂ together with the carbon atoms to which they are attached form a 3-6 membered carbocyclic ring or a 3-6 membered heterocyclyl containing 0-3 heteroatoms selected from N, O or S;
R_{M1} is H, C₁₋₄ alkyl or C₃₋₆ cycloalkyl;
and h is 1 or 2.

26. The nitrogen-containing heterocyclic compound according to claim 24, or the stereoisomer, the solvate, the deuterated form, the pharmaceutically acceptable salt or the cocrystal thereof, wherein,
L₁ is -C(O)CR₃R₄-;
R₃ and R₄ are independently selected from H, halogen and C₁₋₄ alkyl;
A is 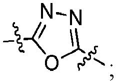
B is 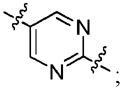
L₂ is NH;
M₂ is 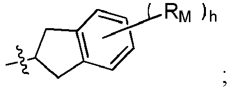
h is 1 or 2;
each R_{M} is independently H, C₁₋₄ alkyl or C₂₋₄ alkynyl; the alkyl or alkynyl is optionally substituted with 1-3 groups selected from halogen and cyano;
and R₃, R₄ and R_{M} are not all H at the same time.

27. The nitrogen-containing heterocyclic compound according to claim 1, or the stereoisomer, the solvate, the deuterated form, the pharmaceutically acceptable salt or the cocrystal thereof, wherein the compound is selected from one of the following structures:

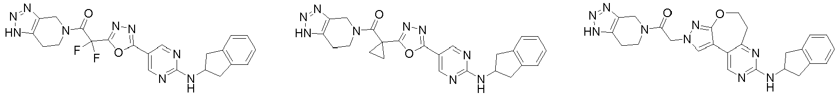

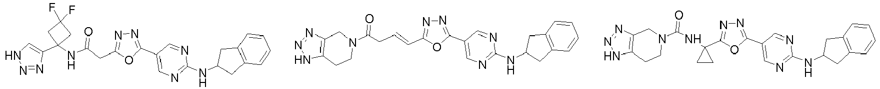

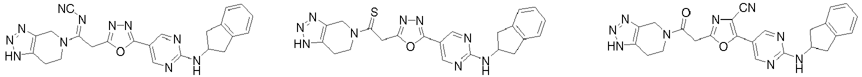

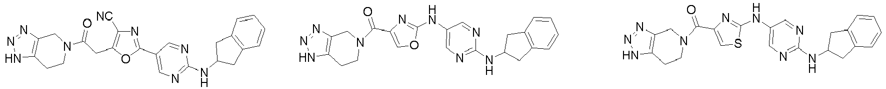

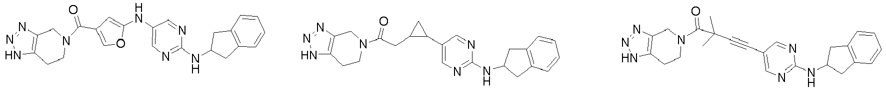

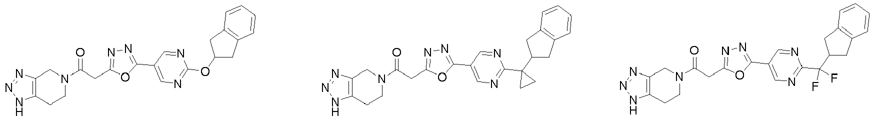

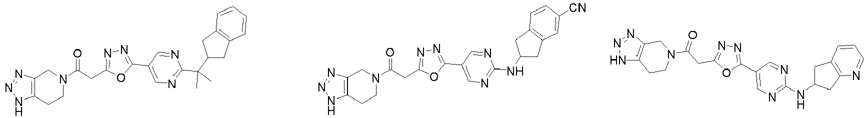

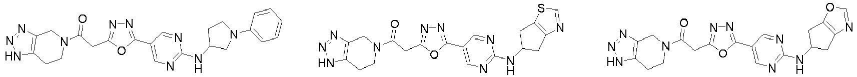

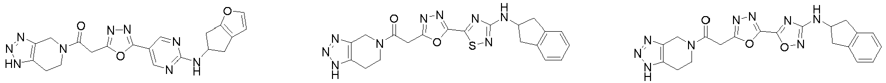

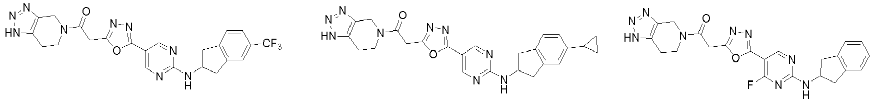

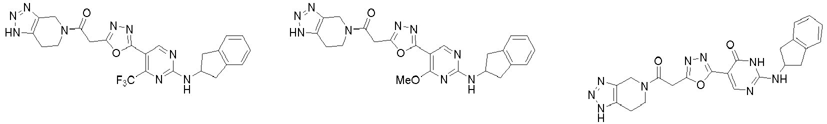

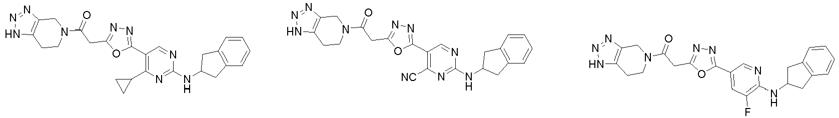

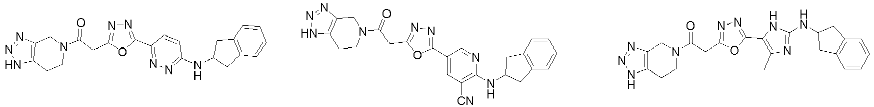

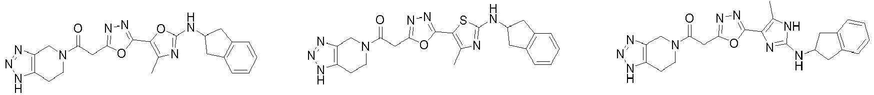

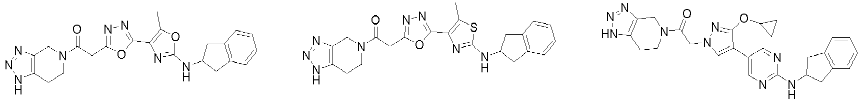

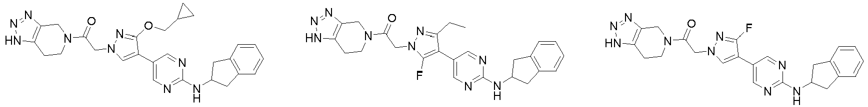

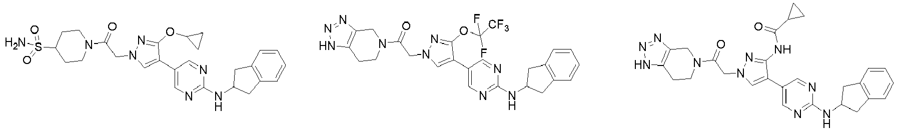

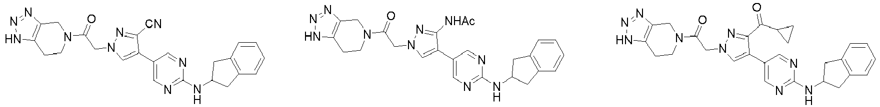

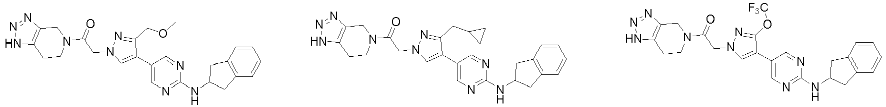

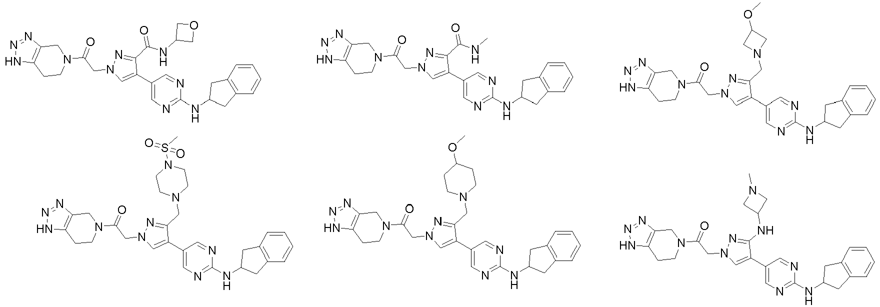

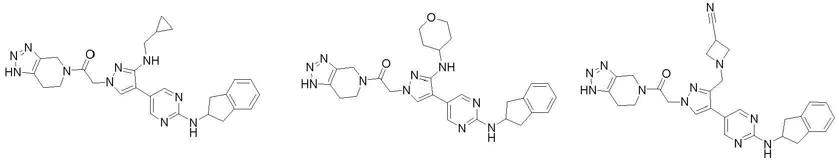

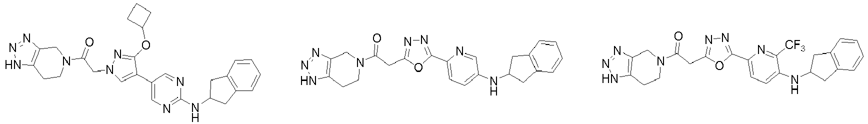

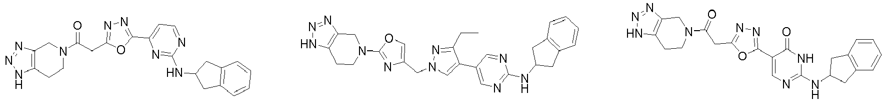

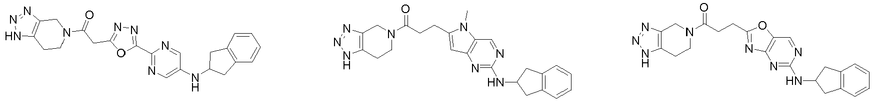

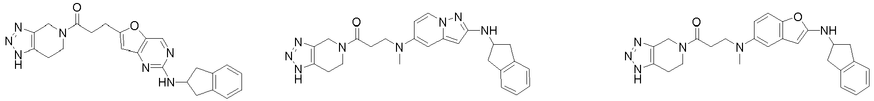

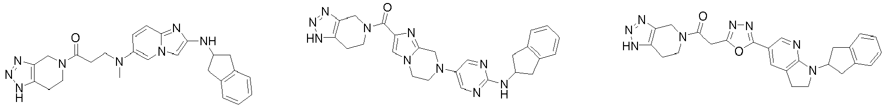

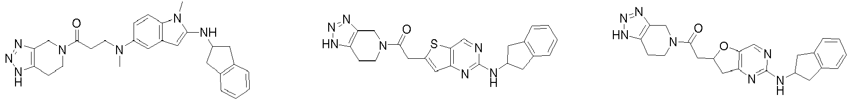

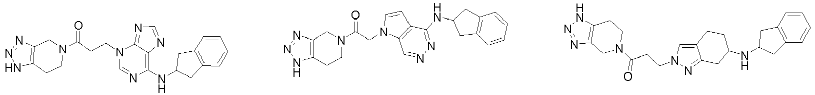

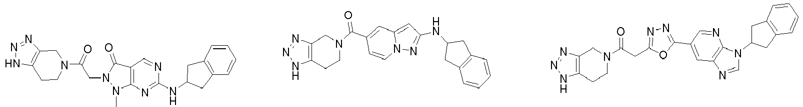

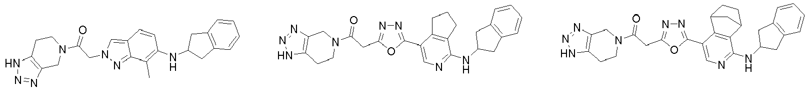

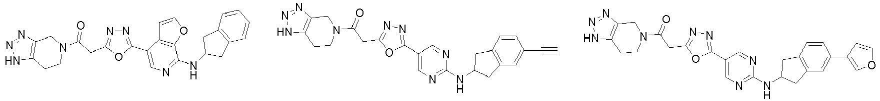

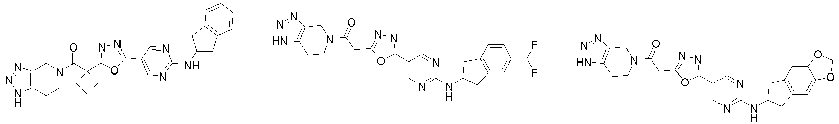

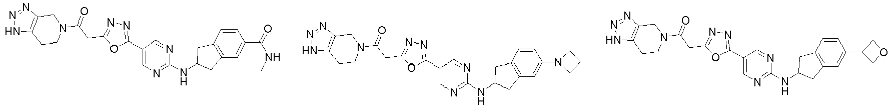

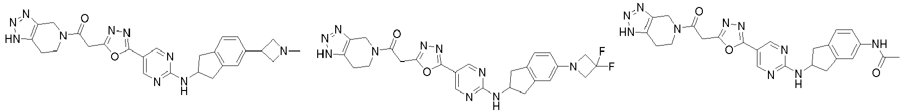

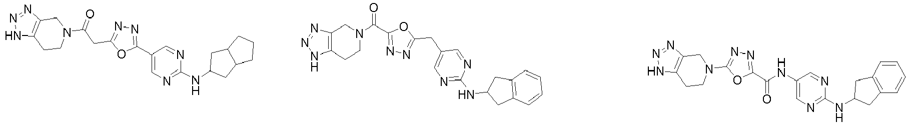

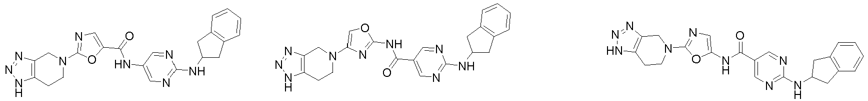

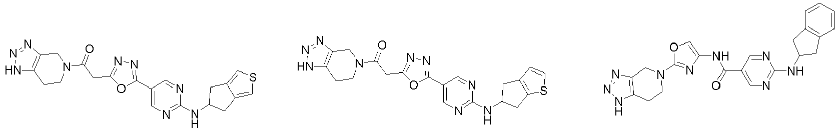

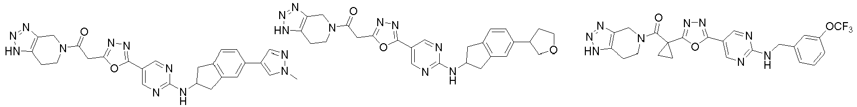

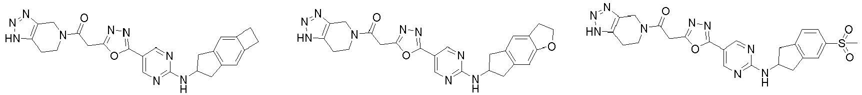

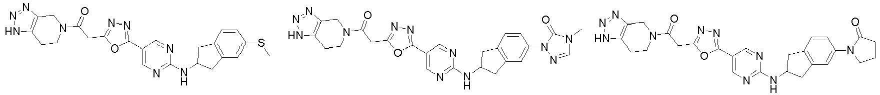

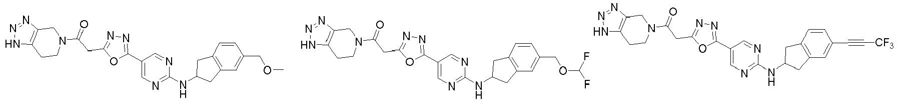

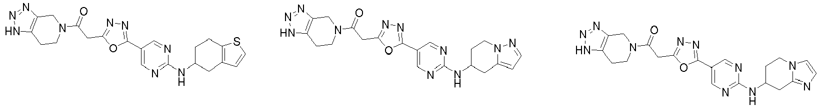

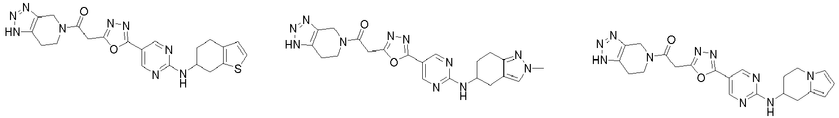

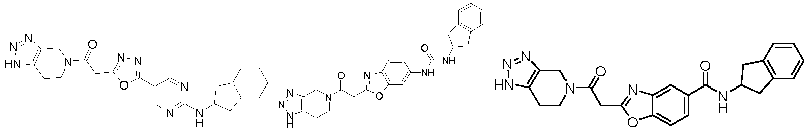

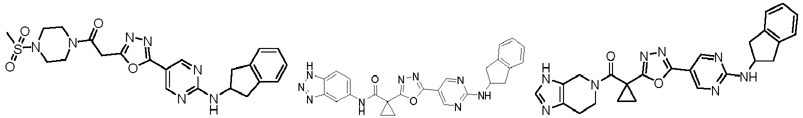

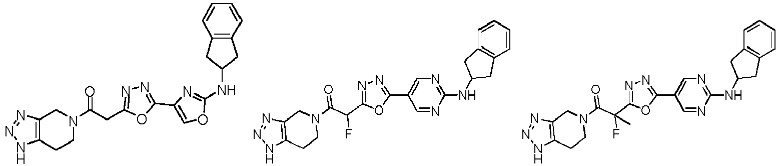

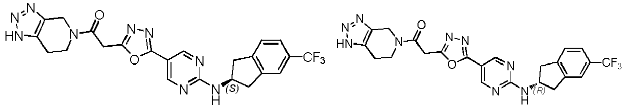

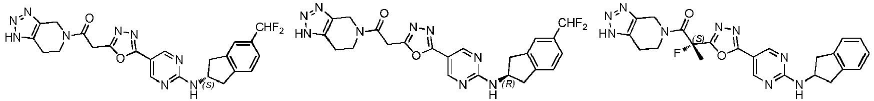

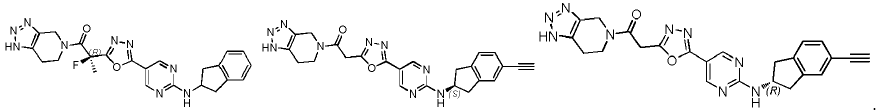

28. A pharmaceutical composition, comprising a pharmaceutically effective amount of the nitrogen-containing heterocyclic compound of any one of claims 1-27, or the stereoisomer, the solvate, the deuterated form, the pharmaceutically acceptable salt or the cocrystal thereof, and a pharmaceutically acceptable adjuvant and/or carrier.

29. Use of the nitrogen-containing heterocyclic compound of any one of claims 1-27, or the stereoisomer, the solvate, the deuterated form, the pharmaceutically acceptable salt or the cocrystal thereof, or the composition of claim 28 in the preparation of a drug for treating/preventing diseases mediated by autotaxin.

30. Use of the nitrogen-containing heterocyclic compound of any one of claims 1-27, or the stereoisomer, the solvate, the deuterated form, the pharmaceutically acceptable salt or the cocrystal thereof, or the composition of claim 28 for treating or preventing diseases mediated by autotaxin.

31. Use according to claim 29 or 30, wherein the diseases mediated by autotaxin are selected from cardiovascular conditions, cancers, metabolism disorders, kidney conditions, hepatic conditions, inflammatory conditions, nervous system conditions, respiratory system conditions, fibrotic diseases, ophthalmic conditions, cholestasis and other forms of chronic itch and acute or chronic organ-graft rejection.

32. Use according to claim 31, wherein the inflammatory conditions are selected from arthritis, atopic dermatitis, arthritis and asthma.
